# EUROPEAN PATENT SPECIFICATION

(11) **EP 3 126 361 B1**
(45) Date of publication and mention of the grant of the patent: **06.11.2019**
(21) Application number: 15714951.9
(22) Date of filing: 18.03.2015
(51) Int. Cl.: C07D 471/04, A61K 31/4985, A61P 25/28

(54) **CHROMENE AND 1,1A,2,7B-TETRAHYDROCYCLOPROPA[C]CHROMENE PYRIDOPYRAZINEDIONES AS GAMMA-SECRETASE MODULATORS**
CHROMEN UND 1,1A,2,7B-TETRAHYDROCYCLOPROPA[C]CHROMEN-PYRIDOPYRAZIN-DIONE ALS GAMMA-SEKRETASE-MODULATOREN
CHROMÈNE ET 1,1A,2,7B-TÉTRAHYDROCYCLOPROPA[C]CHROMÈNE PYRIDOPYRAZINEDIONES COMME MODULATEURS DE GAMMA-SÉCRÉTASE

(30) Priority: 01.04.2014 US 201461973436 P
(43) Date of publication of application: 08.02.2017
(73) Proprietor: Pfizer Inc., New York, NY 10017 (US)
(72) Inventor: AM ENDE, Christopher William, Mystic, Connecticut 06355 (US); HUMPHREY, John Michael, Mystic, Connecticut 06355 (US); JOHNSON, Douglas Scott, Concord, Massachusetts 01742 (US); KAUFFMAN, Gregory Wayne, East Greenwich, Rhode Island 02818 (US); PETTERSSON, Martin Youngjin, Littleton, Massachusetts 01460 (US); RANKIC, Danica Antonia, Niantic, Connecticut 06357 (US); STEPAN, Antonia Friederike, Brookline, Massachusetts 02446 (US); VERHOEST, Patrick Robert, Newton, Massachusetts 02460 (US)
(74) Representative: Pfizer
(86) International application number: PCT/IB2015/051988
(87) International publication number: WO 2015/150957

(56) References cited:
- WO-A1-2013/171712
- WO-A1-2014/111457
- WO-A1-2015/049616
- US-A1- 2012 252 758
- US-A1- 2014 088 111
- MARTIN PETTERSSON ET AL: "Design, Synthesis, and Pharmacological Evaluation of a Novel Series of Pyridopyrazine-1,6-dione [gamma]-Secretase Modulators", JOURNAL OF MEDICINAL CHEMISTRY, vol. 57, no. 3, 13 February 2014 (2014-02-13), pages 1046-1062, XP055156862, ISSN: 0022-2623, DOI: 10.1021/jm401782h

## Description

### FIELD OF THE INVENTION

The present invention relates to novel chromene and cyclopropachromene pyridopyrazinedione compounds of Formula I useful for the treatment of neurodegenerative and/or neurological disorders, such as Alzheimer's disease and Down's syndrome.

### BACKGROUND OF THE INVENTION

Dementia results from a wide variety of distinctive pathological processes. The most common pathological processes causing dementia are Alzheimer's disease (AD), cerebral amyloid angiopathy (CM) and prion-mediated diseases (see, e.g., Haan et al., Clin. Neurol. Neurosurg. 1990, 92(4):305-310; Glenner et al., J. Neurol. Sci. 1989, 94:1-28). AD affects nearly half of all people past the age of 85, the most rapidly growing portion of the United States population. As such, the number of AD patients in the United States is expected to increase from about 4 million to about 14 million by 2050.

Certain γ-secretase modulators are already known, for example those described in WO 2013/171712, Petterson et al. (J. Med. Chem., vol. 57, no. 3, 13 February 2014, pages 1046-1062), US 2012/252758 or US 2014/088111. There is however a need for further γ-secretase modulators. The present invention thus relates to a group of γ-secretase modulators, useful for the treatment of neurodegenerative and/or neurological disorders such as Alzheimer's disease and Down's syndrome. (see Ann. Rep. Med. Chem. 2007, Olsen et al., 42: 27-47).

### SUMMARY OF THE INVENTION

The present invention is directed to γ-secretase modulators of Formula I: 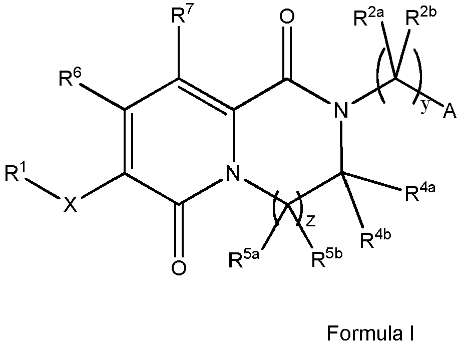 or pharmaceutically acceptable salts thereof as defined in the appended claims.

Compounds of the invention include Examples 1-37, 39-56 and 58-66 or a pharmaceutically acceptable salt thereof as described herein.

Also provided herein are compositions comprising a pharmaceutically effective amount of one or more of the compounds described herein and a pharmaceutically acceptable vehicle, carrier or excipient.

The compounds of Formula I are γ-secretase modulators. γ-Secretase plays a role in the production of amyloid beta protein (Aβ) plaques associated with Alzheimer's disease. Accordingly, the compounds of Formula I are believed to be useful in treating a variety of neurodegenerative and/or neurological disorders related to Aβ production.

Other features and advantages of this invention will be apparent from this specification and the appending claims which describe the invention.

### DETAILED DESCRIPTION OF THE INVENTION

### Definitions and Exemplifications

As used throughout this application, including the claims, the following terms have the meanings defined below, unless specifically indicated otherwise. The plural and singular should be treated as interchangeable, other than the indication of number:
The term "(C₁-C₆)alkyl" refers to a linear or branched-chain saturated hydrocarbyl substituent (i.e., a substituent obtained from a hydrocarbon by removal of a hydrogen) containing from 1 to 6 carbon atoms. Examples of such substituents include methyl, ethyl, propyl (including n-propyl and isopropyl), butyl (including n-butyl, isobutyl, sec-butyl and *tert*-butyl), pentyl, and hexyl.

The term hydroxy(C₁-C₆)alkyl refers to a (C₁-C₆)alkyl as defined above wherein at least one hydrogen atom is replaced with a hydroxy, as defined below. Representative examples of a hydroxy(C₁-C₆)alkyl include, but are not limited to, hydroxymethyl, hydroxyethyl, hydroxypropyl, 2-hydroxypropan-2-yl, 2-hydroxy-2-methylpropyl, hydroxybutyl, hydroxypentyl and hydroxyhexyl.

The term "(C₁-C₃)alkyl" refers to a linear or branched-chain saturated hydrocarbyl substituent (i.e., a substituent obtained from a hydrocarbon by removal of a hydrogen) containing from 1 to 3 carbon atoms. Examples of such substituents include methyl, ethyl, and propyl (including n-propyl and isopropyl).

The term hydroxy(C₁-C₃)alkyl refers to a (C₁-C₃)alkyl as defined above wherein at least one hydrogen atom is replaced with a hydroxy, as defined below. Representative examples of a hydroxy(C₁-C₆)alkyl include, but are not limited to, hydroxymethyl, hydroxyethyl, hydroxypropyl, and 2-hydroxypropan-2-yl.

The term "(C₂-C₆)alkenyl" refers to an aliphatic hydrocarbon having from 2 to 6 carbon atoms and having at least one carbon-carbon double bond, including straight chain or branched chain groups having at least one carbon-carbon double bond. Representative examples include, but are not limited to, ethenyl, 1-propenyl, 2-propenyl (allyl), isopropenyl, 2-methyl-1-propenyl, 1-butenyl, and 2-butenyl. When the compounds of the invention contain a (C₂-C₆)alkenyl group, the compound may exist as the pure E (entgegen) form, the pure Z (zusammen) form, or any mixture thereof.

The term "(C₂-C₆)alkynyl" refers to an aliphatic hydrocarbon having from 2 to 6 carbon atoms and having at least one carbon-carbon triple bond, including straight chain or branched chain groups having at least one carbon-carbon triple bond. Representative examples of an alkynyl include, but are not limited to, acetylenyl, 1-propynyl, 2-propynyl, 3-butynyl, 2-pentynyl, and 1-butynyl.

The term "halogen" refers to fluorine (which may be depicted as -F), chlorine (which may be depicted as -Cl), bromine (which may be depicted as -Br), or iodine (which may be depicted as -I).

The term "halo(C₁-C₆)alkyl" as used herein, refers to a (C₁-C₆)alkyl group, as defined above, wherein at least one hydrogen atom is replaced with a halogen, as defined above. Representative examples of a halo(C₁-C₆)alkyl include, but are not limited to, fluoromethyl, 2-fluoroethyl, trifluoromethyl, pentafluoroethyl, and 2-chloro-3-fluoropentyl.

The term "(C₁-C₆)alkoxy" as used herein, means a (C₁-C₆)alkyl group, as defined above, attached to the parent molecular moiety through an oxygen atom. Examples include, but are not limited to, methoxy, ethoxy, propoxy, 2-propoxy, butoxy, *tert-butoxy,* pentyloxy, and hexyloxy.

The term "(C₁-C₆)alkoxy(C₁-C₆)alkyl" as used herein, means a (C₁-C₆)alkoxy group, as defined above, attached to the parent moiety through a (C₁-C₆)alkyl group, as defined above. Examples include, but are not limited to, methoxymethyl, methoxyethyl and the like.

The term "halo(C₁-C₆)alkoxy" as used herein, refers to a (C₁-C₆)alkoxy group, as defined above, wherein at least one hydrogen atom is replaced with a halogen, as defined above. Representative examples of a halo(C₁-C₆)alkoxy include, but are not limited to, fluoromethoxy, 2-fluoroethoxy, trifluoromethoxy, and pentafluoroethoxy.

The term "(C₁-C₆)alkylthio" as used herein, means a (C₁-C₆)alkyl group, as defined above, appended to the parent molecular moiety through a sulfur atom. Representative examples of (C₁-C₆)alkylthio include, but are not limited to, methylthio, ethylthio, *tert*-butylthio, and hexylthio.

The term "(C₃-C₈)cycloalkyl" refers to a carbocyclic substituent obtained by removing a hydrogen from a saturated carbocyclic molecule having from 3 to 8 carbon atoms. A "(C₃-C₆)cycloalkyl" refers to a carbocyclic substituent obtained by removing a hydrogen from a saturated carbocyclic molecule having from 3 to 6 carbon atoms. A "(C₃-C₈)cycloalkyl" may be a monocyclic ring, examples of which include cyclopropyl, cyclobutyl, cyclopentyl, cyclohexyl, cycloheptyl, and cyclooctyl. Alternatively, a cycloalkyl may contain more than one ring, such as a (C₄-C₈)bicycloalkyl. The term "(C₄-C₈)bicycloalkyl" refers to a bicyclic system containing 4 to 8 carbon atoms. The bicycloalkyl may be fused, such as bicyclo[1.1.0]butane, bicyclo[2.1.0]pentane, bicyclo[2.2.0]hexane, bicyclo[3.1.0]hexane, bicylco[3.2.0]heptane and bicyclo[3.3.0]octane. The term "bicycloalkyl" also includes bridged bicycloalkyl systems such as, but not limited to, bicyclo[2.2.1]heptane and bicyclo[1.1.1]pentane.

The term "(C₃-C₆)cycloalkylchromenyl" refers to a "(C₃-C₆)cycloalkyl moiety as described above, wherein the "(C₃-C₆)cycloalkyl moiety is fused to a chromenyl moiety.

The term "(C₃-C₆)cycloalkyisochromenyl" refers to a "(C₃-C₆)cycloalkyl moiety as described above, wherein the "(C₃-C₆)cycloalkyl moiety is fused to a isochromenyl, moiety.

The term "(C₃-C₆)cycloalkylbenzofuranyl" refers to a "(C₃-C₆)cycloalkyl moiety as described above, wherein the "(C₃-C₆)cycloalkyl moiety is fused to a benzofuranyl moiety.

The term "(C₃-C₆)cycloalkylindenyl" refers to a "(C₃-C₆)cycloalkyl moiety as described above, wherein the "(C₃-C₆)cycloalkyl moiety is fused to a indenyl moiety.

The term "(C₆-C₁₀)aryl" refers to an aromatic substituent containing from 6 to 10 carbon atoms, including one ring or two fused rings. Examples of such aryl substituents include, but are not limited to, phenyl and naphthyl. The (C₆-C₁₀)aryl may also include phenyl and naphthyl substituents that are optionally fused to a (C₃-C₆)cycloalkyl ring (e.g., bicyclo[4.2.0]octa-1,3,5-trienyl) or a (5- to 6-membered)heterocycloalkyl ring (e.g., dihydrobenzofuranyl, benzodioxolyl, and oxoisoindolinyl) as defined herein, wherein a group having such a fused aryl group as a substituent is attached to a carbon atom of the aryl.

The term "heterocycloalkyl," as used herein, refers to a cycloalkyl as defined above, wherein at least one of the ring carbon atoms is replaced with a heteroatom selected from nitrogen, oxygen or sulfur. A "(4- to 10-membered)heterocycloalkyl" refers to a heterocycloalkyl substituent obtained by removing a hydrogen from a saturated or partially saturated ring structure containing a total of 4 to 10 ring atoms, wherein at least one of the ring atoms is a heteroatom selected from oxygen, nitrogen, or sulfur. A heterocycloalkyl may be a single ring with up to 10 total members. Alternatively, a heterocycloalkyl as defined above may comprise 2 or 3 rings fused together, wherein at least one such ring contains a heteroatom as a ring atom (i.e., nitrogen, oxygen, or sulfur). In a group that has a heterocycloalkyl substituent, the ring atom of the heterocycloalkyl substituent that is attached to the group may be the at least one heteroatom, when the heteroatom is a nitrogen having the appropriate valence, or it may be a ring carbon atom, where the ring carbon atom may be in the same ring as the at least one heteroatom or where the ring carbon atom may be in a different ring from the at least one heteroatom. Similarly, if the heterocycloalkyl substituent is in turn substituted with a group or substituent, the group or substituent may be bound to the at least one heteroatom when the heteroatom is a nitrogen having the appropriate valence, or it may be bound to a ring carbon atom, where the ring carbon atom may be in the same ring as the at least one heteroatom or where the ring carbon atom may be in a different ring from the at least one heteroatom.

Also included in the definition of "heterocycloalkyl" are heterocycloalkyls that are fused to a (C₆-C₁₀)aromatic ring or a (5- to 10-membered)heteroaromatic ring. When such a fused heterocycloalkyl group is substituted with one or more substituents, the one or more substituents, unless otherwise specified, are each bound to a heteroatom of the heterocycloalkyl group when the heteroatom is nitrogen having the appropriate valence or to a carbon atom of the heterocycloalkyl group. Examples of heterocycloalkyl rings include, but are not limited to, azetidinyl, dihydrofuranyl, dihydrothiophenyl, tetrahydrothiophenyl, tetrahydrofuranyl, tetrahydrotriazinyl, tetrahydropyrazolyl, tetrahydrooxazinyl, tetrahydropyrimidinyl, octahydrobenzofuranyl, octahydrobenzimidazolyl, octaohydrobenzothiazolyl, imidazolidinyl, pyrrolidinyl, piperidinyl, piperazinyl, oxazolidinyl, thiazolidinyl, pyrazolidinyl, thiomorpholinyl, tetrahydropyranyl, tetrahydrothiazinyl, tetrahydrothiadiazinyl, tetrahydro-oxazolyl, morpholinyl, oxetanyl, tetrahydrodiazinyl, oxazinyl, oxathiazinyl, quinuclidinyl, chromanyl, isochromanyl, benzoxazinyl, indolinyl, isoindolinyl, dihydrobenzofuranyl, tetrahydroquinolyl, isochromyl, dihydro-1H-isoindolyl, 2-azabicyclo[2.2.1]heptanonyl, 3-azabicyclo[3.1.0]hexanyl, 3-azabicyclo [4.1.0]heptanyl and the like. Further examples of heterocycloalkyl rings include tetrahydrofuran-2-yl, tetrahydrofuran-3-yl, imidazolidin-1-yl, imidazolidin-2-yl, imidazolidin-4-yl, pyrrolidin-1-yl, pyrrolidin-2-yl, pyrrolidin-3-yl, piperidin-1-yl, piperidin-2-yl, piperidin-3-yl, piperidin-4-yl, piperazin-1-yl, piperazin-2-yl, 1,3-oxazolidin-3-yl, 1,4-oxazepan-1-yl, isothiazolidinyl, 1,3-thiazolidin-3-yl, 1,2-pyrazolidin-2-yl, 1,2-tetrahydrothiazin-2-yl, 1,3-thiazinan-3-yl, 1,2-tetrahydrodiazin-2-yl, 1,3 tetrahydrodiazin-1-yl, 1,4-oxazin-4-yl, oxazolidinonyl, 2-oxo-piperidinyl (e.g., 2-oxo-piperidin-1-yl), and the like.

The term "(5- to 14-membered)heteroaryl" refers to a heteroaryl ring having from 5 to 14 ring atoms in which at least one of the ring atoms is a heteroatom (i.e., oxygen, nitrogen, or sulfur), with the remaining ring atoms being independently selected from the group consisting of carbon, oxygen, nitrogen, and sulfur. A "(5- to 6-membered)heteroaryl" refers to a heteroaryl ring having from 5 to 6 ring atoms in which at least one of the ring atoms is a heteroatom (i.e., oxygen, nitrogen, or sulfur), with the remaining ring atoms being independently selected from the group consisting of carbon, oxygen, nitrogen, and sulfur. A "(6-membered)heteroaryl" refers to a hetroaryl ring having 6 ring atoms. A "(5-membered)heteroaryl" refers to a heteroaryl ring having 5 ring atoms in which at least one of the ring atoms is a heteroatom. A heteroaryl may be a single ring or 2 or 3 fused rings. Examples of heteroaryls include, but are not limited to, 6-membered ring substituents such as pyridinyl, pyrazinyl, pyrimidinyl and pyridazinyl; 5-membered heteroaryls such as triazolyl, imidazolyl, furanyl, isoxazolyl, isothiazolyl, 1,2,3-, 1,2,4, 1,2,5-, or 1,3,4-oxadiazolyl, oxazolyl, thiophenyl, thiazolyl, isothiazolyl, and pyrazolyl; 6/5-membered fused ring substituents such as indolyl, indazolyl, benzofuranyl, benzimidazolyl, benzothienyl, benzoxadiazolyl, benzothiazolyl, isobenzothiofuranyl, benzothiofuranyl, benzothiophenyl, benzisoxazolyl, benzoxazolyl, furanopyridinyl, purinyl, imidazopyridinyl, pyrrolopyridinyl, pyrazolopyridinyl, thienopyridinyl, triazolopyrimidinyl, triazolopyridinyl (e.g., 5,6,7,8-tetrahydro[1,2,4]triazolo[1,5-a]pyridin-2-yl), and anthranilyl; and 6/6-membered fused ring substituents such as quinolinyl, isoquinolinyl, cinnolinyl, quinazolinyl, oxochromenyl, and 1,4-benzoxazinyl. In a group that has a heteroaryl substituent, the ring atom of the heteroaryl substituent that is bound to the group may be the at least one heteroatom when the heteroatom is nitrogen having the appropriate valence, or it may be a ring carbon atom, where the ring carbon atom may be in the same ring as the at least one heteroatom or where the ring carbon atom may be in a different ring from the at least one heteroatom. Similarly, if the heteroaryl substituent is in turn substituted with a group or substituent, the group or substituent may be bound to the at least one heteroatom when the heteroatom is a nitrogen having the appropriate valence or it may be bound to a ring carbon atom, where the ring carbon atom may be in the same ring as the at least one heteroatom, or where the ring carbon atom may be in a different ring from the at least one heteroatom.

It is to be understood that the "(5- to 14-membered)heteroaryl" may be optionally fused to a (C₃-C₈)cycloalkyl group, or to a (4- to 10-membered)heterocycloalkyl group, as defined herein. A group having such a fused heteroaryl group as a substituent is attached to an aromatic carbon of the heteroaryl group or to a heteroatom of the heteroaryl group when the heteroatom is nitrogen having the appropriate valence. When such a fused heteroaryl group is substituted with up to four substituents, the substituents, unless otherwise specified, are each bound to an aromatic carbon of the heteroaryl group or to a heteroatom of the heteroaryl group when the heteroatom is nitrogen having the appropriate valence.

The term "hydrogen" refers to a hydrogen substituent, and may be depicted as -H.

The term "hydroxy" or "hydroxyl" refers to -OH. When used in combination with another term(s), the prefix "hydroxy" indicates that the substituent to which the prefix is attached is substituted with one or more hydroxy substituents. Compounds bearing a carbon to which one or more hydroxy substituents are attached include, for example, alcohols, enols and phenol.

The term "cyano" (also referred to as "nitrile") means -CN, which also may be depicted:

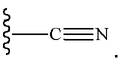

If a substituent is described as being "substituted," a non-hydrogen substituent is in the place of a hydrogen substituent on a carbon or nitrogen of the substituent. Thus, for example, a substituted alkyl substituent is an alkyl substituent wherein at least one non-hydrogen substituent is in the place of a hydrogen substituent on the alkyl substituent. To illustrate, monofluoroalkyl is alkyl substituted with a fluoro substituent, and difluoroalkyl is alkyl substituted with two fluoro substituents. It should be recognized that if there is more than one substitution on a substituent, each non-hydrogen substituent may be identical or different (unless otherwise stated).

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent. As a further example, when there are optional substituents that can be present, e.g., R¹¹ or R¹³, those substituents are as specified in the present specification, and when not present, the group to which the optional substituent could be attached (i.e., C or N) would have the requisite number of hydrogens attached.

This specification uses the terms "substituent," "radical," and "group" interchangeably.

If a substituent is described as being optionally substituted with up to a particular number of non-hydrogen substituents, that substituent may be either (1) not substituted; or (2) substituted by up to that particular number of non-hydrogen substituents or by up to the maximum number of substitutable positions on the substituent, whichever is less. Thus, for example, if a substituent is described as a heteroaryl optionally substituted with up to 3 non-hydrogen substituents, then any heteroaryl with less than 3 substitutable positions would be optionally substituted by up to only as many non-hydrogen substituents as the heteroaryl has substitutable positions. To illustrate, tetrazolyl (which has only one substitutable position) would be optionally substituted with up to one non-hydrogen substituent. To illustrate further, if an amino nitrogen is described as being optionally substituted with up to 2 non-hydrogen substituents, then the nitrogen will be optionally substituted with up to 2 non-hydrogen substituents if the amino nitrogen is a primary nitrogen, whereas the amino nitrogen will be optionally substituted with up to only 1 non-hydrogen substituent if the amino nitrogen is a secondary nitrogen.

If substituents are described as being "independently selected" from a group, each substituent is selected independent of the other(s). Each substituent therefore may be identical to or different from the other substituent(s).

It is to be understood that a variable following a substituent shown in parenthesis [i.e., "(R¹³ )₀₋₃"] with a numerical range means that the variable represents an integer (in this case selected from 0, 1, 2, or 3). The substituent "(R¹³)₀₋₁" means that the variable is present as an integer selected from 0 or 1, such that either one R¹³ group is present, or no R¹³ group is present.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

"Patient" refers to warm-blooded animals such as, for example, pigs, cows, chickens, horses, guinea pigs, mice, rats, gerbils, cats, rabbits, dogs, monkeys, chimpanzees, and humans.

"Treating" or "treat", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above. The term "treating" also includes adjuvant and neo-adjuvant treatment of a subject.

"Pharmaceutically acceptable" indicates that the substance or composition must be compatible, chemically and/or toxicologically, with the other ingredients comprising a formulation, and/or the mammal being treated therewith.

"Isomer" means "stereoisomer" and "geometric isomer" as defined below.

"Stereoisomer" refers to compounds that possess one or more chiral centers, which may each exist in the R or S configuration. Stereoisomers include all diastereomeric, enantiomeric and epimeric forms as well as racemates and mixtures thereof.

"Geometric isomer" refers to compounds that may exist in cis, trans, anti, *entgegen* (E), and *zusammen* (Z) forms as well as mixtures thereof.

As used herein the terms "Formula I", "Formula la", "Formula Ib", and "Formula Ic" may be hereinafter referred to as "compound(s) of the invention." Such terms are also defined to include all forms of the compound of Formulas I through Ic including hydrates, solvates, isomers, crystalline and non-crystalline forms, isomorphs, polymorphs, and metabolites thereof. For example, the compounds of Formulas I through Ic, or pharmaceutically acceptable salts thereof, may exist in unsolvated and solvated forms with pharmaceutically acceptable solvents such as water, ethanol and the like. When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm. In general, the solvated forms are considered equivalent to the unsolvated forms for the purposes of the present invention.

The compounds of the invention may exist as clathrates or other complexes. Included within the scope of the invention are complexes such as clathrates, drug-host inclusion complexes wherein the drug and host are present in stoichiometric or non-stoichiometric amounts. Also included are complexes of the compounds of the present invention containing two or more organic and/or inorganic components, which may be in stoichiometric or non-stoichiometric amounts. The resulting complexes may be ionized, partially ionized, or non-ionized. For a review of such complexes, see J. Pharm. Sci., 64 (8), 1269-1288 by Haleblian (August 1975).

Compounds of the invention may exist as geometric isomers. The compounds of the invention may possess one or more asymmetric centers, thus existing as two, or more, stereoisomeric forms. The present invention includes all the individual stereoisomers and geometric isomers of the compounds of the invention and mixtures thereof. Individual enantiomers can be obtained by resolution, chiral chromatography, or other methods well-known to those skilled in the art, or by using the relevant enantiomeric reactant or reagent in the synthesis.

The carbon-carbon bonds of the compounds of the invention may be depicted herein using a solid line (-), a solid wedge ( 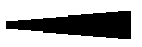 ), or a dotted wedge ( 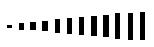 ). The use of a solid line to depict bonds to asymmetric carbon atoms is meant to indicate that all possible stereoisomers (e.g., specific enantiomers, racemic mixtures, etc.) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Racemic compounds possessing such indicated relative stereochemistry are marked with (+/-). For example, unless stated otherwise, it is intended that the compounds of the invention can exist as stereoisomers, which include cis and trans isomers, optical isomers such as R and S enantiomers, diastereomers, geometric isomers, rotational isomers, conformational isomers, atropisomers, and mixtures thereof. The compounds of the invention may exhibit more than one type of isomerism, and consist of mixtures thereof (such as racemates and diastereomeric pairs). Also included are acid addition or base addition salts wherein the counterion is optically active, for example, D-lactate or L-lysine, or racemic, for example, DL-tartrate or DL-arginine.

When any racemate crystallizes, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer.

The present invention also includes all pharmaceutically acceptable isotopically labeled compounds, which are identical to those recited in Formulas I through Ic except that one or more atoms are replaced by an atom having the same atomic number, but an atomic mass or mass number different from the atomic mass or mass number which predominates in nature. Examples of isotopes suitable for inclusion in the compounds of the present invention include, but are not limited to, isotopes of hydrogen, such as ²H, ³H; carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with heavier isotopes such as deuterium, i.e., ²H, may afford certain therapeutic advantages resulting from greater metabolic stability, for example, increased in vivo half-life or reduced dosage requirements and, hence, may be preferred in some circumstances. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by conventional techniques known to those skilled in the art or by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*. Compounds of the present invention, as well as the compounds exemplified in Examples 1-66 described below, include isotopically labeled versions of these compounds, such as, but not limited to, the deuterated and tritiated isotopes and all other isotopes discussed above.

The compounds of this invention may be used in the form of salts derived from inorganic or organic acids. Depending on the particular compound, a salt of the compound may be advantageous due to one or more of the salt's physical properties, such as enhanced pharmaceutical stability in differing temperatures and humidities, or a desirable solubility in water or oil. In some instances, a salt of a compound also may be used as an aid in the isolation, purification, and/or resolution of the compound.

Where a salt is intended to be administered to a patient (as opposed to, for example, being used in an in vitro context), the salt preferably is pharmaceutically acceptable. The term "pharmaceutically acceptable salt" refers to a salt prepared by combining a compound of the invention with an acid whose anion, or a base whose cation, is generally considered suitable for human consumption. Pharmaceutically acceptable salts are particularly useful as products of the methods of the present invention because of their greater aqueous solubility relative to the parent compound.

Suitable pharmaceutically acceptable acid addition salts of the compounds of the present invention when possible include those derived from inorganic acids, such as hydrochloric, hydrobromic, hydrofluoric, boric, fluoroboric, phosphoric, metaphosphoric, nitric, carbonic, sulfonic, and sulfuric acids, and organic acids such as acetic, benzenesulfonic, benzoic, citric, ethanesulfonic, fumaric, gluconic, glycolic, isothionic, lactic, lactobionic, maleic, malic, methanesulfonic, trifluoromethanesulfonic, succinic, toluenesulfonic, tartaric, and trifluoroacetic acids. Suitable organic acids generally include but are not limited to aliphatic, cycloaliphatic, aromatic, araliphatic, heterocyclic, carboxylic, and sulfonic classes of organic acids.

Specific examples of suitable organic acids include but are not limited to acetate, trifluoroacetate, formate, propionate, succinate, glycolate, gluconate, digluconate, lactate, malate, tartrate, citrate, ascorbate, glucuronate, maleate, fumarate, pyruvate, aspartate, glutamate, benzoate, anthranilate, stearate, salicylate, p-hydroxybenzoate, phenylacetate, mandelate, embonate (pamoate), methanesulfonate, ethanesulfonate, benzenesulfonate, pantothenate, toluenesulfonate, 2-hydroxyethanesulfonate, sufanilate, cyclohexylaminosulfonate, β-hydroxybutyrate, galactarate, galacturonate, adipate, alginate, butyrate, camphorate, camphorsulfonate, cyclopentanepropionate, dodecylsulfate, glycoheptanoate, glycerophosphate, heptanoate, hexanoate, nicotinate, 2-naphthalenesulfonate, oxalate, palmoate, pectinate, 3-phenylpropionate, picrate, pivalate, thiocyanate, and undecanoate.

Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. In another embodiment, base salts are formed from bases which form non-toxic salts, including aluminum, arginine, benzathine, choline, diethylamine, diolamine, glycine, lysine, meglumine, olamine, tromethamine and zinc salts.

Organic salts may be made from secondary, tertiary or quaternary amine salts, such as tromethamine, diethylamine, *N,N'*-dibenzylethylenediamine, chloroprocaine, choline, diethanolamine, ethylenediamine, meglumine (*N*-methylglucamine), and procaine. Basic nitrogen-containing groups may be quaternized with agents such as lower alkyl (C₁-C₆) halides (e.g., methyl, ethyl, propyl, and butyl chlorides, bromides, and iodides), dialkyl sulfates (e.g., dimethyl, diethyl, dibutyl, and diamyl sulfates), long chain halides (e.g., decyl, lauryl, myristyl, and stearyl chlorides, bromides, and iodides), arylalkyl halides (e.g., benzyl and phenethyl bromides), and others.

In one embodiment, hemisalts of acids and bases may also be formed, for example, hemisulfate and hemicalcium salts.

Certain derivatives of the compound of the invention that may have little or no pharmacological activity themselves can, when administered into or onto the body, be converted into the compound of the invention having the desired activity, for example, by hydrolytic cleavage. Such derivatives are referred to as "prodrugs." Further information on the use of prodrugs may be found in "Pro-drugs as Novel Delivery Systems, Vol. 14, ACS Symposium Series (T. Higuchi and V. Stella) and "Bioreversible Carriers in Drug Design," Pergamon Press, 1987 (ed. E. B. Roche, American Pharmaceutical Association). Prodrugs can, for example, be produced by replacing appropriate functionalities present in the compounds of the present invention with certain moieties known to those skilled in the art as "pro-moieties" as described, for example, in "Design of Prodrugs" by H. Bundgaard (Elsevier, 1985).

One skilled in the art will appreciate that compounds of the invention can also be prepared with certain protecting groups that are useful for purification or storage and can be removed before administration to a patient. The protection and deprotection of functional groups is described in "Protective Groups in Organic Chemistry", edited by J. W. F. McOmie, Plenum Press (1973) and "Protective Groups in Organic Synthesis", 3rd edition, T. W. Greene and P. G. M. Wuts, Wiley-Interscience (1999).

Typically, a compound of the invention is administered in an amount effective to treat a condition as described herein. The compounds of the invention are administered by any suitable route in the form of a pharmaceutical composition adapted to such a route, and in a dose effective for the treatment intended. Therapeutically effective doses of the compounds required to treat the progress of the medical condition are readily ascertained by one of ordinary skill in the art using preclinical and clinical approaches familiar to the medicinal arts. The term "therapeutically effective amount" as used herein refers to that amount of the compound being administered which will relieve to some extent one or more of the symptoms of the disorder being treated.

### Compounds

The compounds of Formula I, as depicted above, have a fused bicyclic core represented by 3,4-dihydro-2*H*-pyrido[1,2-*a*]pyrazine-1,6-dione. On the left side of the core, the pyridinone ring is substituted with R⁶, R⁷, and a (5- to 14-membered)heteroaryl moiety represented by X, wherein X is further substituted with R¹; and on the right side of the core the pyrazinone ring is substituted with R^{4a}, R^{4b}, R^{5a}, R^{5b} and a moiety represented by: 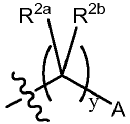 wherein A is represented by a chromenyl, a cyclopropachromenyl, or a cyclopropabenzofuranyl moiety.

In the compounds of Formula I, X is a (5-membered)heteroaryl, wherein the heteroaryl is imidazolyl.

In the compounds of Formula I:
R¹ is a (C₁-C₆)alkyl, wherein the (C₁-C₆)alkyl is methyl; and
R^{2a}, R^{2b}, R^{4a}, R^{4b}, R^{5a} and R^{5b} are each independently
   i) hydrogen; or
   ii) (C₁-C₆)alkyl, wherein the (C₁-C₆)alkyl is methyl.

In certain other embodiments, R¹ is methyl; and R^{2a}, R^{2b}, R^{4a}, R^{4b}, R^{5a} and R^{5b} are each independently hydrogen.

In certain other embodiments, R¹ is methyl; R^{2a}, R^{2b}, R^{5a} and R^{5b} are each independently hydrogen; and one of R^{4a} and R^{4b} is hydrogen and the other is methyl.

In another embodiment, R¹ is methyl; one of R^{2a} and R^{2b} is hydrogen and the other is methyl; and R^{4a}, R^{4b}, R^{5a} and R^{5b} are each independently hydrogen.

In the compounds of Formula I A is represented by a Formula selected from the group consisting of A1a, A1b, and A3a: 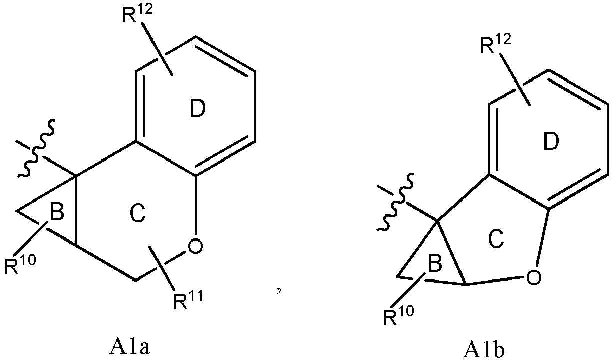 or 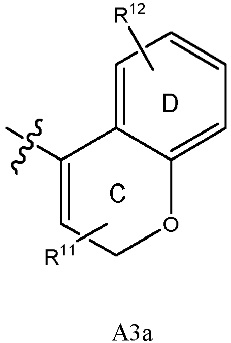 wherein:
X is a (5-membered)heteroaryl selected from imidazolyl;
R¹ is (C₁-C₆)alkyl wherein the alkyl is methyl;
R^{2a}, R^{2b}, R^{4a}, R^{4b}, R^{5a} and R^{5b} are each independently selected from hydrogen or (C₁-C₆)alkyl wherein the (C₁-C₆)alkyl is methyl;
R⁶ and R⁷ are each independently hydrogen;
z and y are each 1;
ring B is optionally substituted with up to three R¹⁰, wherein each R¹⁰ is independently selected from halogen or (C₁-C₆)alkyl;
ring C is optionally substituted with up to three R¹¹ such that substitution occurs at any carbon atom that is chemically permissible, and wherein each R¹¹ is independently selected from halogen, (C₁-C₆)alkyl, or halo(C₁-C₆)alkyl; or two R¹¹ taken together with the carbon atom(s) to which they are attached form a (C₃-C₆)cycloalkyl or a (4- to 6-membered) heterocycloalkyl, wherein the (C₃-C₆)cycloalkyl and (4- to 6-membered) heterocycloalkyl moieties are each optionally substituted with one to three substituents independently selected from halogen or (C₁-C₆)alkyl; and
ring D is optionally substituted with up to three R¹², wherein each R¹² is independently selected from the group consisting of halogen, (C₁-C₆)alkyl, (C₁-C₆)alkoxy, halo(C₁-C₆)alkyl, halo(C₁-C₆)alkoxy, -SF₅, and (C₃-C₆)cycloalkyl, wherein the (C₃-C₆)cycloalkyl is optionally substituted with one to three substituents independently selected from halogen, (C₁-C₆)alkyl, or halo(C₁-C₆)alkyl.

In certain other embodiments, in Formula I, as described above, A is represented by Formula A1a: 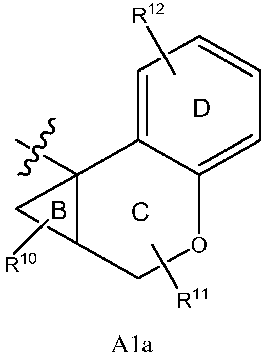 wherein:
X is a (5- membered)heteroaryl, and the (5- membered)heteroaryl is imidazolyl;
R¹ is methyl;
R^{2a}, R^{2b}, R^{4a}, R^{4b}, R^{5a} and R^{5b} are each independently selected from hydrogen or methyl;
R⁶ and R⁷ are each independently hydrogen;
z and y are each 1;
ring B is optionally substituted with one to two R¹⁰, wherein each R¹⁰ is selected from halogen or (C₁-C₆)alkyl; ring C is optionally substituted with one to two R¹¹, wherein each R¹¹ is selected from (C₁-C₆)alkyl or halo(C₁-C₆)alkyl; or two R¹¹ taken together with the carbon atom(s) to which they are attached form a (C₃-C₆)cycloalkyl or a (4- to 6-membered)heterocycloalkyl; and ring D is optionally substituted with one to three R¹², wherein each R¹² is selected from halogen, (C₁-C₆)alkyl, (C₁-C₆)alkoxy, halo(C₁-C₆)alkyl, halo(C₁-C₆)alkoxy, -SF₅, and (C₃-C₆)cycloalkyl optionally substituted with one or two substituents selected from methyl or trifluoromethyl.

In certain embodiments, Formula I is as immediately described above, wherein:
each R¹⁰ is selected from:
   i) halogen selected from fluoro or chloro, or
   ii) (C₁-C₆)alkyl, wherein the (C₁-C₆)alkyl is methyl;
each R¹¹ is selected from:
   i) (C₁-C₆)alkyl, wherein the (C₁-C₆)alkyl is methyl; or
   ii) halo(C₁-C₆)alkyl, wherein the halo(C₁-C₆)alkyl is selected from fluoromethyl, trifluoromethyl, or trifluoroethyl; or
two R¹¹ taken together with the carbon atom(s) to which they are attached form:
   i) a (C₃-C₆)cycloalkyl, wherein the (C₃-C₆)cycloalkyl is cyclobutyl; or
   ii) a (4- to 6-membered)heterocycloalkyl wherein the (4- to 6-membered)heterocycloalkyl is an oxetanyl, and
each R¹² is selected from:
   i) halogen selected from fluoro or chloro;
   ii) (C₁-C₆)alkyl, wherein the (C₁-C₆)alkyl is methyl;
   iii) (C₁-C₆)alkoxy, wherein the (C₁-C₆)alkoxy is methoxy;
   iv) halo(C₁-C₆)alkyl, wherein the halo(C₁-C₆)alkyl is selected from fluoromethyl, trifluoromethyl, or trifluoroethyl;
   v) halo(C₁-C₆)alkoxy, wherein the halo(C₁-C₆)alkoxy is selected from fluoromethoxy, fluoroethoxy, or difluoroethoxy;
   vi) -SF₅; or
   vii) (C₃-C₆)cycloalkyl, wherein the (C₃-C₆)cycloalkyl is cyclopropyl optionally substituted with one to two substituents selected from methyl and trifluoromethyl.

In certain other embodiments, in Formula I, A is represented by Formula A1b: 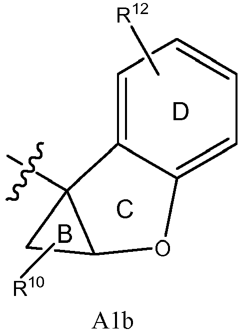 wherein:
X is a (5- membered)heteroaryl, and the (5- membered)heteroaryl is imidazolyl;
R¹ is methyl;
R^{2a}, R^{2b}, R^{4a}, R^{4b}, R^{5a} and R^{5b} are each independently selected from hydrogen or methyl;
R⁶ and R⁷ are each independently hydrogen;
z and y are each 1;
ring B is optionally substituted with one to two R¹⁰, wherein each R¹⁰ is selected from halogen or (C₁-C₆)alkyl; and ring D is optionally substituted with one to three R¹², wherein each R¹² is selected from halogen, (C₁-C₆)alkyl, (C₁-C₆)alkoxy, halo(C₁-C₆)alkyl, halo(C₁-C₆)alkoxy, -SF₅, and (C₃-C₆)cycloalkyl optionally substituted with one or two substituents selected from methyl or trifluoromethyl.

In certain embodiments, Formula I is as immediately described above:
R¹⁰ is selected from:
   i) halogen selected from fluoro or chloro, or
   ii) (C₁-C₆)alkyl, wherein the (C₁-C₆)alkyl is methyl; and
R¹² is selected from:
   i) halogen selected from fluoro or chloro;
   ii) (C₁-C₆)alkyl, wherein the (C₁-C₆)alkyl is methyl;
   iii) (C₁-C₆)alkoxy, wherein the (C₁-C₆)alkoxy is methoxy;
   iv) halo(C₁-C₆)alkyl, wherein the halo(C₁-C₆)alkyl is selected from fluoromethyl, trifluoromethyl, or trifluoroethyl;
   v) halo(C₁-C₆)alkoxy, wherein the halo(C₁-C₆)alkoxy is selected from fluoromethoxy, fluoroethoxy, or difluoroethoxy;
   vi) -SF₅; or
   vii) (C₃-C₆)cycloalkyl, wherein the (C₃-C₆)cycloalkyl is cyclopropyl optionally substituted with one to two substituents selected from methyl and trifluoromethyl.

In certain other embodiments, in Formula I, A is represented by Formula A3a: 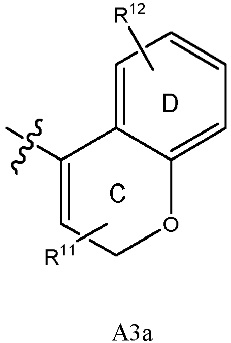 wherein:
X is a (5- membered)heteroaryl, and the (5- membered)heteroaryl is imidazolyl;
R¹ is methyl;
R^{2a}, R^{2b}, R^{4a}, R^{4b}, R^{5a} and R^{5b} are each independently selected from hydrogen or methyl;
R⁶ and R⁷ are each independently hydrogen;
z and y are each 1;
ring C is optionally substituted with one to three R¹¹, wherein each R¹¹ is selected from (C₁-C₆)alkyl or halo(C₁-C₆)alkyl; or two R¹¹ taken together with the carbon atom(s) to which they are attached form a (C₃-C₆)cycloalkyl or a (4- to 6-membered)heterocycloalkyl; and ring D is optionally substituted with one to three R¹², wherein each R¹² is selected from halogen, (C₁-C₆)alkyl, (C₁-C₆)alkoxy, halo(C₁-C₆)alkyl, halo(C₁-C₆)alkoxy, -SF₅, and (C₃-C₆)cycloalkyl optionally substituted with one or two substituents selected from methyl or trifluoromethyl.

In certain embodiments, Formula I is as immediately described above:
each R¹¹ is selected from:
   i) (C₁-C₆)alkyl, wherein the (C₁-C₆)alkyl is methyl; or
   ii) halo(C₁-C₆)alkyl, wherein the halo(C₁-C₆)alkyl is selected from fluoromethyl, trifluoromethyl, or trifluoroethyl; or
two R¹¹ taken together with the carbon atom to which they are attached form:
   i) a (C₃-C₆)cycloalkyl, wherein the (C₃-C₆)cycloalkyl is cyclobutyl; or
   ii) a (4- to 6-membered)heterocycloalkyl wherein the (4- to 6-membered)heterocycloalkyl is an oxetanyl, and
each R¹² is selected from:
   i) halogen selected from fluoro or chloro;
   ii) (C₁-C₆)alkyl, wherein the (C₁-C₆)alkyl is methyl;
   iii) (C₁-C₆)alkoxy, wherein the (C₁-C₆)alkoxy is methoxy;
   iv) halo(C₁-C₆)alkyl, wherein the halo(C₁-C₆)alkyl is selected from fluoromethyl, trifluoromethyl, or trifluoroethyl;
   v) halo(C₁-C₆)alkoxy, wherein the halo(C₁-C₆)alkoxy is selected from fluoromethoxy, fluoroethoxy, or difluoroethoxy;
   vi) -SF₅; or
   vii) (C₃-C₆)cycloalkyl, wherein the (C₃-C₆)cycloalkyl is cyclopropyl optionally substituted with one to two substituents selected from methyl and trifluoromethyl.

To further elucidate the compounds of the present invention, wherein X is a (5-membered)heteroaryl ring and the (5-membered)heteroaryl ring is imidazolyl, and A is a cyclopropachromenyl, a cyclopropabenzofuranyl or a chromenyl moiety, the following subgenuses are described below:

Formula Ia, as depicted below, is a subset of Formula I, as depicted above, wherein A is A1a as depicted above, X is a (5-membered)heteroaryl wherein the heteroaryl is imidazolyl, R¹ is a (C₁-C₆)alkyl wherein the (C₁-C₆)alkyl is methyl, R⁶ and R⁷ are each hydrogen, and y and z are each an integer of 1:

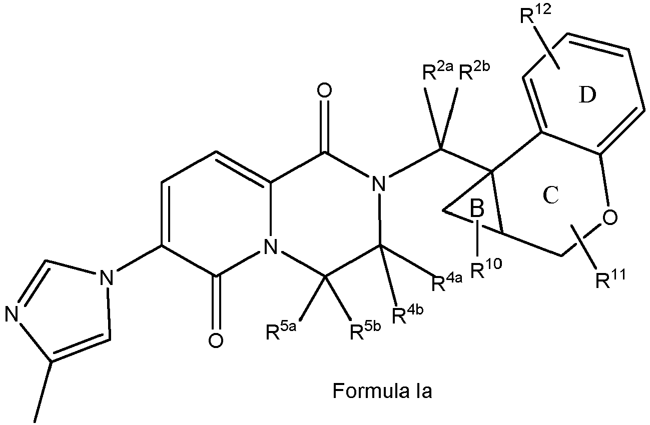

In certain embodiments, in Formula Ia, as depicted above, or a pharmaceutically acceptable salt thereof:
R^{2a}, R^{2b}, R^{4a}, R^{4b}, R^{5a} and R^{5b} are each independently selected from hydrogen or methyl;
ring B is optionally substituted with up to two R¹⁰, wherein each R¹⁰ is independently selected from halogen or (C₁-C₆)alkyl;
ring C is optionally substituted with up to two R¹¹, wherein each R¹¹ is independently selected from (C₁-C₆)alkyl or halo(C₁-C₆)alkyl; or two R¹¹ taken together with the carbon atom(s) to which they are attached form a (C₃-C₆)cycloalkyl or a (4- to 6-membered)heteroaryl; and
ring D is optionally substituted with up to three R¹², wherein each R¹² is independently selected from halogen, (C₁-C₆)alkyl, (C₁-C₆)alkoxy, halo(C₁-C₆)alkyl, halo(C₁-C₆)alkoxy, -SF₅, or (C₃-C₆)cycloalkyl, wherein the (C₃-C₆)cycloalkyl is optionally substituted with one to two substituents independently selected from halogen or (C₁-C₆)alkyl.

In certain embodiments, Formula Ia is as immediately described above:
R^{2a}, R^{2b}, R^{4a}, R^{4b}, R^{5a} and R^{5b} are independently selected from hydrogen or (C₁-C₆)alkyl, wherein the (C₁-C₆)alkyl is methyl;
ring B is optionally substituted with up to two R¹⁰, wherein each R¹⁰ is selected from:
   i) halogen selected from fluoro or chloro, or
   ii) (C₁-C₆)alkyl, wherein the (C₁-C₆)alkyl is methyl;
ring C is optionally substituted with up to two R¹¹, wherein each R¹¹ is selected from:
   i) (C₁-C₆)alkyl, wherein the (C₁-C₆)alkyl is methyl; or
   ii) halo(C₁-C₆)alkyl, wherein the halo(C₁-C₆)alkyl is selected from fluoromethyl, trifluoromethyl, or trifluoroethyl; or
two R¹¹ taken together with the carbon atom(s) to which they are attached form:
   i) a (C₃-C₆)cycloalkyl wherein the (C₃-C₆)cycloalkyl is cyclopropyl; or
   ii) a (4- to 6-membered)heteroaryl, wherein the (4- to 6-membered)heteroaryl is oxetanyl; and
ring D is optionally substituted with up to three R¹², wherein each R¹² is selected from:
   i) halogen selected from fluoro or chloro;
   ii) (C₁-C₆)alkyl, wherein the (C₁-C₆)alkyl is methyl;
   iii) (C₁-C₆)alkoxy, wherein the (C₁-C₆)alkoxy is methoxy;
   iv) halo(C₁-C₆)alkyl, wherein the halo(C₁-C₆)alkyl is selected from fluoromethyl, trifluoromethyl, or trifluoroethyl;
   v) halo(C₁-C₆)alkoxy, wherein the halo(C₁-C₆)alkoxy is selected from fluoromethoxy, fluoroethoxy, or difluoroethoxy;
   vi) -SF₅; or
   vii) (C₃-C₆)cycloalkyl, wherein the (C₃-C₆)cycloalkyl is cyclopropyl optionally substituted with one to two substituents selected from methyl and trifluoromethyl.

Formula Ib, as depicted below, is a subset of Formula I as depicted above, wherein A is A1b as depicted above, X is a (5-membered)heteroaryl, wherein the heteroaryl is imidazolyl, R¹ is a (C₁-C₆)alkyl, wherein the (C₁-C₆)alkyl is methyl, R⁶ and R⁷ are each hydrogen, and y and z are each an integer of 1:

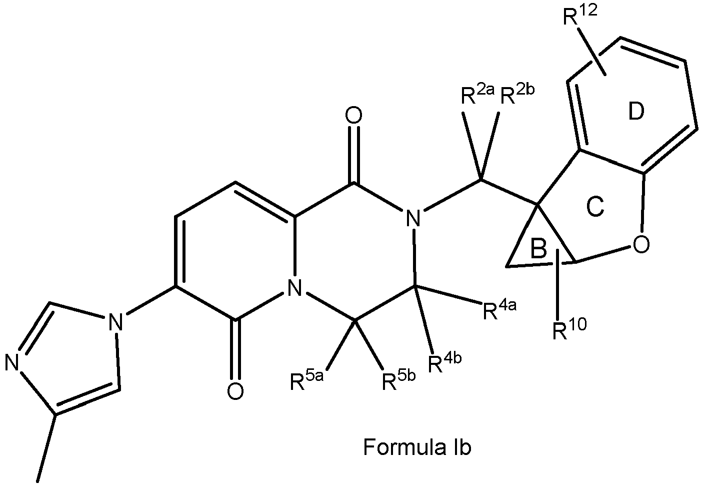

In certain embodiments, in Formula Ib, as depicted above, or a pharmaceutically acceptable salt thereof:
R^{2a}, R^{2b}, R^{4a}, R^{4b}, R^{5a} and R^{5b} are each independently selected from hydrogen or methyl;
ring B is optionally substituted with up to two R¹⁰, wherein each R¹⁰ is independently selected from halogen or (C₁-C₆)alkyl; and
ring D is optionally substituted with up to three R¹², wherein each R¹² is independently selected from halogen, (C₁-C₆)alkyl, (C₁-C₆)alkoxy, halo(C₁-C₆)alkyl, halo(C₁-C₆)alkoxy, -SF₅, or (C₃-C₆)cycloalkyl, wherein the (C₃-C₆)cycloalkyl is optionally substituted with one to two substituents independently selected from halogen or (C₁-C₆)alkyl.

In certain embodiments, Formula Ib is as immediately described above:
R^{2a}, R^{2b}, R^{4a}, R^{4b}, R^{5a} and R^{5b} are independently selected from hydrogen or (C₁-C₆)alkyl, wherein the (C₁-C₆)alkyl is methyl;
ring B is optionally substituted with up to three R¹⁰, wherein each R¹⁰ is selected from:
   i) halogen selected from fluoro or chloro, or
   ii) (C₁-C₆)alkyl, wherein the (C₁-C₆)alkyl is methyl; and
ring D is optionally substituted with up to three R¹², wherein each R¹² is selected from:
   i) halogen selected from fluoro or chloro;
   ii) (C₁-C₆)alkyl, wherein the (C₁-C₆)alkyl is methyl;
   iii) (C₁-C₆)alkoxy, wherein the (C₁-C₆)alkoxy is methoxy;
   iv) halo(C₁-C₆)alkyl, wherein the halo(C₁-C₆)alkyl is selected from fluoromethyl, trifluoromethyl, or trifluoroethyl;
   v) halo(C₁-C₆)alkoxy, wherein the halo(C₁-C₆)alkoxy is selected from fluoromethoxy, fluoroethoxy, or difluoroethoxy;
   vi) -SF₅; or
   vii) (C₃-C₆)cycloalkyl, wherein the (C₃-C₆)cycloalkyl is cyclopropyl optionally substituted with one to two substituents selected from methyl and trifluoromethyl.

Formula Ic, as depicted below, is a subset of Formula I as depicted above, wherein A is A3a as depicted above, X is a (5-membered)heteroaryl, wherein the heteroaryl is imidazolyl, R¹ is a (C₁-C₆)alkyl, wherein the (C₁-C₆)alkyl is methyl, R⁶ and R⁷ are each hydrogen, and y and z are each an integer of 1:

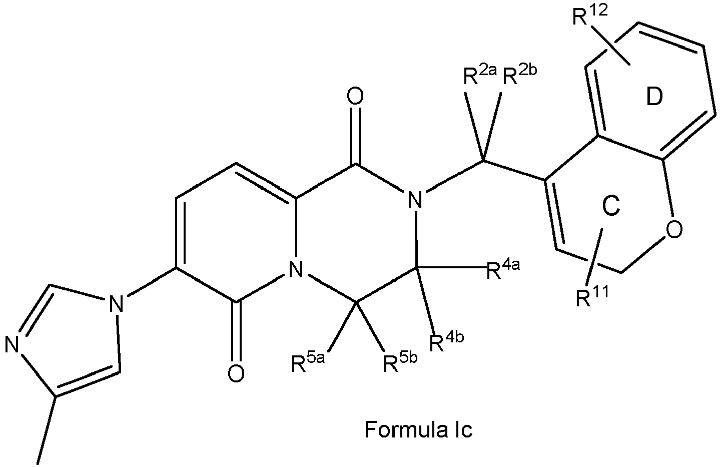

In certain embodiments, in Formula Ic, as depicted above, or a pharmaceutically acceptable salt thereof:
R^{2a}, R^{2b}, R^{4a}, R^{4b}, R^{5a} and R^{5b} are each independently selected from hydrogen or methyl;
ring C is optionally substituted with up to three R¹¹, wherein each R¹¹ is independently selected from (C₁-C₆)alkyl or halo(C₁-C₆)alkyl; or two R¹¹ taken together with the carbon atom(s) to which they are attached form a (C₃-C₆)cycloalkyl or a (4- to 6-membered)heteroaryl; and
ring D is optionally substituted with up to three R¹², wherein each R¹² is independently selected from halogen, (C₁-C₆)alkyl, (C₁-C₆)alkoxy, halo(C₁-C₆)alkyl, halo(C₁-C₆)alkoxy, -SF₅, or (C₃-C₆)cycloalkyl, wherein the (C₃-C₆)cycloalkyl is optionally substituted with one to two substituents independently selected from halogen or (C₁-C₆)alkyl.

In certain embodiments, Formula Ic is as immediately described above:
R^{2a}, R^{2b}, R^{4a}, R^{4b}, R^{5a} and R^{5b} are independently selected from hydrogen or (C₁-C₆)alkyl, wherein the (C₁-C₆)alkyl is methyl;
ring C is optionally substituted with up to three R¹¹, wherein each R¹¹ is selected from:
   i) (C₁-C₆)alkyl, wherein the (C₁-C₆)alkyl is methyl; or
   ii) halo(C₁-C₆)alkyl, wherein the halo(C₁-C₆)alkyl is selected from fluoromethyl, trifluoromethyl, or trifluoroethyl; or
two R¹¹ taken together with the carbon atom(s) to which they are attached form:
   i) a (C₃-C₆)cycloalkyl wherein the (C₃-C₆)cycloalkyl is cyclopropyl; or
   ii) a (4- to 6-membered)heteroaryl, wherein the (4- to 6-membered)heteroaryl is oxetanyl; and
ring D is optionally substituted with up to three R¹², wherein each R¹² is selected from:
   i) halogen selected from fluoro or chloro;
   ii) (C₁-C₆)alkyl, wherein the (C₁-C₆)alkyl is methyl;
   iii) (C₁-C₆)alkoxy, wherein the (C₁-C₆)alkoxy is methoxy;
   iv) halo(C₁-C₆)alkyl, wherein the halo(C₁-C₆)alkyl is selected from fluoromethyl, trifluoromethyl, or trifluoroethyl;
   v) halo(C₁-C₆)alkoxy, wherein the halo(C₁-C₆)alkoxy is selected from fluoromethoxy, fluoroethoxy, or difluoroethoxy;
   vi) -SF₅;
   vii) (C₃-C₆)cycloalkyl, wherein the (C₃-C₆)cycloalkyl is cyclopropyl optionally substituted with one to two substituents selected from methyl and trifluoromethyl.

In another embodiment, selected compounds of the present invention, or pharmaceutically acceptable salts thereof, may be useful for the treatment of neurodegeneration and psychiatric disorders, including Alzheimer's disease or Niemann-Pick disease type C.

In certain embodiments, selected compounds of the present invention may be useful for treating Alzheimer's disease or Niemann-Pick disease type C in a patient, by administration of a therapeutically effective amount of a compound of the present invention or a pharmaceutically acceptable salt thereof, to a patient in need thereof.

In certain embodiments, the present invention is directed to a pharmaceutical composition comprising selected compounds of the present invention, or a pharmaceutically acceptable salt thereof, and a pharmaceutically acceptable excipient.

### Pharmacology

Alzheimer's disease (AD) research indicates that the disease is associated with the buildup of plaques in variable shapes and sizes in the brain. The primary plaques associated with AD are composed of amyloid beta protein (Aβ). Aβ is produced when the amyloid protein precursor (APP) undergoes successive proteolysis by β- and γ-secretase (Haas et al., "Trafficking and proteolytic processing of APP." Cold Spring Harbor Perspect. Med., 2011). γ-Secretase is a large complex of four different integral proteins, one of which has been identified as a catalytic component that comprises an unusual membrane-embedded component (De Strooper, Bart et al., "Presenilins and γ-Secretase: Structure, Function, and Role in Alzheimer's Disease. "Cold Spring Harbor Perspect. Med. 2012;2:a006304). The catalytic components, known as presenilins, were first discovered as sites of missense mutations responsible for early-onset Alzheimer's disease. The encoded multipass membrane proteins were subsequently found to be the catalytic components of γ-secretases, membrane-embedded aspartyl protease complexes responsible for generating the carboxyl terminus of the amyloid beta protein from the amyloid protein precursor. (De Strooper, Bart et al.; 2012). Accordingly, targeting γ-secretase proteins for drug discovery has become a main focus of Alzheimer's disease research.

The compounds of the present invention are believed to be γ-secretase modulators and can be used for treating conditions or diseases of the central nervous system identified to have enhanced γ-secretase activity, such as Niemann-Pick disease type C; neurological disorders (such as migraine; epilepsy; Alzheimer's disease; Parkinson's disease; brain injury; stroke; cerebrovascular diseases (including cerebral arteriosclerosis, cerebral amyloid angiopathy, hereditary cerebral hemorrhage, and brain hypoxia-ischemia); cognitive disorders (including amnesia, senile dementia, HIV-associated dementia, Alzheimer's disease, Huntington's disease, Lewy body dementia, vascular dementia, drug-related dementia, myoclonus, dystonia, delirium, Pick's disease, Creutzfeldt-Jacob disease, HIV disease, Gilles de la Tourette's syndrome, epilepsy, and mild cognitive impairment); tardive dyskinesia; muscular spasms and disorders associated with muscular spasticity or weakness including tremors; mental deficiency (including spasticity, Down's syndrome and fragile X syndrome); sleep disorders (including hypersomnia, circadian rhythm sleep disorder, insomnia, parasomnia, and sleep deprivation) and psychiatric disorders such as anxiety (including acute stress disorder, generalized anxiety disorder, social anxiety disorder, panic disorder, post-traumatic stress disorder, agoraphobia, and obsessive-compulsive disorder); factitious disorders (including acute hallucinatory mania); impulse control disorders (including compulsive gambling and intermittent explosive disorder); mood disorders (including bipolar I disorder, bipolar II disorder, mania, mixed affective state, major depression, chronic depression, seasonal depression, psychotic depression, premenstrual syndrome (PMS), premenstrual dysphoric disorder (PDD), and postpartum depression); psychomotor disorders; psychotic disorders (including schizophrenia, schizoaffective disorder, schizophreniform, and delusional disorder); drug dependence (including narcotic dependence, alcoholism, amphetamine dependence, cocaine addiction, nicotine dependence, and drug withdrawal syndrome); eating disorders (including anorexia, bulimia, binge eating disorder, hyperphagia, obesity, compulsive eating disorders and pagophagia); sexual dysfunction disorders; urinary incontinence; neuronal damage disorders (including ocular damage, retinopathy or macular degeneration of the eye; tinnitus, hearing impairment and loss; and brain edema) and pediatric psychiatric disorders (including attention deficit disorder, attention deficit/hyperactive disorder, conduct disorder, and autism) in a mammal, preferably a human, comprising administering to said mammal a therapeutically effective amount of a compound of the present invention or a pharmaceutically acceptable salt thereof.

In certain embodiments, the compounds of the present invention can be utilized for treating a neurological disorder (such as migraine; epilepsy; Alzheimer's disease; Parkinson's disease; Niemann Pick type C; brain injury; stroke; cerebrovascular disease; cognitive disorder; sleep disorder) or a psychiatric disorder (such as anxiety; factitious disorder; impulse control disorder; mood disorder; psychomotor disorder; psychotic disorder; drug dependence; eating disorder; and pediatric psychiatric disorder) in a mammal, preferably a human, comprising administering to said mammal a therapeutically effective amount of a compound of the invention or pharmaceutically acceptable salt thereof.

Compounds of the present invention may also be useful for improving memory (both short term and long term) and learning ability.

The text revision of the fourth edition of the Diagnostic and Statistical Manual of Mental Disorders (DSM-IV-TR) (2000, American Psychiatric Association, Washington D.C.) provides a diagnostic tool for identifying many of the disorders described herein. The skilled artisan will recognize that there are alternative nomenclatures, nosologies, and classification systems for disorders described herein, including those as described in the DMS-IV and that terminology and classification systems evolve with medical scientific progress.

### Formulations

The compounds of the invention may be administered orally. Oral administration may involve swallowing, so that the compound enters the gastrointestinal tract, or buccal or sublingual administration may be employed, by which the compound enters the blood stream directly from the mouth.

In another embodiment, the compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

In another embodiment, the compounds of the invention may also be administered topically to the skin or mucosa, that is, dermally or transdermally. In another embodiment, the compounds of the invention can also be administered intranasally or by inhalation. In another embodiment, the compounds of the invention may be administered rectally or vaginally. In another embodiment, the compounds of the invention may also be administered directly to the eye or ear.

The dosage regimen for the compounds and/or compositions containing the compounds is based on a variety of factors, including the type, age, weight, sex and medical condition of the patient; the severity of the condition; the route of administration; and the activity of the particular compound employed. Thus the dosage regimen may vary widely. Dosage levels of the order from about 0.01 mg to about 100 mg per kilogram of body weight per day are useful in the treatment of the above-indicated conditions. In one embodiment, the total daily dose of a compound of the invention (administered in single or divided doses) is typically from about 0.01 to about 100 mg/kg. In another embodiment, the total daily dose of the compound of the invention is from about 0.1 to about 50 mg/kg, and in another embodiment, from about 0.5 to about 30 mg/kg (i.e., mg compound of the invention per kg body weight). In one embodiment, dosing is from 0.01 to 10 mg/kg/day. In another embodiment, dosing is from 0.1 to 1.0 mg/kg/day. Dosage unit compositions may contain such amounts or submultiples thereof to make up the daily dose. In many instances, the administration of the compound will be repeated a plurality of times in a day (typically no greater than 4 times). Multiple doses per day typically may be used to increase the total daily dose, if desired.

For oral administration, the compositions may be provided in the form of tablets containing 0.01, 0.05, 0.1, 0.5, 1.0, 2.5, 5.0, 10.0, 15.0, 25.0, 50.0, 75.0, 100, 125, 150, 175, 200, 250 and 500 milligrams of the active ingredient for the symptomatic adjustment of the dosage to the patient. A medicament typically contains from about 0.01 mg to about 500 mg of the active ingredient, or in another embodiment, from about 1 mg to about 100 mg of active ingredient. Intravenously, doses may range from about 0.1 to about 10 mg/kg/minute during a constant rate infusion.

Suitable subjects according to the present invention include mammalian subjects. Mammals according to the present invention include, but are not limited to, canine, feline, bovine, caprine, equine, ovine, porcine, rodents, lagomorphs, primates, and the like, and encompass mammals in utero. In one embodiment, humans are suitable subjects. Human subjects may be of either gender and at any stage of development.

In another embodiment, the invention comprises the use of one or more compounds of the invention for the preparation of a medicament for the treatment of the conditions recited herein.

For the treatment of the conditions referred to above, the compounds of the invention can be administered as compound per se. Alternatively, pharmaceutically acceptable salts are suitable for medical applications because of their greater aqueous solubility relative to the parent compound.

In another embodiment, the present invention comprises pharmaceutical compositions. Such pharmaceutical compositions comprise a compound of the invention presented with a pharmaceutically acceptable carrier. The carrier can be a solid, a liquid, or both, and may be formulated with the compound as a unit-dose composition, for example, a tablet, which can contain from 0.05% to 95% by weight of the active compounds. A compound of the invention may be coupled with suitable polymers as targetable drug carriers. Other pharmacologically active substances can also be present.

The compounds of the present invention may be administered by any suitable route, preferably in the form of a pharmaceutical composition adapted to such a route, and in a dose effective for the treatment intended. The active compounds and compositions, for example, may be administered orally, rectally, parenterally, or topically.

Oral administration of a solid dose form may be, for example, presented in discrete units, such as hard or soft capsules, pills, cachets, lozenges, or tablets, each containing a predetermined amount of at least one compound of the present invention. In another embodiment, the oral administration may be in a powder or granule form. In another embodiment, the oral dose form is sub-lingual, such as, for example, a lozenge. In such solid dosage forms, the compounds of the invention are ordinarily combined with one or more adjuvants. Such capsules or tablets may contain a controlled-release formulation. In the case of capsules, tablets, and pills, the dosage forms also may comprise buffering agents or may be prepared with enteric coatings.

In another embodiment, oral administration may be in a liquid dose form. Liquid dosage forms for oral administration include, for example, pharmaceutically acceptable emulsions, solutions, suspensions, syrups, and elixirs containing inert diluents commonly used in the art (i.e., water). Such compositions also may comprise adjuvants, such as wetting, emulsifying, suspending, flavoring (e.g., sweetening), and/or perfuming agents.

In another embodiment, the present invention comprises a parenteral dose form. "Parenteral administration" includes, for example, subcutaneous injections, intravenous injections, intraperitoneal injections, intramuscular injections, intrasternal injections, and infusion. Injectable preparations (i.e., sterile injectable aqueous or oleaginous suspensions) may be formulated according to the known art using suitable dispersing, wetting, and/or suspending agents.

In another embodiment, the present invention comprises a topical dose form. "Topical administration" includes, for example, transdermal administration, such as via transdermal patches or iontophoresis devices, intraocular administration, or intranasal or inhalation administration. Compositions for topical administration also include, for example, topical gels, sprays, ointments, and creams. A topical formulation may include a compound which enhances absorption or penetration of the active ingredient through the skin or other affected areas. When the compounds of this invention are administered by a transdermal device, administration will be accomplished using a patch either of the reservoir and porous membrane type or of a solid matrix variety. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, Finnin and Morgan, J. Pharm. Sci., 88 (10), 955-958 (1999).

Formulations suitable for topical administration to the eye include, for example, eye drops wherein the compound of this invention is dissolved or suspended in a suitable carrier. A typical formulation suitable for ocular or aural administration may be in the form of drops of a micronized suspension or solution in isotonic, pH-adjusted, sterile saline. Other formulations suitable for ocular and aural administration include ointments, biodegradable (e.g., absorbable gel sponges, collagen) and non-biodegradable (e.g., silicone) implants, wafers, lenses and particulate or vesicular systems, such as niosomes or liposomes. A polymer such as crossed-linked polyacrylic acid, polyvinyl alcohol, hyaluronic acid, a cellulosic polymer, for example, hydroxypropylmethyl cellulose, hydroxyethyl cellulose, or methyl cellulose, or a heteropolysaccharide polymer, for example, gelan gum, may be incorporated together with a preservative, such as benzalkonium chloride. Such formulations may also be delivered by iontophoresis.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant. Formulations suitable for intranasal administration are typically administered in the form of a dry powder (either alone; as a mixture, for example, in a dry blend with lactose; or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

In another embodiment, the present invention comprises a rectal dose form. Such rectal dose form may be in the form of, for example, a suppository. Cocoa butter is a traditional suppository base, but various alternatives may be used as appropriate.

Other carrier materials and modes of administration known in the pharmaceutical art may also be used. Pharmaceutical compositions of the invention may be prepared by any of the well-known techniques of pharmacy, such as effective formulation and administration procedures. The above considerations in regard to effective formulations and administration procedures are well known in the art and are described in standard textbooks. Formulation of drugs is discussed in, for example, Hoover, John E., Remington's Pharmaceutical Sciences, Mack Publishing Co., Easton, Pennsylvania, 1975; Liberman et al., Eds., Pharmaceutical Dosage Forms, Marcel Decker, New York, N.Y., 1980; and Kibbe et al., Eds., Handbook of Pharmaceutical Excipients (3rd Ed.), American Pharmaceutical Association, Washington, 1999.

The compounds of the present invention can be used, alone or in combination with other therapeutic agents, in the treatment of various conditions or disease states. The compound(s) of the present invention and other therapeutic agent(s) may be administered simultaneously (either in the same dosage form or in separate dosage forms) or sequentially. An exemplary therapeutic agent may be, for example, a metabotropic glutamate receptor agonist.

The administration of two or more compounds "in combination" means that the two compounds are administered closely enough in time that the presence of one alters the biological effects of the other. The two or more compounds may be administered simultaneously, concurrently or sequentially. Additionally, simultaneous administration may be carried out by mixing the compounds prior to administration or by administering the compounds at the same point in time but at different anatomic sites or using different routes of administration.

The phrases "concurrent administration," "co-administration," "simultaneous administration," and "administered simultaneously" mean that the compounds are administered in combination.

The present invention includes the use of a combination of a γ-secretase modulator compound as provided by the compounds of the invention and one or more additional pharmaceutically active agent(s). If a combination of active agents is administered, then they may be administered sequentially or simultaneously, in separate dosage forms or combined in a single dosage form. Accordingly, the present invention also includes pharmaceutical compositions comprising an amount of: (a) a first agent comprising a compound of the present invention or a pharmaceutically acceptable salt of the compound; (b) a second pharmaceutically active agent; and (c) a pharmaceutically acceptable carrier, vehicle or diluent.

Various pharmaceutically active agents may be selected for use in conjunction with the compounds of the present invention, depending on the disease, disorder, or condition to be treated. Pharmaceutically active agents that may be used in combination with the compositions of the present invention include, without limitation:
(i) acetylcholinesterase inhibitors, such as donepezil hydrochloride (ARICEPT, MEMAC), physostigmine salicylate (ANTILIRIUM), physostigmine sulfate (ESERINE), metrifonate, neostigmine, ganstigmine, pyridostigmine (MESTINON), ambenonium (MYTELASE), demarcarium, Debio 9902 (also known as ZT-1; Debiopharm), rivastigmine (EXELON), ladostigil, NP-0361, galantamine hydrobromide (RAZADYNE, RIMINYL, NIVALIN), tacrine (COGNEX), tolserine, velnacrine maleate, memoquin, huperzine A (HUP-A; NeuroHitech), phenserine, edrophonium (ENLON, TENSILON), and INM-176;
(ii) amyloid-β (or fragments thereof), such as Aβ₁₋₁₅ conjugated to pan HLA DR-binding epitope (PADRE), ACC-001 (Elan/Wyeth), ACI-01, ACI-24, AN-1792, Affitope AD-01, CAD106, and V-950;
(iii) antibodies to amyloid-β (or fragments thereof), such as ponezumab, solanezumab, bapineuzumab (also known as AAB-001), AAB-002 (Wyeth/Elan), ACI-01-Ab7, BAN-2401, intravenous Ig (GAMMAGARD), LY2062430 (humanized m266; Lilly), R1450 (Roche), ACU-5A5, huC091, and those disclosed in International Patent Publication Nos WO04/032868, WO05/025616, WO06/036291, WO06/069081, WO06/118959, in US Patent Publication Nos US2003/0073655, US2004/0192898, US2005/0048049, US2005/0019328, in European Patent Publication Nos EP0994728 and 1257584, and in US Patent No 5,750,349;
(iv) amyloid-lowering or -inhibiting agents (including those that reduce amyloid production, accumulation and fibrillization) such as dimebon, davunetide, eprodisate, leuprolide, SK-PC-B70M, celecoxib, lovastatin, anapsos, oxiracetam, pramiracetam, varenicline, nicergoline, colostrinin, bisnorcymserine (also known as BNC), NIC5-15 (Humanetics), E-2012 (Eisai), pioglitazone, clioquinol (also known as PBT1), PBT2 (Prana Biotechnology), flurbiprofen (ANSAID, FROBEN) and its R-enantiomer tarenflurbil (FLURIZAN), nitroflurbiprofen, fenoprofen (FENOPRON, NALFON), ibuprofen (ADVIL, MOTRIN, NUROFEN), ibuprofen lysinate, meclofenamic acid, meclofenamate sodium (MECLOMEN), indomethacin (INDOCIN), diclofenac sodium (VOLTAREN), diclofenac potassium, sulindac (CLINORIL), sulindac sulfide, diflunisal (DOLOBID), naproxen (NAPROSYN), naproxen sodium (ANAPROX, ALEVE), ARC031 (Archer Pharmaceuticals), CAD-106 (Cytos), LY450139 (Lilly), insulin-degrading enzyme (also known as insulysin), the gingko biloba extract EGb-761 (ROKAN, TEBONIN), tramiprosate (CEREBRIL, ALZHEMED), eprodisate (FIBRILLEX, KIACTA), compound W (3,5-bis(4-nitrophenoxy)benzoic acid), NGX-96992, neprilysin (also known as neutral endopeptidase (NEP)), scyllo-inositol (also known as scyllitol), atorvastatin (LIPITOR), simvastatin (ZOCOR), KLVFF-(EEX)3, SKF-74652, ibutamoren mesylate, BACE inhibitors such as ASP-1702, SCH-745966, JNJ-715754, AMG-0683, AZ-12304146, BMS-782450, GSK-188909, NB-533, E2609 and TTP-854; gamma secretase modulators such as ELND-007; and RAGE (receptor for advanced glycation end-products) inhibitors, such as TTP488 (Transtech) and TTP4000 (Transtech), and those disclosed in US Patent No 7,285,293, including PTI-777;
(v) alpha-adrenergic receptor agonists, such as guanfacine (INTUNIV, TENEX), clonidine (CATAPRES), metaraminol (ARAMINE), methyldopa (ALDOMET, DOPAMET, NOVOMEDOPA), tizanidine (ZANAFLEX), phenylephrine (also known as neosynephrine), methoxamine, cirazoline, guanfacine (INTUNIV), lofexidine, xylazine, modafinil (PROVIGIL), adrafinil, and armodafinil (NUVIGIL);
(vi) beta-adrenergic receptor blocking agents (beta blockers), such as carteolol, esmolol (BREVIBLOC), labetalol (NORMODYNE, TRANDATE), oxprenolol (LARACOR, TRASACOR), pindolol (VISKEN), propanolol (INDERAL), sotalol (BETAPACE, SOTALEX, SOTACOR), timolol (BLOCADREN, TIMOPTIC), acebutolol (SECTRAL, PRENT), nadolol (CORGARD), metoprolol tartrate (LOPRESSOR), metoprolol succinate (TOPROL-XL), atenolol (TENORMIN), butoxamine, and SR 59230A (Sanofi);
(vii) anticholinergics, such as amitriptyline (ELAVIL, ENDEP), butriptyline, benztropine mesylate (COGENTIN), trihexyphenidyl (ARTANE), diphenhydramine (BENADRYL), orphenadrine (NORFLEX), hyoscyamine, atropine (ATROPEN), scopolamine (TRANSDERM-SCOP), scopolamine methylbromide (PARMINE), dicycloverine (BENTYL, BYCLOMINE, DIBENT, DILOMINE), tolterodine (DETROL), oxybutynin (DITROPAN, LYRINEL XL, OXYTROL), penthienate bromide, propantheline (PRO-BANTHINE), cyclizine, imipramine hydrochloride (TOFRANIL), imipramine maleate (SURMONTIL), lofepramine, desipramine (NORPRAMIN), doxepin (SINEQUAN, ZONALON), trimipramine (SURMONTIL), and glycopyrrolate (ROBINUL);
(viii) anticonvulsants, such as carbamazepine (TEGRETOL, CARBATROL), oxcarbazepine (TRILEPTAL), phenytoin sodium (PHENYTEK), fosphenytoin (CEREBYX, PRODILANTIN), divalproex sodium (DEPAKOTE), gabapentin (NEURONTIN), pregabalin (LYRICA), topirimate (TOPAMAX), valproic acid (DEPAKENE), valproate sodium (DEPACON), 1-benzyl-5-bromouracil, progabide, beclamide, zonisamide (TRERIEF, EXCEGRAN), CP-465022, retigabine, talampanel, and primidone (MYSOLINE);
(ix) antipsychotics, such as lurasidone (LATUDA, also known as SM-13496; Dainippon Sumitomo), aripiprazole (ABILIFY), chlorpromazine (THORAZINE), haloperidol (HALDOL), iloperidone (FANAPTA), flupentixol decanoate (DEPIXOL, FLUANXOL), reserpine (SERPLAN), pimozide (ORAP), fluphenazine decanoate, fluphenazine hydrochloride, prochlorperazine (COMPRO), asenapine (SAPHRIS), loxapine (LOXITANE), molindone (MOBAN), perphenazine, thioridazine, thiothixine, trifluoperazine (STELAZINE), ramelteon, clozapine (CLOZARIL), norclozapine (ACP-104), risperidone (RISPERDAL), paliperidone (INVEGA), melperone, olanzapine (ZYPREXA), quetiapine (SEROQUEL), talnetant, amisulpride, ziprasidone (GEODON), blonanserin (LONASEN), and ACP-103 (Acadia Pharmaceuticals);
(x) calcium channel blockers such as lomerizine, ziconotide, nilvadipine (ESCOR, NIVADIL), diperdipine, amlodipine (NORVASC, ISTIN, AMLODIN), felodipine (PLENDIL), nicardipine (CARDENE), nifedipine (ADALAT, PROCARDIA), MEM 1003 and its parent compound nimodipine (NIMOTOP), nisoldipine (SULAR), nitrendipine, lacidipine (LACIPIL, MOTENS), lercanidipine (ZANIDIP), lifarizine, diltiazem (CARDIZEM), verapamil (CALAN, VERELAN), AR-R 18565 (AstraZeneca), and enecadin;
(xi) catechol O-methyltransferase (COMT) inhibitors, such as nitecapone, tolcapone (TASMAR), entacapone (COMTAN), and tropolone;
(xii) central nervous system stimulants, such as atomoxetine, reboxetine, yohimbine, caffeine, phenmetrazine, phendimetrazine, pemoline, fencamfamine (GLUCOENERGAN, REACTIVAN), fenethylline (CAPTAGON), pipradol (MERETRAN), deanol (also known as dimethylaminoethanol), methylphenidate (DAYTRANA), methylphenidate hydrochloride (RITALIN), dexmethylphenidate (FOCALIN), amphetamine (alone or in combination with other CNS stimulants, e.g. ADDERALL (amphetamine aspartate, amphetamine sulfate, dextroamphetamine saccharate, and dextroamphetamine sulfate)), dextroamphetamine sulfate (DEXEDRINE, DEXTROSTAT), methamphetamine (DESOXYN), lisdexamfetamine (VYVANSE), and benzphetamine (DIDREX);
(xiii) corticosteroids, such as prednisone (STERAPRED, DELTASONE), prednisolone (PRELONE), prednisolone acetate (OMNIPRED, PRED MILD, PRED FORTE), prednisolone sodum phosphate (ORAPRED ODT), methylprednisolone (MEDROL); methylprednisolone acetate (DEPO-MEDROL), and methylprednisolone sodium succinate (A-METHAPRED, SOLU-MEDROL);
(xiv) dopamine receptor agonists, such as apomorphine (APOKYN), bromocriptine (PARLODEL), cabergoline (DOSTINEX), dihydrexidine, dihydroergocryptine, fenoldopam (CORLOPAM), lisuride (DOPERGIN), terguride spergolide (PERMAX), piribedil (TRIVASTAL, TRASTAL), pramipexole (MIRAPEX), quinpirole, ropinirole (REQUIP), rotigotine (NEUPRO), SKF-82958 (GlaxoSmithKline), cariprazine, pardoprunox and sarizotan;
(xv) dopamine receptor antagonists, such as chlorpromazine, fluphenazine, haloperidol, loxapine, risperidone, thioridazine, thiothixene, trifluoperazine, tetrabenazine (NITOMAN, XENAZINE), 7-hydroxyamoxapine, droperidol (INAPSINE, DRIDOL, DROPLETAN), domperidone (MOTILIUM), L-741742, L-745870, raclopride, SB-277011A, SCH-23390, ecopipam, SKF-83566, and metoclopramide (REGLAN);
(xvi) dopamine reuptake inhibitors such as bupropion, safinamide, nomifensine maleate (MERITAL), vanoxerine (also known as GBR-12909) and its decanoate ester DBL-583, and amineptine;
(xvii) gamma-aminobutyric acid (GABA) receptor agonists, such as baclofen (LIORESAL, KEMSTRO), siclofen, pentobarbital (NEMBUTAL), progabide (GABRENE), and clomethiazole;
(xviii) histamine 3 (H3) antagonists such as ciproxifan, tiprolisant, S-38093, irdabisant, pitolisant, GSK-239512, GSK-207040, JNJ-5207852, JNJ-17216498, HPP-404, SAR-110894, trans-*N*-ethyl-3-fluoro-3-[3-fluoro-4-(pyrrolidin-1-ylmethyl)phenyl]cyclobutanecarboxamide (PF-3654746 and those disclosed in US Patent Publication Nos US2005-0043354, US2005-0267095, US2005-0256135, US2008-0096955, US2007-1079175, and US2008-0176925; International Patent Publication Nos WO2006/136924, WO2007/063385, WO2007/069053, WO2007/088450, WO2007/099423, WO2007/105053, WO2007/138431, and WO2007/088462; and US Patent No 7,115,600);
(xix) immunomodulators such as glatiramer acetate (also known as copolymer-1; COPAXONE), MBP-8298 (synthetic myelin basic protein peptide), dimethyl fumarate, fingolimod (also known as FTY720), roquinimex (LINOMIDE), laquinimod (also known as ABR-215062 and SAIK-MS), ABT-874 (human anti-IL-12 antibody; Abbott), rituximab (RITUXAN), alemtuzumab (CAMPATH), daclizumab (ZENAPAX), and natalizumab (TYSABRI);
(xx) immunosuppressants such as methotrexate (TREXALL, RHEUMATREX), mitoxantrone (NOVANTRONE), mycophenolate mofetil (CELLCEPT), mycophenolate sodium (MYFORTIC), azathioprine (AZASAN, IMURAN), mercaptopurine (PURI-NETHOL), cyclophosphamide (NEOSAR, CYTOXAN), chlorambucil (LEUKERAN), cladribine (LEUSTATIN, MYLINAX), alpha-fetoprotein, etanercept (ENBREL), and 4-(benzyloxy)-5-[(5-undecyl-2*H*-pyrrol-2-ylidene)methyl]-1*H*,1'*H*-2,2'-bipyrrole (also known as PNU-156804);
(xxi) interferons, including interferon beta-1a (AVONEX, REBIF) and interferon beta-1b (BETASERON, BETAFERON);
(xxii) levodopa (or its methyl or ethyl ester), alone or in combination with a DOPA decarboxylase inhibitor (e.g., carbidopa (SINEMET, CARBILEV, PARCOPA), benserazide (MADOPAR), α-methyldopa, monofluoromethyldopa, difluoromethyldopa, brocresine, or m-hydroxybenzylhydrazine);
(xxiii) *N*-methyl-D-aspartate (NMDA) receptor antagonists, such as memantine (NAMENDA, AXURA, EBIXA), amantadine (SYMMETREL), acamprosate (CAMPRAL), besonprodil, ketamine (KETALAR), delucemine, dexanabinol, dexefaroxan, dextromethorphan, dextrorphan, traxoprodil, CP-283097, himantane, idantadol, ipenoxazone, L-701252 (Merck), lancicemine, levorphanol (DROMORAN), LY-233536 and LY-235959 (both Lilly), methadone, (DOLOPHINE), neramexane, perzinfotel, phencyclidine, tianeptine (STABLON), dizocilpine (also known as MK-801), EAB-318 (Wyeth), ibogaine, voacangine, tiletamine, riluzole (RILUTEK), aptiganel (CERESOTAT), gavestinel, and remacimide;
(xxiv) monoamine oxidase (MAO) inhibitors, such as selegiline (EMSAM), selegiline hydrochloride (L-deprenyl, ELDEPRYL, ZELAPAR), desmethylselegiline, brofaromine, phenelzine (NARDIL), tranylcypromine (PARNATE), moclobemide (AURORIX, MANERIX), befloxatone, safinamide, isocarboxazid (MARPLAN), nialamide (NIAMID), rasagiline (AZILECT), iproniazid (MARSILID, IPROZID, IPRONID), CHF-3381 (Chiesi Farmaceutici), iproclozide, toloxatone (HUMORYL, PERENUM), bifemelane, desoxypeganine, harmine (also known as telepathine or banasterine), harmaline, linezolid (ZYVOX, ZYVOXID), and pargyline (EUDATIN, SUPIRDYL);
(xxv) muscarinic receptor (particularly M1 subtype) agonists, such as cevimeline, levetiracetam, bethanechol chloride (DUVOID, URECHOLINE), itameline, pilocarpine (SALAGEN), NGX267, arecoline, L-687306 (Merck), L-689660 (Merck), furtrethonium iodide (FURAMON, FURANOL), furtrethonium benzensulfonate, furtrethonium *p-*toluenesulfonate, McN-A-343, oxotremorine, sabcomeline, AC-90222 (Acadia Pharmaceuticals), and carbachol (CARBASTAT, MIOSTAT, CARBOPTIC);
(xxvi) neuroprotective drugs such as bosutinib, condoliase, airmoclomol, lamotrigine, perampanel, aniracetam, minaprime, 2,3,4,9-tetrahydro-1*H*-carbazol-3-one oxime, desmoteplase, anatibant, astaxanthin, neuropeptide NAP (e.g., AL-108 and AL-208; both Allon Therapeutics), neurostrol, perampenel, ispronicline, bis(4-β-D-glucopyranosyloxybenzyl)-2-β-D-glucopyranosyl-2-isobutyltartrate (also known as dactylorhin B or DHB), formobactin, xaliproden (XAPRILA), lactacystin, dimeboline hydrochloride (DIMEBON), disufenton (CEROVIVE), arundic acid (ONO-2506, PROGLIA, CEREACT), citicoline (also known as cytidine 5'-diphosphocholine), edaravone (RADICUT), AEOL-10113 and AEOL-10150 (both Aeolus Pharmaceuticals), AGY-94806 (also known as SA-450 and Msc-1), granulocyte-colony stimulating factor (also known as AX-200), BAY-38-7271 (also known as KN-387271; Bayer AG), ancrod (VIPRINEX, ARWIN), DP-b99 (D-Pharm Ltd), HF-0220 (17-β-hydroxyepiandrosterone; Newron Pharmaceuticals), HF-0420 (also known as oligotropin), pyridoxal 5'-phosphate (also known as MC-1), microplasmin, S-18986, piclozotan, NP031112, tacrolimus, L-seryl-L-methionyl-L-alanyl-L-lysyl-L-glutamyl-glycyl-L-valine, AC-184897 (Acadia Pharmaceuticals), ADNF-14 (National Institutes of Health), stilbazulenyl nitrone, SUN-N8075 (Daiichi Suntory Biomedical Research), and zonampanel;
(xxvii) nicotinic receptor agonists, such as epibatidine, bupropion, CP-601927, varenicline, ABT-089 (Abbott), ABT-594, AZD-0328 (AstraZeneca), EVP-6124, R3487 (also known as MEM3454; Roche/Memory Pharmaceuticals), R4996 (also known as MEM63908; Roche/Memory Pharmaceuticals), TC-4959 and TC-5619 (both Targacept), and RJR-2403;
(xxviii) norepinephrine (noradrenaline) reuptake inhibitors, such as atomoxetine (STRATTERA), doxepin (APONAL, ADAPIN, SINEQUAN), nortriptyline (AVENTYL, PAMELOR, NORTRILEN), amoxapine (ASENDIN, DEMOLOX, MOXIDIL), reboxetine (EDRONAX, VESTRA), viloxazine (VIVALAN), maprotiline (DEPRILEPT, LUDIOMIL, PSYMION), bupropion (WELLBUTRIN), and radaxafine;
(xxix) phosphodiesterase (PDE) inhibitors, including but not limited to, (a) PDE1 inhibitors (e.g., vinpocetine (CAVINTON, CERACTIN, INTELECTOL) and those disclosed in US Patent No 6,235,742), (b) PDE2 inhibitors (e.g., erythro-9-(2-hydroxy-3-nonyl)adenine (EHNA), BAY 60-7550, and those described in US Patent No. 6,174,884), (c) PDE3 inhibitors (e.g., anagrelide, cilostazol, milrinone, olprinone, parogrelil, and pimobendan), (d) PDE4 inhibitors (e.g., apremilast, ibudilast, roflumilast, rolipram, Ro 20-1724, ibudilast (KETAS), piclamilast (also known as RP73401), CDP840, cilomilast (ARIFLO), roflumilast, tofimilast, oglemilast (also known as GRC 3886), tetomilast (also known as OPC-6535), lirimifast, theophylline (UNIPHYL, THEOLAIR), arofylline (also known as LAS-31025), doxofylline, RPR-122818, or mesembrine), and (e) PDE5 inhibitors (e.g., sildenafil (VIAGRA, REVATIO), tadalafil (CIALIS), vardenafil (LEVITRA, VIVANZA), udenafil, avanafil, dipyridamole (PERSANTINE), E-4010, E-4021, E-8010, zaprinast, iodenafil, mirodenafil, DA-8159, and those disclosed in International Patent Applications WO2002/020521, WO2005/049616, WO2006/120552, WO2006/126081, WO2006/126082, WO2006/126083, and WO2007/122466), (f) PDE7 inhibitors; (g) PDE8 inhibitors; (h) PDE9 inhibitors (e.g., BAY 73-6691 (Bayer AG) and those disclosed in US Patent Publication Nos US2003/0195205, US2004/0220186, US2006/0111372, US2006/0106035, and USSN 12/118,062 (filed May 9, 2008)), (i) PDE10 inhibitors such as 2-({4-[1-methyl-4-(pyridin-4-yl)-1*H*-pyrazol-3-yl]phenoxy}methyl)quinoline (PF-2545920), and SCH-1518291, and (j) PDE11 inhibitors;
(xxx) quinolines, such as quinine (including its hydrochloride, dihydrochloride, sulfate, bisulfate and gluconate salts), chloroquine, sontoquine, hydroxychloroquine (PLAQUENIL), mefloquine (LARIAM), and amodiaquine (CAMOQUIN, FLAVOQUINE);
(xxxi) β-secretase inhibitors, such as ASP-1702, SCH-745966, JNJ-715754, AMG-0683, AZ-12304146, BMS-782450, GSK-188909, NB-533, LY-2886721, E-2609, HPP-854, (+)-phenserine tartrate (POSIPHEN), LSN-2434074 (also known as LY-2434074), KMI-574, SCH-745966, Ac-rER (*N*²-acetyl-D-arginyl-L-arginine), loxistatin (also known as E64d), and CA074Me;
(xxxii) γ-secretase inhibitors and modulators, such as BMS-708163 (Avagacest), WO20060430064 (Merck), DSP8658 (Dainippon), ITI-009, L-685458 (Merck), ELAN-G, ELAN-Z, 4-chloro-*N*-[2-ethyl-1(*S*)-(hydroxymethyl)butyl]benzenesulfonamide;
(xxxiii) serotonin (5-hydroxytryptamine) 1A (5-HT_{1A}) receptor antagonists, such as spiperone, *levo*-pindolol, BMY 7378, NAD-299, *S*(-)-UH-301, NAN 190, lecozotan;
(xxxiv) serotonin (5-hydroxytryptamine) 2C (5-HT_{2c}) receptor agonists, such as vabicaserin, and zicronapine;
(xxxv) serotonin (5-hydroxytryptamine) 4 (5-HT₄) receptor agonists, such as PRX-03140 (Epix);
(xxxvi) serotonin (5-hydroxytryptamine) 6 (5-HT₆) receptor antagonists, such as A-964324, AVI-101, AVN-211, mianserin (TORVOL, BOLVIDON, NORVAL), methiothepin (also known as metitepine), ritanserin, ALX-1161, ALX-1175, MS-245, LY-483518 (also known as SGS518; Lilly), MS-245, Ro 04-6790, Ro 43-68544, Ro 63-0563, Ro 65-7199, Ro 65-7674, SB-399885, SB-214111, SB-258510, SB-271046, SB-357134, SB-699929, SB-271046, SB-742457 (GlaxoSmithKline), Lu AE58054 (Lundbeck A/S), and PRX-07034 (Epix);
(xxxvii) serotonin (5-HT) reuptake inhibitors such as alaproclate, citalopram (CELEXA, CIPRAMIL), escitalopram (LEXAPRO, CIPRALEX), clomipramine (ANAFRANIL), duloxetine (CYMBALTA), femoxetine (MALEXIL), fenfluramine (PONDIMIN), norfenfluramine, fluoxetine (PROZAC), fluvoxamine (LUVOX), indalpine, milnacipran (IXEL), paroxetine (PAXIL, SEROXAT), sertraline (ZOLOFT, LUSTRAL), trazodone (DESYREL, MOLIPAXIN), venlafaxine (EFFEXOR), zimelidine (NORMUD, ZELMID), bicifadine, desvenlafaxine (PRISTIQ), brasofensine, vilazodone, cariprazine, neuralstem and tesofensine;
(xxxviii) trophic factors, such as nerve growth factor (NGF), basic fibroblast growth factor (bFGF; ERSOFERMIN), neurotrophin-3 (NT-3), cardiotrophin-1, brain-derived neurotrophic factor (BDNF), neublastin, meteorin, and glial-derived neurotrophic factor (GDNF), and agents that stimulate production of trophic factors, such as propentofylline, idebenone, PYM50028 (COGANE; Phytopharm), and AIT-082 (NEOTROFIN);
(xxxix) Glycine transporter-1 inhibitors such as paliflutine, ORG-25935, JNJ-17305600, and ORG-26041;
(xl) AMPA-type glutamate receptor modulators such as perampanel, mibampator, selurampanel, GSK-729327, and *N*-{(3*S*,4*S*)-4-[4-(5-cyanothiophen-2-yl)phenoxy] tetrahydrofuran-3-yl}propane-2-sulfonamide;
and the like.

Also provided are kits that are suitable for use in performing the methods of treatment described above. In one embodiment, the kit contains a first dosage form comprising one or more of the compounds of the present invention and a container for the dosage, in quantities sufficient to carry out the methods of the present invention.

In another embodiment, the kit of the present invention comprises one or more compounds of the invention.

The compounds of the present invention, or their pharmaceutically acceptable salts, may be prepared by the methods described below, together with synthetic methods known in the art of organic chemistry, or modifications and derivatizations that are familiar to those of ordinary skill in the art. The starting materials used herein are commercially available or may be prepared by routine methods known in the art [such as those methods disclosed in standard reference books such as the COMPENDIUM OF ORGANIC SYNTHETIC METHODS, Vol. I-XII (published by Wiley-Interscience)]. Preferred methods include, but are not limited to, those described below.

During any of the following synthetic sequences, it may be necessary and/or desirable to protect sensitive or reactive groups on any of the molecules concerned. This can be achieved by means of conventional protecting groups, such as those described in T. W. Greene, Protective Groups in Organic Chemistry, John Wiley & Sons, 1981; T. W. Greene and P. G. M. Wuts, Protective Groups in Organic Chemistry, John Wiley & Sons, 1991; and T. W. Greene and P. G. M. Wuts, Protective Groups in Organic Chemistry, John Wiley & Sons, 1999, which are hereby incorporated by reference.

Compounds of the present invention, or their pharmaceutically acceptable salts, can be prepared according to the reaction Schemes discussed herein below. Unless otherwise indicated, the substituents in the Schemes are defined as above. Isolation and purification of the products is accomplished by standard procedures, which are known to a chemist of ordinary skill.

It will be understood by one skilled in the art that the various symbols, superscripts and subscripts used in the schemes, methods and examples are used for convenience of representation and/or to reflect the order in which they are introduced in the schemes, and are not intended to necessarily correspond to the symbols, superscripts or subscripts in the appended claims. The schemes are representative of methods useful in synthesizing the compounds of the present invention. They are not to constrain the scope of the invention in any way.

### Schemes

When intermediates used to synthesize compounds of the present invention incorporate a basic center, their suitable acid addition salts may be employed in synthetic pathways. Such suitable addition salts include but are not limited to those derived from inorganic acids, such as hydrochloric, hydrobromic, hydrofluoric, hydroiodic, boric, fluoroboric, phosphoric, nitric, carbonic, and sulfuric acids, and organic acids such as acetic, benzenesulfonic, benzoic, ethanesulfonic, fumaric, lactic, maleic, methanesulfonic, trifluoromethanesulfonic, succinic, toluenesulfonic, and trifluoroacetic acids. Suitable organic acids generally include but are not limited to aliphatic, cycloaliphatic, aromatic, araliphatic, heterocyclic, carboxylic, and sulfonic classes of organic acids.

Specific examples of suitable organic acids include but are not limited to acetate, trifluoroacetate, formate, propionate, succinate, lactate, maleate, fumarate, benzoate, p-hydroxybenzoate, phenylacetate, mandelate, methanesulfonate, ethanesulfonate, benzenesulfonate, toluenesulfonate, adipate, butyrate, camphorate, cyclopentanepropionate, dodecylsulfate, heptanoate, hexanoate, nicotinate, 2-naphthalenesulfonate, oxalate, 3-phenylpropionate, pivalate, and undecanoate.

Furthermore, where intermediates used to prepare compounds of the invention carry an acidic moiety, suitable salts thereof may be employed for synthesis. Such salts include alkali metal salts, e.g., lithium, sodium or potassium salts; alkaline earth metal salts, e.g., calcium or magnesium salts; and salts formed with suitable organic ligands such as amines or quaternary ammonium cations. Organic salts of such acidic intermediates may be made from primary, secondary or tertiary amines such as methylamine, diethylamine, ethylenediamine or trimethylamine. Quaternary amines may be prepared by reaction of tertiary amines with agents such as lower alkyl (C₁-C₆) halides (e.g., methyl, ethyl, propyl, and butyl chlorides, bromides, and iodides), dialkyl sulfates (e.g., dimethyl, diethyl, dibutyl, and diamyl sulfates), arylalkyl halides (e.g., benzyl and phenethyl bromides), and others.

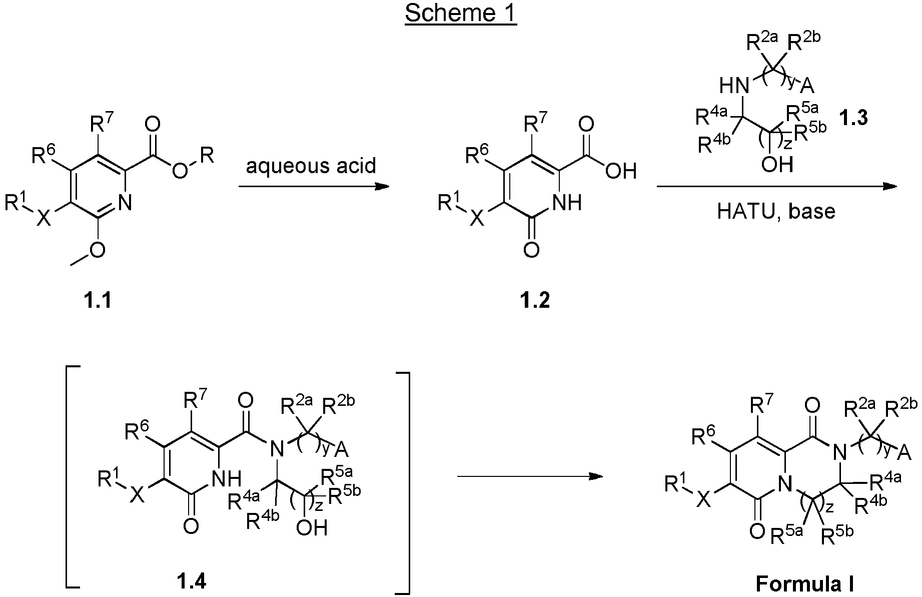

Scheme 1 above illustrates one synthetic sequence for the preparation of compounds depicted by Formula I. In the initial step of the synthesis, as depicted, an appropriate ester of a compound of Formula 1.1, wherein R, typically a (C₁-C₆)alkyl such as methyl, ethyl, *tert*-butyl and the like, is heated in the presence of an aqueous acid such as hydrochloric acid to furnish the corresponding pyridinone acid of Formula **1.2.** During this initial step, the R¹-X, R⁶ and R⁷ substituents of Formula **1.1** should be represented by the same moieties as are desired in the final product, or a protected variation thereof. For example, the final product of Example **1** can be prepared utilizing reaction Scheme 1, where R¹ is represented by methyl, X is represented by imidazolyl, and R⁶ and R⁷ of Formula **1.1** are each represented by hydrogen.

Next, the acid intermediate of Formula **1.2** is subjected to an amide coupling and *in situ* cyclization reaction with an amino alcohol of Formula **1.3** using an appropriate amide coupling reagent such as HATU [*O*-(7-azabenzotriazol-1-yl)-*N,N,N',N*'-tetramethyluronium hexafluorophosphate]. The reaction is carried out in the presence of a suitable base such as *N,N*-diisopropylethylamine, and in a solvent such as dichloromethane or *N,N*-dimethylformamide. During this step, y and z of Formula **1.3** should be represented by an integer as desired in the final product, and the A, R^{2a}, R^{2b}, R^{4a}, R^{4b}, R^{5a}, R^{5b} substituents should be represented by the same moieties as are desired in the final product, or a protected variation thereof. For example, the final product of Example **1** can be prepared utilizing reaction Scheme 1, where R^{2a}, R^{2b}, R^{4a}, R^{4b}, R^{5a}, and R^{5b} are each hydrogen, each of y and z are 1, and A represents 6-chloro-8-fluoro-2,2-dimethyl-2*H*-chromene-4-yl.

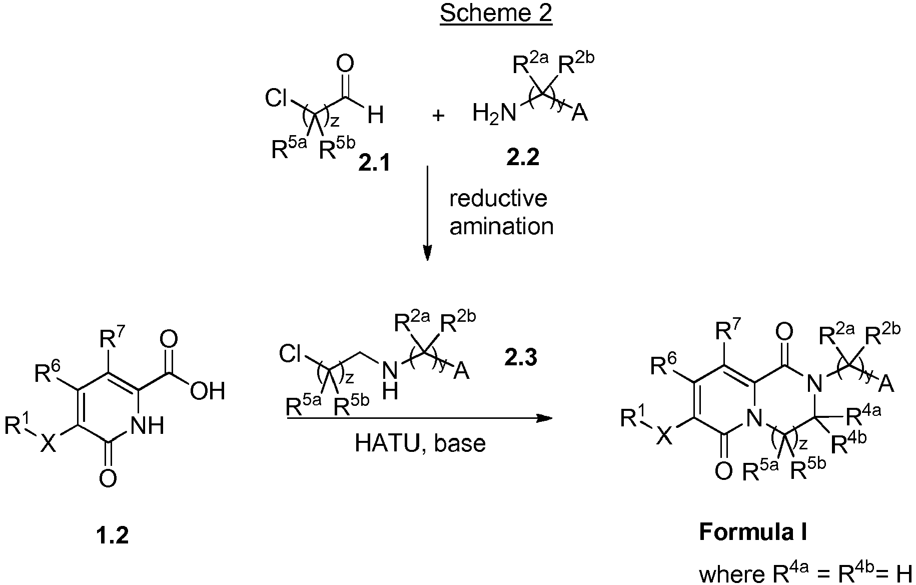

Scheme 2 above illustrates another synthetic sequence for the preparation of compounds of Formula **I.** Reaction of a chloroaldehyde of Formula **2.1** and an amine of Formula **2.2** using one of many reductive amination protocols known to those skilled in the art provides the chloroalkylamine of compound **2.3.** For example, this reaction may be carried out by using a reducing agent such as sodium triacetoxyborohydride in a suitable solvent such as methanol. During this step, z of the chloroaldehyde of Formula **2.1** and y of the amine of Formula **2.2** should be represented by an integer as desired in the final product. The R^{5a} and R^{5b} substituents of Formula **2.1** and the A, R^{2a}, and R^{2b} substituents of the amine of Formula **2.2** should also be represented by the same moieties as are desired in the final product, or a protected variation thereof.

Following purification, the resultant chloroalkylamine of Formula **2.3** may be isolated and stored as its hydrochloride salt. The final compound of Formula **I** may then be prepared by treating a mixture of the chloroalkylamine of Formula **2.3,** the acid of Formula **1.2** (Scheme 1), and a base such as *N,N*-diisopropylethylamine with a suitable amide coupling reagent such as BOP-CI [bis(2-oxo-3-oxazolidinyl)phosphonic chloride], T3P [2,4,6-tripropyl-1,3,5,2,4,6-trioxatriphosphinane 2,4,6-trioxide] or HATU (preferably HATU) in a solvent such as dichloromethane. During this step the R¹-X, R⁶ and R⁷ substituents of Formula **1.2** should be represented by the same moieties as are desired in the final product, or a protected variation thereof.

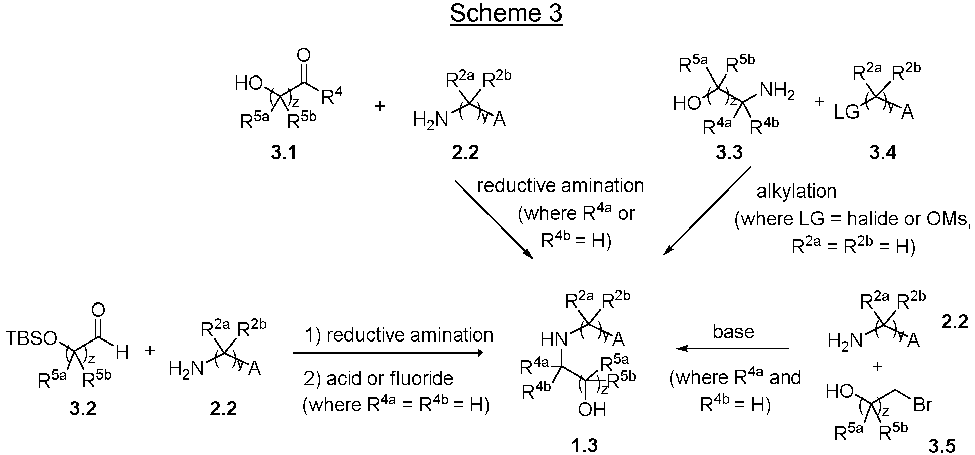

Scheme 3 above represents several synthetic sequences for the preparation of the aminoalcohol of Formula **1.3,** which can readily be envisioned and developed by one skilled in the art. For example, the aminoalcohol of Formula **1.3** may be prepared by carrying out a reductive amination of a ketone of Formula **3.1** with an amine of Formula **2.2** using one of many procedures well known to those skilled in the art.

Another method involves reductive amination of an aldehyde of Formula **3.2** with an amine of Formula **2.2,** followed by removal of the TBS protecting group by using a suitable procedure including treatment with methanolic hydrogen chloride or tetrabutylammonium fluoride.

Another method for the synthesis of an aminoalcohol of Formula **1.3** involves alkylation of an amine of Formula **3.3** with a halide or mesylate of Formula **3.4.**

Yet another method involves alkylation of an amine of Formula **2.2** with a bromoalcohol of Formula **3.5.** Methods of synthesis for various amines of Formula **2.2,** as well as alternative methods of preparation of aminoalcohols of Formula **1.3,** are exemplified in the Experimental Section.

A person skilled in the art, utilizing these disclosures in combination with what is commonly known in the art, may further generalize those syntheses to allow access to a wide variety of amines of Formula **2.2** and aminoalcohols of Formula **1.3,** including but not limited to variations in which y and z are represented by an integer as desired in the final product, and the A, R^{2a}, R^{2b}, R^{4a}, R^{4b}, R^{5a}, and R^{5b} substituents are represented by the same moieties as are desired in the final product, or a protected variation thereof.

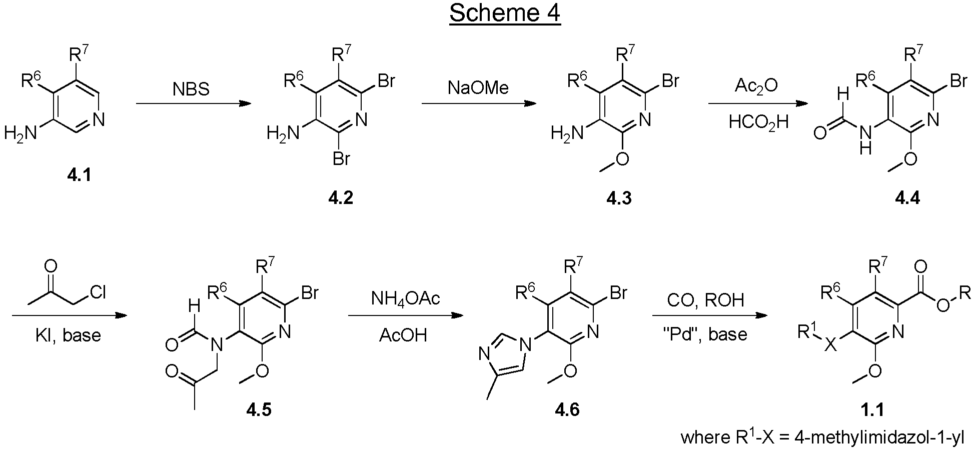

Scheme 4 illustrates one synthetic sequence for the preparation of compounds of Formula **1.1** where R¹-X = 4-methylimidazol-1-yl. A 3-aminopyridine compound of Formula **4.1** is brominated using *N*-bromosuccinimide (NBS) in a solvent such as a mixture of dimethyl sulfoxide (DMSO) and water. During this initial step the R⁶ and R⁷ substituents are represented by the same moieties as are desired in the final product, or a protected variation thereof. The resulting intermediate of Formula **4.2** is then heated with sodium methoxide in a suitable solvent such as 1,4-dioxane to afford the methoxy compound of Formula **4.3.** The intermediate of Formula **4.3** is then treated with a mixture of acetic anhydride and formic acid to afford a formamide of Formula **4.4,** which is alkylated with chloroacetone in the presence of potassium iodide and a base such as cesium carbonate in a suitable solvent such as *N,N*-dimethylformamide. The resulting intermediate of Formula **4.5** is then heated in the presence of NH₄OAc in acetic acid to furnish the imidazole derivative of Formula **4.6.** Finally, the compound of Formula **1.1** can be prepared by subjecting the intermediate of Formula **4.6** to a carbonylation/esterification reaction. This transformation may be carried out by heating a solution of the bromo compound of Formula **4.6** and a base such as triethylamine in an appropriate alcohol solvent ("ROH"), wherein R is typically a (C₁-C₆)alkyl such as methanol or ethanol, under an atmosphere of CO in the presence of a suitable palladium catalyst such as Pd(dppf)Cl₂·dichloromethane {[1,1'-bis(diphenylphosphino)ferrocene] dichloropalladium(II), dichloromethane complex} to provide the ester of Formula **1.1.** 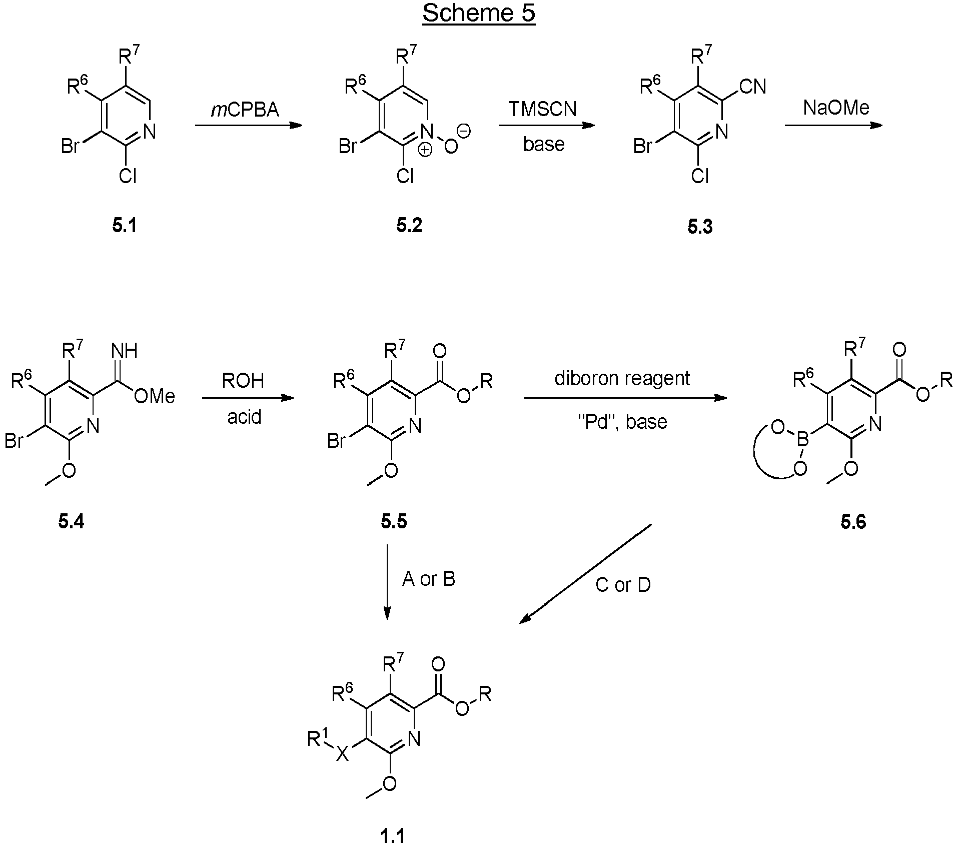
A) Suzuki coupling: R¹X-B(OH)₂, "Pd", base
B) CH-activation: "Pd", 5-membered heteroaryls such as 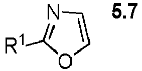 provide compounds of Formula **1.1** wherein R¹-X- is 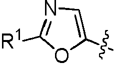
C) Chan-Lam coupling: Cu₂O or Cu(OAc)₂, 5-membered heteroaryls such as provide compounds of Formula **1.1** wherein R¹-X- is 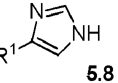 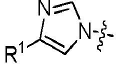
D) Suzuki coupling: R¹X-Br, "Pd", base
where X = a 5- to 6-membered heteroaryl ring

Scheme 5 above depicts alternative synthetic sequences for the preparation of compounds of Formula **1.1.** In a first step, a pyridyl derivative of Formula **5.1** is oxidized with an oxidizing agent such as *m*CPBA [3-chloroperoxybenzoic acid] in a suitable solvent such as dichloroethane to afford the corresponding *N*-oxide of Formula **5.2.** During this initial step the R⁶ and R⁷ substituents of Formula **5.1** are represented by the same moieties as are desired in the final product, or a protected variation thereof. The *N*-oxide of Formula **5.2** is then heated in the presence of TMSCN [trimethylsilyl cyanide] and a base such as triethylamine in a solvent such as acetonitrile to afford the nitrile intermediate of Formula **5.3.** The corresponding ester may then be prepared from Formula **5.3** in two steps by subjecting Formula **5.3** to sodium methoxide in a solvent such as THF, followed by treatment with an appropriate alcohol solvent ("ROH"), wherein R is typically a (C₁-C₆)alkyl such as methyl, ethyl and the like, and an acid such as hydrochloric acid. The ester of Formula **5.5** is a versatile intermediate that allows introduction of a variety of heterocycles R¹-X. For example, Formula **5.5** may be subjected to a Suzuki coupling with a heteroarylboronic acid, using methods well known to those skilled in the art [see Tetrahedron 2002, 58, 9633-9695]. Alternatively, the compound of Formula **5.5** may be coupled to a heterocycle X using a direct arylation approach [see D. Lapointe et al., J. Org. Chem. 2011, 76, 749-759, and references therein]. For example, **5.5** may be coupled to 2-methyl-1,3-oxazole [Formula **5.7** where R¹ = Me] by heating in the presence of a suitable palladium catalyst such as allylpalladium chloride dimer and a base such as potassium carbonate in a solvent such as 1,4-dioxane, to afford the intermediate of Formula **1.1** where R¹-X = 2-methyl-1,3-oxazol-5-yl.

Alternatively, the compound of Formula **5.5** may be converted to the corresponding boronate of Formula **5.6,** using a palladium-catalyzed cross coupling with a diboron reagent such as 5,5,5',5'-tetramethyl-2,2'-bi-1,3,2-dioxaborinane in the presence of potassium acetate and a palladium catalyst such as Pd(dppf)Cl₂·dichloromethane in a solvent such as 1,4-dioxane. The resulting boronate intermediate of Formula **5.6** can in turn be subjected to a Suzuki coupling with a heteroaryl halide to afford the final compound of Formula **1.1.** Another method for the introduction of a heterocycle X involves the use of a Chan-Lam coupling [see Tetrahedron Lett. 2003, 44, 3863-3865, and Synthesis 2008, 5, 795-799]. For example, the boronate of Formula **5.6** may be coupled to a substituted imidazole of Formula **5.8** by heating with a suitable copper source such as copper(I) oxide or copper(II) acetate in a solvent such as methanol in the presence of air to afford the intermediate of Formula **1.1** where X = imidazol-1-yl. 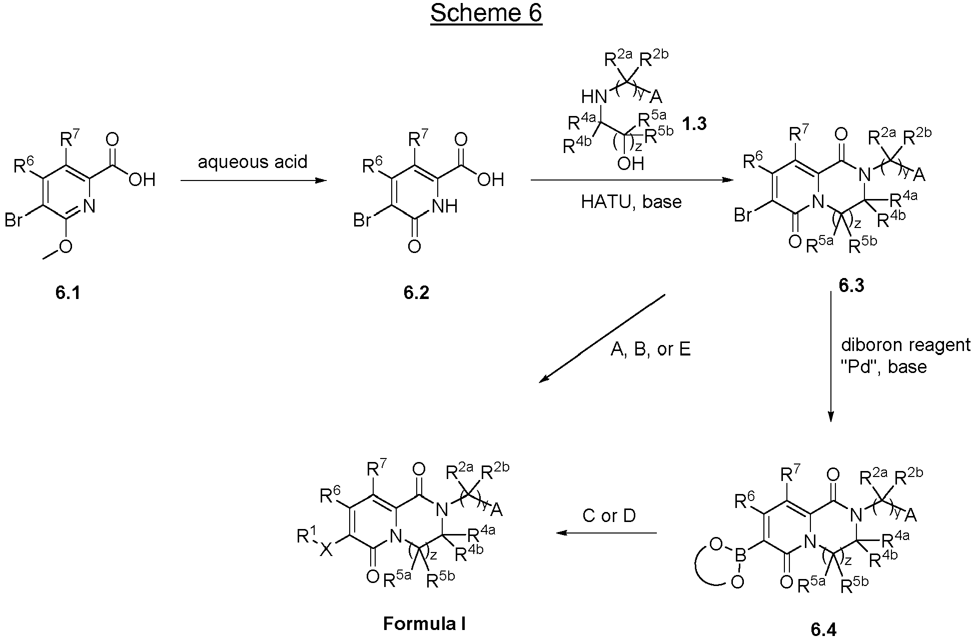
A) Suzuki coupling: R¹X-B(OH)₂, "Pd", base
B) CH-activation: "Pd", 5-membered heteroaryls such as 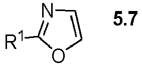 provide compounds of Formula I wherein R¹-X- is 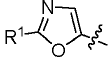
C) Chan-Lam coupling: Cu₂O or Cu(OAc)₂, 5-membered heteroaryls such as 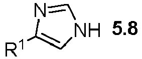 provide compounds of Formula **I**wherein R¹-X- is 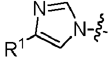
D) Suzuki coupling: R¹X-Br, "Pd", base where X = a 5- to 6-membered heteroaryl ring
E) Base, and a heteroaryl such as 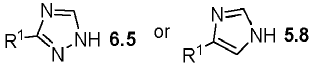 provide compounds of Formula I wherein R¹-X- is

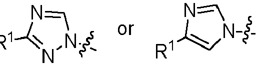

Scheme 6 above illustrates yet another set of synthetic sequences for the preparation of compounds of Formula **I.** The initial step commences by heating the compound of Formula **6.1** in an acid such as hydrochloric acid to afford the pyridinone acid intermediate of Formula **6.2.** During this initial step, the R⁶ and R⁷ substituents of Formula **6.1** are represented by the same moieties as are desired in the final product, or a protected variation thereof. Next, the acid of Formula **6.2** may be subjected to a coupling/cyclization reaction with an aminoalcohol of Formula **1.3** (Scheme 1) to afford an intermediate of Formula **6.3** using chemistry described in Scheme 1. During this step, y and z of Formula **1.3** should be represented by an integer as desired in the final product, and the A, R^{2a}, R^{2b}, R^{4a}, R^{4b}, R^{5a}, and R^{5b} substituents should be represented by the same moieties as are desired in the final product, or a protected variation thereof.

The final compound, Formula **I,** may then be formed directly from Formula **6.3** or via the boronate of Formula **6.4,** using the strategies discussed in Scheme 5. Alternatively, compounds of Formula I where heterocycle X is linked to the pyridinone ring via a C-N bond may be formed by nucleophilic aromatic substitution. For example, the triazole of Formula **6.5** may be coupled to Formula **6.3** by heating in the presence of a base such as potassium carbonate and a solvent such as DMSO to afford the final compound of Formula I where X = triazol-1-yl.

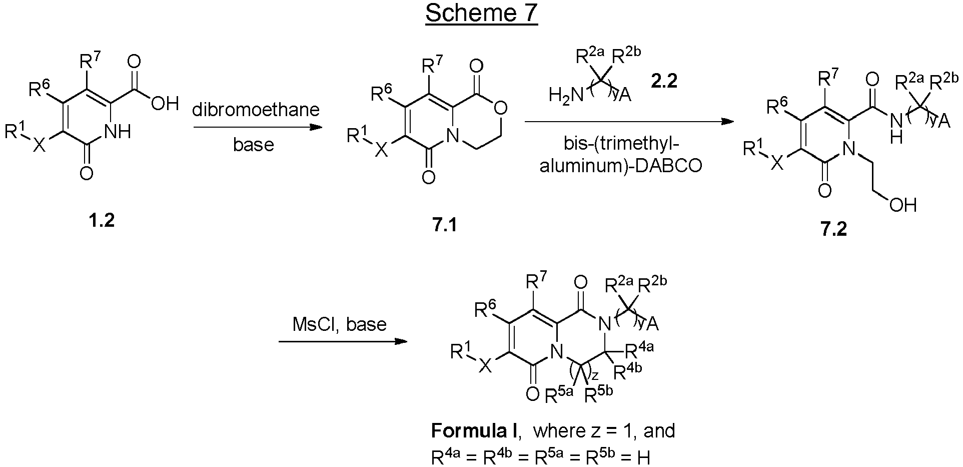

Scheme 7 illustrates another synthetic sequence for the preparation of compounds of Formula **I,** where z = 1 and R^{4a} = R^{4b} = R^{5a} = R^{5b} = H. The method involves heating a mixture of a compound of Formula **1.2** (Scheme 1), dibromoethane, and a base such as cesium carbonate in a solvent such as *N,N*-dimethylformamide to afford the lactone intermediate of Formula **7.1.** During this initial step, the R⁶ and R⁷ substituents of Formula **1.2** are represented by the same moieties as are desired in the final product, or a protected variation thereof. The lactone of Formula **7.1** may then be reacted with an amine of Formula **2.2** (Scheme 2) in the presence of a reagent such as DIBAL (diisobutylaluminum hydride) or bis(trimethylaluminum)-1,4-diazabicyclo[2.2.2]octane adduct in a solvent such as THF to afford the amide alcohol of Formula **7.2**. During this step, y of Formula **2.2** (Scheme 2) should be represented by an integer as desired in the final product, and the A, R^{2a}, and R^{2b} substituents should be represented by the same moieties as are desired in the final product, or a protected variation thereof. This intermediate, in turn, may be reacted with methanesulfonyl chloride in the presence of a base such as triethylamine in a solvent such as THF, followed by treatment with a base such as 1,3,4,6,7,8-hexahydro-2*H*-pyrimido[1,2-*a*]pyrimidine (TBD) to afford the compound of Formula **I** wherein z = 1 and R^{4a} = R^{4b} = R^{5a} = R^{5b} = H. Alternatively, the ring closure may be carried out in a stepwise fashion by first converting the alcohol of Formula **7.2** into the corresponding chloride by treatment with thionyl chloride, followed by deprotonation of the amide NH with a suitable base such as lithium bis(trimethylsilyl)amide to afford the final compound of Formula **I**. Alternatively, a solution of lactam **7.1** and amine **2.2** in *N*,*N*-dimethylformamide may be treated with 1,3,4,6,7,8-hexahydro-2*H*-pyrimido[1,2-*a*]pyrimidine in *N,N-*dimethylformamide to form intermediate **7.2**, which is then directly converted to Formula **I** by addition of ethyl trifluoroacetate.

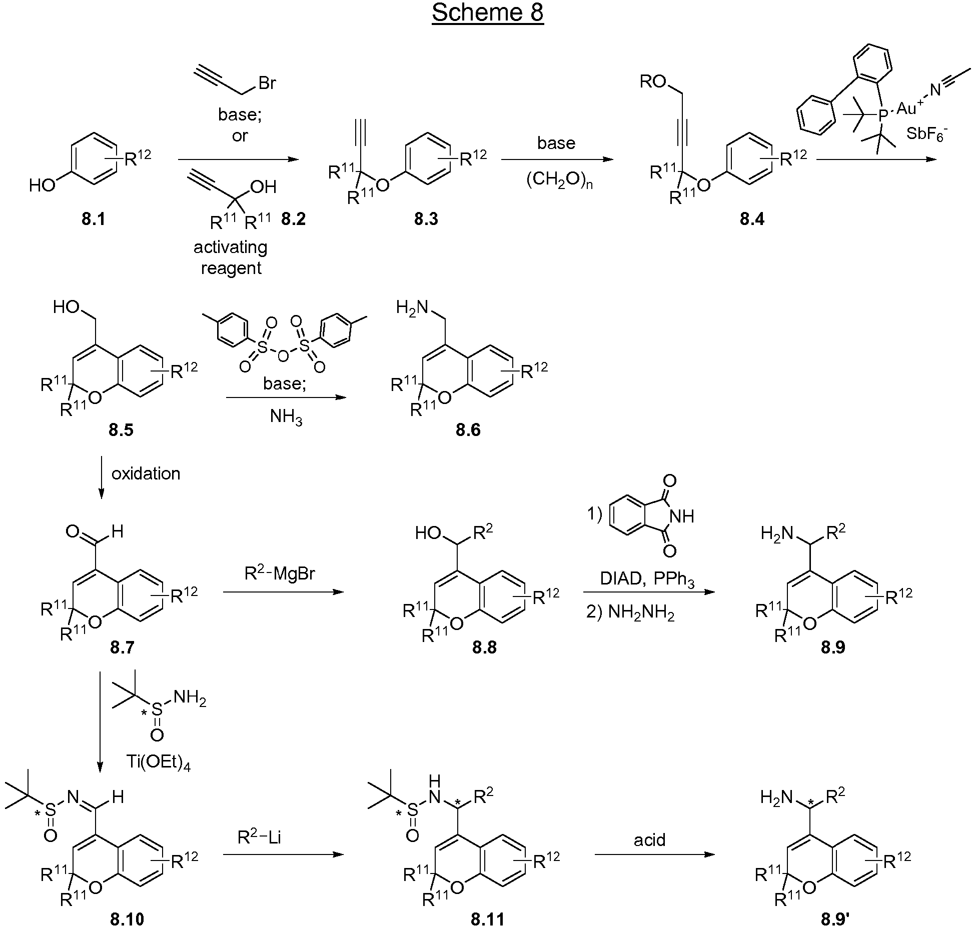

Various methods for preparation of amine coupling partners of the general Formula **2.2** (Scheme 2) can be readily envisioned by those skilled in the art. A number of synthetic methods are described in the Experimental Section that may be generalized to provide many analogous coupling partners of the general Formula **2.2**. Scheme 8 highlights a subset of possible synthetic approaches to the specific amine coupling partners **8.6**, **8.9**, and **8.9'**, which incorporate a chromene ring system. The syntheses commence with alkylation of a suitably substituted phenol of Formula **8.1**. In the case where both R¹¹ substituents are hydrogen atoms, the alkylation can be carried out with 3-bromoprop-1-yne in a suitable solvent such as *N*,*N*-dimethylformamide and a base such as cesium carbonate to afford a propargyl ether of general Formula **8.3**. Alternatively, installation of the propargyl unit may be accomplished via a Mitsunobu reaction with but-2-yne-1,4-diol to directly generate the propargyl alcohol of Formula **8.4** where R = H. In other instances, when the R¹¹ substituents are not hydrogen, phenol **8.1** may be alkylated with a suitably substituted propargyl alcohol derivative **8.2** in the presence of activating reagents such as trifluoroacetic anhydride, copper(II) chloride and a base such as 1,8-diazabicyclo[5.4.0]undec-7-ene (DBU) in a suitable solvent such as acetonitrile. The resultant propargyl ether of general Formal **8.3** may then be treated with a base such as *n*-butyllithium in a solvent such as THF followed by alkylation with paraformaldehyde to afford **8.4** where R = H. Alternatively, the reaction mixture may subsequently be treated with acetyl chloride to afford **8.4** where R = acetate. The propargyl ether intermediate of general Formula **8.4** may then undergo cyclization to afford the chromene intermediate of Formula **8.5**. This transformation may be carried out using a variety of conditions including but not limited to those exemplified in the Experimental Section. For example, propargyl ether derivative **8.4** may be treated with a catalyst such as (acetonitrile)[(2-biphenyl)di-*tert*-butylphosphine]gold(I) hexafluoroantimonate in a solvent such as 1,2-dichloroethane or dichloromethane. In the case where R = acetate, this protecting group is then readily cleaved by addition of methanol and a base such as potassium carbonate to afford chromene derivative **8.5**. Conversion of the primary alcohol of **8.5** to the amine **8.6** can be accomplished by a variety of methods known to those skilled in the art. For example, **8.5** may be treated with p-toluenesulfonic anhydride in the presence of a base such as triethylamine in a solvent such as dichloromethane to afford the corresponding *p*-toluenesulfonate derivative, which in turn is treated with a solution of ammonia in methanol to afford the amine of Formula **8.6**. Alternatively, alcohol **8.5** may be reacted with the ylide derived from carbon tetrabromide and triphenylphosphine in a solvent such as dichloromethane, followed by treatment with methanolic ammonia, to afford amine **8.6**.

The chromane alcohol derivative of Formula **8.5** may be oxidized to the corresponding aldehyde **8.7** using one of several oxidation conditions. For example, **8.5** may be treated with Dess-Martin periodinane in a solvent such as dichloromethane. The resulting aldehyde of Formula **8.7** is then treated with a suitably substituted organometallic reagent to install substituent R². For example, aldehyde **8.7** may be treated with a Grignard reagent such as methylmagnesium bromide in a solvent such as tetrahydrofuran to afford alcohol derivative **8.8** where R² = methyl. Conversion of the alcohol functionality in **8.8** to the amine **8.9** may be carried out using one of several methods known to those skilled in the art. For example, a Mitsunobu reaction of **8.8** with phthalimide using triphenylphosphine (PPh₃) and diisopropyl azodicarboxylate (DIAD) in a solvent such as THF is then followed by treatment with hydrazine in a solvent such as ethanol to afford the racemic amine of Formula **8.9**. The enantiomers of amine **8.9** may be separated using chiral preparative HPLC. Alternatively, the enantiomers can be separated following conversion to Formula **I**.

Preparation of single enantiomer **8.9'** can also be accomplished via intermediates of Formula **8.10** and **8.11**. Aldehyde **8.7** can be converted to sulfinamide **8.10** by treatment with a single enantiomer of 2-methyl-2-propanesulfinamide and a Lewis acid such as titanium(IV) ethoxide in a solvent such as THF. The resulting sulfinamide of Formula **8.10** is then treated with a suitably substituted organometallic reagent such as methyllithium in a solvent such as THF to install the R² substituent and afford **8.11**. Finally, the sulfinamide chiral auxiliary of **8.11** may be removed by exposure to hydrochloric acid in a solvent such as 1,4-dioxane to afford the enantioenriched amine of Formula **8.9'**. The enantiomeric excess may be enhanced by separation using chiral preparative HPLC. During any of the steps described above, R¹¹ and R¹² should be represented by the same moieties as are desired in the final product, or a protected variation thereof. For example, the final product of Example **1** can be prepared utilizing reaction Scheme 1, where each R¹¹ is a methyl group and R¹² represents two substituents, one of which is chloro and the other of which is fluoro.

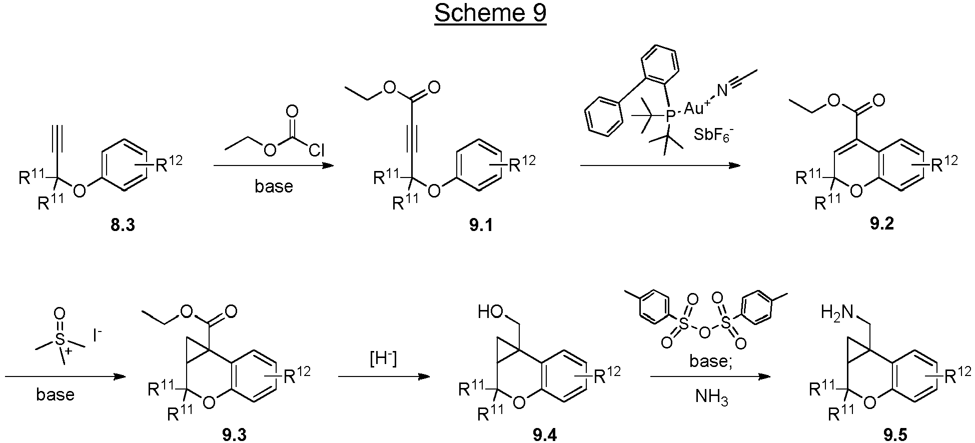

Multiple methods for preparation of amine coupling partners of the general Formula **2.2** (Scheme 2) can be envisioned by those skilled in the art. Scheme 9 depicts a synthetic approach to an amine intermediate of Formula **9.5**. Starting with the propargyl ether intermediate of Formula **8.3**, deprotonation with a base such as *n-*butyllithium in a solvent such as THF, followed by acylation with ethyl chloroformate, affords an alkynoate of Formula **9.1**. During this initial step, R¹¹ and R¹² should be represented by the same moieties as are desired in the final product, or a protected variation thereof. The intermediate of Formula **9.1** may undergo cyclization by exposure to (acetonitrile)[(2-biphenyl)di-*tert*-butylphosphine]gold(I) hexafluoroantimonate in a solvent such as 1,2-dichloroethane or dichloromethane. The resultant chromene intermediate of Formula **9.2** is then subjected to cyclopropanation by exposure to trimethylsulfoxonium iodide and a base such as potassium *tert*-butoxide in a suitable solvent such as THF to afford cyclopropyl chromane ester derivative **9.3.** Reduction of **9.3** to the corresponding alcohol can be carried out using a suitable reducing agent such as diisobutylaluminum hydride in a solvent such as THF. An alternative procedure for preparation of cyclopropyl alcohol intermediate **9.4** is to subject the chromene alcohol intermediate of Formula **8.5** to cyclopropanation using diiodomethane and diethylzinc in a solvent such as dichloromethane. Finally, conversion of the alcohol **9.4** to amine **9.5** may can be accomplished by a variety of methods known to those skilled in the art. For example, **9.4** may be treated with *p*-toluenesulfonic anhydride in the presence of a base such as triethylamine in a solvent such as dichloromethane to afford the corresponding *p*-toluenesulfonate derivative, which in turn is treated with a solution of ammonia in methanol to afford the amine of Formula **9.5**. Alternatively, alcohol **9.4** may be reacted with the ylide derived from carbon tetrabromide and triphenylphosphine in a solvent such as dichloromethane, followed by treatment with methanolic ammonia to afford amine **9.5**. The enantiomers of amine **9.4** may be separated using chiral preparative HPLC. Alternatively, the enantiomers can be separated following conversion to Formula **I**.

### Experimental Procedures and Working Examples

The following illustrate the synthesis of various compounds of the present invention. Additional compounds within the scope of this invention may be prepared using the methods illustrated in these Examples, either alone or in combination with techniques generally known in the art.

Experiments were generally carried out under inert atmosphere (nitrogen or argon), particularly in cases where oxygen- or moisture-sensitive reagents or intermediates were employed. Commercial solvents and reagents were generally used without further purification. Anhydrous solvents were employed where appropriate, generally AcroSeal® products from Acros Organics or DriSolv® products from EMD Chemicals. Products were generally dried under vacuum before being carried on to further reactions or submitted for biological testing. Mass spectrometry data is reported from either liquid chromatography-mass spectrometry (LCMS), atmospheric pressure chemical ionization (APCI) or gas chromatography-mass spectrometry (GCMS) instrumentation. Chemical shifts for nuclear magnetic resonance (NMR) data are expressed in parts per million (ppm, δ) referenced to residual peaks from the deuterated solvents employed. In some examples, chiral separations were carried out to separate enantiomers of certain compounds of the invention. In some examples, the optical rotation of an enantiomer was measured using a polarimeter. According to its observed rotation data (or its specific rotation data), an enantiomer with a clockwise rotation was designated as the (+)-enantiomer and an enantiomer with a counter-clockwise rotation was designated as the (-)-enantiomer.

Reactions proceeding through detectable intermediates were generally followed by LCMS, and allowed to proceed to full conversion prior to addition of subsequent reagents. For syntheses referencing procedures in other Examples or Methods, reaction conditions (reaction time and temperature) may vary. In general, reactions were followed by thin layer chromatography or mass spectrometry, and subjected to work-up when appropriate. Purifications may vary between experiments: in general, solvents and the solvent ratios used for eluents/gradients were chosen to provide appropriate R_{f}s or retention times.

### Example 1

### 2-[(6-Chloro-8-fluoro-2,2-dimethyl-2H-chromen-4-yl)methyl]-7-(4-methyl-1H-imidazol-1-yl)-3,4-dihydro-2H-pyrido[1,2-a]pyrazine-1,6-dione (1)

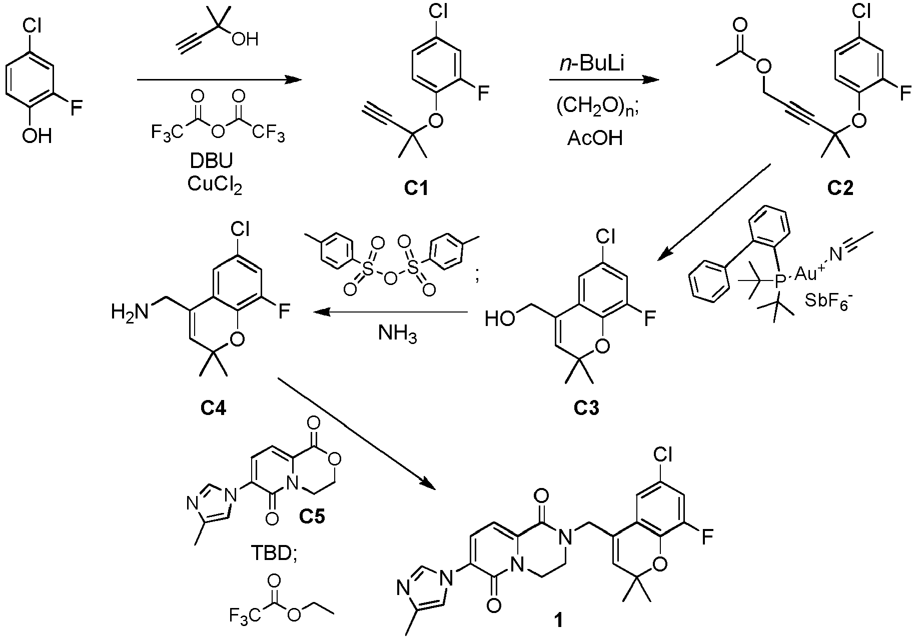

### Step 1. Synthesis of 4-chloro-2-fluoro-1-[(2-methylbut-3-yn-2-yl)oxy]benzene (C1).

A solution of 2-methylbut-3-yn-2-ol (5.0 mL, 51.6 mmol) and 1,8-diazabicyclo[5.4.0]undec-7-ene (DBU, 97%, 10.5 mL, 68.1 mmol) in acetonitrile (24 mL) was cooled to -5 °C to -10 °C in an ice-salt bath. Trifluoroacetic anhydride (7.3 mL, 51.4 mmol) was added drop-wise over 30 minutes, at a rate that maintained the internal reaction temperature <1 °C over the course of the addition. This solution was kept at 0 °C for 30 minutes. In a separate flask, a solution of 4-chloro-2-fluorophenol (3.6 mL, 34 mmol) in acetonitrile (24 mL) was cooled to -5 °C to -10 °C in an ice-salt bath. 1,8-Diazabicyclo[5.4.0]undec-7-ene (97%, 9.3 mL, 60.3 mmol) and copper(II) chloride (23 mg, 0.17 mmol) were added. The solution containing the 2-methylbut-3-yn-2-ol was then added drop-wise via cannula over 15 minutes, while keeping the reaction mixture at -5 °C. After being stirred at 0 °C for 3 hours, the reaction mixture was allowed to warm to room temperature, whereupon it was concentrated *in vacuo* to remove most of the acetonitrile. The residue was poured onto a pad of silica gel atop a thin layer of diatomaceous earth and eluted with heptane (250 mL). Concentration of the filtrate under reduced pressure afforded the product; as this material was slightly volatile, it was not placed on a vacuum line. By ¹H NMR analysis, the product contained 16% heptane by weight. Yield: 6.24 g, 24.6 mmol (corrected for heptane), 72%. ¹H NMR (400 MHz, CDCl₃) δ 7.39 (dd, *J*=8.7, 8.7 Hz, 1H), 7.12 (dd, *J*=10.1, 2.5 Hz, 1H), 7.03-7.07 (m, 1H), 2.56 (s, 1H), 1.66 (s, 6H).

### Step 2. Synthesis of 4-(4-chloro-2-fluorophenoxy)-4-methylpent-2-yn-1-yl acetate (C2).

*n*-Butyllithium (2.5 M solution in hexanes, 7.11 mL, 17.8 mmol) was added drop-wise over 5 minutes to a -78 °C solution of **C1** (3.0 g, 12 mmol, corrected for 16% heptane contaminant by weight) in tetrahydrofuran (60 mL), and the reaction mixture was allowed to stir for 15 minutes at -78 °C. Paraformaldehyde (0.534 g, 17.8 mmol) was added, and the reaction mixture was allowed to warm to room temperature. After 30 minutes, it was cooled to -78 °C, treated with acetyl chloride (1.28 mL, 17.7 mmol), stirred for 10 minutes, and then allowed to warm to room temperature over 2 hours. The reaction was quenched with aqueous ammonium chloride solution and extracted three times with ethyl acetate. The combined organic layers were washed with brine, dried over sodium sulfate, filtered, and concentrated *in vacuo.* Silica gel chromatography (Gradient: 0% to 50% ethyl acetate in heptane) provided the product as a yellow oil. Yield: 800 mg, 2.81 mmol, 23%. ¹H NMR (400 MHz, CDCl₃) δ 7.34 (dd, *J*=8.7, 8.6 Hz, 1H), 7.12 (dd, *J*=10.1, 2.5 Hz, 1H), 7.03-7.08 (m, 1H), 4.68 (s, 2H), 2.10 (s, 3H), 1.64 (s, 6H).

### Step 3. Synthesis of (6-chloro-8-fluoro-2,2-dimethyl-2H-chromen-4-yl)methanol (C3).

(Acetonitrile)[(2-biphenyl)di-*tert*-butylphosphine]gold(I) hexafluoroantimonate (21.6 mg, 28.0 µmol) was added to a solution of **C2** (800 mg, 2.81 mmol) in 1,2-dichloroethane (4.7 mL), and the reaction mixture was stirred at room temperature for 5 days. Additional (acetonitrile)[(2-biphenyl)di-*tert*-butylphosphine]gold(I) hexafluoroantimonate (21.6 mg, 28.0 µmol) was added, and the reaction mixture was heated at 50 °C for 18 hours. After it had cooled to room temperature, the reaction mixture was diluted with methanol (5 volumes) and treated with potassium carbonate (5 equivalents). After 1 hour, water was added, and the mixture was extracted three times with diethyl ether. The combined organic layers were washed with water and with brine, dried over sodium sulfate, filtered, and concentrated *in vacuo.* Silica gel chromatography (Gradient: 0% to 40% ethyl acetate in heptane) provided the product as an amber oil. Yield: 395 mg, 1.63 mmol, 58%. ¹H NMR (400 MHz, CDCl₃) δ 6.97-7.02 (m, 2H), 5.80 (s, 1H), 4.46 (s, 2H), 1.49 (s, 6H).

### Step 4. Synthesis of 1-(6-chloro-8-fluoro-2,2-dimethyl-2H-chromen-4-yl)methanamine (C4).

A solution of **C3** (100 mg, 0.412 mmol) and *p*-toluenesulfonic anhydride (202 mg, 0.619 mmol) in dichloromethane (2 mL) was cooled to 0 °C and treated drop-wise with triethylamine (96%, 0.12 mL, 0.83 mmol). After 10 minutes at 0 °C, the reaction mixture was allowed to warm to room temperature and stirred for 15 minutes. A solution of ammonia in methanol (7 M, 5 mL) was added, and stirring was continued for 16 hours. After removal of solvents under reduced pressure, the residue was partitioned between diethyl ether and 1 M aqueous sodium hydroxide solution. The aqueous layer was extracted three times with diethyl ether, and the combined organic layers were washed with brine, dried over magnesium sulfate, filtered, and concentrated *in vacuo* to provide the crude product, which was taken directly into the following step. ¹H NMR (400 MHz, CDCl₃) δ 6.94-7.02 (m, 2H), 5.77 (s, 1H), 3.67 (s, 2H), 1.47 (s, 6H).

### Step 5. Synthesis of 2-[(6-chloro-8-fluoro-2,2-dimethyl-2H-chromen-4-yl)methyl]-7-(4-methyl-1H-imidazol-1-yl)-3,4-dihydro-2H-pyrido[1,2-a]pyrazine-1,6-dione (1).

Compound **C4** (from the previous step, <0.412 mmol) and 7-(4-methyl-1*H-*imidazol-1-yl)-3,4-dihydropyrido[2,1-*c*][1,4]oxazine-1,6-dione (**C5**, see C. W. am Ende et al., PCT Int. Appl. 2012131539, Oct 4, 2012) (132 mg, 0.538 mmol) were suspended in *N*,*N*-dimethylformamide (0.4 mL). 1,3,4,6,7,8-Hexahydro-2*H*-pyrimido[1,2-*a*]pyrimidine (TBD, 91.0 mg, 0.621 mmol) was added, and the reaction mixture was stirred at room temperature for 1 hour; ethyl trifluoroacetate (0.247 mL, 2.07 mmol) was then added drop-wise and stirring was continued for 30 minutes. After addition of aqueous sodium hydroxide solution (1 M, 2 mL), the reaction mixture was stirred for 5 minutes, then diluted with water (2 mL) and filtered. The collected solid was washed with water (2 x 2 mL) and with diethyl ether (2 x 2 mL). Trituration with a 1:2 mixture of ethyl acetate and diethyl ether afforded the product as a solid. Yield: 61.6 mg, 0.131 mmol, 32% over 2 steps. LCMS *m*/*z* 469.1, 471.1 [M+H]+. ¹H NMR (400 MHz, CDCl₃) δ 8.66 (br s, 1H), 7.60 (d, *J*=7.8 Hz, 1H), 7.38 (d, *J*=7.6 Hz, 1H), 7.20 (br s, 1H), 6.99-7.06 (m, 2H), 5.78 (s, 1H), 4.56 (s, 2H), 4.25-4.31 (m, 2H), 3.54-3.61 (m, 2H), 2.39 (s, 3H), 1.51 (s, 6H).

### Examples 2, 3 and 4

### rel-2-{[(1aS,7bS)-2,2-Dimethyl-6-(trifluoromethyl)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (2), 2-{[(1aS,7bS)-2,2-Dimethyl-6-(trifluoromethyl)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (3), and 2-{[(1aR,7bR)-2,2-Dimethyl-6-(trifluoromethyl)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (4)

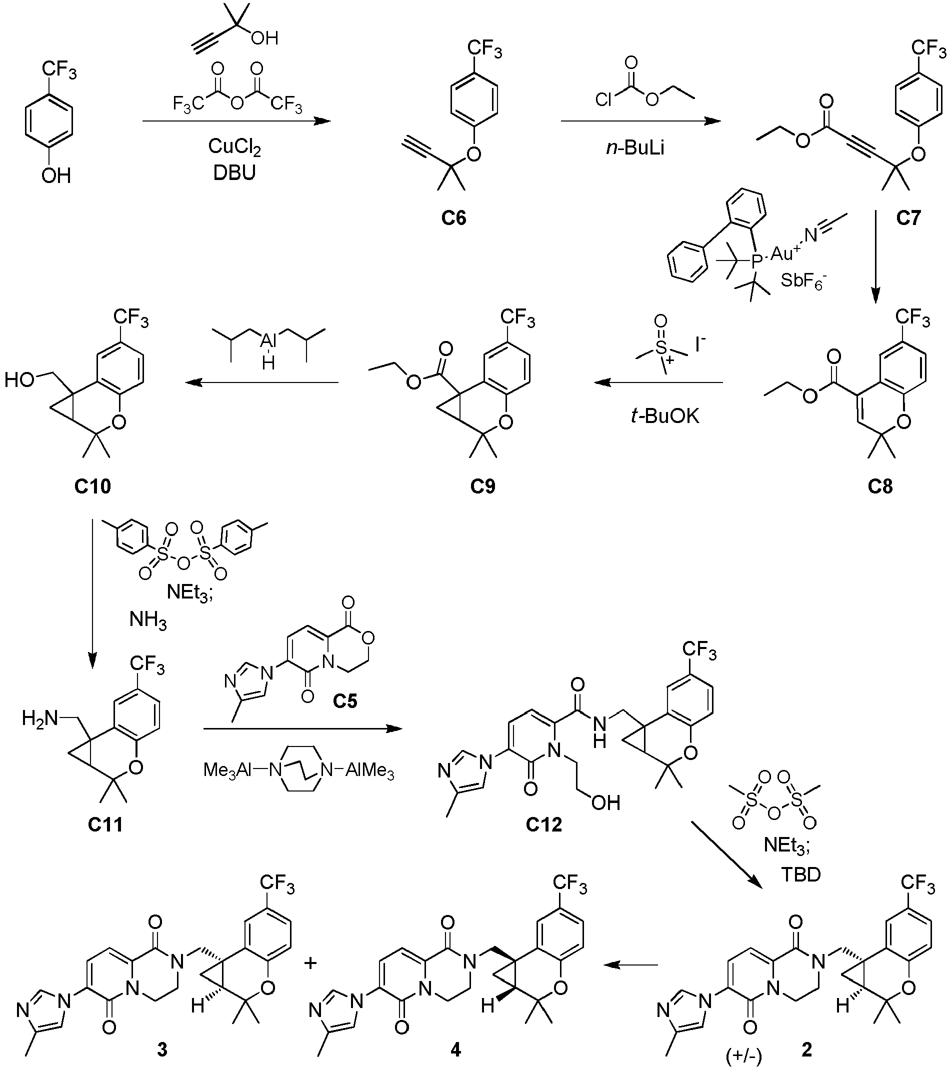

### Step 1. Synthesis of 1-[(2-methylbut-3-yn-2-yl)oxy]-4-(trifluoromethyl)benzene (C6).

The product was synthesized from 4-(trifluoromethyl)phenol using the method described for synthesis of **C1** in Example 1. Yield: 88.6 g (corrected for 13% residual heptane by weight), 388 mmol, 78%. ¹H NMR (400 MHz, CDCl₃) δ 7.55 (br d, *J*=8.6 Hz, 2H), 7.30 (br d, *J*=8.8 Hz, 2H), 2.63 (s, 1H), 1.70 (s, 6H).

### Step 2. Synthesis of ethyl 4-methyl-4-[4-(trifluoromethyl)phenoxy]pent-2-ynoate (C7).

*n*-Butyllithium (2.5 M solution in hexanes, 16.0 mL, 40.0 mmol) was added drop-wise to a -78 °C solution of **C6** (8.2 g, corrected for 13% heptane contaminant by weight, 36 mmol) in tetrahydrofuran (100 mL), while keeping the reaction temperature below -60 °C. The reaction mixture was stirred for 15 minutes at -78 °C, whereupon ethyl chloroformate (97%, 5.30 mL, 53.7 mmol) was added drop-wise, at a rate that maintained the reaction temperature below -70 °C. After 15 minutes at -78 °C, the reaction mixture was warmed to 0 °C and stirred at that temperature for 30 minutes. Saturated aqueous ammonium chloride solution (50 mL) was added at 0 °C, and the mixture was allowed to warm to room temperature, at which point it was diluted with *tert-*butyl methyl ether (500 mL). The organic layer was washed with water, dried over magnesium sulfate, filtered, and concentrated *in vacuo.* Silica gel chromatography (Gradient: 0% to 5% ethyl acetate in heptane) afforded the product as an oil. Yield: 10 g, 33 mmol, 92%. ¹H NMR (400 MHz, CDCl₃) δ 7.55-7.59 (m, 2H), 7.24-7.29 (m, 2H), 4.25 (q, *J*=7.1 Hz, 2H), 1.72 (s, 6H), 1.33 (t, *J*=7.1 Hz, 3H).

### Step 3. Synthesis of ethyl 2,2-dimethyl-6-(trifluoromethyl)-2H-chromene-4-carboxylate (C8).

(Acetonitrile)[(2-biphenyl)di-*tert*-butylphosphine]gold(I) hexafluoroantimonate (250 mg, 0.32 mmol) was added to a solution of **C7** (10 g, 33 mmol) in 1,2-dichloroethane (100 mL), and the reaction mixture was heated at 80 °C for 16 hours. After it had cooled to room temperature, the reaction mixture was filtered through a pad of silica gel, and the pad was rinsed with dichloromethane (3 x 200 mL). The filtrate was concentrated *in vacuo* to provide the product as a thick oil. Yield: 9.6 g, 32 mmol, 97%. ¹H NMR (400 MHz, CDCl₃) δ 8.30 (br d, *J*=2 Hz, 1H), 7.41-7.45 (m, 1H), 6.90 (br d, *J*=8.5 Hz, 1H), 6.71 (s, 1H), 4.35 (q, *J*=7.1 Hz, 2H), 1.50 (s, 6H), 1.40 (t, *J*=7.1 Hz, 3H).

### Step 4. Synthesis of ethyl 2,2-dimethyl-6-(trifluoromethyl)-1a,2-dihydrocyclopropa[c]chromene-7b(1H)-carboxylate (C9).

Potassium *tert*-butoxide (1.0 M solution in tetrahydrofuran, 35 mL, 35 mmol) was added to a suspension of trimethylsulfoxonium iodide (98%, 7.6 g, 34 mmol) in tetrahydrofuran (75 mL), and the mixture was stirred for 30 minutes. A solution of **C8** (7.00 g, 23.3 mmol) in tetrahydrofuran (25 mL) was added, and the reaction mixture was stirred for 30 minutes, whereupon it was partitioned between saturated aqueous ammonium chloride solution (100 mL) and *tert*-butyl methyl ether (500 mL). The organic layer was washed with water (100 mL), dried over magnesium sulfate, filtered, and concentrated *in vacuo* to afford the product as a thick oil, which was used without additional purification. Yield: 7.3 g, 23 mmol, 99%. ¹H NMR (400 MHz, CDCl₃) δ 8.16-8.18 (m, 1H), 7.35 (ddq, *J*=8.5, 2.3, 0.7 Hz, 1H), 6.83-6.86 (m, 1H), 4.31 (dq, half of ABX₃ pattern, *J*=10.8, 7.1 Hz, 1H), 4.23 (dq, half of ABX₃ pattern, *J*=10.8, 7.1 Hz, 1H), 2.07 (dd, half of ABX pattern, *J*=9.0, 6.6 Hz, 1H), 1.99 (dd, half of ABX pattern, *J*=9.0, 4.4 Hz, 1H), 1.53 (s, 3H), 1.33 (t, *J*=7.1 Hz, 3H), 1.29 (dd, *J*=6.6, 4.4 Hz, 1H), 1.28 (s, 3H).

### Step 5. Synthesis of [2,2-dimethyl-6-(trifluoromethyl)-1a,2-dihydrocyclopropa[c]chromen-7b(1H)-yl]methanol (C10).

Diisobutylaluminum hydride (1.5 M solution in toluene, 50 mL, 75 mmol) was added drop-wise over 30 minutes to a -78 °C solution of **C9** (7.3 g, 23 mmol) in tetrahydrofuran (100 mL). After 15 minutes at -78 °C, the reaction mixture was warmed to room temperature, stirred for 30 minutes, and cooled in an ice bath. Half-saturated aqueous citric acid (50 mL) was added, the ice bath was removed, and the mixture was stirred at room temperature for 16 hours, whereupon it was extracted with diethyl ether (500 mL). The organic layer was washed with water (100 mL), dried over magnesium sulfate, filtered, and concentrated *in vacuo.* Silica gel chromatography (Gradient: 5% to 30% ethyl acetate in heptane) provided the product as a thick oil. Yield: 5.7 g, 21 mmol, 91%. ¹H NMR (400 MHz, CDCl₃) δ 7.81 (br d, *J*=2.2 Hz, 1H), 7.35 (ddq, *J*=8.4, 2.2, 0.7 Hz, 1H), 6.84-6.87 (m, 1H), 4.12 (d, *J*=11.7 Hz, 1H), 3.73 (d, *J*=11.7 Hz, 1H), 1.58 (dd, *J*=8.6, 5.7 Hz, 1H), 1.52 (s, 3H), 1.22 (s, 3H), 1.12 (br dd, *J*=5.6, 5.1 Hz, 1H), 1.05 (dd, *J*=8.5, 5.0 Hz, 1H).

### Step 6. Synthesis of 1-[2,2-dimethyl-6-(trifluoromethyl)-1a,2-dihydrocyclopropa[c]chromen-7b(1H)-yl]methanamine (C11).

p-Toluenesulfonic anhydride (7.19 g, 22.0 mmol) was added in portions over 10 minutes to a 0 °C solution of **C10** (5.00 g, 18.4 mmol) in dichloromethane (100 mL), and the reaction mixture was stirred under ice cooling for 10 minutes. Triethylamine (4.0 mL, 29 mmol) was added drop-wise, and stirring was continued at 0 °C for 30 minutes, whereupon the reaction mixture was allowed to warm to room temperature and stir for 1 hour. *tert*-Butyl methyl ether (500 mL) was added, and the mixture was washed with water (100 mL), dried over sodium sulfate, filtered, and concentrated *in vacuo,* providing the intermediate tosylated compound as a thick oil. Yield: 7.80 g, 18.3 mmol, 99%. This material was dissolved in methanol (50 mL), added to a solution of ammonia in methanol (7 M, 300 mL, 2.1 mol) and stirred at room temperature for 24 hours. Volatiles were removed under reduced pressure, and the residue was partitioned between *tert-*butyl methyl ether (500 mL) and aqueous sodium hydroxide solution (1 M, 100 mL) with vigorous stirring for 15 minutes. The aqueous layer was extracted with *tert*-butyl methyl ether (500 mL) and the combined organic layers were dried over sodium sulfate, filtered, and concentrated *in vacuo.* Chromatography on silica gel (Gradient: 0% to 5% methanol in dichloromethane) provided the product as a thick oil. Yield: 3.43 g, 12.6 mmol, 69%. LCMS *m*/*z*271.9 [M+H]+. ¹H NMR (400 MHz, CDCl₃) δ 7.66-7.69 (m, 1H), 7.31-7.36 (m, 1H), 6.86 (br d, *J*=8.3 Hz, 1H), 3.62 (d, *J*=13.8 Hz, 1H), 2.51 (d, *J*=13.8 Hz, 1H), 1.51 (s, 3H), 1.49 (dd, *J*=8.4, 5.7 Hz, 1H), 1.24 (s, 3H), 1.06 (dd, *J*=5.5, 5.0 Hz, 1H), 0.97 (dd, *J*=8.5, 5.0 Hz, 1H).

### Step 7. Synthesis of N-{[2,2-dimethyl-6-(trifluoromethyl)-1a,2-dihydrocyclopropa[c]chromen-7b(1H)-yl]methyl}-1-(2-hydroxyethyl)-5-(4-methyl-1H-imidazol-1-yl)-6-oxo-1,6-dihydropyridine-2-carboxamide (C12).

Compound **C11** (3.60 g, 13.3 mmol) was dissolved in tetrahydrofuran (50 mL), and bis(trimethylaluminum)-1,4-diazabicyclo[2.2.2]octane adduct (97%, 4.5 g, 17 mmol) was added portion-wise. The reaction mixture was warmed to 45 °C for 45 minutes, whereupon **C5** (4.63 g, 18.9 mmol) was added. The resulting mixture was heated at reflux for 2 hours, cooled in an ice bath and quenched via slow addition of water (10 mL). Aqueous sodium hydroxide solution (1 M, 50 mL) was introduced, and the mixture was stirred at room temperature for 15 minutes, then extracted with ethyl acetate. The combined organic layers were dried over sodium sulfate, filtered, and concentrated *in vacuo*; the residue was suspended in diethyl ether and collected via filtration to afford the product as an off-white solid. Yield: 6.10 g, 11.8 mmol, 89%. LCMS *m*/*z* 517.3 [M+H]+. ¹H NMR (400 MHz, CD₃OD), characteristic peaks: δ 8.16 (d, *J*=1.3 Hz, 1H), 7.85 (br d, *J*=2 Hz, 1H), 7.62 (d, *J*=7.5 Hz, 1H), 7.37 (ddq, *J*=8.4, 2.2, 0.7 Hz, 1H), 7.21-7.23 (m, 1H), 6.86-6.90 (m, 1H), 6.37 (d, *J*=7.5 Hz, 1H), 4.32-4.39 (m, 3H), 3.72-3.82 (m, 2H), 2.22 (d, *J*=1.0 Hz, 3H), 1.90 (dd, *J*=8.6, 5.9 Hz, 1H), 1.50 (s, 3H), 1.23 (s, 3H), 1.18 (dd, *J*=8.7, 4.9 Hz, 1H), 1.07 (dd, *J*=5.7, 5.1 Hz, 1H).

### Step 8. Synthesis of rel-2-{[(1aS,7bS)-2,2-dimethyl-6-(trifluoromethyl)-1a,2-dihydrocyclopropa[cjchromen-7b(1H)-yl]methyl}-7-(4-methyl-1H-imidazol-1-yl)-3,4-dihydro-2H-pyrido[1,2-a]pyrazine-1,6-dione (2), 2-{[(1aS,7bS)-2,2-dimethyl-6-(trifluoromethyl)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (3), and 2-{[(1aR,7bR)-2,2-dimethyl-6-(trifluoromethyl)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (4).

Triethylamine (2.5 mL, 18 mmol) was added to a 0 °C suspension of **C12** (6.10 g, 11.8 mmol) in tetrahydrofuran (100 mL). Methanesulfonic anhydride (2.5 g, 14 mmol) was then added portion-wise, and the reaction mixture was stirred under ice cooling for 45 minutes. Additional triethylamine (1 mL, 7 mmol) and methanesulfonic anhydride (1 g, 6 mmol) were introduced, and stirring was continued for 2 hours. After addition of triethylamine (1 mL, 7 mmol) and methanesulfonic anhydride (0.5 g, 3 mmol) and a further 30 minutes of stirring, 1,3,4,6,7,8-hexahydro-2*H*-pyrimido[1,2-*a*]pyrimidine (TBD, 97%, 6.0 g, 42 mmol) was added and the reaction was allowed to continue for 30 minutes at 0 °C. Additional 1,3,4,6,7,8-hexahydro-2*H*-pyrimido[1,2-*a*]pyrimidine (97%, 2 g, 14 mmol) was introduced, and after 30 minutes at 0 °C, another charge of 1,3,4,6,7,8-hexahydro-2*H*-pyrimido[1,2-*a*]pyrimidine (97%, 3 g, 21 mmol) was added. After 30 minutes, the reaction mixture was partitioned between water (100 mL) and ethyl acetate (750 mL). The organic layer was washed with water (100 mL), dried over sodium sulfate, filtered, and concentrated under reduced pressure. Silica gel chromatography (Gradient: 0% to 10% methanol in dichloromethane) provided partially purified material (4.8 g); this was treated with diethyl ether (50 mL), warmed to reflux for 10 minutes, cooled to room temperature, and filtered to provide the racemic product **2** as a pale yellow solid. Yield: 3.5 g, 7.0 mmol, 59%. ¹H NMR (400 MHz, CDCl₃) δ 8.21 (d, *J*=0.9 Hz, 1H), 7.74 (d, *J*=1.8 Hz, 1H), 7.45 (d, *J*=7.7 Hz, 1H), 7.33-7.37 (m, 1H), 7.30 (d, *J*=7.8 Hz, 1H), 7.12 (br s, 1H), 6.86 (d, *J*=8.6 Hz, 1H), 4.96 (d, *J*=14.8 Hz, 1H), 4.35 (ddd, *J*=14.3, 6.7, 4.0 Hz, 1H), 4.19 (ddd, *J*=14.3, 8.1, 4.2 Hz, 1H), 3.64-3.79 (m, 2H), 3.20 (d, *J*=14.7 Hz, 1H), 2.28 (s, 3H), 1.80 (dd, *J*=8.7, 5.8 Hz, 1H), 1.53 (s, 3H), 1.27 (s, 3H), 1.17 (dd, *J*=5.7, 5.4 Hz, 1H), 1.08 (dd, *J*=8.8, 5.3 Hz, 1H). Compound **2** was separated into its enantiomers via supercritical fluid chromatography (Column: Phenomenex Lux Cellulose-1, 5 µm; Eluent: 3:2 carbon dioxide / 0.2% [7 M solution of ammonia in ethanol] in methanol). The first-eluting enantiomer (1.8 g) was suspended in ethyl acetate (25 mL), heated to reflux and treated with additional ethyl acetate (10 mL). After cooling to room temperature, a solid was removed via filtration, and the filtrate was concentrated under reduced pressure to provide an off-white solid. This was dissolved in ethyl acetate (10 mL), heated to reflux and treated with heptane (20 mL); the mixture was cooled to room temperature and the resulting solid was isolated via filtration and washed with heptane, affording material assigned as Example **3**. The indicated stereochemistry was assigned on the basis of single crystal X-ray determination on **3**, see below. Yield: 1.36 g, 2.73 mmol, 23%. This material proved to be crystalline by powder X-ray diffraction. **3**: LCMS *m*/*z* 499.3 [M+H]+. ¹H NMR (400 MHz, CD₃OD) δ 8.27-8.28 (m, 1H), 7.86-7.89 (m, 1H), 7.77 (d, *J*=7.8 Hz, 1H), 7.31-7.35 (m, 1H), 7.26-7.30 (m, 2H), 6.86 (d, *J*=8.4 Hz, 1H), 5.17 (d, *J*=14.8 Hz, 1H), 4.28-4.36 (m, 1H), 4.14-4.22 (m, 1H), 3.73-3.85 (m, 2H), 3.05 (d, *J*=14.6 Hz, 1H), 2.23 (s, 3H), 2.05 (dd, J=8.6, 5.9 Hz, 1H), 1.52 (s, 3H), 1.30 (s, 3H), 1.10 (dd, *J*=8.6, 5.0 Hz, 1H), 1.06 (dd, *J*=5.5, 5.4 Hz, 1H).Retention time: 8.35 minutes (Column: Phenomenex Lux Cellulose-1, 4.6 x 250 mm, 5 µm; Mobile phase A: carbon dioxide; Mobile phase B: 0.2% [7 M solution of ammonia in ethanol] in methanol; Gradient: 5% B from 0 to 1.0 minute, then linear from 5% to 60% B for 8.5 minutes; Flow rate: 3.0 mL/minute).

The second-eluting enantiomer was assigned as Example **4**. Yield: 1.8 g, 3.6 mmol, 30%. **4**: LCMS *m*/*z* 499.3 [M+H]+. ¹H NMR (400 MHz, CD₃OD) δ 8.27 (br s, 1H), 7.86-7.89 (m, 1H), 7.77 (d, *J*=7.8 Hz, 1H), 7.31-7.35 (m, 1H), 7.26-7.30 (m, 2H), 6.86 (d, *J*=8.6 Hz, 1H), 5.17 (d, *J*=14.6 Hz, 1H), 4.28-4.36 (m, 1H), 4.14-4.22 (m, 1H), 3.73-3.85 (m, 2H), 3.05 (d, *J*=14.6 Hz, 1H), 2.23 (s, 3H), 2.05 (dd, *J*=8.5, 6.0 Hz, 1H), 1.52 (s, 3H), 1.30 (s, 3H), 1.04-1.13 (m, 2H). Retention time: 9.56 minutes, using conditions identical to those described above for Example **3.**

### Single-crystal X-ray determination of 3

### Single Crystal X-Ray Analysis

Data collection was performed on a Bruker APEX diffractometer at room temperature. Data collection consisted of omega and phi scans.

The structure was solved by direct methods using the SHELX software suite in the space group P2₁2₁2₁. The structure was subsequently refined by the full-matrix least squares method. All non-hydrogen atoms were found and refined using anisotropic displacement parameters.

The molecule is disordered in several locations; C23-N4, C16, and the CF₃ group were modeled with two occupancies. The O3 atom is also disordered, but no model was tested here.

All hydrogen atoms were placed in calculated positions and were allowed to ride on their carrier atoms. The final refinement included isotropic displacement parameters for all hydrogen atoms.

Analysis of the absolute structure using likelihood methods (Hooft 2008) was performed using PLATON (Spek 2010). The results indicate that the absolute structure has been correctly assigned. The method calculates that the probability that the structure is correct is 100.0. The Hooft parameter is reported as 0.06 with an esd of 0.08.

The final R-index was 5.75%. A final difference Fourier revealed no missing or misplaced electron density.

Pertinent crystal, data collection and refinement are summarized in Table 1. Atomic coordinates, bond lengths, bond angles, torsion angles and displacement parameters are listed in Tables 2 - 5.

### Software and References

SHELXTL, Version 5.1, Bruker AXS, 1997.
PLATON, A. L. Spek, J. Appl. Cryst. 2003, 36, 7-13.
MERCURY, C. F. Macrae, P. R. Edington, P. McCabe, E. Pidcock, G. P. Shields, R. Taylor, M. Towler and J. van de Streek, J. Appl. Cryst. 2006, 39, 453-457.
OLEX2, O. V. Dolomanov, L. J. Bourhis, R. J. Gildea, J. A. K. Howard, H. Puschmann, J. Appl. Cryst. 2009, 42, 339-341.
R. W. Hooft et al., J. Appl. Cryst. 2008, 41, 96-103.
H. D. Flack, Acta Cryst. 1983, A39, 867-881.

**Table 1. Crystal data and structure refinement for 3.**

| | | |
|---|---|---|
| Empirical formula | C₂₆H₂₅F₃N₄O₃ | |
| Formula weight | 498.50 | |
| Temperature | 296(2) K | |
| Wavelength | 1.54178 Å | |
| Crystal system | Orthorhombic | |
| Space group | P2(1)2(1)2(1) | |
| Unit cell dimensions | a = 6.1848(2) Å | α = 90° |
| | b = 13.6417(3) Å | β = 90° |
| | c = 27.9922(7) Å | γ = 90° |
| Volume | 2361.74(11) Å³ | |
| Z | 4 | |
| Density (calculated) | 1.402 Mg/m³ | |
| Absorption coefficient | 0.923 mm⁻¹ | |
| F(000) | 1040 | |
| Crystal size | 0.31 x 0.15 x 0.10 mm³ | |
| Theta range for data collection | 3.16 to 68.46°. | |
| Index ranges | -7<=h<=6, -16<=k<=16, 33<=I<=33 | |
| Reflections collected | 28654 | |
| Independent reflections | 4323 [R(int) = 0.0638] | |
| Completeness to theta = 68.46° | 99.9 % | |
| Absorption correction | Empirical | |
| Refinement method | Full-matrix least-squares or F² | |
| Data / restraints / parameters | 4323 / 2 / 383 | |
| Goodness-of-fit on F² | 1.025 | |
| Final R indices [I>2sigma(I)] | R1 = 0.0575, wR2 = 0.1480 | |
| R indices (all data) | R1 = 0.0612, wR2 = 0.1520 | |
| Absolute structure parameter | 0.1(3) | |
| Extinction coefficient | 0.0009(2) | |
| Largest diff. peak and hole | 0.729 and -0.669 e.Å⁻³ | |

**Table 2. Atomic coordinates (x 10⁴) and equivalent isotropic displacement parameters (Å² x 10³) for 3. U(eq) is defined as one-third of the trace of the orthogonalized U^{ij} tensor.**

| | x | y | z | U(eq) |
|---|---|---|---|---|
| C(1) | 1643(5) | -238(2) | 8356(1) | 33(1) |
| C(2) | 2786(5) | -1082(2) | 8476(1) | 36(1) |
| C(3) | 4293(5) | -1474(2) | 8163(1) | 37(1) |
| C(4) | 4678(6) | -1043(2) | 7723(1) | 44(1) |
| C(5) | 3552(6) | -210(3) | 7598(1) | 45(1) |
| C(6) | 2067(5) | 196(2) | 7909(1) | 37(1) |
| C(7) | 5524(6) | -2360(2) | 8319(1) | 46(1) |
| C(8) | 329(5) | 1720(2) | 8114(1) | 34(1) |
| C(9) | -756(5) | 1228(2) | 8534(1) | 37(1) |
| C(10) | -25(5) | 204(2) | 8670(1) | 39(1) |
| C(11) | 2325(5) | 2255(2) | 8279(1) | 36(1) |
| C(12) | -1224(6) | 2388(3) | 7850(1) | 50(1) |
| C(13) | -2235(6) | 384(3) | 8443(2) | 59(1) |
| C(14) | -82(5) | -101(2) | 9186(1) | 47(1) |
| C(15) | 2706(8) | 1059(2) | 9504(1) | 56(1) |
| C(17) | 4959(6) | -456(2) | 9947(1) | 40(1) |
| C(18) | 2959(6) | -689(2) | 9672(1) | 44(1) |
| C(19) | 7323(6) | 778(2) | 10306(2) | 53(1) |
| C(20) | 8594(7) | -19(2) | 10489(1) | 49(1) |
| C(21) | 8038(10) | -963(3) | 10390(1) | 75(2) |
| C(22) | 6218(8) | -1182(2) | 10116(1) | 63(1) |
| C(24) | 13290(8) | 69(3) | 11217(1) | 66(1) |
| C(25) | 11912(6) | -423(3) | 10927(2) | 64(1) |
| C(26) | 15196(8) | -267(4) | 11494(2) | 85(2) |
| C(23A) | 10580(10) | 988(4) | 11022(2) | 49(2) |
| C(23B) | 11885(18) | 1239(6) | 10819(3) | 39(3) |
| C(16C) | 4014(7) | 1291(3) | 9966(2) | 33(1) |
| C(16D) | 4487(16) | 1217(6) | 9635(3) | 33(1) |
| F(1) | 6604(17) | -2235(5) | 8708(4) | 80(3) |
| F(2) | 4117(12) | -3093(8) | 8449(4) | 66(2) |
| F(3) | 6710(30) | -2749(7) | 7986(3) | 82(4) |
| F(1B) | 4760(40) | -3160(8) | 8219(12) | 106(9) |
| F(2B) | 7520(30) | -2370(20) | 8124(9) | 112(6) |
| F(3B) | 6180(40) | -2307(11) | 8768(6) | 121(7) |
| N(1) | 1922(4) | 69(2) | 9455(1) | 40(1) |
| N(2) | 5544(5) | 502(2) | 10040(1) | 45(1) |
| N(3A) | 10231(11) | 150(5) | 10772(3) | 34(1) |
| N(4A) | 12323(9) | 930(4) | 11283(2) | 59(2) |
| N(3B) | 10850(30) | 383(11) | 10770(6; | 34(1) |
| N(4B) | 13629(14) | 1209(6) | 11092(3) | 36(2) |
| O(1) | 938(4) | 996(2) | 7758(1) | 47(1) |
| O(2) | 2353(5) | -1542(2) | 9642(1) | 62(1) |
| O(3) | 7730(5) | 1645(2) | 10387(2) | 107(2) |

**Table 3. Bond lengths [Å] and angles [°] for 3.**

| | | | |
|---|---|---|---|
| C(1)-C(2) | 1.392(4) | C(8)-C(11) | 1.506(4) |
| C(1)-C(6) | 1.410(4) | C(8)-C(9) | 1.510(4) |
| C(1)-C(10) | 1.483(4) | C(8)-C(12) | 1.516(4) |
| C(2)-C(3) | 1.388(4) | C(9)-C(13) | 1.493(5) |
| C(3)-C(4) | 1.385(5) | C(9)-C(10) | 1.517(4) |
| C(3)-C(7) | 1.495(4) | C(10)-C(14) | 1.503(5) |
| C(4)-C(5) | 1.377(4) | C(10)-C(13) | 1.528(5) |
| C(5)-C(6) | 1.381(4) | C(14)-N(1) | 1.468(5) |
| C(6)-O(1) | 1.362(4) | C(15)-C(16D) | 1.181(11) |
| C(7)-F(1B) | 1.221(13) | C(15)-N(1) | 1.442(4) |
| C(7)-F(1) | 1.288(8) | C(15)-C(16C) | 1.557(6) |
| C(7)-F(3) | 1.302(8) | C(17)-C(22) | 1.345(5) |
| C(7)-F(3B) | 1.323(14) | C(17)-N(2) | 1.380(4) |
| C(7)-F(2B) | 1.350(14) | C(17)-C(18) | 1.492(5) |
| C(7)-F(2) | 1.374(8) | C(18)-O(2) | 1.226(4) |
| C(8)-O(1) | 1.453(3) | C(18)-N(1) | 1.360(4) |
| C(19)-O(3) | 1.231(5) | F(1)-C(7)-F(3) | 111.5(8) |
| C(19)-N(2) | 1.381(5) | F(1B)-C(7)-F(3B) | 112.7(12) |
| C(19)-C(20) | 1.436(4) | F(1)-C(7)-F(3B) | 14.3(13) |
| C(20)-N(3A) | 1.306(8) | F(3)-C(7)-F(3B) | 122.0(9) |
| C(20)-C(21) | 1.361(5) | F(1B)-C(7)-F(2B) | 104.6(8) |
| C(20)-N(3B) | 1.692(16) | F(1)-C(7)-F(2B) | 82.5(12) |
| C(21)-C(22) | 1.395(7) | F(3)-C(7)-F(2B) | 35.7(10) |
| C(24)-N(4A) | 1.330(6) | F(3B)-C(7)-F(2B) | 96.0(11) |
| C(24)-C(25) | 1.355(6) | F(1B)-C(7)-F(2) | 33.5(14) |
| C(24)-C(26) | 1.483(6) | F(1)-C(7)-F(2) | 101.6(6) |
| C(24)-N(4B) | 1.607(9) | F(3)-C(7)-F(2) | 104.6(5) |
| C(25)-N(3B) | 1.356(18) | F(3B)-C(7)-F(2) | 89.0(10) |
| C(25)-N(3A) | 1.372(9) | F(2B)-C(7)-F(2) | 132.7(9) |
| C(23A)-N(4A) | 1.304(7) | F(1B)-C(7)-C(3) | 117.4(6) |
| C(23A)-N(3A) | 1.359(9) | F(1)-C(7)-C(3) | 113.8(4) |
| C(23B)-N(4B) | 1.322(11) | F(3)-C(7)-C(3) | 114.1(5) |
| C(23B)-N(3B) | 1.340(16) | F(3B)-C(7)-C(3) | 112.9(6) |
| C(16C)-N(2) | 1.449(5) | F(2B)-C(7)-C(3) | 110.8(7) |
| C(16D)-N(2) | 1.632(9) | F(2)-C(7)-C(3) | 110.1(5) |
| C(2)-C(1)-C(6) | 117.8(3) | O(1)-C(8)-C(11) | 109.0(2) |
| C(2)-C(1)-C(10) | 123.1(3) | O(1)-C(8)-C(9) | 110.2(2) |
| C(6)-C(1)-C(10) | 119.0(3) | C(11)-C(8)-C(9) | 110.0(2) |
| C(3)-C(2)-C(1) | 120.5(3) | O(1)-C(8)-C(12) | 103.8(2) |
| C(4)-C(3)-C(2) | 121.0(3) | C(11)-C(8)-C(12) | 112.2(3) |
| C(4)-C(3)-C(7) | 121.0(3) | C(9)-C(8)-C(12) | 111.4(3) |
| C(2)-C(3)-C(7) | 118.0(3) | C(13)-C(9)-C(8) | 118.9(3) |
| C(5)-C(4)-C(3) | 119.2(3) | C(13)-C(9)-C(10) | 61.0(2) |
| C(4)-C(5)-C(6) | 120.4(3) | C(8)-C(9)-C(10) | 118.2(2) |
| O(1)-C(6)-C(5) | 117.9(3) | C(1)-C(10)-C(14) | 118.2(3) |
| O(1)-C(6)-C(1) | 121.0(3) | C(1)-C(10)-C(9) | 115.6(3) |
| C(5)-C(6)-C(1) | 121.0(3) | C(14)-C(10)-C(9) | 119.4(3) |
| F(1B)-C(7)-F(1) | 120.8(11) | C(1)-C(10)-C(13) | 116.1(3) |
| F(1B)-C(7)-F(3) | 72.0(13) | C(14)-C(10)-C(13) | 115.0(3) |
| C(9)-C(10)-C(13) | 58.7(2) | N(4B)-C(23B)-N(3B) | 115.0(11) |
| C(9)-C(13)-C(10) | 60.3(2) | N(2)-C(16C)-C(15) | 107.8(3) |
| N(1)-C(14)-C(10) | 115.3(2) | C(15)-C(16D)-N(2) | 118.7(7) |
| C(16D)-C(15)-N(1) | 121.0(5) | C(18)-N(1)-C(15) | 120.7(3) |
| C(16D)-C(15)-C(16C) | 38.8(5) | C(18)-N(1)-C(14) | 120.4(3) |
| N(1)-C(15)-C(16C) | 116.5(3) | C(15)-N(1)-C(14) | 118.8(3) |
| C(22)-C(17)-N(2) | 118.6(4) | C(17)-N(2)-C(19) | 124.6(3) |
| C(22)-C(17)-C(18) | 120.3(3) | C(17)-N(2)-C(16C) | 120.4(3) |
| N(2)-C(17)-C(18) | 121.1(3) | C(19)-N(2)-C(16C) | 113.2(3) |
| O(2)-C(18)-N(1) | 123.2(4) | C(17)-N(2)-C(16D) | 109.3(4) |
| O(2)-C(18)-C(17) | 119.4(3) | C(19)-N(2)-C(16D) | 122.1(4) |
| N(1)-C(18)-C(17) | 117.4(3) | C(16C)-N(2)-C(16D) | 36.3(4) |
| O(3)-C(19)-N(2) | 121.6(3) | C(20)-N(3A)-C(23A) | 125.8(6) |
| O(3)-C(19)-C(20) | 123.4(4) | C(20)-N(3A)-C(25) | 132.8(6) |
| N(2)-C(19)-C(20) | 115.0(3) | C(23A)-N(3A)-C(25) | 101.3(6) |
| N(3A)-C(20)-C(21) | 119.1(4) | C(23A)-N(4A)-C(24) | 110.4(5) |
| N(3A)-C(20)-C(19) | 120.5(4) | C(23B)-N(3B)-C(25) | 116.2(13) |
| C(21)-C(20)-C(19) | 120.4(4) | C(23B)-N(3B)-C(20) | 136.5(14) |
| N(3A)-C(20)-N(3B) | 12.1(7) | C(25)-N(3B)-C(20) | 106.7(8) |
| C(21)-C(20)-N(3B) | 127.6(7) | C(23B)-N(4B)-C(24) | 92.9(6) |
| C(19)-C(20)-N(3B) | 111.8(7) | C(6)-O(1)-C(8) | 117.8(2) |
| C(20)-C(21)-C(22) | 121.2(3) | | |
| C(17)-C(22)-C(21) | 120.2(3) | | |
| N(4A)-C(24)-C(25) | 103.7(4) | | |
| N(4A)-C(24)-C(26) | 123.9(5) | | |
| C(25)-C(24)-C(26) | 131.3(5) | | |
| N(4A)-C(24)-N(4B) | 40.1(4) | | |
| C(25)-C(24)-N(4B) | 115.5(4) | | |
| C(26)-C(24)-N(4B) | 108.0(5) | | |
| C(24)-C(25)-N(3B) | 95.6(7) | | |
| C(24)-C(25)-N(3A) | 112.6(5) | | |
| N(3B)-C(25)-N(3A) | 21.0(5) | | |
| N(4A)-C(23A)-N(3A) | 111.6(6) | | |

Symmetry transformations used to generate equivalent atoms.

**Table 4. Anisotropic displacement parameters (Å² x 10³) for 3. The anisotropic displacement factor exponent takes the form: -2π²[h² a*²U¹¹ + ... + 2 h k a* b* U¹²].**

| | U¹¹ | U²² | U³³ | U²³ | U¹³ | U¹² |
|---|---|---|---|---|---|---|
| C(1) | 25(1) | 28(1) | 47(2) | -12(1) | -4(1) | -3(1) |
| C(2) | 31(2) | 26(1) | 52(2) | -6(1) | 1(1) | -5(1) |
| C(3) | 36(2) | 31(1) | 44(2) | -15(1) | -8(1) | 4(1) |
| C(4) | 50(2) | 50(2) | 31(1) | -22(1) | -5(1) | 17(2) |
| C(5) | 57(2) | 51(2) | 29(1) | -16(1) | -10(1) | 17(2) |
| C(6) | 35(2) | 39(2) | 38(2) | -15(1) | -12(1) | 10(1) |
| C(7) | 46(2) | 36(2) | 58(2) | -10(1) | 2(2) | 6(2) |
| C(8) | 38(2) | 33(1) | 32(1) | -10(1) | -2(1) | 7(1) |
| C(9) | 24(1) | 34(2) | 53(2) | -6(1) | 6(1 ) | 0(1) |
| C(10) | 24(1) | 30(1) | 62(2) | 2(1) | 12(1) | -5(1) |
| C(11) | 43(2) | 37(2) | 28(1) | 4(1) | 3(1) | -5(1) |
| C(12) | 57(2) | 54(2) | 40(2) | -8(2) | -12(2) | 23(2) |
| C(13) | 26(2) | 43(2) | 108(3) | -8(2) | 4(2) | -1(1) |
| C(14) | 33(2) | 35(2) | 73(2) | 11(2) | 31(2) | 2(1) |
| C(15) | 113(4) | 23(1) | 32(2) | 1(1) | -7(2) | 7(2) |
| C(17) | 63(2) | 23(1) | 34(1) | 7(1) | 31(2) | 11(1) |
| C(18) | 53(2) | 28(1) | 52(2) | 11(1) | 31(2) | 7(1) |
| C(19) | 37(2) | 33(2) | 89(3) | 24(2) | 28(2) | 5(1) |
| C(20) | 84(3) | 43(2) | 20(1) | 5(1) | 17(2) | 25(2) |
| C(21) | 158(5) | 39(2) | 29(2) | -5(1) | -21(2) | 45(3) |
| C(22) | 139(4) | 28(2) | 22(1) | 0(1) | -5(2) | 26(2) |
| C(24) | 86(3) | 91(3) | 23(2) | 16(2) | 23(2) | 31(3) |
| C(25) | 42(2) | 65(2) | 87(3) | 40(2) | 17(2) | 6(2) |
| C(26) | 59(3) | 125(4) | 73(3) | 51(3) | 18(2) | 5(3) |
| C(23A) | 56(4) | 49(3) | 42(3) | -21(2) | 20(3) | -11(3) |
| C(23B) | 60(7) | 22(4) | 35(5) | -3(4) | -17(5) | -2(4) |
| C(16C) | 44(2) | 22(2) | 31(2) | 4(2) | 5(2) | 5(2) |
| C(16D) | 44(2) | 22(2) | 31(2) | 4(2) | 5(2) | 5(2) |
| F(1) | 89(5) | 38(3) | 112(8) | -27(3) | -68(5) | 6(3) |
| F(2) | 52(3) | 38(3) | 108(5) | 15(3) | 5(3) | 0(2) |
| F(3) | 117(9) | 63(5) | 66(4) | 6(3) | 42(4) | 58(5) |
| F(1B) | 113(14) | 28(4) | 178(19 | -31(7) | -67(14) | 12(6) |
| F(2B) | 84(8) | 104(12 | 148(13 | 43(9) | 22(8) | 57(8) |
| F(3B) | 211(16) | 97(9) | 55(6) | -3(5) | -17(7) | 114(10) |
| N(1) | 45(2) | 25(1) | 49(1) | 8(1) | 28(1) | 3(1) |
| N(2) | 37(2) | 26(1) | 72(2) | 15(1) | 22(1) | 6(1) |
| N(3A) | 49(4) | 28(3) | 24(1) | 3(2) | 14(3) | -7(2) |
| N(4A) | 59(3) | 79(3) | 39(2) | -24(2) | 11(3) | -14(3) |
| N(3B) | 49(4) | 28(3) | 24(1) | 3(2) | 14(3) | -7(2) |
| N(4B) | 46(5) | 39(4) | 22(4) | -4(3) | -2(4) | -6(4) |
| O(1) | 59(2) | 50(1) | 32(1) | -15(1) | -15(1) | 24(1) |
| O(2) | 63(2) | 25(1) | 97(2) | 17(1) | 28(2) | -1(1) |
| O(3) | 57(2) | 35(1) | 230(5) | 46(2) | -44(3) | -13(1) |

**Table 5. Hydrogen coordinates (x 10⁴) and isotropic displacement parameters (Å² x 10³) for 3.**

| | x | y | z | U(eq) |
|---|---|---|---|---|
| H(006) | 2538 | -1385 | 8769 | 44 |
| H(015) | 5686 | -1313 | 7514 | 52 |
| H(021) | 3794 | 80 | 7302 | 55 |
| H(022) | -1158 | 1659 | 8800 | 45 |
| H(01C) | 3007 | 2564 | 8010 | 54 |
| H(01D) | 3311 | 1797 | 8422 | 54 |
| H(01E) | 1930 | 2744 | 8509 | 54 |
| H(03A) | -2438 | 2014 | 7739 | 75 |
| H(03B) | -498 | 2679 | 7582 | 75 |
| H(03C) | -1718 | 2895 | 8062 | 75 |
| H(03D) | -2440 | 178 | 8114 | 71 |
| H(03E) | -3501 | 319 | 8645 | 71 |
| H(01A) | -1246 | 249 | 9343 | 56 |
| H(01B) | -423 | -795 | 9201 | 56 |
| H(02A) | 1475 | 1499 | 9492 | 67 |
| H(02B) | 3614 | 1206 | 9231 | 67 |
| H(028) | 8888 | -1472 | 10507 | 90 |
| H(025) | 5874 | -1831 | 10049 | 75 |
| H(029) | 12087 | -1078 | 10843 | 77 |
| H(03F) | 14861 | -254 | 11829 | 128 |
| H(03G) | 16398 | 159 | 11432 | 128 |
| H(03H) | 15562 | -923 | 11401 | 128 |
| H(23A) | 9687 | 1536 | 11010 | 59 |
| H(23B) | 11413 | 1813 | 10673 | 47 |
| H(16A) | 4774 | 1909 | 9931 | 39 |
| H(16B) | 3047 | 1342 | 10237 | 39 |
| H(16C) | 4525 | 1882 | 9758 | 39 |
| H(16D) | 5423 | 1200 | 9357 | 39 |

### Example 5

### rel-2-{[(1aS,7bS)-6-(1-Methylcyclopropyl)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (5)

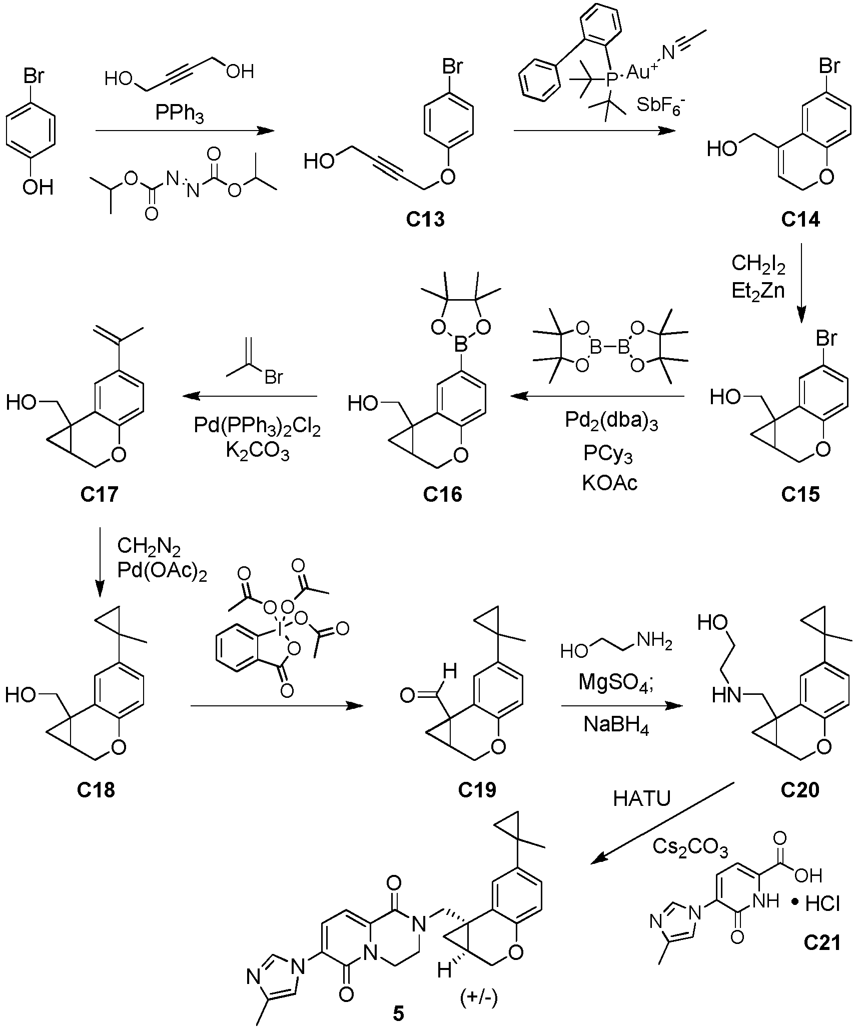

### Step 1. Synthesis of 4-(4-bromophenoxy)but-2-yn-1-ol (C13).

4-Bromophenol (50 g, 290 mmol) and triphenylphosphine (91 g, 350 mmol) were dissolved in tetrahydrofuran (500 mL). But-2-yne-1,4-diol (24.9 g, 289 mmol) was added and the reaction mixture was allowed to stir for 15 minutes, whereupon it was cooled to 0 °C and treated in a drop-wise manner with diisopropyl azodicarboxylate (70.0 g, 346 mmol). The resulting solution was stirred at room temperature for 12 hours, then quenched by addition of ice water. After the mixture had been concentrated *in vacuo,* the residue was partitioned between ethyl acetate and water; the organic layer was washed with water and with brine, dried over sodium sulfate, filtered, and concentrated under reduced pressure. Silica gel chromatography (Eluent: 20% ethyl acetate in hexanes) provided the product as an off-white solid. Yield: 45 g, 0.19 mmol, 66%. GCMS *m*/*z* 240, 242 (M⁺). ¹H NMR (400 MHz, DMSO-*d₆*) δ 7.47 (br d, *J*=9.0 Hz, 2H), 6.95 (br d, *J*=9.0 Hz, 2H), 5.23 (t, *J*=6.0 Hz, 1H), 4.83 (t, *J*=1.6 Hz, 2H), 4.10 (dt, *J*=6.0, 1.7 Hz, 2H).

### Step 2. Synthesis of (6-bromo-2H-chromen-4-yl)methanol (C14).

To a solution of **C13** (20 g, 83 mmol) in dichloromethane (200 mL) was added (acetonitrile)[(2-biphenyl)di-*tert*-butylphosphine]gold(I) hexafluoroantimonate (320 mg, 0.414 mmol) and the reaction mixture was stirred at room temperature for 16 hours. After dilution with dichloromethane, the reaction mixture was washed with water and with brine, dried over sodium sulfate, filtered, and concentrated *in vacuo.* Chromatography on silica gel (Eluent: 20% ethyl acetate in hexanes) afforded the product as an off-white solid. Yield: 8.0 g, 33 mmol, 40%. GCMS *m*/*z* 240, 242 (M⁺). ¹H NMR (400 MHz, DMSO-*d₆*) δ 7.35 (d, *J*=2.4 Hz, 1H), 7.27 (dd, *J*=8.6, 2.4 Hz, 1H), 6.74 (d, *J*=8.6 Hz, 1H), 5.88-5.92 (m, 1H), 5.09 (t, *J*=5.4 Hz, 1H), 4.76-4.80 (m, 2H), 4.22-4.27 (m, 2H).

### Step 3. Synthesis of (6-bromo-1a,2-dihydrocyclopropa[c]chromen-7b(1H)-yl)methanol (C15).

To a 0 °C solution of **C14** (8.0 g, 33 mmol) in dichloromethane (80 mL) were added diiodomethane (16.1 mL, 200 mmol) and diethylzinc (1 M solution in hexanes, 100 mL, 100 mmol). The reaction mixture was stirred at room temperature for 3 hours, whereupon it was filtered through a pad of diatomaceous earth. The filtrate was diluted with dichloromethane, washed sequentially with aqueous sodium thiosulfate solution, water, and brine, dried over sodium sulfate, filtered, and concentrated under reduced pressure. Silica gel chromatography (Eluent: 30% ethyl acetate in hexanes) provided the product as a yellow solid. Yield: 5 g, 20 mmol, 61%. GCMS *m*/*z* 254, 256 (M⁺). ¹H NMR (400 MHz, DMSO-*d₆*) δ 7.67 (d, *J*=2.4 Hz, 1H), 7.20 (dd, *J*=8.6, 2.4 Hz, 1H), 6.73 (d, *J*=8.6 Hz, 1H), 4.90 (t, *J*=5.5 Hz, 1H), 4.26 (d, *J*=10.6 Hz, 1H), 3.74-3.80 (m, 2H), 3.59 (dd, *J*=11.8, 5.3 Hz, 1H), 1.69-1.75 (m, 1H), 1.04 (dd, *J*=8.3, 4.6 Hz, 1H), 0.92 (dd, *J*=5, 5 Hz, 1H).

### Step 4. Synthesis of [6-(4,4,5,5-tetramethyl-1,3,2-dioxaborolan-2-yl)-1a,2-dihydrocyclopropa[c]chromen-7b(1H)-yl]methanol (C16).

Tricyclohexylphosphine (PCy₃, 192 mg, 0.685 mmol) and tris(dibenzylideneacetone)dipalladium(0) (269 mg, 0.294 mmol) were suspended in 1,4-dioxane (25 mL), and the mixture was stirred at room temperature for 15 minutes. Compound **C15** (2.5 g, 9.8 mmol), 4,4,4',4',5,5,5',5'-octamethyl-2,2'-bi-1,3,2-dioxaborolane (4.98 g, 19.6 mmol) and potassium acetate (1.93 g, 19.7 mmol) were added, and the reaction mixture was heated to 80 °C for 16 hours. The reaction mixture was then filtered through a pad of diatomaceous earth. The filtrate was concentrated *in vacuo*, and the residue was partitioned between water and ethyl acetate; the aqueous layer was extracted with ethyl acetate, and the combined organic layers were washed with brine, dried over sodium sulfate, filtered, and concentrated under reduced pressure. Chromatography on silica gel (Eluent: 20% ethyl acetate in hexanes) afforded the product as a yellow solid. Yield: 2.2 g, 7.3 mmol, 74%. GCMS *m*/*z* 302 (M⁺). ¹H NMR (400 MHz, DMSO-*d₆*), characteristic peaks: δ 7.36 (br d, *J*=8 Hz, 1H), 6.74 (d, *J*=8.0 Hz, 1H), 4.82 (t, *J*=5.4 Hz, 1H), 4.27 (d, *J*=10.8 Hz, 1H), 3.78-3.86 (m, 2H), 3.59 (dd, *J*=11.5, 5.1 Hz, 1H), 1.68-1.74 (m, 1H), 1.01 (dd, *J*=8.5, 4.2 Hz, 1H), 0.85 (dd, *J*=5, 5 Hz, 1H).

### Step 5. Synthesis of [6-(prop-1-en-2-yl)-1a,2-dihydrocyclopropa[c]chromen-7b(1H)-yl]methanol (C17).

A solution of **C16** (1.0 g, 3.3 mmol) and potassium carbonate (915 mg, 6.62 mmol) in a mixture of tetrahydrofuran and water (7:3, 10 mL) was purged with nitrogen for 5 minutes. 2-Bromoprop-1-ene (0.48 g, 4.0 mmol) and dichlorobis(triphenylphosphine)palladium(II) (23 mg, 33 µmol) were introduced, and the reaction mixture was stirred at room temperature for 16 hours. It was then extracted with ethyl acetate, and the combined organic layers were washed with water, washed with brine, dried over sodium sulfate, filtered, and concentrated *in vacuo.* Silica gel chromatography (Eluent: 10% ethyl acetate in hexanes) provided the product as a yellow gum. Yield: 240 mg, 1.11 mmol, 34%. GCMS *m*/*z* 216 (M⁺). ¹H NMR (400 MHz, CDCl₃) δ 7.69 (d, *J*=2.1 Hz, 1H), 7.22 (dd, *J*=8.4, 2.3 Hz, 1H), 6.81 (d, *J*=8.3 Hz, 1H), 5.31 (br s, 1H), 5.01-5.04 (m, 1H), 4.32 (br d, *J*=10.5 Hz, 1H), 4.16 (br d, *J*=12 Hz, 1H), 3.94 (br d, *J*=10.5 Hz, 1H), 3.76 (dd, *J*=12, 6 Hz, 1H), 2.16 (br s, 3H), 1.67-1.74 (m, 1H), 1.22 (dd, *J*=5, 5 Hz, 1H), 1.05 (dd, *J*=8.4, 5.0 Hz, 1H).

### Step 6. Synthesis of [6-(1-methylcyclopropyl)-1a,2-dihydrocyclopropa[c]chromen-7b(1H)-yl]methanol (C18).

Palladium(II) acetate (254 mg, 1.13 mmol) and a freshly prepared solution of diazomethane in diethyl ether (50 mL) were added to a 0 °C solution of **C17** (700 mg, 3.24 mmol) in diethyl ether (20 mL), and the reaction mixture was stirred at room temperature for 1 hour. It was then filtered through a pad of diatomaceous earth, diluted with ethyl acetate, and washed sequentially with water and with brine. The solution was dried over sodium sulfate, filtered, and concentrated *in vacuo*; silica gel chromatography (Eluent: 10% ethyl acetate in hexanes) afforded the product as a colorless gum. Yield: 450 mg, 1.95 mmol, 60%. GCMS *m*/*z* 230 (M⁺). ¹H NMR (400 MHz, CDCl₃) δ 7.48 (d, *J*=2.0 Hz, 1H), 7.02 (dd, *J*=8.3, 2.1 Hz, 1H), 6.77 (d, *J*=8.3 Hz, 1H), 4.28 (d, *J*=10.5 Hz, 1H), 4.14 (d, *J*=11.6 Hz, 1H), 3.90 (d, *J*=10.5 Hz, 1H), 3.72 (d, *J*=11.6 Hz, 1H), 1.63-1.71 (m, 1H), 1.40 (s, 3H), 1.21 (dd, *J*=5.3, 4.9 Hz, 1H), 1.01 (dd, *J*=8.4, 4.8 Hz, 1H), 0.81-0.87 (m, 2H), 0.66-0.73 (m, 2H).

### Step 7. Synthesis of 6-(1-methylcyclopropyl)-1a,2-dihydrocyclopropa[c]chromene-7b(1H)-carbaldehyde (C19).

Dess-Martin periodinane (2.77 g, 6.53 mmol) was added to a 0 °C solution of **C18** (500 mg, 2.17 mmol) in dichloromethane (15 mL) and the reaction mixture was stirred at room temperature for 3 hours. It was then filtered through a pad of diatomaceous earth; the filtrate was washed with water and with brine, dried over sodium sulfate, filtered, and concentrated *in vacuo.* Chromatography on silica gel (Eluent: 5% ethyl acetate in hexanes) provided the product as a greenish gum. Yield: 300 mg, 1.31 mmol, 60%. GCMS *m*/*z* 228 (M⁺). ¹H NMR (400 MHz, CDCl₃) δ 9.72 (s, 1H), 7.72 (d, *J*=2.1 Hz, 1H), 7.08 (dd, *J*=8.5, 2.0 Hz, 1H), 6.81 (d, *J*=8.3 Hz, 1H), 4.31 (br d, *J*=11.4 Hz, 1H), 4.01 (br d, *J*=11 Hz, 1H), 2.16-2.24 (m, 1H), 1.96 (dd, *J*=8.9, 4.6 Hz, 1H), 1.64 (dd, *J*=6.5, 4.8 Hz, 1H), 1.41 (s, 3H), 0.81-0.87 (m, 2H), 0.68-0.74 (m, 2H).

### Step 8. Synthesis of 2-({[6-(1-methylcyclopropyl)-1a,2-dihydrocyclopropa[c]chromen-7b(1H)-yl]methyl}amino)ethanol (C20).

2-Aminoethanol (79 µL, 1.3 mmol) and magnesium sulfate (2.64 g, 21.9 mmol) were added to a solution of **C19** (250 mg, 1.10 mmol) in 1,2-dichloroethane (20 mL), and the reaction mixture was stirred at room temperature for 16 hours. The reaction mixture was filtered through a pad of diatomaceous earth, and the filtrate was concentrated under reduced pressure. The residue was dissolved in methanol (10 mL), cooled to 0 °C, and treated with sodium borohydride (46 mg, 1.2 mmol). After being stirred at room temperature for 1 hour, the reaction mixture was quenched with ice water and concentrated *in vacuo.* The residue was diluted with water and extracted with dichloromethane; the combined organic layers were washed with water and with brine, dried over sodium sulfate, filtered, and concentrated under reduced pressure. Silica gel chromatography (Eluent: 5% methanol in dichloromethane) afforded the product as an off-white solid. Yield: 170 mg, 0.62 mmol, 56%. LCMS *m*/*z* 274.0 [M+H]+. ¹H NMR (400 MHz, DMSO-*d₆*) δ 7.39 (d, *J*=2.0 Hz, 1H), 6.87 (dd, *J*=8.2, 2.1 Hz, 1H), 6.63 (d, *J*=8.3 Hz, 1H), 4.42-4.52 (br s, 1H), 4.20 (d, *J*=10.8 Hz, 1H), 3.75 (d, *J*=10.3 Hz, 1H), 3.41-3.51 (m, 2H), 3.25 (d, *J*=12.5 Hz, 1H), 2.60-2.68 (m, 2H), 2.57 (d, *J*=12.4 Hz, 1H), 1.62-1.70 (m, 1H), 1.33 (s, 3H), 0.94 (dd, *J*=8.2, 4.0 Hz, 1H), 0.86 (dd, *J*=5.3, 4.6 Hz, 1H), 0.71-0.80 (m, 2H), 0.63-0.71 (m, 2H).

### Step 9. Synthesis of rel-2-{[(1aS,7bS)-6-(1-methylcyclopropyl)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (5).

To a solution of 5-(4-methyl-1*H*-imidazol-1-yl)-6-oxo-1,6-dihydropyridine-2-carboxylic acid, hydrochloride salt (**C21**, 66 mg, 0.26 mmol) in acetonitrile (10 mL) were added *O*-(7-azabenzotriazol-1-yl)-*N,N,N',N'*-tetramethyluronium hexafluorophosphate (HATU, 224 mg, 0.589 mmol), cesium carbonate (208 mg, 0.638 mmol) and **C20** (70 mg, 0.26 mmol). The reaction mixture was heated at 50 °C for 16 hours, whereupon it was concentrated *in vacuo.* The residue was partitioned between water and ethyl acetate, and the organic layer was washed sequentially with aqueous sodium bicarbonate solution and with brine, dried over sodium sulfate, filtered, and concentrated under reduced pressure. Purification via reversed phase HPLC (Column: YMC-Actus Triart C18, 5 µm; Mobile phase A: 20 mM ammonium bicarbonate in water; Mobile phase B: acetonitrile; Gradient: 10% to 100% B) provided the product as an off-white solid. Yield: 22 mg, 48 µmol, 18%. LCMS *m*/*z* 457.0 [M+H]+. ¹H NMR (400 MHz, DMSO-*d₆*) δ 8.20 (s, 1H), 7.79 (d, *J*=7.8 Hz, 1H), 7.36 (br s, 1H), 7.25 (br s, 1H), 7.08 (d, *J*=7.7 Hz, 1H), 6.88 (br d, *J*=8.3 Hz, 1H), 6.65 (d, *J*=8.2 Hz, 1H), 5.18 (d, *J*=14.6 Hz, 1H), 4.22-4.33 (m, 2H), 3.89-3.99 (m, 2H), 3.72-3.81 (m, 1H), 3.43-3.53 (m, 1H), 2.81 (d, *J*=14.6 Hz, 1H), 2.13 (s, 3H), 1.99-2.06 (m, 1H), 1.27 (s, 3H), 1.05 (dd, J=8.3, 5.0 Hz, 1H), 0.94 (dd, J=5, 5 Hz, 1H), 0.74-0.79 (m, 1H), 0.57-0.66 (m, 3H).

### Example 6

### 2-{[2,2-Dimethyl-6-(trifluoromethyl)-2H-chromen-4-yl]methyl}-7-(4-methyl-1H-imidazol-1-yl)-3,4-dihydro-2H-pyrido[1,2-a]pyrazine-1,6-dione (6)

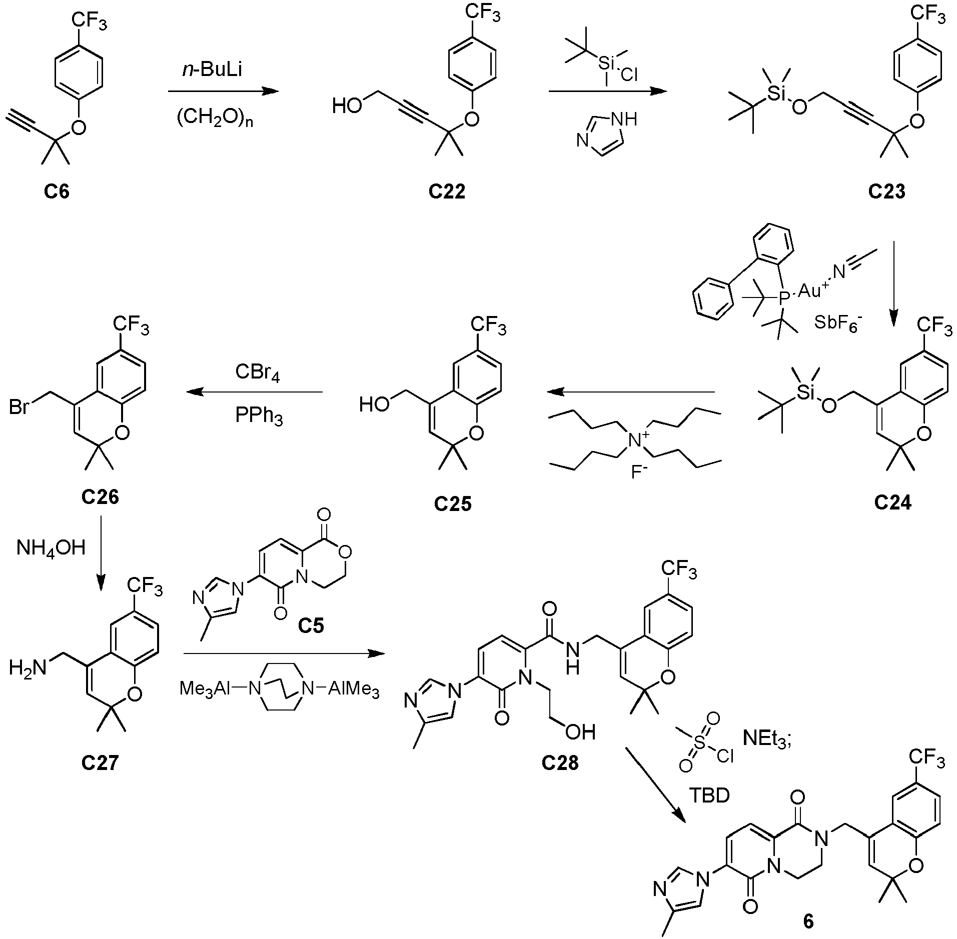

### Step 1. Synthesis of 4-methyl-4-[4-(trifluoromethyl)phenoxy]pent-2-yn-1-ol (C22).

A solution of **C6** (7.40 g, 32.4 mmol) in tetrahydrofuran (125 mL) was cooled to -78 °C and treated drop-wise with *n*-butyllithium (2.5 M solution in hexanes, 15.7 mL, 39.2 mmol) over 20 minutes. After the reaction mixture had stirred for 15 minutes at -78 °C, paraformaldehyde (1.46 g, 48.6 mmol) was added portion-wise, and the reaction mixture was allowed to warm to room temperature over 16 hours. It was then quenched by addition of aqueous ammonium chloride solution, and the resulting mixture was extracted three times with diethyl ether. The combined organic layers were washed with brine, dried over sodium sulfate, filtered, and concentrated *in vacuo.* Silica gel chromatography (Gradient: 0% to 50% ethyl acetate in heptane) afforded the product as a light yellow oil. Yield: 5.44 g, 21.1 mmol, 65%. ¹H NMR (400 MHz, CDCl₃) δ 7.54 (d, *J*=8.7 Hz, 2H), 7.27 (d, *J*=8.6 Hz, 2H), 4.32 (s, 2H), 1.69 (s, 6H).

### Step 2. Synthesis of tert-butyl(dimethyl)({4-methyl-4-[4-(trifluoromethyl)phenoxy]pent-2-yn-1-yl}oxy)silane (C23).

1*H*-Imidazole (99%, 2.34 g, 34.0 mmol) was added to a 0 °C solution of **C22** (5.86 g, 22.7 mmol) in dichloromethane (90 mL). *tert*-Butyl(dimethyl)silyl chloride (5.13 g, 34.0 mmol) was then added slowly, in a portion-wise manner, and the reaction mixture was allowed to stir at room temperature for 4 hours. Aqueous 1 M hydrochloric acid was added, and the aqueous layer was extracted twice with dichloromethane; the combined organic layers were dried over magnesium sulfate, filtered, and concentrated *in vacuo.* Chromatography on silica gel (Gradient: 0% to 10% ethyl acetate in heptane) provided the product as a pale yellow oil. Yield: 9.66 g, assumed quantitative; this material was used in the following step. ¹H NMR (400 MHz, CDCl₃) δ 7.52 (d, *J*=8.7 Hz, 2H), 7.28 (d, *J*=8.6 Hz, 2H, assumed; partially obscured by solvent peak), 4.35 (s, 2H), 1.68 (s, 6H), 0.90 (s, 9H), 0.09 (s, 6H).

### Step 3. Synthesis of tert-butyl{[2,2-dimethyl-6-(trifluoromethyl)-2H-chromen-4-yl]methoxy}dimethylsilane (C24).

(Acetonitrile)[(2-biphenyl)di-*tert*-butylphosphine]gold(I) hexafluoroantimonate (191 mg, 0.247 mmol) was added to a solution of **C23** (from the previous step, ≤22.7 mmol) in dichloromethane (100 mL), and the reaction mixture was stirred at room temperature for 18 hours. Additional gold catalyst (0.5 mol percent) was introduced, and stirring was continued for 24 hours, whereupon more catalyst (0.5 mol percent) was added, and stirring was maintained for a further 24 hours. Water (250 mL) was added, and the mixture was stirred for 15 minutes. The aqueous layer was extracted twice with dichloromethane, and the combined organic layers were dried over magnesium sulfate; they were then filtered through a pad of silica gel on top of a pad of diatomaceous earth. The filtrate was concentrated *in vacuo* to afford the product as a yellow oil. Yield: 8.24 g, 22.1 mmol, 97% over two steps. ¹H NMR (400 MHz, CDCl₃) δ 7.41 (br s, 1H), 7.38 (br d, *J*=8.5 Hz, 1H), 6.86 (d, *J*=8.4 Hz, 1H), 5.70-5.73 (m, 1H), 4.49 (d, *J*=1.5 Hz, 2H), 1.45 (s, 6H), 0.93 (s, 9H), 0.12 (s, 6H).

### Step 4. Synthesis of [2,2-dimethyl-6-(trifluoromethyl)-2H-chromen-4-yl]methanol (C25).

Tetrabutylammonium fluoride (1 M solution in tetrahydrofuran, 16.2 mL, 16.2 mmol) was added drop-wise to a 0 °C solution of **C24** (4.02 g, 10.8 mmol) in dichloromethane (43 mL), and the reaction mixture was stirred for 4 hours. Water was added, and the aqueous layer was extracted twice with dichloromethane; the combined organic layers were dried over sodium sulfate, filtered through a 1 inch plug of silica gel, and concentrated *in vacuo.* Chromatography on silica gel (Gradient: 0% to 50% ethyl acetate in heptane) provided the product as an off-white solid. Yield: 2.26 g, 8.75 mmol, 81%. ¹H NMR (400 MHz, CDCl₃) δ 7.45 (br s, 1H), 7.40 (br d, *J*=8.4 Hz, 1H), 6.88 (d, *J*=8.4 Hz, 1H), 5.77 (br s, 1H), 4.52 (br s, 2H), 1.46 (s, 6H).

### Step 5. Synthesis of 4-(bromomethyl)-2,2-dimethyl-6-(trifluoromethyl)-2H-chromene (C26).

Carbon tetrabromide (97%, 5.26 g, 15.0 mmol) was added to a 0 °C solution of **C25** (2.52 g, 9.76 mmol) in dichloromethane (50 mL). A solution of triphenylphosphine (98.5%, 4.06 g, 15.2 mmol) in dichloromethane was then added drop-wise over 15 minutes, and the reaction mixture was stirred at 0 °C for 15 minutes, then allowed to warm to room temperature. It was adsorbed onto diatomaceous earth and purified via silica gel chromatography (Gradient: 0% to 50% ethyl acetate in heptane) to afford the product as a colorless oil. Yield: 2.08 g, 6.48 mmol, 66%. ¹H NMR (400 MHz, CDCl₃) δ 7.56 (br d, *J*=1.7 Hz, 1H), 7.43 (br dd, *J*=8.6, 1.9 Hz, 1H), 6.89 (d, *J*=8.5 Hz, 1H), 5.87 (s, 1H), 4.25 (s, 2H), 1.46 (s, 6H).

### Step 6. Synthesis of 1-[2,2-dimethyl-6-(trifluoromethyl)-2H-chromen-4-yl]methanamine (C27).

Concentrated aqueous ammonium hydroxide solution (42 mL) was added to a solution of **C26** (2.08 g, 6.48 mmol) in 1,4-dioxane (42 mL), and the reaction mixture was heated at 50 °C for 16 hours. Water was added, and the mixture was extracted four times with dichloromethane; the combined organic layers were dried over magnesium sulfate, filtered, and concentrated *in vacuo.* Chromatography on silica gel (Gradient: 0% to 10% methanol in dichloromethane) afforded the product as a colorless oil. Yield: 1.16 g, 4.51 mmol, 70%. ¹H NMR (400 MHz, CDCl₃) δ 7.37-7.41 (m, 2H), 6.88 (d, *J*=8.0 Hz, 1H), 5.70-5.72 (m, 1H), 3.70 (d, *J*=1.4 Hz, 2H), 1.45 (s, 6H), 1.31 (br s, 2H).

### Step 7. Synthesis of N-{[2,2-dimethyl-6-(trifluoromethyl)-2H-chromen-4-yl]methyl}-1-(2-hydroxyethyl)-5-(4-methyl-1H-imidazol-1-yl)-6-oxo-1,6-dihydropyridine-2-carboxamide (C28).

Bis(trimethylaluminum)-1,4-diazabicyclo[2.2.2]octane adduct (97%, 2.09 g, 7.91 mmol) was added to a solution of **C27** (1.36 g, 5.27 mmol) in tetrahydrofuran (50 mL), and the mixture was stirred for 5 minutes. Compound **C5** (1.42 g, 5.79 mmol) was introduced, and the reaction mixture was heated at 70 °C for 2 hours, then stirred at room temperature for 16 hours, whereupon it was cautiously poured into 1 M aqueous sodium hydroxide solution. After four extractions with dichloromethane, the combined organic layers were dried over magnesium sulfate, filtered, and concentrated *in vacuo* to provide the product as a foamy yellow solid, which was taken directly into the following reaction.. Yield: 2.43 g, 4.84 mmol, 92%. LCMS *m*/*z* 503.1 [M+H]+. ¹H NMR (400 MHz, CDCl₃) δ 9.35 (br t, *J*=5 Hz, 1H), 7.83 (br s, 1H), 7.53 (br s, 1H), 7.43 (br d, *J*=8.6 Hz, 1H), 7.11 (d, *J*=7.6 Hz, 1H), 6.93 (br s, 1H), 6.91 (d, *J*=8.6 Hz, 1H), 6.41 (d, *J*=7.6 Hz, 1H), 5.83 (s, 1H), 4.45 (d, *J*=5.1 Hz, 2H), 4.19-4.26 (m, 2H), 3.98-4.05 (m, 2H), 1.86 (s, 3H), 1.47 (s, 6H).

### Step 8. Synthesis of 2-{[2,2-dimethyl-6-(trifluoromethyl)-2H-chromen-4-yl]methyl}-7-(4-methyl-1H-imidazol-1-yl)-3,4-dihydro-2H-pyrido[1,2-a]pyrazine-1,6-dione(6).

Triethylamine (1.19 mL, 8.54 mmol) was added to a suspension of **C28** (from the previous step, 2.43 g, 4.84 mmol) in tetrahydrofuran (40 mL), and the mixture was cooled to -20 °C. A solution of methanesulfonyl chloride (98%, 0.575 mL, 7.25 mmol) in tetrahydrofuran (10 mL) was added drop-wise over 15 minutes, and the reaction mixture was stirred for 10 minutes at -20 °C, whereupon it was allowed to warm to room temperature. 1,3,4,6,7,8-Hexahydro-2*H*-pyrimido[1,2-*a*]pyrimidine (95%, 1.98 g, 13.5 mmol) was added and stirring was continued. After 3.5 hours, water was added, and the mixture was extracted four times with dichloromethane. The combined organic layers were dried over magnesium sulfate, filtered, and concentrated *in vacuo.* Chromatography on silica gel (Gradient: 0% to 10% methanol in dichloromethane) provided incomplete purification; the fractions were recombined, dissolved in tetrahydrofuran, treated with 1,3,4,6,7,8-hexahydro-2*H*-pyrimido[1,2-*a*]pyrimidine (95%, 1.98 g, 13.5 mmol) and allowed to stir for 16 hours. After dilution with water, the mixture was extracted four times with dichloromethane. The combined organic layers were washed with water, dried over magnesium sulfate, filtered, and concentrated under reduced pressure. The residue was triturated with ethyl acetate and heptane and filtered to afford a yellow solid (760 mg). This was purified via silica gel chromatography (Gradient: 0% to 10% methanol in dichloromethane), then slurried in ethyl acetate and heptane to afford the product as an off-white powder (668 mg). The filtrate from the trituration was concentrated *in vacuo* and recrystallized from ethyl acetate / heptane, then subjected to silica gel chromatography (Gradient: 0% to 10% methanol in dichloromethane) and a similar slurry in ethyl acetate and heptane, providing the product as a yellow solid (527 mg). Combined yield: 1.20 g, 2.48 mmol, 51%. LCMS *m*/*z* 485.3 [M+H]+. ¹H NMR (400 MHz, CDCl₃) δ 8.31 (br s, 1H), 7.52 (br d, *J*=2 Hz, 1H), 7.48 (d, *J*=7.7 Hz, 1H), 7.41 (br dd, *J*=8.5, 2 Hz, 1H), 7.34 (d, *J*=7.7 Hz, 1H), 7.12-7.14 (m, 1H), 6.91 (br d, *J*=8.6 Hz, 1H), 5.74 (br s, 1H), 4.62 (d, *J*=1.0 Hz, 2H), 4.25-4.29 (m, 2H), 3.52-3.57 (m, 2H), 2.31 (d, *J*=1.0 Hz, 3H), 1.49 (s, 6H).

### Example 7

### (3S)-2-{[2,2-Dimethyl-6-(trifluoromethyl)-2H-chromen-4-yl]methyl}-3-methyl-7-(4-methyl-1H-imidazol-1-yl)-3,4-dihydro-2H-pyrido[1,2-a]pyrazine-1,6-dione (7)

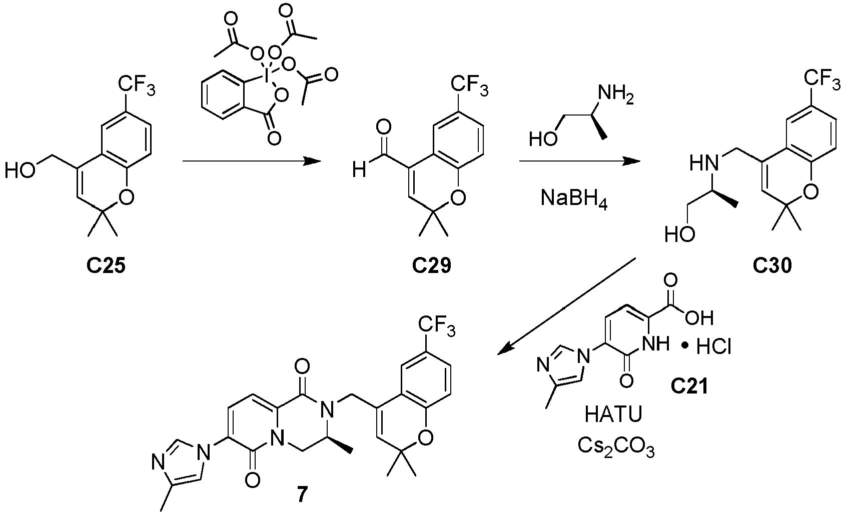

### Step 1. Synthesis of 2,2-dimethyl-6-(trifluoromethyl)-2H-chromene-4-carbaldehyde (C29).

To a solution of **C25** (2.2 g, 8.5 mmol) in dichloromethane (30 mL) was added Dess-Martin periodinane (9.03 g, 21.3 mmol) in two portion at 0 °C, and the reaction mixture was stirred at 0 °C for 2 hours. The reaction mixture was filtered through a pad of diatomaceous earth and the pad was washed with dichloromethane; the combined filtrates were dried over sodium sulfate, filtered, and concentrated *in vacuo.* Silica gel chromatography (Eluent: 15% ethyl acetate in hexanes) afforded the product as a white solid. Yield: 1.98 g, 7.73 mmol, 91%. GCMS *m*/*z* 256 (M⁺). ¹H NMR (400 MHz, CDCl₃) δ 9.68 (s, 1H), 8.54 (br s, 1H), 7.48 (br dd, *J*=8.6, 2 Hz, 1H), 6.94 (d, *J*=8.6 Hz, 1H), 6.53 (s, 1H), 1.57 (s, 6H).

### Step 2. Synthesis of (2S)-2-({[2,2-dimethyl-6-(trifluoromethyl)-2H-chromen-4-yl]methyl}amino)propan-1-ol (C30).

To a 0 °C solution of **C29** (200 mg, 0.781 mmol) in 1,2-dichloroethane (10 mL) was added (2S)-2-aminopropan-1-ol (0.122 mL, 1.57 mmol), and the reaction mixture was stirred at room temperature for 16 hours. It was then cooled to 0 °C, treated with sodium borohydride (59.4 mg, 1.57 mmol), and stirred at room temperature for 14 hours. Additional sodium borohydride (2 equivalents) was added at 0 °C and the reaction mixture was allowed to stir at room temperature for 64 hours, whereupon it was partitioned between dichloromethane and water. The aqueous layer was extracted with dichloromethane, and the combined organic layers were washed with brine, dried over sodium sulfate, filtered, and concentrated *in vacuo.* Chromatography on silica gel (Eluent: 50% ethyl acetate in hexanes) provided the product as a sticky yellow solid. Yield: 50 mg, 0.16 mmol, 20%. LCMS *m*/*z* 316.0 [M+H]⁺. ¹H NMR (400 MHz, CDCl₃) δ 7.48 (br s, 1H), 7.38 (br d, *J*=8.3 Hz, 1H), 6.87 (d, *J*=8.3 Hz, 1H), 5.71 (s, 1H), 3.71 (br d, *J*=14 Hz, 1H), 3.64 (dd, *J*=10.6, 4.0 Hz, 1H), 3.53 (br d, *J*=14 Hz, 1H), 3.32 (dd, *J*=10.6, 7.3 Hz, 1H), 2.84-2.94 (m, 1H), 1.45 (s, 6H), 1.13 (d, *J*=6.4 Hz, 3H).

### Step 3. Synthesis of (3S)-2-{[2,2-dimethyl-6-(trifluoromethyl)-2H-chromen-4-yl]methyl}-3-methyl-7-(4-methyl-1H-imidazol-1-yl)-3,4-dihydro-2H-pyrido[1,2-a]pyrazine-1,6-dione (7).

A mixture of **C30** (50 mg, 0.16 mmol) and **C21** (60.8 mg, 0.238 mmol) in acetonitrile (5 mL) was treated with *O*-(7-azabenzotriazol-1-yl)-*N*,*N*,*N'*,*N'-*tetramethyluronium hexafluorophosphate (HATU, 151 mg, 0.397 mmol) followed by cesium carbonate (155 mg, 0.476 mmol). The reaction mixture was heated at 55 °C for 14 hours, whereupon it was concentrated to dryness and partitioned between ethyl acetate and water. After extraction of the aqueous layer with ethyl acetate, the combined organic layers were washed with water, dried, filtered, and concentrated *in vacuo.* Silica gel chromatography, followed by reversed phase HPLC (Column: Waters Xterra RP18, 10 µm; Mobile phase A: 20 mM aqueous ammonium bicarbonate; Mobile phase B: acetonitrile; Gradient: 10% to 100% B), afforded the product as a white solid. Yield: 10 mg, 20 µmol, 12%. LCMS *m*/*z* 499.0 [M+H]⁺. ¹H NMR (400 MHz, DMSO-*d₆*) δ 8.33 (br s, 1H), 7.83 (d, *J*=7.8 Hz, 1H), 7.66 (br s, 1H), 7.50 (br d, *J*=8 Hz, 1H), 7.44 (s, 1H), 7.13 (d, *J*=7.8 Hz, 1H), 6.97 (d, *J*=8.3 Hz, 1H), 5.96 (s, 1H), 4.72 (br d, *J*=15 Hz, 1H), 4.65 (br d, *J*=14 Hz, 1H), 4.38 (br d, *J*=15 Hz, 1H), 3.85-3.98 (m, 2H), 2.16 (s, 3H), 1.43 (s, 3H), 1.42 (s, 3H), 1.09 (d, *J*=6.4 Hz, 3H).

### Example 8

### (3R)-2-{[2,2-Dimethyl-6-(trifluoromethyl)-2H-chromen-4-yl]methyl}-3-methyl-7-(4-methyl-1H-imidazol-1-yl)-3,4-dihydro-2H-pyrido[1,2-a]pyrazine-1,6-dione (8)

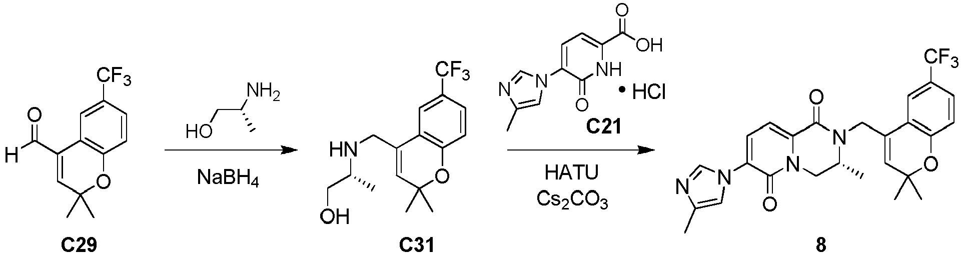

### Step 1. Synthesis of (2R)-2-({[2,2-dimethyl-6-(trifluoromethyl)-2H-chromen-4-yl]methyl}amino)propan-1-ol (C31).

To a 0 °C solution of **C29** (500 mg, 1.95 mmol) in 1,2-dichloroethane (10 mL) was added (2*R*)-2-aminopropan-1-ol (0.38 mL, 4.8 mmol) and the reaction mixture was stirred at room temperature for 16 hours. It was then cooled to 0 °C and treated with sodium borohydride (186 mg, 4.92 mmol), whereupon it was stirred at room temperature for 14 hours and partitioned between dichloromethane and water. The aqueous layer was extracted with dichloromethane, and the combined organic layers were washed with brine, dried over sodium sulfate, filtered, and concentrated *in vacuo.* Silica gel chromatography (Gradient: 5% to 10% methanol in dichloromethane) provided the product as a yellow solid. Yield: 100 mg, 0.317 mmol, 16%. LCMS *m*/*z* 316.6 [M+H]⁺. ¹H NMR (400 MHz, DMSO-*d₆*) δ 7.69 (br s, 1H), 7.45 (br d, *J*=8 Hz, 1H), 6.92 (d, *J*=8.3 Hz, 1H), 5.84 (s, 1H), 4.54 (dd, *J*=5.4, 5.1 Hz, 1H), 3.53 (br AB quartet, *J*_{AB}=14 Hz, Δν_{AB}=42 Hz, 2H), 3.22-3.3 (m, 2H, assumed; partially obscured by water peak), 2.62-2.72 (m, 1H), 1.39 (s, 6H), 0.97 (d, *J*=6.4 Hz, 3H).

### Step 2. Synthesis of (3R)-2-{[2,2-dimethyl-6-(trifluoromethyl)-2H-chromen-4-yl]methyl}-3-methyl-7-(4-methyl-1H-imidazol-1-yl)-3,4-dihydro-2H-pyrido[1,2-a]pyrazine-1,6-dione (8).

The product, obtained as a white solid, was synthesized from **C31** according to the method described for synthesis of **7** in Example 7. Yield: 50 mg, 0.10 mmol, 32%. LCMS *m*/*z* 499.4 [M+H]⁺. ¹H NMR (400 MHz, DMSO-*d₆*) δ 8.26 (d, *J*=1.2 Hz, 1H), 7.82 (d, *J*=7.6 Hz, 1H), 7.66 (br s, 1H), 7.50 (br d, *J*=8 Hz, 1H), 7.41-7.43 (m, 1H), 7.13 (d, *J*=7.7 Hz, 1H), 6.97 (br d, *J*=8.4 Hz, 1H), 5.96 (br s, 1H), 4.72 (br d, *J*=16 Hz, 1H), 4.65 (br dd, *J*=14, 2 Hz, 1H), 4.37 (br d, *J*=16 Hz, 1H), 3.85-3.99 (m, 2H), 2.15 (s, 3H), 1.43 (s, 3H), 1.42 (s, 3H), 1.09 (d, *J*=6.6 Hz, 3H).

### Examples 9, 10 and 11

### rel-2-{[(1aS,7bS)-2,2-Dimethyl-6-(trifluoromethoxy)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (9), 2-{[(1aS,7bS)-2,2-Dimethyl-6-(trifluoromethoxy)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (10), and 2-{[(1aR,7bR)-2,2-Dimethyl-6-(trifluoromethoxy)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (11)

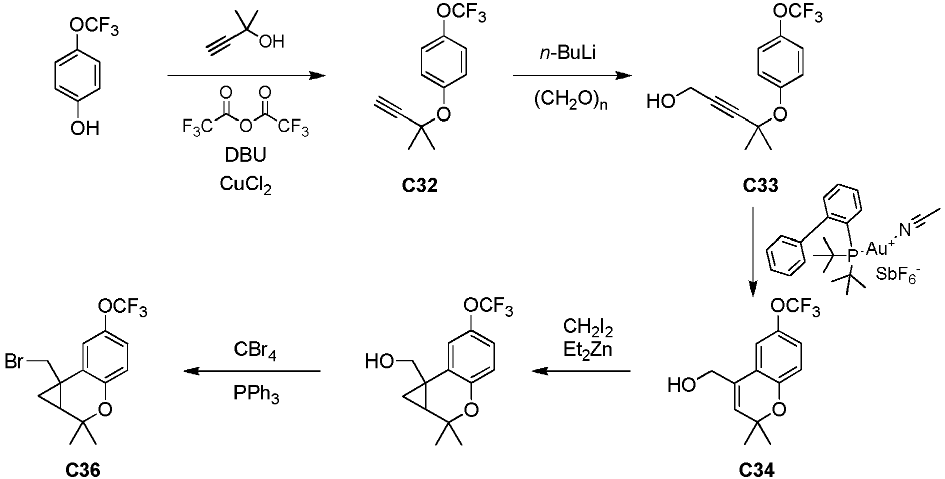

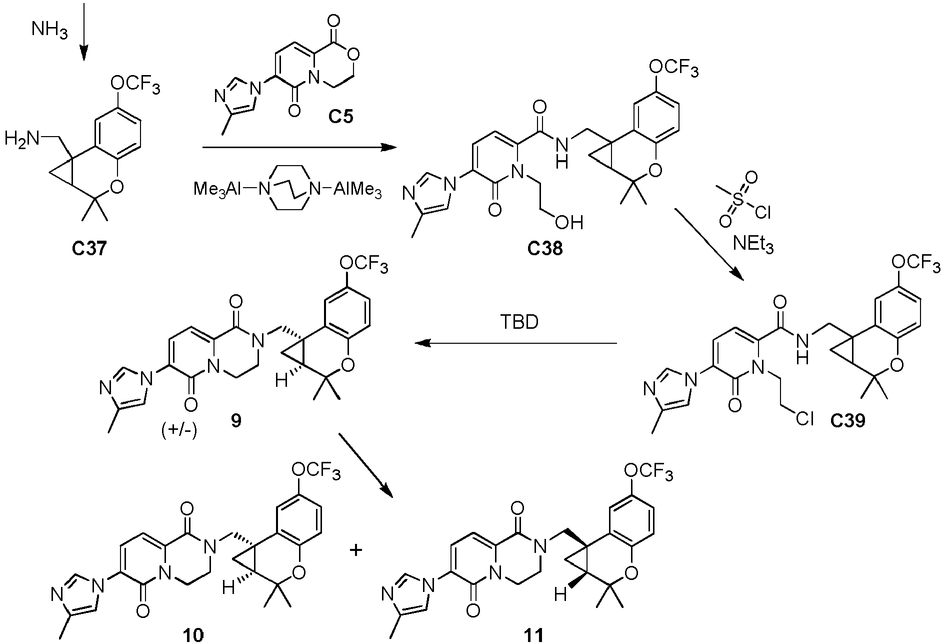

### Step 1. Synthesis of 1-[(2-methylbut-3-yn-2-yl)oxyl-4-(trifluoromethoxy)benzene (C32).

4-(Trifluoromethoxy)phenol was converted to the product according to the method described for synthesis of **C1** in Example 1. The product was obtained as a colorless liquid. Yield: 7.5 g, 30.7 mmol, 68%. GCMS *m*/*z* 244 (M⁺). ¹H NMR (400 MHz, CDCl₃) δ 7.22 (br d, half of AB quartet, *J*=9.3 Hz, 2H), 7.13 (br d, half of AB quartet, *J*=9 Hz, 2H), 2.59 (s, 1H), 1.65 (s, 6H).

### Step 2. Synthesis of 4-methyl-4-[4-(trifluoromethoxy)phenoxy]pent-2-yn-1-ol (C33).

To a -78 °C solution of **C32** (7.15 gm, 29.3 mmol) in tetrahydrofuran (70 mL) was added n-butyllithium (2.24 M in hexanes, 19.6 mL, 43.9 mmol) drop-wise. After the reaction mixture had stirred at -78 °C for 30 minutes, paraformaldehyde (1.92 g, 63.9 mmol) was added. The cooling bath was removed and the reaction mixture was allowed to warm to room temperature and stir for 2 hours, whereupon it was quenched with ice and extracted with diethyl ether. The combined organic layers were dried over sodium sulfate, filtered, and concentrated *in vacuo.* Silica gel chromatography (Gradient: 3% to 7% ethyl acetate in hexanes) afforded the product as an off-white solid. Yield: 6.0 g, 22 mmol, 75%. ¹H NMR (400 MHz, CDCl₃) δ 7.16 (AB quartet, *J*_{AB}=9.2 Hz, Δν_{AB}=22.4 Hz, 4H), 4.32 (d, *J*=6.2 Hz, 2H), 1.64 (s, 6H).

### Step 3. Synthesis of [2,2-dimethyl-6-(trifluoromethoxy)-2H-chromen-4-yl]methanol (C34).

Compound **C33** was converted to the product using the method described for synthesis of **C14** in Example 5. The product was obtained as an off-white solid. Yield: 3.5 g, 13 mmol, 71%. GCMS *m*/*z* 274 (M⁺). ¹H NMR (400 MHz, CDCl₃) δ 7.08 (br d, *J*=2 Hz, 1H), 7.00 (br d, *J*=9 Hz, 1H), 6.81 (d, *J*=8.7 Hz, 1H), 5.76 (br s, 1H), 4.48 (dd, *J*=5.8, 1.1 Hz, 2H), 1.55 (t, *J*=5.9 Hz, 1H), 1.45 (s, 6H).

### Step 4. Synthesis of [2,2-dimethyl-6-(trifluoromethoxy)-1a,2-dihydrocyclopropa[c]chromen-7b(1H)-yl]methanol (C35).

Diiodomethane (4.4 mL, 55 mmol) was added to a 0 °C solution of **C34** (2.5 g, 9.1 mmol) in dichloromethane (40 mL). After this mixture had stirred at 0 °C for 10 minutes, diethylzinc (1 M in solution in hexanes, 27.3 mL, 27.3 mmol) was added and the reaction mixture was allowed to warm to room temperature and stir for 4 hours. Additional dichloromethane was introduced, followed by aqueous sodium bisulfate solution, and the organic layer was washed with brine, dried over sodium sulfate, filtered, and concentrated under reduced pressure. Silica gel chromatography (Gradient: 15% to 20% ethyl acetate in hexanes) provided the product as an off-white solid. Yield: 1.9 g, 6.6 mmol, 72%. ¹H NMR (400 MHz, CDCl₃) δ 7.42 (br d, *J*=2.6 Hz, 1H), 6.94 (br d, *J*=8.7 Hz, 1H), 6.77 (d, *J*=8.7 Hz, 1H), 4.04 (dd, *J*=11.7, 4.5 Hz, 1H), 3.70 (dd, *J*=11.9, 6.6 Hz, 1H), 1.50-1.56 (m, 2H), 1.50 (s, 3H), 1.19 (s, 3H), 1.15 (dd, *J*=5.4, 5.3 Hz, 1 H), 1.02 (dd, *J*=8.6, 4.9 Hz, 1H).

### Step 5. Synthesis of 7b-(bromomethyl)-2,2-dimethyl-6-(trifluoromethoxy)-1,1a,2,7b-tetrahydrocyclopropa[c]chromene (C36).

To a 0 °C solution of **C35** (1.9 g, 6.6 mmol) in dichloromethane (20 mL) was added carbon tetrabromide (2.6 g, 7.8 mmol), followed by drop-wise addition of a solution of triphenylphosphine (2.0 g, 7.6 mmol) in dichloromethane. The reaction mixture was then allowed to warm to room temperature and stir for 16 hours. At this point, dichloromethane and water were added, and the organic layer was washed with aqueous sodium bicarbonate solution and with brine, dried over sodium sulfate, filtered, and concentrated *in vacuo.* Chromatography on silica gel (Gradient: 0% to 5% ethyl acetate in hexanes) afforded the product as a brown liquid. Yield: 1.4 g, 4.0 mmol, 61%. GCMS *m*/*z* 350, 352 (M⁺). ¹H NMR (400 MHz, CDCl₃) δ 7.26-7.30 (m, 1H, assumed; obscured by solvent peak), 6.97 (br d, *J*=9 Hz, 1H), 6.78 (d, *J*=8.8 Hz, 1H), 4.18 (d, *J*=11.0 Hz, 1H), 3.22 (d, *J*=11.2 Hz, 1H), 1.61 (dd, *J*=8.7, 6.2 Hz, 1H), 1.48 (s, 3H), 1.46-1.51 (m, 1H), 1.24 (s, 3H), 1.20-1.26 (m, 1H).

### Step 6. Synthesis of 1-[2,2-dimethyl-6-(trifluoromethoxy)-1a,2-dihydrocyclopropa[c]chromen-7b(1H)-yl]methanamine (C37).

A solution of **C36** (1.4 g, 4.0 mmol) in methanolic ammonia (25 mL) was heated in a sealed tube at 80 °C for 5 hours. The reaction mixture was then concentrated *in vacuo*; the residue was washed with pentane and dissolved in dichloromethane. After basification with aqueous sodium bicarbonate solution, the mixture was extracted with a solution of 5% methanol in dichloromethane. The combined organic layers were washed with water, dried over sodium sulfate, filtered, and concentrated under reduced pressure to afford the product as a brown liquid. Yield: 600 mg, 2.09 mmol, 52%. LCMS *m*/*z* 287.8 [M+H]⁺. ¹H NMR (400 MHz, DMSO-*d₆*) δ 7.48 (d, *J*=2.7 Hz, 1H), 7.00 (br d, *J*=9 Hz, 1H), 6.77 (d, *J*=8.8 Hz, 1H), 3.20 (d, *J*=13.4 Hz, 1H), 2.60 (d, *J*=13.2 Hz, 1H), 1.61 (dd, *J*=8.4, 5.5 Hz, 1H), 1.41 (s, 3H), 1.11 (s, 3H), 1.00 (dd, *J*=8.3, 4.4 Hz, 1H), 0.81 (dd, *J*=5.4, 4.6 Hz, 1H).

### Step 7. Synthesis of N-{[2,2-dimethyl-6-(trifluoromethoxy)-1a,2-dihydrocyclopropa[c]chromen-7b(1H)-yl]methyl}-1-(2-hydroxyethyl)-5-(4-methyl-1H-imidazol-1-yl)-6-oxo-1,6-dihydropyridine-2-carboxamide (C38).

Compound **C37** (600 mg, 2.09 mmol) was dissolved in tetrahydrofuran (6.0 mL), and bis(trimethylaluminum)-1,4-diazabicyclo[2.2.2]octane adduct (1.0 g, 3.9 mmol) was added. The reaction mixture was warmed to 40 °C for 45 minutes, whereupon **C5** (512 mg, 2.09 mmol) was introduced, and the reaction mixture was heated to 65 °C for 5 hours. Aqueous sodium hydroxide solution (1 M, 3 mL) was added, and the resulting slurry was diluted with water and extracted with a solution of 5% methanol in dichloromethane. The combined organic layers were washed with brine, dried over sodium sulfate, filtered, and concentrated under reduced pressure. The residue was triturated with pentane to afford the product as an off-white solid. Yield: 800 mg, 1.50 mmol, 72%. LCMS *m*/*z* 533.1 [M+H]⁺. ¹H NMR (400 MHz, DMSO-*d₆*) δ 9.11 (br t, *J*=6 Hz, 1H), 8.10 (d, *J*=1.1 Hz, 1H), 7.67 (d, *J*=7.5 Hz, 1H), 7.48-7.51 (m, 1H), 7.28-7.31 (m, 1H), 7.05 (br d, *J*=8.5 Hz, 1H), 6.81 (d, *J*=8.7 Hz, 1H), 6.18 (d, *J*=7.6 Hz, 1H), 4.88 (dd, *J*=5.6, 5.4 Hz, 1H), 4.16-4.28 (m, 2H), 4.13 (dd, *J*=14, 6.5 Hz, 1H), 3.53-3.6 (m, 2H), 3.21 (dd, *J*=14, 5 Hz, 1H), 2.13 (s, 3H), 1.88 (dd, *J*=8.3, 5.6 Hz, 1H), 1.43 (s, 3H), 1.17 (dd, *J*=8.4, 4.9 Hz, 1H), 1.14 (s, 3H), 0.95 (dd, *J*=5.5, 5.0 Hz, 1H).

### Step 8. Synthesis of 1-(2-chloroethyl)-N-{[2,2-dimethyl-6-(trifluoromethoxy)-1a,2-dihydrocyclopropa[c]chromen-7b(1H)-yl]methyl}-5-(4-methyl-1H-imidazol-1-yl)-6-oxo-1,6-dihydropyridine-2-carboxamide (C39).

To a -10 °C solution of **C38** (800 mg, 1.50 mmol) in dichloromethane (10 mL) was added triethylamine (524 µL, 3.76 mmol), followed by drop-wise addition of methanesulfonyl chloride (175 µL, 2.26 mmol). After 2 hours at room temperature, the reaction mixture was diluted with dichloromethane, washed with aqueous sodium bicarbonate solution and with brine, dried over sodium sulfate, filtered, and concentrated *in vacuo* to provide the crude product as a brown solid (800 mg). This material was taken directly into the following step. LCMS *m*/*z* 551.4, 553.4 [M+H]⁺.

### Step 9. Synthesis of rel-2-{[(1aS,7bS)-2,2-dimethyl-6-(trifluoromethoxy)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (9), 2-{[(1aS,7bS)-2,2-dimethyl-6-(trifluoromethoxy)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (10), and 2-{[(1aR,7bR)-2,2-dimethyl-6-(trifluoromethoxy)-1a,2-dihydrocyclopropa[c]chromen-7b(1H)-yl]methyl}-7-(4-methyl-1H-imidazol-1-yl)-3,4-dihydro-2H-pyrido[1,2-a]pyrazine-1,6-dione (11).

To a solution of **C39** (from the previous step, 800 mg, ≤1.5 mmol) in tetrahydrofuran (10 mL) was added 1,3,4,6,7,8-hexahydro-2*H*-pyrimido[1,2-*a*]pyrimidine (1.0 g, 7.2 mmol). The reaction mixture was allowed to stir at room temperature for 16 hours, whereupon it was concentrated *in vacuo,* diluted with water, and extracted with ethyl acetate. The combined organic layers were washed with brine, dried over sodium sulfate, filtered, and concentrated under reduced pressure. Preparative HPLC yielded the racemic material **9** as a white solid. Yield: 220 mg, 0.428 mmol, 29% over two steps. LCMS *m*/*z* 515.2 [M+H]⁺. ¹H NMR (400 MHz, DMSO-*d₆*) δ 8.22 (d, *J*=1.0 Hz, 1H), 7.79 (d, *J*=7.7 Hz, 1H), 7.51-7.54 (m, 1H), 7.38 (br s, 1H), 7.09 (d, *J*=7.7 Hz, 1H), 6.99-7.04 (m, 1H), 6.80 (d, *J*=8.7 Hz, 1H), 4.96 (d, *J*=14.4 Hz, 1H), 4.06-4.23 (m, 2H), 3.65-3.81 (m, 2H), 3.00 (d, *J*=14.9 Hz, 1H), 2.14 (s, 3H), 2.07 (dd, *J*=8, 6 Hz, 1H), 1.44 (s, 3H), 1.20 (s, 3H), 1.07 (dd, *J*=8.6, 4.8 Hz, 1H), 0.93 (dd, *J*=5, 5 Hz, 1H). The enantiomers were separated via chiral HPLC (Column: Chiral Technologies CHIRALPAK® IC, 5 µm; Mobile phase: 0.1% diethylamine in methanol). Example **10** was the first-eluting enantiomer, obtained as an off-white solid. Yield: 50 mg, 97 µmol, 23%. Example **11** was the second-eluting enantiomer, also isolated as an off-white solid. Yield: 50 mg, 97 µmol, 23%. The absolute configurations of these compounds were assigned in accordance with those of Examples **3** and **4**, using their relative biological activities (see Table 8).
**10**: LCMS *m*/*z* 515.2 [M+H]⁺. ¹H NMR (400 MHz, DMSO-*d₆*) δ 8.22 (br s, 1H), 7.79 (d, *J*=7.8 Hz, 1H), 7.51-7.54 (m, 1H), 7.38 (br s, 1H), 7.09 (d, *J*=7.7 Hz, 1H), 7.02 (br d, *J*=9 Hz, 1H), 6.80 (d, *J*=8.7 Hz, 1H), 4.96 (d, *J*=14.8 Hz, 1H), 4.06-4.23 (m, 2H), 3.65-3.81 (m, 2H), 3.00 (d, *J*=14.9 Hz, 1H), 2.14 (s, 3H), 2.08 (dd, *J*=8.6, 5.9 Hz, 1H), 1.44 (s, 3H), 1.20 (s, 3H), 1.07 (dd, *J*=8.6, 4.8 Hz, 1H), 0.93 (dd, *J*=5.6, 5.1 Hz, 1H). Retention time: 8.93 minutes (Column: Chiral Technologies CHIRALPAK® IC, 4.6 x 250 mm, 5 µm; Mobile phase: 0.1% diethylamine in methanol; Flow rate: 1.0 mL/minute).
**11:** LCMS *m*/*z* 515.2 [M+H]⁺. ¹H NMR (400 MHz, DMSO-*d₆*) δ 8.22 (br s, 1H), 7.79 (d, *J*=7.8 Hz, 1H), 7.51-7.54 (m, 1H), 7.38 (br s, 1H), 7.09 (d, *J*=7.8 Hz, 1H), 7.02 (br d, *J*=9 Hz, 1H), 6.80 (d, *J*=8.8 Hz, 1H), 4.96 (d, *J*=14.9 Hz, 1H), 4.06-4.23 (m, 2H), 3.65-3.81 (m, 2H), 3.01 (d, *J*=14.9 Hz, 1H), 2.14 (s, 3H), 2.04-2.10 (m, 1H), 1.44 (s, 3H), 1.21 (s, 3H), 1.07 (dd, *J*=8.7, 4.8 Hz, 1H), 0.93 (dd, *J*=5.4, 4.9 Hz, 1H). Retention time: 11.10 minutes, using HPLC conditions identical to those described for **10**.

### Example 12

### 7-(4-Methyl-1H-imidazol-1-yl)-2-{[6-(trifluoromethyl)spiro[chromene-2,1'-cyclobutan]-4-yl]methyl}-3,4-dihydro-2H-pyrido[1,2-a]pyrazine-1,6-dione (12)

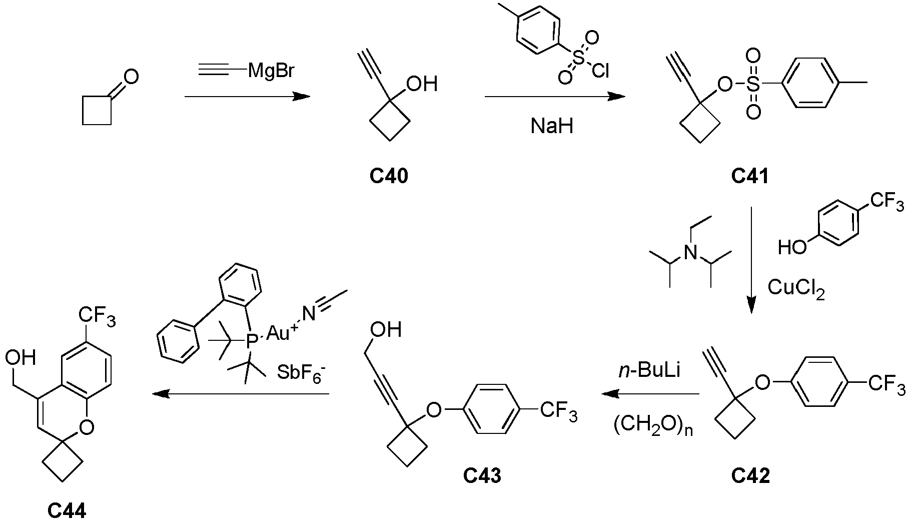

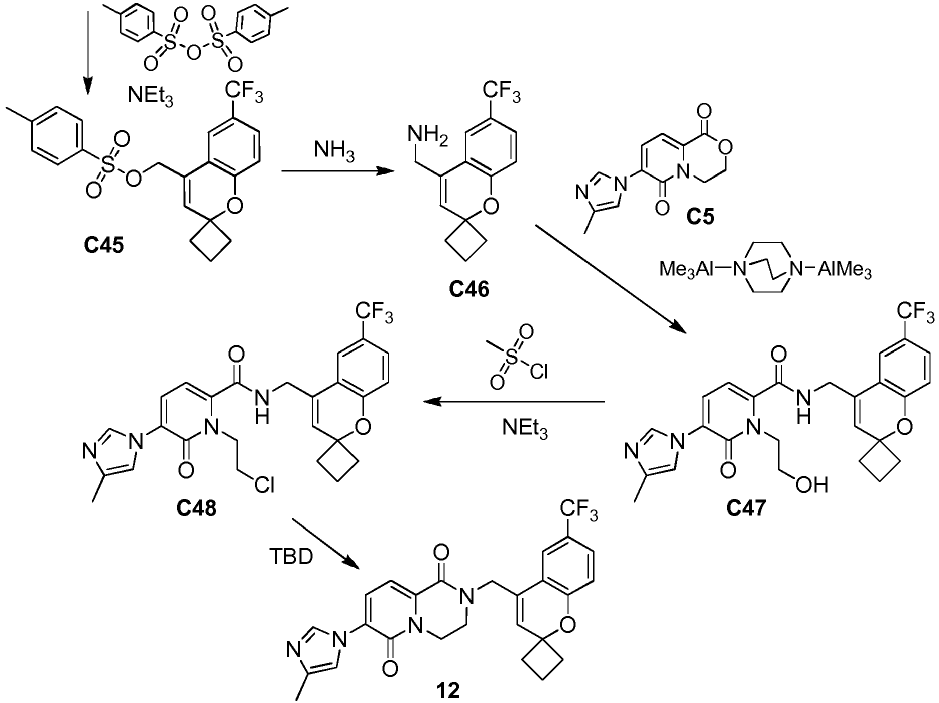

### Step 1. Synthesis of 1-ethynylcyclobutanol (C40).

Ethynylmagnesium bromide (0.5 M solution in tetrahydrofuran, 21.4 mL, 10.7 mmol) was added drop-wise over 5 minutes to a 0 °C solution of cyclobutanone (500 mg, 7.13 mmol) in tetrahydrofuran (20 mL). The reaction mixture was stirred for 1.5 hours at 0 °C, whereupon it was quenched with ice water (10 mL), and the resulting suspension was concentrated *in vacuo.* The residual syrup was diluted with water (5 mL), treated with a saturated aqueous solution of ammonium chloride, and extracted with ethyl acetate (3 x 15 mL). The combined organic layers were washed with brine (10 mL), dried over sodium sulfate, filtered, and concentrated under reduced pressure to afford the product as a brown liquid. Yield: 500 mg, 5.20 mmol, 73%. ¹H NMR (400 MHz, CDCl₃) δ 2.55 (s, 1H), 2.41-2.49 (m, 2H), 2.22-2.32 (m, 2H), 1.79-1.89 (m, 2H).

### Step 2. Synthesis of 1-ethynylcyclobutyl 4-methylbenzenesulfonate (C41).

To a 0 °C solution of **C40** (2.2 g, 23 mmol) and p-toluenesulfonyl chloride (4.36 g, 22.9 mmol) in tetrahydrofuran (100 mL) was added sodium hydride (60% in mineral oil, 1.09 g, 27.2 mmol). The reaction mixture was allowed to stir for 5 hours at room temperature, whereupon it was quenched with ice water (50 mL) and extracted with ethyl acetate (2 x 100 mL). The combined organic layers were washed with brine (50 mL), dried over sodium sulfate, filtered, and concentrated *in vacuo* to provide the product. Yield: 5.2 g, 21 mmol, 91%. ¹H NMR (400 MHz, CDCl₃), characteristic peaks: δ 7.83 (br d, *J*=8.3 Hz, 2H), 7.32 (br d, *J*=8.0 Hz, 2H), 2.65-2.76 (m, 2H), 2.45 (s, 3H).

### Step 3. Synthesis of 1-[(1-ethynylcyclobutyl)oxy]-4-(trifluoromethyl)benzene (C42).

Copper(II) chloride (20 mg, 150 µmol) was added to a 0 °C solution of **C41** (2.2 g, 8.8 mmol) and 4-(trifluoromethyl)phenol (1.56 g, 9.62 mmol) in acetonitrile (100 mL). A solution of *N*,*N*-diisopropylethylamine (1.53 mL, 8.78 mmol) in acetonitrile (20 mL) was then introduced drop-wise, and the reaction mixture was stirred for 12 hours while it was allowed to warm to room temperature. After removal of acetonitrile under reduced pressure, hexanes were added and the organic layer was washed sequentially with water, aqueous sodium hydroxide solution, dilute aqueous hydrochloric acid, aqueous sodium bicarbonate solution, and brine. It was then dried over sodium sulfate, filtered, and concentrated *in vacuo*; chromatography on silica gel (Eluent: hexanes) afforded the product as a yellow liquid. Yield: 1.1 g, 4.6 mmol, 52%. ¹H NMR (400 MHz, CDCl₃) δ 7.54 (br d, *J*=8.6 Hz, 2H), 7.03 (br d, *J*=8.3 Hz, 2H), 2.61-2.72 (m, 3H), 2.48-2.60 (m, 2H), 1.92-2.13 (m, 2H).

### Step 4. Synthesis of 3-{1-[4-(trifluoromethyl)phenoxy]cyclobutyl}prop-2-yn-1-ol (C43).

To a -78 °C solution of **C42** (1.1 g, 4.6 mmol) in tetrahydrofuran (20 mL) was added n-butyllithium (2.4 M solution in hexanes, 2.8 mL, 6.7 mmol). After 30 minutes, paraformaldehyde (0.288 g, 9.59 mmol) was added to the -78 °C reaction mixture, and it was allowed to warm to room temperature and stir for 2 hours. The reaction was then quenched with ice water (20 mL), diluted with saturated aqueous ammonium chloride solution (20 mL) and extracted with ethyl acetate (3 x 100 mL). The combined organic layers were washed with brine (20 mL), dried over sodium sulfate, filtered, and concentrated *in vacuo.* Silica gel chromatography (Gradient: 5% to 10% ethyl acetate in hexanes) provided the product as a yellow liquid. Yield: 0.88 g, 3.2 mmol, 70%. ¹H NMR (400 MHz, CDCl₃) δ 7.53 (d, *J*=8.6 Hz, 2H), 7.01 (d, *J*=8.4 Hz, 2H), 4.32 (d, *J*=6.2 Hz, 2H), 2.60-2.69 (m, 2H), 2.49-2.59 (m, 2H), 1.92-2.11 (m, 2H), 1.52 (t, *J*=6.3 Hz, 1H).

### Step 5. Synthesis of [6-(trifluoromethyl)spiro[chromene-2,1'-cyclobutan]-4-yl]methanol (C44).

(Acetonitrile)[(2-biphenyl)di-*tert*-butylphosphine]gold(I) hexafluoroantimonate (31 mg, 40 µmol) was added to a solution of **C43** (1.1 g, 4.1 mmol) in dichloromethane (25 mL). The reaction mixture was stirred for 5 hours at room temperature, whereupon it was diluted with ice water (30 mL) and extracted with dichloromethane (3 x 50 mL). The combined organic layers were washed with brine (20 mL), dried over sodium sulfate, filtered, and concentrated *in vacuo.* Silica gel chromatography (Gradient: 10% to 20% ethyl acetate in hexanes) afforded the product as a yellow liquid. Yield: 0.90 g, 3.3 mmol, 80%. GCMS *m*/*z* 270 (M⁺). ¹H NMR (400 MHz, CDCl₃) δ 7.42 (br s, 1H), 7.39 (br d, *J*=8.3, 1H), 6.90 (d, *J*=8.4 Hz, 1H), 6.13-6.15 (m, 1H), 4.54 (dd, *J*=5.8, 1.3 Hz, 2H), 2.45-2.56 (m, 2H), 2.22-2.30 (m, 2H), 1.84-1.95 (m, 1H), 1.66-1.79 (m, 1H), 1.58 (t, *J*=5.8 Hz, 1H, assumed; partially obscured by water peak).

### Step 6. Synthesis of [6-(trifluoromethyl)spiro[chromene-2,1'-cyclobutan]-4-yl]methyl 4-methylbenzenesulfonate (C45).

Triethylamine (0.155 mL, 1.11 mmol) was added to a 0 °C solution of **C44** (200 mg, 0.74 mmol) and p-toluenesulfonic anhydride (628 mg, 1.92 mmol) in dichloromethane (5 mL). After the reaction mixture had been stirred for 4 hours at room temperature, it was diluted with ice water (50 mL) and saturated aqueous sodium bicarbonate solution (10 mL). The resulting mixture was extracted with dichloromethane (3 x 20 mL), and the combined organic layers were washed with brine (10 mL), dried over sodium sulfate, filtered, and concentrated under reduced pressure; chromatography on silica gel (Gradient: 5% to 10% ethyl acetate in hexanes) afforded the product as an off-white solid. Yield: 200 mg, 0.47 mmol, 64%. ¹H NMR (400 MHz, CDCl₃) δ 7.80 (br d, *J*=8.3 Hz, 2H), 7.29-7.37 (m, 3H), 7.12 (br d, *J*=2 Hz, 1H), 6.82 (d, *J*=8.6 Hz, 1H), 6.11 (br s, 1H), 4.87 (d, *J*=0.9 Hz, 2H), 2.44 (s, 3H), 2.41-2.51 (m, 2H), 2.16-2.25 (m, 2H), 1.82-1.94 (m, 1H), 1.62-1.75 (m, 1H).

### Step 7. Synthesis of 1-[6-(trifluoromethyl)spiro[chromene-2,1'-cyclobutan]-4-yl]methanamine (C46).

Compound **C45** (250 mg, 0.589 mmol) and methanolic ammonia (5.0 mL) were combined in a sealed tube, and the reaction mixture was stirred at room temperature for 12 hours. Volatiles were removed *in vacuo,* and the residue was diluted with cold water (10 mL) and extracted with 10% methanol in dichloromethane (2 x 50 mL). The combined organic layers were washed with brine (10 mL), dried over sodium sulfate, filtered, and concentrated under reduced pressure. Silica gel chromatography (Gradient: 5% to 10% methanol in dichloromethane) provided the product as a yellow liquid. Yield: 130 mg, 0.483 mmol, 82%. ¹H NMR (400 MHz, CDCl₃) δ 7.34-7.41 (m, 2H), 6.90 (d, *J*=8 Hz, 1H), 6.08 (br s, 1H), 3.72 (br s, 2H), 2.43-2.55 (m, 2H), 2.19-2.29 (m, 2H), 1.81-1.95 (m, 1H), 1.6-1.79 (m, 1H, assumed; partially obscured by water peak).

### Step 8. Synthesis of 1-(2-hydroxyethyl)-5-(4-methyl-1H-imidazol-1-yl)-6-oxo-N-{[6-(trifluoromethyl)spiro[chromene-2,1'-cyclobutan]-4-yl]methyl}-1,6-dihydropyridine-2-carboxamide (C47).

To a solution of **C46** (150 mg, 0.557 mmol) in tetrahydrofuran (10 mL) was added bis(trimethylaluminum)-1,4-diazabicyclo[2.2.2]octane adduct (286 mg, 1.12 mmol) in portions. After completion of the addition, the reaction mixture was stirred for 1 hour at 70 °C, whereupon it was treated with **C5** (205 mg, 0.836 mmol) and stirring was continued at 70 °C for 12 hours. Solvent was removed *in vacuo,* and the residue was diluted with dichloromethane (50 mL). Water (10 mL) was slowly added to the room temperature mixture, followed by dichloromethane (10 mL), and the mixture was stirred for 30 minutes, then filtered. The aqueous layer of the filtrate was extracted with dichloromethane (3 x 10 mL), and the combined organic layers were dried over sodium sulfate, filtered, and concentrated under reduced pressure. Chromatography on silica gel (Eluent: 10:1 dichloromethane / methanol) afforded the product as a light yellow solid. Yield: 150 mg, 0.29 mmol, 52%. LCMS *m*/*z* 515.0 [M+H]+. ¹H NMR (400 MHz, CDCl₃) δ 9.14-9.21 (m, 1H), 7.83 (br s, 1H), 7.50 (br s, 1H), 7.42 (br d, *J*=8.5 Hz, 1H), 7.11 (d, *J*=7.6 Hz, 1H), 6.91-6.95 (m, 2H), 6.41 (d, *J*=7.7 Hz, 1H), 6.19 (s, 1H), 4.48 (d, *J*=5.1 Hz, 2H), 4.21-4.27 (m, 2H), 4.01-4.07 (m, 2H), 2.46-2.57 (m, 2H), 2.23-2.32 (m, 2H), 1.86-1.97 (m, 1H), 1.86 (s, 3H), 1.66-1.80 (m, 1H).

### Step 9. Synthesis of 1-(2-chloroethyl)-5-(4-methyl-1H-imidazol-1-yl)-6-oxo-N-{[6-(trifluoromethyl)spiro[chromene-2,1'-cyclobutan]-4-yl]methyl}-1,6-dihydropyridine-2-carboxamide (C48)

To a 0 °C solution of **C47** (148 mg, 0.288 mmol) in dichloromethane (10 mL) was added triethylamine (80 µL, 0.57 mmol) followed by methanesulfonyl chloride (27 µL, 0.35 mmol). After 3 hours at room temperature, the reaction mixture was diluted with ice water (10 mL) and extracted with dichloromethane (3 x 20 mL). The combined organic layers were washed with saturated aqueous sodium bicarbonate solution (10 mL) and with brine (10 mL), dried over sodium sulfate, filtered, and concentrated *in vacuo* to afford the product as a yellow liquid. This material was used in the next step without additional purification. Yield: 140 mg, 0.263 mmol, 91%. LCMS *m*/*z* 533.2 [M+H]⁺.

### Step 10. Synthesis of 7-(4-methyl-1H-imidazol-1-yl)-2-{[6-(trifluoromethyl)spiro[chromene-2,1'-cyclobutan]-4-yl]methyl}-3,4-dihydro-2H-pyrido[1,2-a]pyrazine-1,6-dione (12).

To a solution of **C48** (from the previous step, 140 mg, 0.263 mmol) in tetrahydrofuran (10 mL) was added 1,3,4,6,7,8-hexahydro-2*H*-pyrimido[1,2-*a*]pyrimidine (131 mg, 0.941 mmol). After 8 hours at room temperature, the reaction mixture was diluted with aqueous sodium hydroxide solution (1 M, 5 mL) and extracted with ethyl acetate (3 x 20 mL). The combined organic layers were washed with brine (10 mL), dried over sodium sulfate, filtered, and concentrated under reduced pressure. Silica gel chromatography (Eluent: 10% methanol in dichloromethane) was followed by further purification via reversed phase HPLC (Column: XTerra RP18, 10 µm; Mobile phase A: 5 mM aqueous ammonium acetate; Mobile phase B: acetonitrile; Gradient: 10% to 100% B) to afford the product as an off-white solid. Yield: 22.0 mg, 44.3 µmol, 17%. LCMS *m*/*z* 497.0 [M+H]⁺. ¹H NMR (400 MHz, CDCl₃) δ 8.21-8.31 (br s, 1H), 7.50 (br s, 1H), 7.47 (d, *J*=7.7 Hz, 1H), 7.40 (br d, *J*=8 Hz, 1H), 7.34 (d, *J*=7.7 Hz, 1H), 7.13 (br s, 1H), 6.93 (d, *J*=8.3 Hz, 1H), 6.10 (s, 1H), 4.64 (s, 2H), 4.24-4.30 (m, 2H), 3.53-3.59 (m, 2H), 2.49-2.59 (m, 2H), 2.29 (s, 3H), 2.23-2.31 (m, 2H), 1.88-2.00 (m, 1H), 1.7-1.79 (m, 1H, assumed; partially obscured by water peak).

### Examples 13, 14 and 15

### 2-{1-[2,2-dimethyl-6-(trifluoromethoxy)-2H-chromen-4-yl]ethyl}-7-(4-methyl-1H-imidazol-1-yl)-3,4-dihydro-2H-pyrido[1,2-a]pyrazine-1,6-dione (13), 2-{(1S)-1-[2,2-Dimethyl-6-(trifluoromethoxy)-2H-chromen-4-yl]ethyl}-7-(4-methyl-1H-imidazol-1-yl)-3,4-dihydro-2H-pyrido[1,2-a]pyrazine-1,6-dione (14), and 2-[(1R)-1-[2,2-Dimethyl-6-(trifluoromethoxy)-2H-chromen-4-yl]ethyl}-7-(4-methyl-1H-imidazol-1-yl)-3,4-dihydro-2H-pyrido[1,2-a]pyrazine-1,6-dione (15)

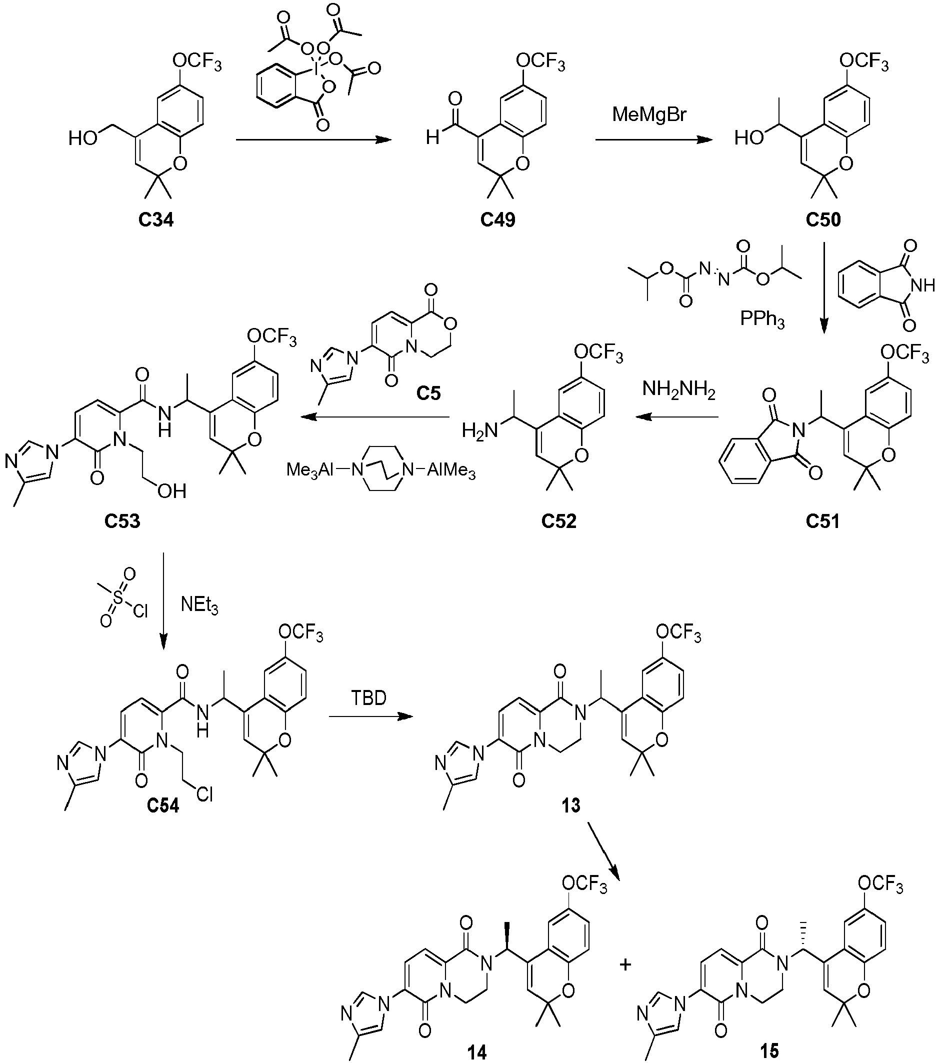

### Step 1. Synthesis of 2,2-dimethyl-6-(trifluoromethoxy)-2H-chromene-4-carbaldehyde (C49).

To a 0 °C solution of **C34** (2.2 g, 8.0 mmol) in dichloromethane (100 mL) was added Dess-Martin periodinane (6.80 g, 16.0 mmol), and the reaction mixture was stirred for 3 hours at room temperature, whereupon it was diluted with ice water (50 mL) and saturated aqueous sodium bicarbonate solution (50 mL). This mixture was extracted with dichloromethane (3 x 100 mL), and the combined organic layers were washed with brine (50 mL), dried over sodium sulfate, filtered, and concentrated *in vacuo.* Silica gel chromatography (Eluent: 30% ethyl acetate in hexanes) afforded the product as a yellow liquid. Yield: 1.5 g, 5.5 mmol, 69%. GCMS *m*/*z* 272 (M⁺). ¹H NMR (400 MHz, CDCl₃) δ 9.65 (s, 1H), 8.14-8.17 (m, 1H), 7.08 (br d, *J*=8.8 Hz, 1H), 6.86 (d, *J*=8.8 Hz, 1H), 6.51 (s, 1H), 1.55 (s, 6H).

### Step 2. Synthesis of 1-[2,2-dimethyl-6-(trifluoromethoxy)-2H-chromen-4-yl]ethanol (C50).

To a 0 °C solution of **C49** (1.5 g, 5.5 mmol) in tetrahydrofuran (100 mL) was added methylmagnesium bromide (3 M solution in diethyl ether, 2.75 mL, 8.25 mmol). The reaction mixture was stirred for 2 hours at room temperature, quenched with ice water (50 mL) and saturated aqueous ammonium chloride solution (50 mL), and extracted with ethyl acetate (3 x 100 mL). The combined organic layers were washed with brine (50 mL), dried over sodium sulfate, filtered, and concentrated *in vacuo.* Chromatography on silica gel (Eluent: 30% ethyl acetate in hexanes) provided the product as a yellow liquid. Yield: 1.1 g, 3.8 mmol, 69%. GCMS *m*/*z* 288 (M⁺). ¹H NMR (400 MHz, CDCl₃) δ 7.17 (br d, *J*=2.6 Hz, 1H), 6.99 (br d, *J*=8.7 Hz, 1H), 6.81 (d, *J*=8.7 Hz, 1H), 5.79 (br s, 1H), 4.79 (br q, *J*=6.4 Hz, 1H), 1.46 (d, *J*=6.5 Hz, 3H), 1.44 (s, 3H), 1.42 (s, 3H).

### Step 3. Synthesis of 2-{1-[2,2-dimethyl-6-(trifluoromethoxy)-2H-chromen-4-yl]ethyl}-1H-isoindole-1,3(2H)-dione (C51).

To a 0 °C solution of **C50** (1.5 g, 5.2 mmol) in tetrahydrofuran (100 mL) was added triphenylphosphine (1.5 g, 5.7 mmol) and phthalimide (0.84 g, 5.7 mmol), followed by diisopropyl azodicarboxylate (1.13 mL, 5.74 mmol), and the reaction mixture was stirred for 12 hours at room temperature. Ice water (30 mL) was added, and the mixture was extracted with ethyl acetate (3 x 50 mL); the combined organic layers were washed with brine (100 mL), dried over sodium sulfate, filtered, and concentrated *in vacuo.* Purification via silica gel chromatography (Gradient: 5% to 10% ethyl acetate in hexanes) afforded the product as a yellow liquid. Yield: 1.2 g, 2.9 mmol, 56%. GCMS *m*/*z* 417 (M⁺). ¹H NMR (400 MHz, CDCl₃) δ 7.80 (dd, *J*=5.4, 3.1 Hz, 2H), 7.69 (dd, *J*=5.5, 3.1 Hz, 2H), 7.09-7.12 (m, 1H), 6.91 (br d, *J*=9 Hz, 1H), 6.77 (d, *J*=8.7 Hz, 1H), 6.02 (br s, 1H), 5.39 (br q, *J*=7 Hz, 1H), 1.72 (d, *J*=7.0 Hz, 3H), 1.52 (s, 3H), 1.43 (s, 3H).

### Step 4. Synthesis of 1-[2,2-dimethyl-6-(trifluoromethoxy)-2H-chromen-4-yl]ethanamine (C52).

To a 0 °C solution of **C51** (1.5 g, 3.6 mmol) in ethanol (100 mL) was added hydrazine monohydrate (0.90 g, 18 mmol). After 12 hours at room temperature, the reaction mixture was filtered, and the filtrate was concentrated *in vacuo.* The residue was dissolved in dichloromethane (100 mL), washed with water, dried over sodium sulfate, filtered, and concentrated under reduced pressure to afford the product as a light yellow liquid. Yield: 0.90 g, 3.1 mmol, 86%. LCMS *m*/*z* 288.2 [M+H]⁺. ¹H NMR (400 MHz, DMSO-*d₆*) δ 7.38 (br d, *J*=2.2 Hz, 1H), 7.09 (br d, *J*=9 Hz, 1H), 6.84 (d, *J*=8.8 Hz, 1H), 5.86 (br s, 1H), 3.91 (br q, *J*=6.4 Hz, 1H), 1.72 (v br s, 2H), 1.38 (s, 3H), 1.34 (s, 3H), 1.16 (d, *J*=6.4 Hz, 3H).

### Step 5. Synthesis of N-{1-[2,2-dimethyl-6-(trifluoromethoxy)-2H-chromen-4-yl]ethyl}-1-(2-hydroxyethyl)-5-(4-methyl-1H-imidazol-1-yl)-6-oxo-1,6-dihydropyridine-2-carboxamide (C53).

Compound **C52** was converted to the product, which was obtained as a light yellow solid, according to the method described for synthesis of **C47** in Example 12. Yield: 300 mg, 0.56 mmol, 56%. LCMS *m*/*z* 533.2 [M+H]⁺. ¹H NMR (400 MHz, DMSO-*d₆*) δ 9.22 (d, *J*=8.1 Hz, 1H), 8.13 (s, 1H), 7.69 (d, *J*=7.6 Hz, 1H), 7.32 (s, 1H), 7.24-7.27 (m, 1H), 7.16 (br d, *J*=9 Hz, 1H), 6.89 (d, *J*=8.8 Hz, 1H), 6.31 (d, *J*=7.6 Hz, 1H), 5.91 (s, 1H), 4.96-5.05 (m, 1H), 4.91 (dd, *J*=5.3, 5.1 Hz, 1H), 4.19-4.27 (m, 2H), 3.53-3.68 (m, 2H), 2.14 (s, 3H), 1.34-1.41 (m, 9H).

### Step 6. Synthesis of 1-(2-chloroethyl)-N-{1-[2,2-dimethyl-6-(trifluoromethoxy)-2H-chromen-4-yl]ethyl}-5-(4-methyl-1H-imidazol-1-yl)-6-oxo-1,6-dihydropyridine-2-carboxamide (C54).

Compound **C53** (350.0 mg, 0.657 mmol) was converted to the product using the method employed for synthesis of **C39** in Examples 9, 10, and 11. The product was obtained as a yellow liquid, which was taken directly into the following step. LCMS *m*/*z* 551.4 [M+H]⁺.

### Step 7. Synthesis of 2-{1-[2,2-dimethyl-6-(trifluoromethoxy)-2H-chromen-4-yl]ethyl}-7-(4-methyl-1H-imidazol-1-yl)-3,4-dihydro-2H-pyrido[1,2-a]pyrazine-1,6-dione (13), 2-{(1S)-1-[2,2-dimethyl-6-(trifluoromethoxy)-2H-chromen-4-yl]ethyl}-7-(4-methyl-1H-imidazol-1-yl)-3,4-dihydro-2H-pyrido[1,2-a]pyrazine-1,6-dione (14), and 2-{(1R)-1-[2,2-dimethyl-6-(trifluoromethoxy)-2H-chromen-4-yl]ethyl}-7-(4-methyl-1H-imidazol-1-yl)-3,4-dihydro-2H-pyrido[1,2-a]pyrazine-1,6-dione (15).

1,3,4,6,7,8-Hexahydro-2*H*-pyrimido[1,2-*a*]pyrimidine (310 mg, 2.23 mmol) was added to a solution of **C54** (from the previous step, ≤0.657 mmol) in tetrahydrofuran (20 mL) and the reaction mixture was stirred for 12 hours at room temperature. After addition of aqueous sodium hydroxide solution (1 M, 5 mL), the mixture was extracted with dichloromethane (3 x 10 mL), and the combined organic layers were washed with brine (10 mL), dried over sodium sulfate, filtered, and concentrated *in vacuo.* Silica gel chromatography (Eluent: 10% methanol in dichloromethane), followed by preparative HPLC (Column: Dr. Maisch HPLC GmbH Reprosil-Gold C18, 5 µm; Mobile phase A: 20 mM aqueous ammonium acetate; Mobile phase B: acetonitrile; Gradient: 10% to 100% B), provided the racemic material **13** as an off-white solid. Yield: 95 mg, 0.18 mmol, 27% over two steps. LCMS *m*/*z* 515.2 [M+H]⁺. ¹H NMR (400 MHz, CDCl₃) δ 8.20 (s, 1H), 7.45 (d, *J*=7.7 Hz, 1H), 7.32 (d, *J*=7.7 Hz, 1H), 7.12 (br s, 1H), 7.04-7.07 (m, 1H), 6.97-7.02 (m, 1H), 6.84 (d, *J*=8.8 Hz, 1H), 5.87 (br q, *J*=7 Hz, 1H), 5.83 (s, 1H), 4.24 (ddd, *J*=14.5, 7, 4 Hz, 1H), 3.82 (ddd, *J*=14.5, 8, 4 Hz, 1H), 3.38-3.47 (m, 1H), 3.18 (ddd, *J*=13.5, 7, 4 Hz, 1H), 2.28 (s, 3H), 1.59 (s, 3H), 1.47 (d, *J*=6.7 Hz, 3H), 1.38 (s, 3H). Samples of the two enantiomers were obtained via chiral HPLC (Column: Chiral Technologies CHIRALPAK® IC, 5 µm; Mobile phase: 0.1% diethylamine in methanol). Both were obtained as off-white solids. The first-eluting enantiomer was assigned as Example **14**, and the later-eluting enantiomer as Example **15.** The absolute configurations of these compounds were assigned in analogy to those of Examples **24** and **25**, according to their relative biological activities (see Table 8).
**14**: LCMS *m*/*z* 515.2 [M+H]⁺. ¹H NMR (400 MHz, CDCl₃) δ 8.19 (s, 1H), 7.45 (d, *J*=7.7 Hz, 1H), 7.32 (d, *J*=7.7 Hz, 1H), 7.12 (br s, 1H), 7.04-7.07 (m, 1H), 7.00 (br d, *J*=9 Hz, 1H), 6.84 (d, *J*=8.8 Hz, 1H), 5.87 (br q, *J*=7 Hz, 1H), 5.82-5.84 (m, 1H), 4.24 (ddd, *J*=14.2, 7.0, 3.9 Hz, 1H), 3.82 (ddd, *J*=14.2, 8.4, 4.0 Hz, 1H), 3.43 (ddd, *J*=13.4, 8.4, 3.9 Hz, 1H), 3.18 (ddd, *J*=13.4, 7.0, 3.9 Hz, 1H), 2.28 (s, 3H), 1.59 (s, 3H), 1.47 (d, *J*=6.6 Hz, 3H), 1.38 (s, 3H). Retention time: 7.35 minutes (Column: Chiral Technologies CHIRALPAK® IC, 4.6 x 250 mm, 5 µm; Mobile phase: 0.1% diethylamine in methanol; Flow rate: 1.0 mL/minute).
**15**: LCMS *m*/*z* 515.3 [M+H]⁺. ¹H NMR (400 MHz, CDCl₃) δ 8.19 (s, 1H), 7.45 (d, *J*=7.8 Hz, 1H), 7.32 (d, *J*=7.7 Hz, 1H), 7.12 (br s, 1H), 7.05-7.07 (m, 1H), 7.00 (br d, *J*=9 Hz, 1H), 6.84 (d, *J*=8.8 Hz, 1H), 5.87 (br q, *J*=7 Hz, 1H), 5.82-5.84 (m, 1H), 4.24 (ddd, *J*=14.2, 7.0, 3.9 Hz, 1H), 3.82 (ddd, *J*=14.2, 8.4, 3.9 Hz, 1H), 3.43 (ddd, *J*=13.4, 8.2, 4.1 Hz, 1H), 3.18 (ddd, *J*=13.5, 7, 4 Hz, 1H), 2.28 (s, 3H), 1.6 (s, 3H, assumed; obscured by water peak), 1.47 (d, *J*=6.7 Hz, 3H), 1.39 (s, 3H). Retention time: 10.24 minutes, using HPLC conditions identical to those described above for **14**.

### Preparations

### Preparation 1

### 1-(1,1-Difluoroethoxy)-4-(prop-2-yn-1-yloxy)benzene (P1)

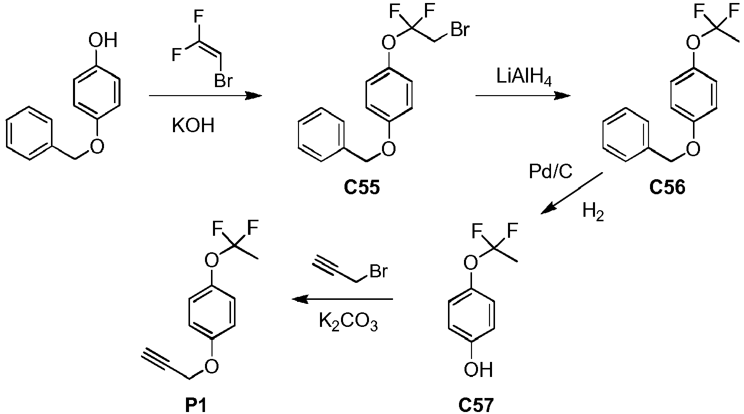

### Step 1. Synthesis of 1-(benzyloxy)-4-(2-bromo-1,1-difluoroethoxy)benzene (C55).

A mixture of 4-(benzyloxy)phenol (25.0 g, 125 mmol), 2-bromo-1,1-difluoroethene (17.8 g, 125 mmol) and potassium hydroxide (7.00 g, 125 mmol) in acetonitrile (150 mL) and water (10 mL) was stirred at 50 °C for 5 hours. The aqueous layer was discarded, and the organic layer was concentrated to a volume of approximately 50 mL. This was diluted with heptane (100 mL) and filtered through a 2 cm pad of silica gel. The pad was further eluted with a 1:10 mixture of ethyl acetate and heptane, and the combined organic filtrates were concentrated *in vacuo.* The residue was recrystallized from heptane to afford the product as a solid. Yield: 36.5 g, 106 mmol, 85%. GCMS *m*/*z* 342, 344 (M⁺). ¹H NMR (400 MHz, CDCl₃) δ 7.31-7.47 (m, 5H), 7.14 (br d, *J*=9.1 Hz, 2H), 6.95 (br d, *J*=9.2 Hz, 2H), 5.06 (s, 2H), 3.75 (t, *J*=8.8 Hz, 2H).

### Step 2. Synthesis of 1-(benzyloxy)-4-(1,1-difluoroethoxy)benzene (C56).

Lithium aluminum hydride (1 M solution in tetrahydrofuran, 119 mL, 119 mmol) was added in a drop-wise manner to a 0 °C solution of **C55** (37.0 g, 108 mmol) in tetrahydrofuran (200 mL), at a rate that maintained the internal reaction temperature at <10 °C. After 10 minutes, the solution was allowed to warm to room temperature and stir for 3 hours, whereupon an aqueous sodium hydroxide solution (1 M, 1 equivalent) was carefully added, the mixture was filtered, and the collected solids were washed with ethyl acetate. The organic layers of the filtrates were combined, washed with brine, dried over magnesium sulfate, filtered, and concentrated *in vacuo.* Recrystallization of the residue from heptane afforded the product as a white solid. Yield: 25.0 g, 94.6 mmol, 88%. GCMS *m*/*z* 264 (M⁺). ¹H NMR (400 MHz, CDCl₃) δ 7.31-7.47 (m, 5H), 7.11 (br d, *J*=9.2 Hz, 2H), 6.93 (br d, *J*=9.2 Hz, 2H), 5.05 (s, 2H), 1.90 (t, *J*=13.2 Hz, 3H).

### Step 3. Synthesis of 4-(1,1-difluoroethoxy)phenol (C57).

Palladium on carbon (10%, 2.5 g) was added to a solution of **C56** (25.0 g, 94.6 mmol) in ethanol (300 mL), and the mixture was hydrogenated at 100 psi for 5 hours. After filtration, the filtrate was concentrated *in vacuo* to afford the product. Yield: 11.5 g, 66.0 mmol, 70%. GCMS *m*/*z* 174 (M⁺). ¹H NMR (400 MHz, CDCl₃) δ 7.06 (br d, *J*=8.9 Hz, 2H), 6.78 (br d, *J*=8.9 Hz, 2H), 4.6-5.0 (br s, 1H), 1.90 (t, *J*=13.2 Hz, 3H).

### Step 4. Synthesis of 1-(1,1-difluoroethoxy)-4-(prop-2-yn-1-yloxy)benzene (P1).

A mixture of **C57** (5.6 g, 32 mmol), 3-bromoprop-1-yne (80%, 3.6 mL, 32 mmol), and potassium carbonate (8.9 g, 64 mmol) in *N*,*N*-dimethylformamide (40 mL) was stirred at room temperature for 3 hours. The reaction mixture was poured into water (100 mL) and extracted with diethyl ether (3 x 50 mL); the combined organic layers were dried over sodium sulfate, filtered, and concentrated *in vacuo* to afford the product as a pale yellow oil. Yield: 5.6 g, 26 mmol, 81%. GCMS *m*/*z* 212 (M⁺). ¹H NMR (400 MHz, CDCl₃) δ 7.12 (br d, *J*=9.1 Hz, 2H), 6.94 (br d, *J*=9.1 Hz, 2H), 4.68 (d, *J*=2.3 Hz, 2H), 2.53 (t, *J*=2.4 Hz, 1H), 1.91 (t, *J*=13.3 Hz, 3H).

### Preparation 2

### (6-Chloro-3-methyl-2H-chromen-4-yl)methanol (P2)

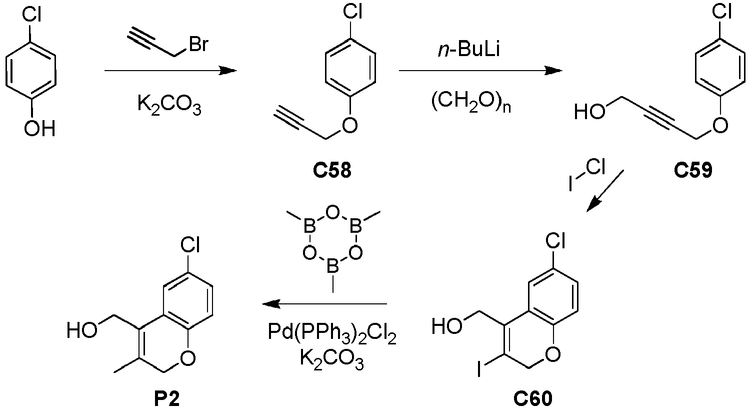

### Step 1. Synthesis of 4-chlorophenyl prop-2-yn-1-yl ether (C58).

Potassium carbonate (22.5 g, 163 mmol) was added to a solution of 4-chlorophenol (15.0 g, 117 mmol) in acetone (200 mL). The reaction mixture was stirred at room temperature for 1 hour, whereupon 3-bromoprop-1-yne (16.5 g, 139 mmol) was introduced and the reaction mixture was heated at 50 °C for 12 hours. After concentration under reduced pressure, the residue was diluted with water (400 mL) and extracted with ethyl acetate (3 x 200 mL); the combined organic layers were dried over sodium sulfate, filtered, and concentrated *in vacuo.* Silica gel chromatography (Eluent: 5% ethyl acetate in hexanes) afforded the product as a light yellow liquid. Yield: 14.0 g, 84.0 mmol, 72%. ¹H NMR (400 MHz, CDCl₃) δ 7.27 (br d, *J*=9.0 Hz, 2H), 6.92 (br d, *J*=9.0 Hz, 2H), 4.68 (d, *J*=2.3 Hz, 2H), 2.53 (t, *J*=2.4 Hz, 1H).

### Step 2. Synthesis of 4-(4-chlorophenoxy)but-2-yn-1-ol (C59).

Compound **C58** was converted to the product using the method described for synthesis of **C43** in Example 12. The product was obtained as an off-white solid. Yield: 3.0 g, 15 mmol, 54%. ¹H NMR (400 MHz, CDCl₃) δ 7.26 (br d, *J*=8.9 Hz, 2H), 6.90 (br d, *J*=8.9 Hz, 2H), 4.72 (t, *J*=1.7 Hz, 2H), 4.32 (t, *J*=1.6 Hz, 2H).

### Step 3. Synthesis of (6-chloro-3-iodo-2H-chromen-4-yl)methanol (C60).

A solution of iodine monochloride (11.6 g, 71.4 mmol) in nitromethane (80 mL) was added drop-wise to a -30 °C solution of **C59** (10 g, 51 mmol) in nitromethane (70 mL), and the reaction mixture was stirred at this temperature for 1 hour. After addition of ethyl acetate (500 mL) and saturated aqueous sodium thiosulfate solution (500 mL), the reaction mixture was allowed to warm to room temperature, whereupon the organic layer was dried over sodium sulfate, filtered, and concentrated *in vacuo.* Chromatography on silica gel (Eluent: 10% ethyl acetate in hexanes) provided the product as a white solid. Yield: 4.1 g, 13 mmol, 25%. GCMS *m*/*z* 322, 324 (M⁺). ¹H NMR (400 MHz, CDCl₃) δ 7.45 (d, *J*=2.3 Hz, 1H), 7.14 (dd, *J*=8.6, 2.4 Hz, 1H), 6.77 (d, *J*=8.6 Hz, 1H), 4.90 (s, 2H), 4.66 (d, *J*=6.1 Hz, 2H), 1.69 (t, *J*=6.1 Hz, 1H).

### Step 4. Synthesis of (6-chloro-3-methyl-2H-chromen-4-yl)methanol (P2).

To a solution of **C60** (2.0 g, 6.2 mmol) in *N*,*N*-dimethylformamide (20 mL) were added trimethylboroxin (778 mg, 6.20 mmol) and dichlorobis(triphenylphosphine)palladium(II) (435 mg, 0.620 mmol), followed by potassium carbonate (1.71 g, 12.4 mmol), and the reaction mixture was heated to 60 °C for 7 hours. It was then diluted with water (100 mL) and filtered through a pad of diatomaceous earth; the filter cake was washed with ethyl acetate (2 x 50 mL). The aqueous layer of the filtrate was extracted with ethyl acetate (3 x 50 mL), and the combined organic layers and flitrates were washed with water (200 mL) and with brine (200 mL), dried over sodium sulfate, filtered, and concentrated *in vacuo.* Silica gel chromatography (Eluent: 10% ethyl acetate in hexanes) afforded the product as a yellow solid. Yield: 280 mg, 1.33 mmol, 21%. GCMS *m*/*z* 210, 212 (M⁺). ¹H NMR (400 MHz, CDCl₃) δ 7.37 (d, *J*=2.3 Hz, 1H), 7.05 (dd, *J*=8.6, 2.4 Hz, 1H), 6.74 (d, *J*=8.6 Hz, 1H), 4.62 (s, 2H), 4.55 (d, *J*=5.3 Hz, 2H), 1.89 (s, 3H), 1.35 (t, *J*=5.4 Hz, 1H).

### Preparation 3

### [6-(Trifluoromethyl)-1,1a,2,7b-tetrahydrocyclopropa[c]chromen-1-yl]methanol (P3)

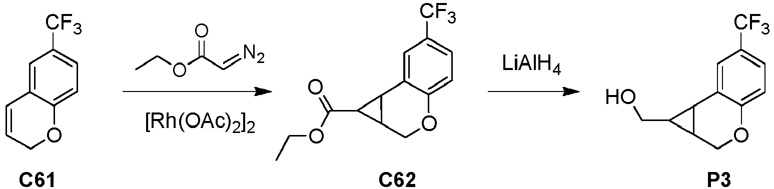

### Step 1. Synthesis of ethyl 6-(trifluoromethyl)-1,1a,2,7b-tetrahydrocyclopropa[c]chromene-1-carboxylate (C62).

Rhodium(II) acetate dimer (0.663 g, 1.50 mmol) was added to a solution of 6-(trifluoromethyl)-2*H*-chromene (**C61**, prepared from 1-(prop-2-yn-1-yloxy)-4-(trifluoromethyl)benzene using the method described for synthesis of **C8** in Examples 2, 3, and 4) (3.0 g, 15 mmol) in 1,2-dichloroethane (75 mL) and the mixture was cooled to 0 °C. A solution of ethyl diazoacetate (3.4 g, 30 mmol) in 1,2-dichloroethane (25 mL) was added drop-wise over 1.5 hours, whereupon the reaction mixture was allowed to warm to room temperature and stir for 5 hours. Volatiles were removed *in vacuo,* and purification via silica gel chromatography (Eluent: hexanes) afforded the product as a liquid. Yield: 800 mg, 2.8 mmol, 19%. GCMS *m*/*z* 286 (M⁺). ¹H NMR (400 MHz, CDCl₃) δ 7.53 (br s, 1H), 7.37 (br d, *J*=8.4 Hz, 1H), 6.88 (d, *J*=8.4 Hz, 1H), 4.44 (d, *J*=11 Hz, 1H), 4.19 (q, *J*=7.2 Hz, 2H), 3.98 (d, *J*=11 Hz, 1H), 2.63 (dd, *J*=9.0, 3.5 Hz, 1H), 2.34-2.40 (m, 1H), 2.30 (dd, *J*=4, 4 Hz, 1H), 1.29 (t, *J*=7.2 Hz, 3H).

### Step 2. Synthesis of [6-(trifluoromethyl)-1,1a,2,7b-tetrahydrocyclopropa[c]chromen-1-yl]methanol (P3).

To a 0 °C solution of **C62** (300 mg, 1.05 mmol) in tetrahydrofuran (5 mL) was added lithium aluminum hydride (1 M solution in tetrahydrofuran, 1.0 mL, 1.0 mmol), and the reaction mixture was allowed to stir at room temperature for 2 hours. After being quenched with saturated aqueous sodium sulfate solution, the mixture was filtered, dried over sodium sulfate, filtered, and concentrated under reduced pressure. Silica gel chromatography (Gradient: 0% to 30% ethyl acetate in hexanes) provided the product as a liquid. Yield: 100 mg, 0.41 mmol, 39% GCMS *m*/*z* 244 (M⁺). ¹H NMR (400 MHz, CDCl₃) δ 7.50 (br d, *J*=1.8 Hz, 1H), 7.32 (br dd, *J*=8.6, 1.8 Hz, 1H), 6.87 (d, *J*=8.6 Hz, 1H), 4.42 (d, *J*=10.5 Hz, 1H), 3.90 (d, *J*=10.6 Hz, 1H), 3.65-3.77 (m, 2H), 1.98-2.04 (m, 1H), 1.72-1.81 (m, 2H).

**Table 6. Method of Synthesis and Physicochemical Data for Examples 16 - 37.**

| Example Number | Method of Synthesis: Example Number; Source of Non-commercial Starting Materials | Structure | ¹H NMR (400 MHz, CDCl₃), δ (ppm); LCMS, observed ion *m*/*z* [M+H]⁺ or HPLC retention time (minutes); LCMS *m*/*z* [M+H]⁺ (unless otherwise indicated) |
|---|---|---|---|
| **16** | Ex 13, 14, and 15¹ | 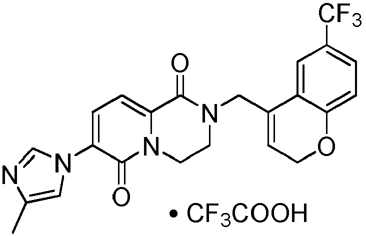 | 8.22 (br s, 1H), 7.49 (br s, 1H), 7.44 (d, *J*=7.8 Hz, 1H), 7.40 (br d, *J*=8.5 Hz, 1H), 7.31 (d, *J*=7.7 Hz, 1H), 7.10 (br s, 1H), 6.89 (d, *J*=8.4 Hz, 1H), 5.89 (br t, *J*=3.5 Hz, 1H), 4.89 (br d, *J*=3.5 Hz, 2H), 4.62 (br s, 2H), 4.24-4.29 (m, 2H), 3.55-3.60 (m, 2H), 2.27 (s, 3H)²; 457.1 |
| **17** | Ex 9, 10 and 11^{1,**3,4,5**} | 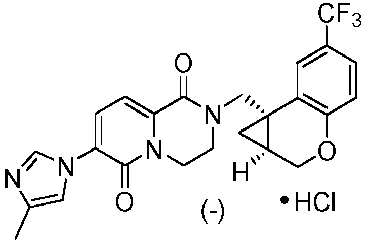 | ¹H NMR (400 MHz, CD₃OD) δ 9.42 (d, *J*=1.7 Hz, 1H), 8.03 (d, *J*=7.8 Hz, 1H), 7.78 (br d, *J*=1.8 Hz, 1H), 7.71-7.73 (m, 1H), 7.33 (br dd, *J*=8.7, 1.8 Hz, 1H), 7.30 (d, *J*=7.8 Hz, 1H), 6.92 (br d, *J*=8.5 Hz, 1H), 5.36 (d, *J*=14.6 Hz, 1H), 4.37-4.44 (m, 2H), 4.06-4.16 (m, 2H), 3.79-3.86 (m, 1H), 3.62 (ddd, *J*=13.5, 9.8, 4.1 Hz, 1H), 2.93 (d, *J*=14.7 Hz, 1H), 2.41 (d, *J*=1.1 Hz, 3H), 2.06-2.12 (m, 1H), 1.14-1.22 (m, 2H); 471.2 |
| **18** | Ex 9, 10 and 11^{1,**3,4,5**} | 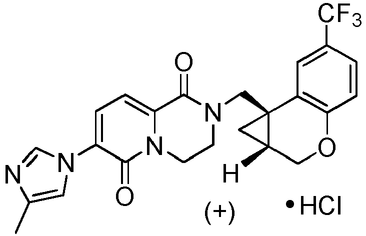 | ¹H NMR (400 MHz, CD₃OD) δ 9.44 (d, *J*=1.4 Hz, 1H), 8.04 (d, *J*=7.8 Hz, 1H), 7.78 (br s, 1H), 7.73 (br s, 1H), 7.30-7.35 (m, 1H), 7.30 (d, *J*=7.8 Hz, 1H), 6.91 (d, *J*=8.4 Hz, 1H), 5.36 (d, *J*=14.6 Hz, 1H), 4.37-4.45 (m, 2H), 4.05-4.17 (m, 2H), 3.83 (ddd, *J*=13.5, 5, 5 Hz, 1H), 3.62 (ddd, *J*=13.5, 9.8, 4.1 Hz, 1H), 2.93 (d, *J*=14.6 Hz, 1H), 2.42 (s, 3H), 2.06-2.14 (m, 1H), 1.12-1.24 (m, 2H); 471.3 |
| **19** | Ex 9, 10 and 11^{6,7} | 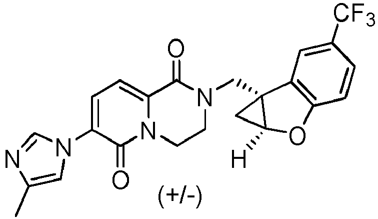 | 8.20 (br s, 1H), 7.63 (br s, 1H), 7.40-7.45 (m, 2H), 7.24-7.28 (m, 1H, assumed; partially obscured by solvent peak), 7.11 (br s, 1H), 6.91 (d, *J*=8 Hz, 1H), 4.90-4.94 (m, 1H), 4.86 (d, *J*=14.7 Hz, 1H), 4.26-4.34 (m, 1H), 4.11-4.20 (m, 1H), 3.54-3.64 (m, 2H), 3.43 (d, *J*=14.8 Hz, 1H), 2.28 (s, 3H), 1.25 (dd, *J*=6.7, 5.9 Hz, 1H), 0.60-0.64 (m, 1H); 457.0 |
| **20** | Ex 6^{8,9,5} | 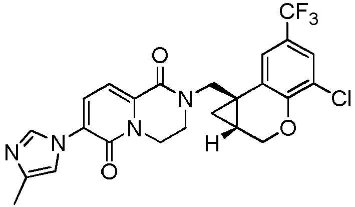 | 4.98 minutes¹⁰; 505.3 |
| **21** | Ex 6^{8,9,5} | 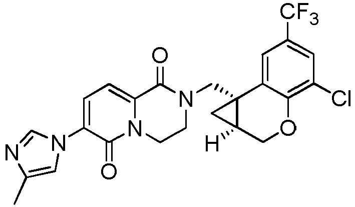 | 5.87 minutes¹⁰; 505.3 |
| **22** | Ex 9, 10 and 11; **P1** | 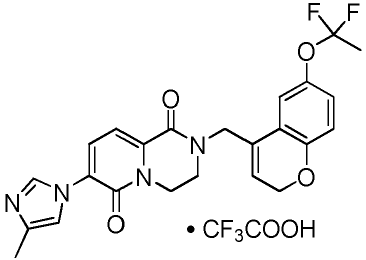 | ¹H NMR (600 MHz, DMSO-*d₆*) δ 9.27 (br s, 1H), 8.03 (d, *J*=7.7 Hz, 1H), 7.77 (br s, 1H), 7.20 (d, *J*=7.9 Hz, 1H), 7.07 (br s, 1H), 6.98 (br d, *J*=8.8 Hz, 1H), 6.83 (d, *J*=8.8 Hz, 1H), 6.02 (br s, 1H), 4.78-4.81 (m, 2H), 4.51 (br s, 2H), 4.21-4.26 (m, 2H), 3.63-3.69 (m, 2H), 2.31 (s, 3H), 1.88 (t, *J*=13.7 Hz, 3H); 469.1 |
| **23** | Ex 13, 14, and 15; **C29** | 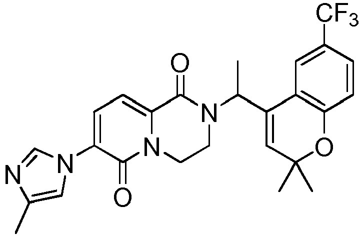 | ¹H NMR (400 MHz, DMSO-*d₆*) δ 8.21 (br s, 1H), 7.79 (d, *J*=7.8 Hz, 1H), 7.43-7.51 (m, 2H), 7.37 (br s, 1H), 7.11 (d, *J*=7.8 Hz, 1H), 6.96 (d, *J*=8.4 Hz, 1H), 6.10 (br s, 1H), 5.74 (br q, *J*=7 Hz, 1H), 3.96-4.11 (m, 2H), 3.54-3.63 (m, 1H), 3.02-3.11 (m, 1H), 2.13 (s, 3H), 1.52 (s, 3H), 1.37-1.44 (m, 6H); 499.2 |
| **24** | Ex 6; **C29**¹¹ | 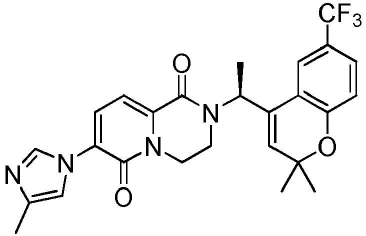 | ¹H NMR (400 MHz, CD₃OD) δ 8.26 (d, *J*=1.3 Hz, 1H), 7.76 (d, *J*=7.8 Hz, 1H), 7.50 (br d, *J*=1.8 Hz, 1H), 7.40 (br dd, *J*=8.5, 2.2 Hz, 1H), 7.30 (d, *J*=7.7 Hz, 1H), 7.27-7.29 (m, 1H), 6.92 (br d, *J*=8.4 Hz, 1H), 6.08 (d, *J*=1.5 Hz, 1H), 5.88 (qd, *J*=6.9, 1.4 Hz, 1H), 4.06-4.12 (m, 2H), 3.63 (ddd, *J*=13.8, 5.8, 4.7 Hz, 1H), 3.11-3.19 (m, 1H), 2.22 (d, *J*=1.1 Hz, 3H), 1.58 (s, 3H), 1.51 (d, *J*=6.8 Hz, 3H), 1.42 (s, 3H); 499.2 |
| **25** | Ex 13, 14, and 15¹²; **C29** | 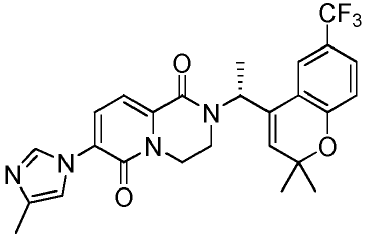 | ¹H NMR (400 MHz, DMSO-*d₆*) δ 8.21 (br s, 1H), 7.79 (d, *J*=7.7 Hz, 1H), 7.43-7.50 (m, 2H), 7.37 (br s, 1H), 7.11 (d, *J*=7.7 Hz, 1H), 6.96 (d, *J*=8.3 Hz, 1H), 6.10 (br s, 1H), 5.74 (br q, *J*=7 Hz, 1H), 3.96-4.11 (m, 2H), 3.54-3.62 (m, 1H), 3.02-3.11 (m, 1H), 2.13 (s, 3H), 1.52 (s, 3H), 1.38-1.44 (m, 6H); 499.0 |
| **26** | Ex 9, 10 and 11 | 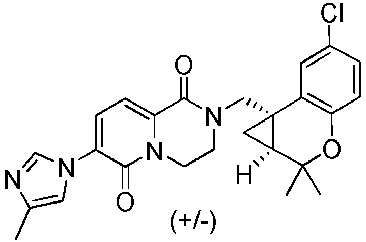 | ¹H NMR (400 MHz, DMSO-*d₆*) δ 8.22 (br s, 1H), 7.78 (d, *J*=7.8 Hz, 1H), 7.57 (d, *J*=2.3 Hz, 1H), 7.38 (br s, 1H), 7.12 (d, *J*=7.8 Hz, 1H), 7.05 (dd, *J*=8.6, 2.4 Hz, 1H), 6.72 (d, *J*=8.6 Hz, 1H), 4.91 (d, *J*=14.8 Hz, 1H), 4.17-4.25 (m, 1H), 4.08-4.17 (m, 1H), 3.65-3.80 (m, 2H), 3.03 (d, *J*=14.7 Hz, 1H), 2.14 (s, 3H), 2.03 (dd, *J*=8.2, 6.0 Hz, 1H), 1.42 (s, 3H), 1.20 (s, 3H), 1.06 (dd, *J*=8.7, 4.5 Hz, 1H), 0.90 (dd, *J*=5, 5 Hz, 1H); 465.4 |
| **27** | Ex 9, 10 and 11¹³ | 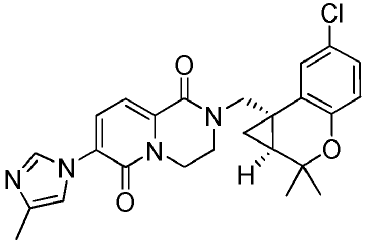 | 8.21 (br s, 1H), 7.46 (d, *J*=7.7 Hz, 1H), 7.43 (d, *J*=2.3 Hz, 1H), 7.32 (d, *J*=7.7 Hz, 1H), 7.12 (br s, 1H), 7.04 (dd, *J*=8.4, 2.4 Hz, 1H), 6.72 (d, *J*=8.6 Hz, 1H), 4.82 (d, *J*=14.9 Hz, 1H), 4.25-4.30 (m, 2H), 3.65-3.81 (m, 2H), 3.22 (d, *J*=14.6 Hz, 1H), 2.29 (s, 3H), 1.74 (dd, *J*=9, 6 Hz, 1H), 1.49 (s, 3H), 1.24 (s, 3H), 1.17 (dd, *J*=6, 5 Hz, 1H), 1.04 (dd, *J*=9, 5 Hz, 1H); 465.2 |
| **28** | Ex 9, 10 and 11¹³ | 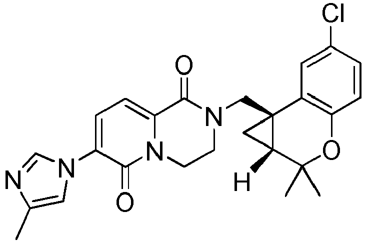 | 8.21 (br s, 1H), 7.46 (d, *J*=7.7 Hz, 1H), 7.43 (d, *J*=2.4 Hz, 1H), 7.32 (d, *J*=7.7 Hz, 1H), 7.12 (br s, 1H), 7.04 (dd, *J*=8.5, 2.5 Hz, 1H), 6.72 (d, *J*=8.4 Hz, 1H), 4.82 (d, *J*=14.8 Hz, 1H), 4.25-4.30 (m, 2H), 3.65-3.81 (m, 2H), 3.22 (d, *J*=14.6 Hz, 1H), 2.29 (s, 3H), 1.74 (dd, J=9, 6 Hz, 1H), 1.49 (s, 3H), 1.24 (s, 3H), 1.17 (dd, J=5, 5 Hz, 1H), 1.04 (dd, J=9, 5 Hz, 1H); 465.0 |
| **29** | Ex 9, 10 and 11; **P2** | 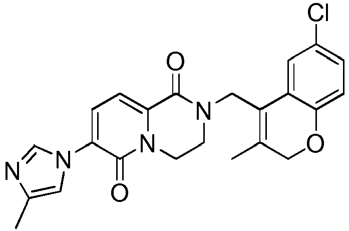 | ¹H NMR (400 MHz, DMSO-*d₆*) δ 8.23 (s, 1H), 7.79 (d, *J*=7.8 Hz, 1H), 7.39 (s, 1H), 7.28 (s, 1H), 7.14 (d, *J*=7.3 Hz, 1H), 7.10 (br d, *J*=9 Hz, 1H), 6.80 (d, *J*=8.3 Hz, 1H), 4.70 (s, 2H), 4.63 (s, 2H), 4.06-4.14 (m, 2H), 3.43-3.50 (m, 2H), 2.14 (s, 3H), 1.93 (s, 3H); 437.2, 439.2 |
| **30** | Ex 9, 10 and 11; **C34** | 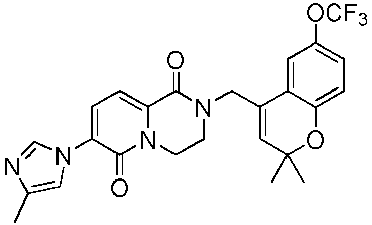 | ¹H NMR (400 MHz, DMSO-*d₆*) δ 8.24 (s, 1H), 7.80 (d, *J*=7.6 Hz, 1H), 7.40 (s, 1H), 7.27 (br s, 1H), 7.10-7.17 (m, 2H), 6.88 (d, *J*=8.6 Hz, 1H), 5.94 (s, 1H), 4.50 (s, 2H), 4.18-4.25 (m, 2H), 3.58-3.65 (m, 2H), 2.15 (s, 3H), 1.40 (s, 6H); 500.9 |
| **31** | Ex 5¹⁴ | 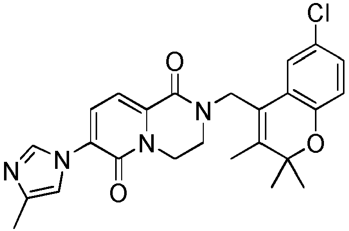 | ¹H NMR (400 MHz, DMSO-*d₆*) δ 8.22 (s, 1H), 7.79 (d, *J*=7.6 Hz, 1H), 7.39 (br s, 1H), 7.31 (d, *J*=2.4 Hz, 1H), 7.14 (d, *J*=7.6 Hz, 1H), 7.11 (dd, *J*=8.6, 2.4 Hz, 1H), 6.80 (d, *J*=8.6 Hz, 1H), 4.64 (s, 2H), 4.07-4.13 (m, 2H), 3.39-3.45 (m, 2H), 2.14 (s, 3H), 1.95 (s, 3H), 1.39 (s, 6H); 465.0 |
| **32** | Ex 12¹⁵ | 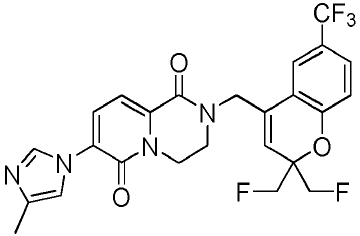 | 8.24-8.32 (br s, 1H), 7.60 (br s, 1H), 7.46-7.51 (m, 2H), 7.34 (d, *J*=7.6 Hz, 1H), 7.10-7.18 (br s, 1H), 7.02 (d, *J*=8.6 Hz, 1H), 5.75 (br s, 1H), 4.69 (s, 2H), 4.47-4.69 (m, 4H), 4.28-4.34 (m, 2H), 3.56-3.63 (m, 2H), 2.30 (s, 3H); 521.1 |
| **33** | Ex 9, 10 and 11^{16,5}; **P1** | 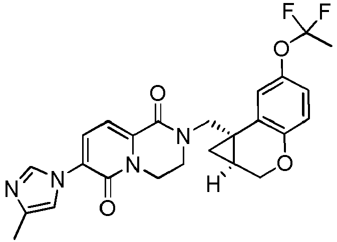 | 5.73 minutes¹⁷; 483.4 |
| **34** | Ex 9, 10 and 11^{16,5} ; **P1** | 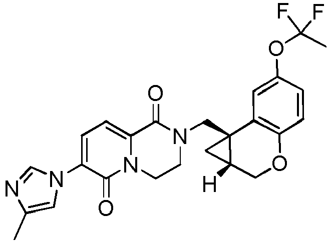 | 4.54 minutes¹⁷; 483.4 |
| **35** | Ex 1¹⁸ | 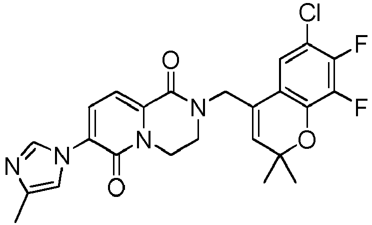 | 8.22 (s, 1H), 7.46 (d, *J*=7.7 Hz, 1H), 7.34 (d, *J*=7.8 Hz, 1H), 7.11-7.16 (m, 2H), 5.74 (s, 1H), 4.54 (s, 2H), 4.25-4.31 (m, 2H), 3.52-3.58 (m, 2H), 2.29 (s, 3H), 1.52 (s, 6H); 487.1, 489.2 |
| **36** | Ex 9, 10 and 11^{14,19,5} | 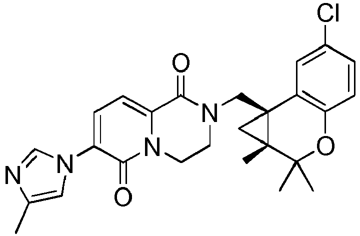 | ¹H NMR (400 MHz, DMSO-*d₆*) δ 8.22 (s, 1H), 7.80 (d, *J*=8.0 Hz, 1H), 7.38-7.41 (m, 2H), 7.15 (d, *J*=7.8 Hz, 1H), 7.07 (dd, *J*=8.6, 2.6 Hz, 1H), 6.73 (d, *J*=8.6 Hz, 1H), 4.79 (d, *J*=15.4 Hz, 1H), 4.18-4.27 (m, 1H), 4.02-4.10 (m, 1H), 3.62-3.71 (m, 1H), 3.51 (d, *J*=15.6 Hz, 1H), 3.5-3.57 (m, 1H), 2.14 (s, 3H), 1.45 (s, 3H), 1.39 (s, 3H), 1.26 (d, *J*=4.7 Hz, 1H), 1.17 (s, 3H), 0.86 (d, *J*=4.6 Hz, 1H); 479.2 |
| **37** | Ex 9, 10 and 11^{14,19,5} | 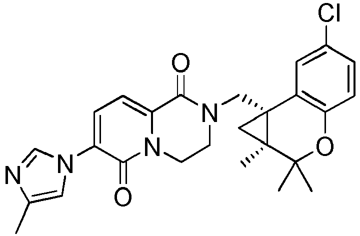 | ¹H NMR (400 MHz, DMSO-*d₆*) δ 8.22 (br s, 1H), 7.80 (d, *J*=7.8 Hz, 1H), 7.38-7.41 (m, 2H), 7.15 (d, *J*=7.7 Hz, 1H), 7.07 (dd, *J*=8.4, 2.5 Hz, 1H), 6.73 (d, *J*=8.7 Hz, 1H), 4.79 (d, *J*=15.5 Hz, 1H), 4.18-4.27 (m, 1H), 4.01-4.10 (m, 1H), 3.62-3.71 (m, 1H), 3.51 (d, *J*=15.5 Hz, 1H), 3.5-3.57 (m, 1H), 2.14 (s, 3H), 1.45 (s, 3H), 1.39 (s, 3H), 1.26 (d, *J*=4.6 Hz, 1H), 1.17 (s, 3H), 0.86 (d, *J*=4.7 Hz, 1H); 479.3 |

| | | | |
|---|---|---|---|
| 1. The requisite [6-(trifluoromethyl)-2*H*-chromen-4-yl]methanol was prepared according to the method for synthesis of **C25** in Example 6, by using 1-(prop-2-yn-1-yloxy)-4-(trifluoromethyl)benzene as starting material. 2. The NMR was obtained on the free base. 3. In this case, ring closure was carried out on the 1-(2-hydroxyethyl)-5-(4-methyl-1*H*-imidazol-1-yl)-6-oxo-1,6-dihydropyridine-2-carboxamide intermediate, using triphenylphosphine and diisopropyl azodicarboxylate. 4. The free bases of enantiomers **17** and **18** were separated via supercritical fluid chromatography (Column: Phenomenex Lux Cellulose-3, 5 µm; Eluent: 4:1 carbon dioxide / methanol). The first-eluting enantiomer was **18**, which displayed a positive (+) optical rotation. Example **17** was the second-eluting enantiomer, and gave a negative (-) optical rotation. 5. The indicated absolute configurations were assigned by analogy with Examples **3** and **4,** according to the relative biological activities of the enantiomers (see Table 8). 6. 5-(Trifluoromethyl)-1-benzofuran was reacted with (chloromethylene)dimethylammonium chloride to provide 5-(trifluoromethyl)-1-benzofuran-3-carbaldehyde. This was reduced using sodium borohydride to afford the requisite [5-(trifluoromethyl)-1-benzofuran-3-yl]methanol. 7. In this case, a mesylate leaving group was used, rather than a bromide, for introduction of the amine group. 8. The requisite [4-chloro-6-(trifluoromethyl)-1a,2-dihydrocyclopropa[c]chromen-7b(1*H*)-yl]methanol was prepared from [6-(trifluoromethyl)-1a,2-dihydrocyclopropa[*c*]chromen-7b(1*H*)-yl]methanol (see Example 17) via reaction with *N*-chlorosuccinimide in acetic acid. 9. Examples **20** and **21** were synthesized as the racemate and then separated via supercritical fluid chromatography (Column: Chiral Technologies CHIRALPAK® OJ-H, 5 µm; Mobile phase: 4:1 carbon dioxide / methanol). Example **20** was the first-eluting enantiomer, and Example **21** was the second-eluting enantiomer. 10. Conditions for analytical HPLC. Column: Chiral Technologies CHIRALPAK® OJ-H, 4.6 x 100 mm, 5 µm; Mobile phase: 4:1 carbon dioxide / methanol; Flow rate: 1.5 mL/minute. 11. Compound **C29** was converted to the requisite (1*S*)-1-[2,2-dimethyl-6-(trifluoromethyl)-2*H*-chromen-4-yl]ethanamine by reaction with *R*-(+)-*tert*-butyl sulfinamide in the presence of titanium(IV) ethoxide; the resulting intermediate was then subjected to reaction with methyllithium. See G. Liu et al., J. Am. Chem. Soc. 1997, 119, 9913-9914 and G. Liu et al., J. Org. Chem. 1999, 64, 1278-1284. 12. In this case, the final compound was synthesized as the racemate (Example **23**) and then subjected to chiral HPLC (Column: Chiral Technologies CHIRALPAK® IC, 5 µm; Mobile phase: 0.1% diethylamine in methanol). Example **25** was the second-eluting enantiomer; Retention time: 11.26 minutes (Column: Chiral Technologies CHIRALPAK® IC, 4.6 x 250 mm, 5 µm; Mobile phase: 0.1% diethylamine in methanol; Flow rate: 1.0 mL/minute). [Under this set of HPLC conditions, enantiomer Example **24** exhibited a retention time of 8.94 minutes.] 13. Examples **27** and **28** were synthesized as the racemate (Example **26**) and then separated via chiral HPLC (Column: Chiral Technologies CHIRALPAK® IC, 5 µm; Mobile phase: 0.1% diethylamine in methanol). Example **27** was the first-eluting enantiomer. Retention time: 17.75 minutes (Column: Chiral Technologies CHIRALPAK® IC, 4.6 x 250 mm, 5 µm; Mobile phase: 0.1% diethylamine in methanol; Flow rate: 1.0 mL/minute). Example **28** was the second-eluting enantiomer, with retention time 21.49 minutes under the same HPLC conditions. 14. 4-(4-Chlorophenoxy)-4-methylpent-2-yn-1-ol was prepared from 4-chlorophenol using the method described for synthesis of **C22** in Example 6. This intermediate was then converted to (6-chloro-2,2,3-trimethyl-2*H*-chromen-4-yl)methanol according to the chemistry described for transformation of **C59** to **P2** in Preparation P2. 15. In this case, the starting material was 1,3-difluoropropan-2-one. 16. Examples **33** and **34** were synthesized as the racemate and then separated via supercritical fluid chromatography (Column: Chiral Technologies CHIRALPAK® AD-H, 5 µm; Mobile phase: 7:3 carbon dioxide / 0.2% ammonium hydroxide in methanol). Example **34** was the first-eluting enantiomer, and Example **33** was the second-eluting enantiomer. 17. Conditions for analytical HPLC. Column: Chiral Technologies CHIRALPAK® AD-H, 4.6 x 100 mm, 5 µm; Mobile phase: 7:3 carbon dioxide / 0.2% ammonium hydroxide in methanol; Flow rate: 1.5 mL/minute. 18. The requisite 4-chloro-2,3-difluorophenol was prepared via reaction of (4-chloro-2,3-difluorophenyl)boronic acid with OXONE® (potassium peroxymonosulfate) in acetone and water. 19. Examples **36** and **37** were synthesized as the racemate and then separated via chiral HPLC. Example **36** was the first-eluting enantiomer. Retention time: 17.84 minutes (Column: YMC Amylose-C, 4.6 x 250 mm, 5 µm; Mobile phase: 0.1% diethylamine in ethanol; Flow rate: 0.5 mL/minute). Example **37** was the second-eluting enantiomer, with retention time 27.27 minutes under the same HPLC conditions. | | | |

**Table 7. Method of Synthesis and Physicochemical Data for Examples 38 - 66.**

| Example Number | Method of Synthesis: Example Number; Source of Non-commercial Starting Materials | Structure | LCMS, *m*/*z* [M+H]⁺ |
|---|---|---|---|
| **38** (ref. ex.) | Ex 13, 14, and 15^{1,2} | 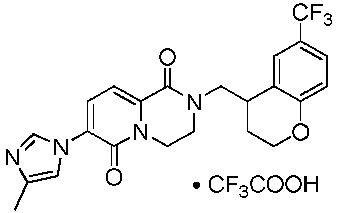 | 459.2 |
| **39** | Ex 9, 10 and 11³ | 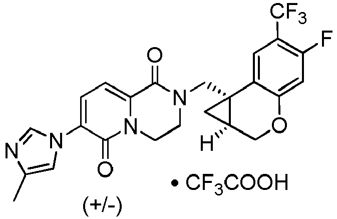 | 489.2 |
| **40** | Ex 9, 10 and 11^{4,2} | 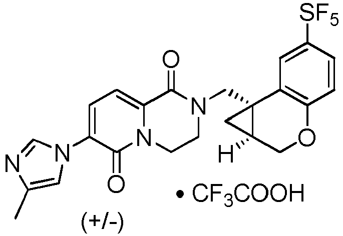 | 529.3 |
| **41** | Ex 39^{5,6} | 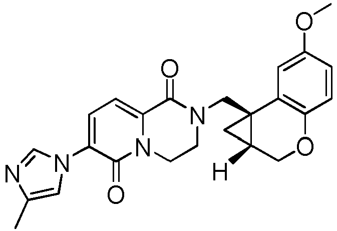 | 434.7 |
| **42** | Ex 39^{5,6} | 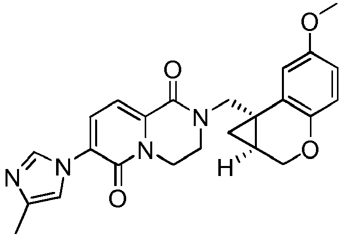 | 434.7 |
| **43** | Ex 39^{7,6} | 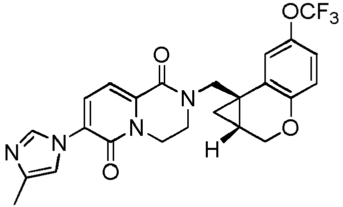 | 488.6 |
| **44** | Ex 39^{7,6} | 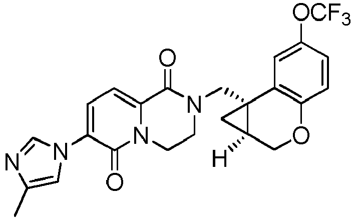 | 488.6 |
| **45** | Ex 6^{8,9,6} | 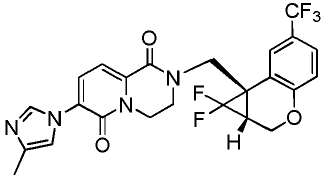 | 508.1 |
| **46** | Ex 6^{8,9,6} | 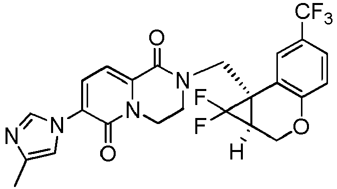 | 508.2 |
| **47** | Ex 9, 10 and 11^{1,10,6} | 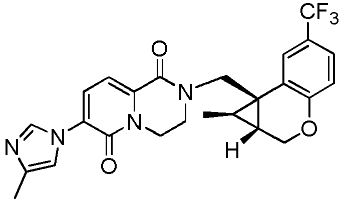 | 486.6 |
| **48** | Ex 9, 10 and 11^{1,10,6} | 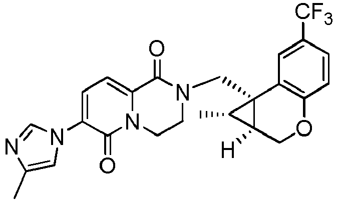 | 486.6 |
| **49** | Ex 9, 10 and 11^{11,6} | 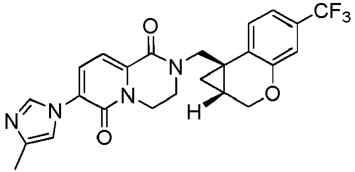 | 471.0 |
| **50** | Ex 9, 10 and 11^{11,6} | 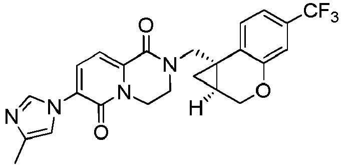 | 471.0 |
| **51** | Ex 9, 10 and 11^{12,13,6} | 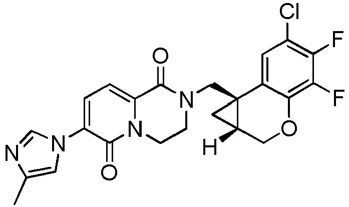 | 473.0 |
| **52** | Ex 9, 10 and 11^{12,13,6} | 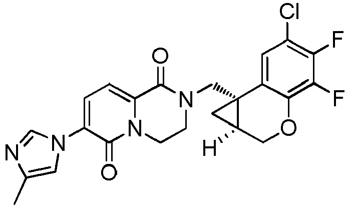 | 473.2 |
| **53** | Ex 9, 10 and 11^{14,6} | 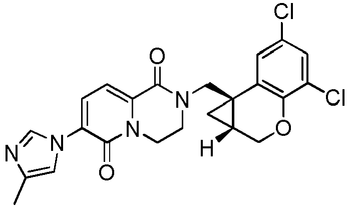 | 471.2, 473.2, 475.2 |
| **54** | Ex 9, 10 and 11^{14,6} | 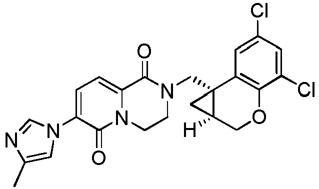 | 471.2, 473.2, 475.3 |
| **55** | Ex 24¹⁵ | 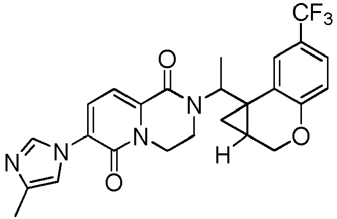 | 485.3 |
| **56** | Ex 24¹⁵ | 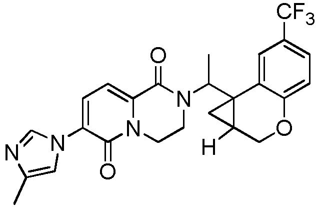 | 485.3 |
| **57** (ref. ex.) | Ex 9, 10 and 11; **P3** | 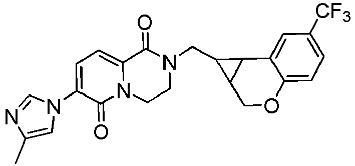 | 471.2 |
| **58** | Ex 9, 10 and 11^{16,6} | 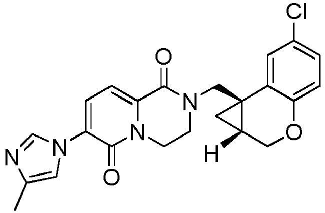 | 436.8 |
| **59** | Ex 9, 10 and 11^{16,6} | 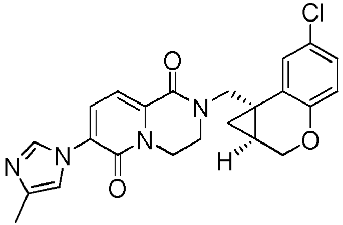 | 437.2, 439.3 |
| **60** | Ex 9, 10 and 11¹⁷ | 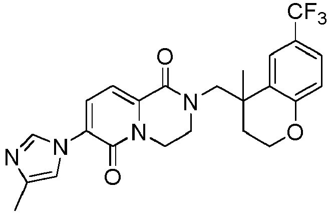 | 473.0 |
| **61** | Ex 9, 10 and 11¹⁸; **C15** | 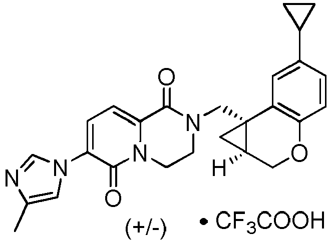 | 443.2 |
| **62** | 9, 10 and 11¹⁹; **C15** | 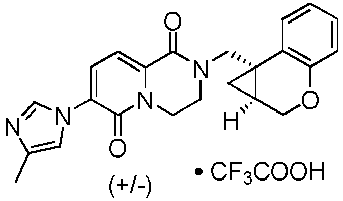 | 403.1 |
| **63** | Ex 5^{20,21} | 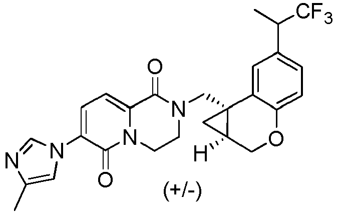 | 499.0 |
| **64** | Ex 9, 10 and 11 | 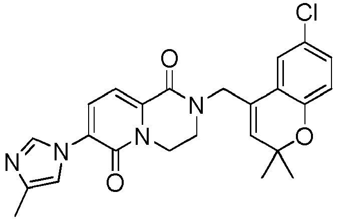 | 450.9 |
| **65** | Ex 9, 10 and 11^{22,23,6} | 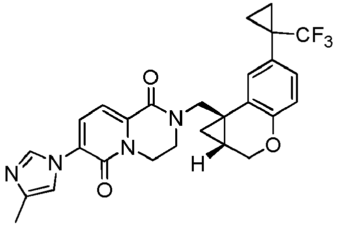 | 511.3 |
| **66** | Ex 9, 10 and 11^{22,23,6} | 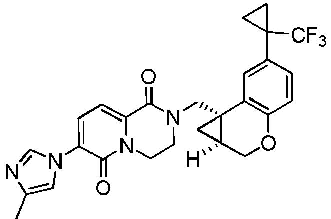 | 511.3 |

| | | | |
|---|---|---|---|
| 1. [6-(Trifluoromethyl)-2*H*-chromen-4-yl]methanol, prepared according to the method for synthesis of **C25** in Example 6, by using 1-(prop-2-yn-1-yloxy)-4-(trifluoromethyl)benzene as starting material, was hydrogenated over palladium hydroxide to afford 1-[6-(trifluoromethyl)-3,4-dihydro-2*H*-chromen-4-yl]methanamine. 2. In this case, ring closure was carried out on the 1-(2-hydroxyethyl)-5-(4-methyl-1*H*-imidazol-1-yl)-6-oxo-1,6-dihydropyridine-2-carboxamide intermediate, using triphenylphosphine and diisopropyl azodicarboxylate. 3. In this case, cyclization of 4-[3-fluoro-4-(trifluoromethyl)phenoxy]but-2-yn-1-ol was carried out using indium(III) iodide in 1,2-dichloroethane at elevated temperature, to afford [7-fluoro-6-(trifluoromethyl)-2*H*-chromen-4-yl]methanol. 4. 4-(Pentafluoro-λ⁶-sulfanyl)phenol was reacted with but-2-yne-1,4-diol according to the method described for synthesis of **C13** in Example 5; the product was cyclized using indium(III) iodide to provide the requisite [6-(pentafluoro-λ⁶-sulfanyl)-2*H*-chromen-4-yl]methanol. 5. Examples **41** and **42** were synthesized as the racemate and then separated via supercritical fluid chromatography. Example **41** was the first-eluting enantiomer. Retention time: 6.80 minutes (Column: Chiral Technologies CHIRALPAK® AS-H, 4.6 x 100 mm, 5 µm; Mobile phase: 4:1 carbon dioxide / methanol; Flow rate: 1.5 mL/minute). Example **42** was the second-eluting enantiomer, with retention time 8.27 minutes under the same chromatographic conditions. 6. The indicated absolute configurations were assigned by analogy with Examples **3** and **4,** according to the relative biological activities of the enantiomers (see Table 8). 7. Examples **43** and **44** were synthesized as the racemate and then separated via supercritical fluid chromatography. Example **43** was the first-eluting enantiomer. Retention time: 4.28 minutes (Column: Chiral Technologies CHIRALPAK® IA, 4.6 x 100 mm, 5 µm; Mobile phase: 7:3 carbon dioxide / ethanol; Flow rate: 1.5 mL/minute). Example **44** was the second-eluting enantiomer, with retention time 5.17 minutes under the same chromatographic conditions. 8. In this case, intermediate *tert*-butyl(dimethyl){[6-(trifluoromethyl)-2*H-*chromen-4-yl]methoxy}silane was subjected to difluorocyclopropanation using the method of F. Wang et al., Angew Chem., Int. Ed. 2011, 50, 7153-7157, to provide *tert*-butyl{[1,1-difluoro-6-(trifluoromethyl)-1a,2-dihydrocyclopropa[*c*]chromen-7b(1*H*)-yl]methoxy}dimethylsilane, which was then taken on using the chemistry described in Example 6. 9. Examples **45** and **46** were synthesized as the racemate and then separated via supercritical fluid chromatography (Column: Chiral Technologies CHIRALPAK® IB, 5 µm; Mobile phase: 4:1 carbon dioxide / methanol). Example **45** was the first-eluting enantiomer; Retention time: 4.37 minutes (Column: Chiral Technologies CHIRALPAK® AD-H, 4.6 x 100 mm, 5 µm; Mobile phase: 4:1 carbon dioxide / methanol; Flow rate: 1.5 mL/minute). Example **46** was the second-eluting enantiomer, with retention time 5.44 minutes under the same HPLC conditions. 10. Examples **47** and **48** were synthesized as the racemate and then separated via supercritical fluid chromatography (Column: Chiral Technologies CHIRALPAK® AD-H, 5 µm; Mobile phase: 4:1 carbon dioxide / methanol). Example **47** was the first-eluting enantiomer; Retention time: 8.60 minutes (Column: Chiral Technologies CHIRALPAK® AD-H, 4.6 x 100 mm, 5 µm; Mobile phase: 4:1 carbon dioxide / methanol; Flow rate: 1.5 mL/minute). Example **48** was the second-eluting enantiomer, with retention time 8.75 minutes under the same HPLC conditions. 11. Examples **49** and **50** were synthesized as the racemate and then separated via chiral HPLC (Column: Chiral Technologies CHIRALPAK® AD-H, 5 µm; Mobile phase: 0.1% diethylamine in methanol). Example **49** was the first-eluting enantiomer. Retention time: 8.57 minutes (Column: Chiral Technologies CHIRALPAK® IA, 4.6 x 250 mm, 5 µm; Mobile phase: 0.1% diethylamine in methanol; Flow rate: 1.0 mL/minute). Example **50** was the second-eluting enantiomer, with retention time 10.41 minutes under the same HPLC conditions. 12. (4-Chloro-2,3-difluorophenyl)boronic acid was treated with hydrogen peroxide to provide 4-chloro-2,3-difluorophenol; reaction with 3-bromoprop-1-yne and potassium carbonate afforded 4-chloro-2,3-difluorophenyl prop-2-yn-1-yl ether. 13. Examples **51** and **52** were synthesized as the racemate and then separated via chiral HPLC (Column: Chiral Technologies CHIRALPAK® IA, 5 µm; Mobile phase: 0.1% diethylamine in [10% methanol in acetonitrile]). Example **51** was the first-eluting enantiomer. Retention time: 5.27 minutes (Column: Chiral Technologies CHIRALPAK® IA, 4.6 x 250 mm, 5 µm; Mobile phase: 0.1% diethylamine in methanol; Flow rate: 1.0 mL/minute). Example **52** was the second-eluting enantiomer, with retention time 6.58 minutes under the same HPLC conditions. 14. Examples **53** and **54** were synthesized as the racemate and then separated via supercritical fluid chromatography (Column: Chiral Technologies CHIRALPAK® AD-H, 5 µm; Mobile phase: 1:1 carbon dioxide / ethanol). Example **53** was the first-eluting enantiomer. Retention time: 7.80 minutes (Column: Chiral Technologies CHIRALPAK® AD-H, 4.6 x 250 mm, 5 µm; Mobile phase A: carbon dioxide; Mobile phase B: ethanol; Gradient: 30% to 50% B over 1.0 minute, then 50% to 80% B over 8 minutes, then hold at 80% B for 0.5 minutes; Flow rate: 3.0 mL/minute). Example **54** was the second-eluting enantiomer, with retention time 9.32 minutes under the same HPLC conditions. 15. In this case, addition of methylmagnesium bromide to the 2-methyl-*N-*{[6-(trifluoromethyl)-1a,2-dihydrocyclopropa[*c*]chromen-7b(1*H*)-yl]methylidene}propane-2-sulfinamide intermediate provided four diastereomers. Two of them were carried on, and were determined to be enantiomers of one another. Examples **55** and **56** are single enantiomers, and enantiomers of one another, but of unknown absolute and relative configurations. 16. Examples **58** and **59** were synthesized as the racemate and then separated via chiral HPLC (Column: Chiral Technologies CHIRALPAK® IA, 5 µm; Mobile phase: 0.1% diethylamine in methanol). Example **58** was the first-eluting enantiomer. Retention time: 8.85 minutes (Column: Chiral Technologies CHIRALPAK® IA, 4.6 x 250 mm, 5 µm; Mobile phase: 0.1% diethylamine in methanol; Flow rate: 1.0 mL/minute). Example **59** was the second-eluting enantiomer, with retention time 11.79 minutes under the same HPLC conditions. 17. Hydrogenation of ethyl 6-(trifluoromethyl)-2*H*-chromene-4-carboxylate afforded ethyl 6-(trifluoromethyl)-3,4-dihydro-2*H*-chromene-4-carboxylate, which was reacted with sodium hydride and iodomethane to provide ethyl 4-methyl-6-(trifluoromethyl)-3,4-dihydro-2*H*-chromene-4-carboxylate. Cleavage of the ester with lithium hydroxide, followed by 1-[3-(dimethylamino)propyl]-3-ethylcarbodiimide hydrochloride-mediated amide formation with ammonium chloride, gave 4-methyl-6-(trifluoromethyl)-3,4-dihydro-2*H*-chromene-4-carboxamide. Lithium aluminum hydride reduction of the amide afforded the requisite 1-[4-methyl-6-(trifluoromethyl)-3,4-dihydro-2*H*-chromen-4-yl]methanamine. 18. *rel*-2-{[(1a*S*,7b*S*)-6-Bromo-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione was reacted with cyclopropylzinc bromide under bis(tri-*tert-*butylphosphine)palladium(0) catalysis to provide the product. 19. *rel*-2-{[(1a*S*,7b*S*)-6-Bromo-1a,2-dihydrocyclopropa[c]chromen-7b(1*H*)-yl]methyl}-7-(4-methyl-1*H*-imidazol-1-yl)-3,4-dihydro-2*H*-pyrido[1,2-*a*]pyrazine-1,6-dione was reduced with tris(trimethylsilyl)silane and 2,2'-azobisisobutyronitrile to provide the product. 20. In this case, intermediate [6-(3,3,3-trifluoroprop-1-en-2-yl)-1a,2-dihydrocyclopropa[*c*]chromen-7b(1*H*)-yl]methanol was hydrogenated rather than cyclopropanated, to afford [6-(1,1,1-trifluoropropan-2-yl)-1a,2-dihydrocyclopropa[*c*]chromen-7b(1*H*)-yl]methanol. 21. Example **63** was isolated as a racemic mixture of diastereomers. 22. 4,4,5,5-Tetramethyl-2-{4-[1-(trifluoromethyl)cyclopropyl]phenyl}-1,3,2-dioxaborolane was reacted with OXONE® (potassium peroxymonosulfate) in acetone and water to afford 4-[1-(trifluoromethyl)cyclopropyl]phenol, which was subjected to potassium carbonate and 3-bromoprop-1-yne to provide 1-(prop-2-yn-1-yloxy)-4-[1-(trifluoromethyl)cyclopropyl]benzene. 23. Examples **65** and **66** were synthesized as the racemate and then separated via supercritical fluid chromatography (Column: Chiral Technologies CHIRALPAK® AD-H, 5 µm; Mobile phase: 3:2 carbon dioxide / ethanol). Example **65** was the first-eluting enantiomer: Retention time: 7.68 minutes (Column: Chiral Technologies CHIRALPAK® AD-H, 4.6 x 250 mm, 5 µm; Mobile phase A: carbon dioxide; Mobile phase B: 0.2% [7 M solution of ammonia in ethanol] in ethanol; Gradient: 5% B over 1.0 minute, then 5% to 60% B over 8 minutes, then hold at 60% B for 0.5 minutes). Example **66** was the second-eluting enantiomer, with retention time 8.96 minutes under the same HPLC conditions. | | | |

### Cell-based γ-secretase assay with ELISA readout

The ability of compounds to modulate production of amyloid beta protein Aβ(1-42) was determined using human WT-APP overexpressing CHO cells. Cells were plated at 22,000 cells/100 µL well in 96 well tissue culture treated, clear plates (Falcon) in DMEM/F12 based medium and incubated for 24 h at 37 °C. Compounds for testing were diluted in 100% DMSO to achieve an eleven point, half log, dose response for IC₅₀ determinations. Compounds were added in fresh medium to achieve 1% final DMSO. Appropriate vehicle or inhibitor controls were added into control wells individually to obtain minimum or maximum inhibition values, respectively, for the assay signal window before the plates were incubated for ∼24 h at 37 °C. This procedure produces conditioned media in each well which is tested for Aβ(1-42) levels in the ELISA detection step described next. The remaining cell cultures in each well are also tested for cell toxicity as described below.

Coating of ELISA assay plates was initiated by addition of 50 µL/well of an in-house Aβ(1-42) specific antibody at (3 µg/mL) in 0.1 M NaHCO₃ (pH 9.0) into black 384-well Maxisorp® plates (Nunc) and incubated overnight at 4 °C. The capture antibody was then aspirated from the ELISA assay plates and plates were washed either 2 x 100 µL with a Matrical Squirt plate washer, or 3 x 90 µL with a Thermo Combi, using Wash Buffer (Dulbecco's PBS, 0.05% Tween 20). 90 µL/well of Blocking Buffer (Dulbecco's PBS, 1.0% BSA (Sigma A7030) was then added to plates. Ambient temperature incubation was allowed to proceed for a minimum of 2 h. Blocking buffer was then removed and 20 µL/well Assay Buffer (Dulbecco's PBS, 1.0% BSA (Sigma A7030), 0.05% Tween 20) was then added. At this point, 35 µL (40 µL prior to August, 2012) (in duplicate) of experimental conditioned media (described above) were transferred into wells of the blocked ELISA plates containing the capture antibody, followed by overnight incubation at 4 °C. Cell toxicity was also measured in the corresponding remaining cells after removal of the conditioned media for the Aβ(1-42) assay by a colorimetric cell proliferation assay (CellTiter 96® AQᵤₑₒᵤₛ One Solution Cell Proliferation Assay, Promega) according to the manufacturer's instructions.

After overnight incubation of the ELISA assay plates at 4 °C, unbound Aβ peptides were removed via either 2 x 100 µL washes with a Matrical Squirt plate washer, or 3 x 90 µL washes with a Thermo Combi, using Wash Buffer. Europium (Eu) labeled (custom labeled, PerkinElmer) Aβ(1-16) 6e10 Monoclonal Antibody (Covance #SIG-39320) was added, (50 µL/well Eu-6e10 @ 1:10,000, 20 uM EDTA) in Assay Buffer. Incubation at ambient temperature for a minimum of 2 h was followed by either 2 x 100 µL washes with a Matrical Squirt plate washer, or 3 x 90 µL washes with a Thermo Combi, using Wash Buffer, before 30 µL/well of Delfia Enhancement Solution (PerkinElmer) was added. Following 30 to 60 min ambient temperature incubation, the plates were read on an EnVision plate reader (PerkinElmer) using standard DELFIA TRF settings. Data analysis including inhibitory IC₅₀ determination was performed using nonlinear regression fit analysis (in-house software) and the appropriate plate mean values for the maximum and minimum inhibition controls.

Biological data for the compounds of Examples 1-66 are found in Table 8 below:

**Table 8. Biological activity and IUPAC names for Examples 1 - 66.**

| Example Number | Aβ 42B IC₅₀ (nM)^{a} | IUPAC Name |
|---|---|---|
| **1** | 7.8 | 2-[(6-chloro-8-fluoro-2,2-dimethyl-2*H*-chromen-4-yl)methyl]-7-(4-methyl-1*H*-imidazol-1-yl)-3,4-dihydro-2*H*-pyrido[1,2-*a*]pyrazine-1,6-dione |
| **2** | 17.5 | *rel*-2-{[(1a*S*,7b*S*)-2,2-dimethyl-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **3** | 4.9 | 2-{[(1a*S*,7b*S*)-2,2-dimethyl-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **4** | 172 | 2-{[(1a*R*,7b*R*)-2,2-dimethyl-6-(trifluoromethyl)-1a,2-dihydrocyclopropa[c]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **5** | 14.1 | *rel*-2-{[(1a*S*,7b*S*)-6-(1-methylcyclopropyl)-1a,2-dihydrocyclopropa[*c*]chromen-7b(1*H*)-yl]methyl}-7-(4-methyl-1 *H*-imidazol-1-yl)-3,4-dihydro-2*H*-pyrido[1,2-*a*]pyrazine-1,6-dione |
| **6** | 13.0 | 2-{[2,2-dimethyl-6-(trifluoromethyl)-2*H*-chromen-4-yl]methyl}-7-(4-methyl-1*H*-imidazol-1-yl)-3,4-dihydro-2*H*-pyrido[1,2-*a*]pyrazine-1,6-dione |
| **7** | 23.4 | (3*S*)-2-{[2,2-dimethyl-6-(trifluoromethyl)-2*H*-chromen-4-yl]methyl}-3-methyl-7-(4-methyl-1*H*-imidazol-1-yl)-3,4-dihydro-2*H*-pyrido[1,2-*a*]pyrazine-1,6-dione |
| **8** | 33.2 | (3*R*)-2-{[2,2-dimethyl-6-(trifluoromethyl)-2*H*-chromen-4-yl]methyl}-3-methyl-7-(4-methyl-1*H*-imidazol-1-yl)-3,4-dihydro-2*H*-pyrido[1,2-*a*]pyrazine-1,6-dione |
| **9** | 6.2 | *rel*-2-{[(1a*S*,7b*S*)-2,2-dimethyl-6-(trifluoromethoxy)-1a,2-dihydrocyclopropa[*c*]chromen-7b(1*H*)-yl]methyl}-7-(4-methyl-1*H*-imidazol-1-yl)-3,4-dihydro-2*H-*pyrido[1,2-*a*]pyrazine-1,6-dione |
| **10** | 3.5 | 2-{[(1a*S*,7b*S*)-2,2-dimethyl-6-(trifluoromethoxy)-1a,2-dihydrocyclopropa[c]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **11** | 150 | 2-{[(1a*R*,7b*R*)-2,2-dimethyl-6-(trifluoromethoxy)-1a,2-dihydrocyclopropa[c]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **12** | 9.4 | 7-(4-methyl-1*H*-imidazol-1-yl)-2-{[6-(trifluoromethyl)spiro[chromene-2,1'-cyclobutan]-4-yl]methyl}-3,4-dihydro-2*H*-pyrido[1,2-*a*]pyrazine-1,6-dione |
| **13** | 16.7 | 2-{1-[2,2-dimethyl-6-(trifluoromethoxy)-2*H*-chromen-4-yl]ethyl}-7-(4-methyl-1*H*-imidazol-1-yl)-3,4-dihydro-2*H*-pyrido[1,2-*a*]pyrazine-1,6-dione |
| **14** | 6.0 | 2-{(1*S*)-1-[2,2-dimethyl-6-(trifluoromethoxy)-2*H-*chromen-4-yl]ethyl}-7-(4-methyl-1*H*-imidazol-1-yl)-3,4-dihydro-2*H*-pyrido[1,2-*a*]pyrazine-1,6-dione |
| **15** | 1350 | 2-{(1*R*)-1-[2,2-dimethyl-6-(trifluoromethoxy)-2*H-*chromen-4-yl]ethyl}-7-(4-methyl-1*H*-imidazol-1-yl)-3,4-dihydro-2*H*-pyrido[1,2-*a*]pyrazine-1,6-dione |
| **16** | 29.6 | 7-(4-methyl-1*H*-imidazol-1-yl)-2-{[6-(trifluoromethyl)-2H-chromen-4-yl]methyl}-3,4-dihydro-2*H*-pyrido[1,2-*a*]pyrazine-1,6-dione, trifluoroacetate salt |
| **17** | 4.2 | (-)-7-(4-methyl-1*H*-imidazol-1-yl)-2-{[(1a*S*,7b*S*)-6-(trifluoromethyl)-1a,2-dihydrocyclopropa[*c*]chromen-7b(1*H*)-yl]methyl}-3,4-dihydro-2*H*-pyrido[1,2-*a*]pyrazine-1,6-dione, hydrochloride salt |
| **18** | 114 | (+)-7-(4-methyl-1*H*-imidazol-1-yl)-2-{[(1a*R*,7b*R*)-6-(trifluoromethyl)-1a,2-dihydrocyclopropa[*c*]chromen-7b(1*H*)-yl]methyl}-3,4-dihydro-2*H*-pyrido[1,2-*a*]pyrazine-1,6-dione, hydrochloride salt |
| **19** | 48.5 | *rel*-7-(4-methyl-1*H*-imidazol-1-yl)-2-{[(1a*S*,6b*S*)-5-(trifluoromethyl)-1,1a-dihydro-6b*H-*cyclopropa[*b*][1]benzofuran-6b-yl]methyl}-3,4-dihydro-2*H*-pyrido[1,2-*a*]pyrazine-1,6-dione |
| **20** | 155 | 2-{[(1a*R*,7b*R*)-4-chloro-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **21** | 8.0 | 2-{[(1a*S*,7b*S*)-4-chloro-6-(trifluoromethyl)-1a,2-dihydrocyclopropa[*c*]chromen-7b(1*H*)-yl]methyl}-7-(4-methyl-1 *H*-imidazol-1-yl)-3,4-dihydro-2*H*-pyrido[1,2-*a*]pyrazine-1,6-dione |
| **22** | 42.5 | 2-{[6-(1,1-difluoroethoxy)-2*H*-chromen-4-yl]methyl}-7-(4-methyl-1*H*-imidazol-1-yl)-3,4-dihydro-2*H-*pyrido[1,2-*a*]pyrazine-1,6-dione, trifluoroacetate salt |
| **23** | 10.5 | 2-{1-[2,2-dimethyl-6-(trifluoromethyl)-2*H*-chromen-4-yl]ethyl}-7-(4-methyl-1*H*-imidazol-1-yl)-3,4-dihydro-2*H*-pyrido[1,2-*a*]pyrazine-1,6-dione |
| **24** | 4.9^{b} | 2-{(1*S*)-1-[2,2-dimethyl-6-(trifluoromethyl)-2*H-*chromen-4-yl]ethyl}-7-(4-methyl-1*H*-imidazol-1-yl)-3,4-dihydro-2*H*-pyrido[1,2-*a*]pyrazine-1,6-dione |
| **25** | 1180 | 2-{(1 *R*)-1-[2,2-dimethyl-6-(trifluoromethyl)-2*H-*chromen-4-yl]ethyl}-7-(4-methyl-1 *H*-imidazol-1-yl)-3,4-dihydro-2*H*-pyrido[1,2-*a*]pyrazine-1,6-dione |
| **26** | 28.9 | *rel*-2-{[(1a*S*,7b*S*)-6-chloro-2,2-dimethyl-1a,2-dihydrocyclopropa[*c*]chromen-7b(1*H*)-yl]methyl}-7-(4-methyl-1 *H*-imidazol-1-yl)-3,4-dihydro-2*H*-pyrido[1,2-*a*]pyrazine-1,6-dione |
| **27** | 8.3 | 2-{[(1a*S*,7b*S*)-6-chloro-2,2-dimethyl-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **28** | 153 | 2-{[(1a*R*,7b*R*)-6-chloro-2,2-dimethyl-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **29** | 17.7 | 2-[(6-chloro-3-methyl-2*H*-chromen-4-yl)methyl]-7-(4-methyl-1*H*-imidazol-1-yl)-3,4-dihydro-2*H*-pyrido[1,2-*a*]pyrazine-1,6-dione |
| **30** | 5.5 | 2-{[2,2-dimethyl-6-(trifluoromethoxy)-2*H*-chromen-4-yl]methyl}-7-(4-methyl-1*H*-imidazol-1-yl)-3,4-dihydro-2*H*-pyrido[1,2-*a*]pyrazine-1,6-dione |
| **31** | 37.4 | 2-[(6-chloro-2,2,3-trimethyl-2*H*-chromen-4-yl)methyl]-7-(4-methyl-1H-imidazol-1-yl)-3,4-dihydro-2*H-*pyrido[1,2-*a*]pyrazine-1,6-dione |
| **32** | 21.1 | 2-{[2,2-bis(fluoromethyl)-6-(trifluoromethyl)-2*H-*chromen-4-yl]methyl}-7-(4-methyl-1*H*-imidazol-1-yl)-3,4-dihydro-2*H*-pyrido[1,2-*a*]pyrazine-1,6-dione |
| **33** | 14.3 | 2-{[(1a*S*,7b*S*)-6-(1,1-difluoroethoxy)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **34** | 266^{c} | 2-{[(1a*R*,7b*R*)-6-(1,1-difluoroethoxy)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **35** | 6.6 | 2-[(6-chloro-7,8-difluoro-2,2-dimethyl-2*H*-chromen-4-yl)methyl]-7-(4-methyl-1*H*-imidazol-1-yl)-3,4-dihydro-2*H*-pyrido[1,2-*a*]pyrazine-1,6-dione |
| **36** | 161 | 2-{[(1a*R*,7b*R*)-6-chloro-1a,2,2-trimethyl-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **37** | 14.6 | 2-{[(1a*S*,7b*S*)-6-chloro-1a,2,2-trimethyl-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **38** (Reference example) | 322 | 7-(4-methyl-1*H*-imidazol-1-yl)-2-{[6-(trifluoromethyl)-3,4-dihydro-2*H*-chromen-4-yl]methyl}-3,4-dihydro-2*H-*pyrido[1,2-*a*]pyrazine-1,6-dione, trifluoroacetate salt |
| **39** | 22.1 | *rel*-2-{[(1a*S*,7b*S*)-5-fluoro-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione, trifluoroacetate salt |
| **40** | 9.4 | *rel*-7-(4-methyl-1*H*-imidazol-1-yl)-2-{[(1 a*S*,7b*S*)-6-(pentafluoro-λ⁶-sulfanyl)-1a,2-dihydrocyclopropa[*c*]chromen-7b(1*H*)-yl]methyl}-3,4-dihydro-2*H*-pyrido[1,2-*a*]pyrazine-1,6-dione, trifluoroacetate salt |
| **41** | 756^{c} | 2-{[(1a*R*,7b*R*)-6-methoxy-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **42** | 181 | 2-{[(1a*S*,7b*S*)-6-methoxy-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **43** | 177 | 7-(4-methyl-1*H*-imidazol-1-yl)-2-{[(1a*R*,7b*R*)-6-(trifluoromethoxy)-1a,2-dihydrocyclopropa[*c*]chromen-7b(1*H*)-yl]methyl}-3,4-dihydro-2*H*-pyrido[1,2-*a*]pyrazine-1,6-dione |
| **44** | 6.8 | 7-(4-methyl-1*H*-imidazol-1-yl)-2-{[(1 a*S*,7b*S*)-6-(trifluoromethoxy)-1a,2-dihydrocyclopropa[*c*]chromen-7b(1*H*)-yl]methyl}-3,4-dihydro-2*H*-pyrido[1,2-*a*]pyrazine-1,6-dione |
| **45** | 361 | 2-{[(1a*R*,7b*S*)-1,1-difluoro-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **46** | 50.9 | 2-{[(1a*S*,7b*R*)-1,1-difluoro-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **47** | 194 | 7-(4-methyl-1*H*-imidazol-1-yl)-2-{[(1*R*,1 a*R*,7b*R*)-1-methyl-6-(trifluoromethyl)-1a,2-dihydrocyclopropa[*c*]chromen-7b(1*H*)-yl]methyl}-3,4-dihydro-2*H*-pyrido[1,2-*a*]pyrazine-1,6-dione |
| **48** | 18.1 | 7-(4-methyl-1*H*-imidazol-1-yl)-2-{[(1*S*,1a*S*,7b*S*)-1-methyl-6-(trifluoromethyl)-1a,2-dihydrocyclopropa[*c*]chromen-7b(1*H*)-yl]methyl}-3,4-dihydro-2*H*-pyrido[1,2-*a*]pyrazine-1,6-dione |
| **49** | 505 | 7-(4-methyl-1*H*-imidazol-1-yl)-2-{[(1a*R*,7b*R*)-5-(trifluoromethyl)-1a,2-dihydrocyclopropa[c]chromen-7b(1*H*)-yl]methyl}-3,4-dihydro-2*H*-pyrido[1,2-*a*]pyrazine-1,6-dione |
| **50** | 52.4 | 7-(4-methyl-1*H*-imidazol-1-yl)-2-{[(1a*S*,7b*S*)-5-(trifluoromethyl)-1a,2-dihydrocyclopropa[*c*]chromen-7b(1*H*)-yl]methyl}-3,4-dihydro-2*H*-pyrido[1,2-*a*]pyrazine-1,6-dione |
| **51** | 219 | 2-{[(1a*R*,7b*R*)-6-chloro-4,5-difluoro-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **52** | 11.3 | 2-{[(1a*S*,7b*S*)-6-chloro-4,5-difluoro-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **53** | 509 | 2-{[(1a*R*,7b*R*)-4,6-dichloro-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **54** | 23.2 | 2-{[(1a*S*,7b*S*)-4,6-dichloro-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **55** | 272 | 7-(4-methyl-1*H*-imidazol-1-yl)-2-{1-[6-(trifluoromethyl)-1a,2-dihydrocyclopropa[*c*]chromen-7b(1*H*)-yl]ethyl}-3,4-dihydro-2*H*-pyrido[1,2-*a*]pyrazine-1,6-dione^{$245} |
| **56** | 62.1 | 7-(4-methyl-1 *H*-imidazol-1-yl)-2-{1-[6-(trifluoromethyl)-1a,2-dihydrocyclopropa[*c*]chromen-7b(1*H*)-yl]ethyl}-3,4-dihydro-2*H*-pyrido[1,2-*a*]pyrazine-1,6-dione^{$245} |
| **57** (Reference example) | 161 | 7-(4-methyl-1*H*-imidazol-1-yl)-2-{[6-(trifluoromethyl)-1,1a,2,7b-tetrahydrocyclopropa[*c*]chromen-1-yl]methyl}-3,4-dihydro-2*H*-pyrido[1,2-*a*]pyrazine-1,6-dione |
| **58** | 248 | 2-{[(1a*R*,7b*R*)-6-chloro-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **59** | 31.1 | 2-{[(1a*S*,7b*S*)-6-chloro-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione |
| **60** | 1090^{c} | 7-(4-methyl-1*H*-imidazol-1-yl)-2-{[4-methyl-6-(trifluoromethyl)-3,4-dihydro-2*H*-chromen-4-yl]methyl}-3,4-dihydro-2*H*-pyrido[1,2-*a*]pyrazine-1,6-dione |
| **61** | 34.3 | *rel*-2-{[(1a*S*,7b*S*)-6-cyclopropyl-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione, trifluoroacetate salt |
| **62** | 702 | *rel*-2-[(1a*S*,7b*S*)-1a,2-dihydrocyclopropa[*c*]chromen-7b(1*H*)-ylmethyl]-7-(4-methyl-1*H*-imidazol-1-yl)-3,4-dihydro-2*H*-pyrido[1,2-*a*]pyrazine-1,6-dione, trifluoroacetate salt |
| **63** | 31.8 | *rel*-7-(4-methyl-1*H*-imidazol-1-yl)-2-{[(1a*S*,7b*S*)-6-(1,1,1-trifluoropropan-2-yl)-1a,2-dihydrocyclopropa[*c*]chromen-7b(1*H*)-yl]methyl}-3,4-dihydro-2*H*-pyrido[1,2-*a*]pyrazine-1,6-dione |
| **64** | 37.0 | 2-[(6-chloro-2,2-dimethyl-2*H*-chromen-4-yl)methyl]-7-(4-methyl-1*H*-imidazol-1-yl)-3,4-dihydro-2*H-*pyrido[1,2-*a*]pyrazine-1,6-dione |
| **65** | 475 | 7-(4-methyl-1*H*-imidazol-1-yl)-2-{[(1a*R*,7b*R*)-6-[1-(trifluoromethyl)cyclopropyl]-1a,2-dihydrocyclopropa[*c*]chromen-7b(1*H*)-yl]methyl}-3,4-dihydro-2*H*-pyrido[1,2-*a*]pyrazine-1,6-dione |
| **66** | 14.0 | 7-(4-methyl-1*H*-imidazol-1-yl)-2-{[(1a*S*,7b*S*)-6-[1-(trifluoromethyl)cyclopropyl]-1a,2-dihydrocyclopropa[*c*]chromen-7b(1*H*)-yl]methyl}-3,4-dihydro-2*H*-pyrido[1,2-*a*]pyrazine-1,6-dione |

| | | |
|---|---|---|
| a. Reported IC₅₀ values are the geometric mean of 2 - 4 determinations. b. Reported IC₅₀ value is the geometric mean of ≥5 determinations. c. IC₅₀ value is from a single determination. | | |

## Claims

1. A compound having the structure of Formula I: 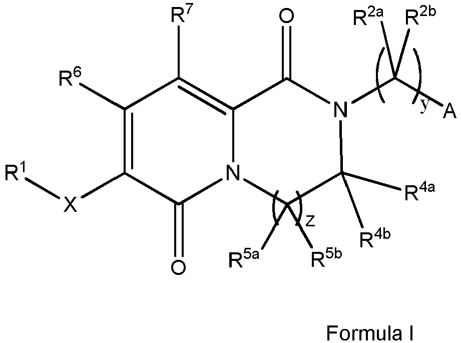 or a pharmaceutically acceptable salt thereof, wherein:
A is selected from the group consisting of A1a, A1b, and A3a: 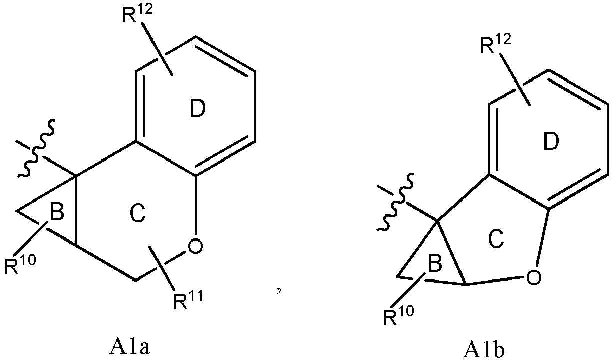 or 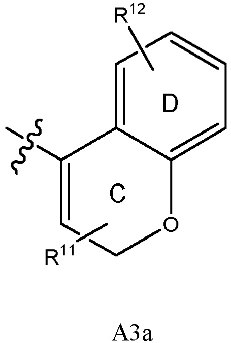
X is a (5-membered)heteroaryl wherein the heteroaryl is imidazolyl;
R¹ is a (C₁-C₆)alkyl, wherein the alkyl is methyl;
R^{2a}, R^{2b}, R^{4a}, R^{4b}, R^{5a} and R^{5b} are each independently:
i) hydrogen; or
ii) (C₁-C₆)alkyl, wherein the (C₁-C₆)alkyl is methyl;
R⁶ and R⁷ are each independently hydrogen;
z and y are each 1;
ring B is optionally substituted with up to three R¹⁰, wherein each R¹⁰ is independently selected from halogen or (C₁-C₆)alkyl;
ring C is optionally substituted with up to three R¹¹ such that substitution occurs at any carbon atom that is chemically permissible, and wherein each R¹¹ is independently selected from halogen, (C₁-C₆)alkyl, or halo(C₁-C₆)alkyl; or two R¹¹ taken together with the carbon atom(s) to which they are attached form a (C₃-C₆)cycloalkyl or a (4- to 6-membered) heterocycloalkyl, wherein the (C₃-C₆)cycloalkyl and (4- to 6-membered) heterocycloalkyl moieties are each optionally substituted with one to three substituents independently selected from halogen or (C₁-C₆)alkyl; and
ring D is optionally substituted with up to three R¹², wherein each R¹² is independently selected from the group consisting of halogen, (C₁-C₆)alkyl, (C₁-C₆)alkoxy, halo(C₁-C₆)alkyl, halo(C₁-C₆)alkoxy, -SF₅, and (C₃-C₆)cycloalkyl, wherein the (C₃-C₆)cycloalkyl is optionally substituted with one to three substituents independently selected from halogen, (C₁-C₆)alkyl, or halo(C₁-C₆)alkyl.

2. The compound according to claim 1, or a pharmaceutically acceptable salt thereof, wherein R^{2a}, R^{2b}, R^{4a}, R^{4b}, R^{5a} and R^{5b} are each independently hydrogen.

3. The compound according to claim 1, or a pharmaceutically acceptable salt thereof, wherein R^{2a}, R^{2b}, R^{5a} and R^{5b} are each independently hydrogen; and one of R^{4a} and R^{4b} is hydrogen and the other is methyl.

4. The compound according to claim 1, or a pharmaceutically acceptable salt thereof, wherein one of R^{2a} and R^{2b} is hydrogen and the other is methyl; R^{4a}, R^{4b}, R^{5a} and R^{5b} are each independently hydrogen.

5. The compound according to claim 1, or a pharmaceutically acceptable salt thereof, wherein A is represented by Formula A1a: 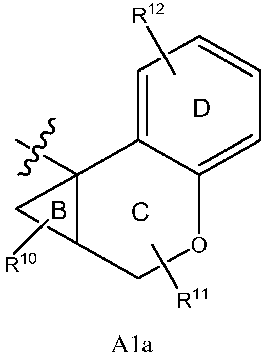 ring B is optionally substituted with one to two R¹⁰, wherein each R¹⁰ is selected from halogen or (C₁-C₆)alkyl; ring C is optionally substituted with one to two R¹¹, wherein each R¹¹ is selected from (C₁-C₆)alkyl or halo(C₁-C₆)alkyl; or two R¹¹ taken together with the carbon atom(s) to which they are attached form a (C₃-C₆)cycloalkyl or a (4- to 6-membered)heterocycloalkyl; and ring D is optionally substituted with one to three R¹², wherein each R¹² is selected from halogen, (C₁-C₆)alkyl, (C₁-C₆)alkoxy, halo(C₁-C₆)alkyl, halo(C₁-C₆)alkoxy, -SF₅, and (C₃-C₆)cycloalkyl optionally substituted with one or two substituents selected from methyl or trifluoromethyl.

6. The compound according to claim 5, or a pharmaceutically acceptable salt thereof, wherein:
each R¹⁰ is:
i) halogen selected from fluoro or chloro, or
ii) (C₁-C₆)alkyl, wherein the (C₁-C₆)alkyl is methyl;
each R¹¹ is:
i) (C₁-C₆)alkyl, wherein the (C₁-C₆)alkyl is methyl; or
ii) halo(C₁-C₆)alkyl, wherein the halo(C₁-C₆)alkyl is selected from fluoromethyl, trifluoromethyl, or trifluoroethyl; or
two R¹¹ taken together with the carbon atom(s) to which they are attached form:
i) a (C₃-C₆)cycloalkyl, wherein the (C₃-C₆)cycloalkyl is cyclobutyl; or
ii) a (4- to 6-membered)heterocycloalkyl wherein the (4- to 6-membered)heterocycloalkyl is an oxetanyl, and
each R¹² is:
i) halogen selected from fluoro or chloro;
ii) (C₁-C₆)alkyl, wherein the (C₁-C₆)alkyl is methyl;
iii) (C₁-C₆)alkoxy, wherein the (C₁-C₆)alkoxy is methoxy;
iv) halo(C₁-C₆)alkyl, wherein the halo(C₁-C₆)alkyl is selected from fluoromethyl, trifluoromethyl, or trifluoroethyl;
v) halo(C₁-C₆)alkoxy, wherein the halo(C₁-C₆)alkoxy is selected from fluoromethoxy, fluoroethoxy, or difluoroethoxy;
vi) -SF₅; or
vii) (C₃-C₆)cycloalkyl, wherein the (C₃-C₆)cycloalkyl is cyclopropyl optionally substituted with one to two substituents selected from methyl and trifluoromethyl.

7. The compound according to claim 1, or a pharmaceutically acceptable salt thereof, wherein A is represented by Formula A1b: 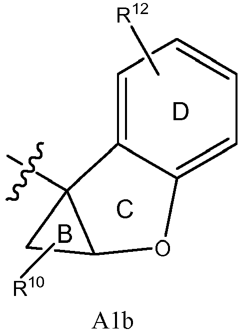 wherein:
ring B is optionally substituted with one to two R¹⁰, wherein each R¹⁰ is selected from halogen or (C₁-C₆)alkyl; and ring D is optionally substituted with one to three R¹², wherein each R¹² is selected from halogen, (C₁-C₆)alkyl, (C₁-C₆)alkoxy, halo(C₁-C₆)alkyl, halo(C₁-C₆)alkoxy, -SF₅, and (C₃-C₆)cycloalkyl optionally substituted with one or two substituents selected from methyl or trifluoromethyl.

8. The compound according to claim 1, or a pharmaceutically acceptable salt thereof, wherein A is represented by Formula A3a: 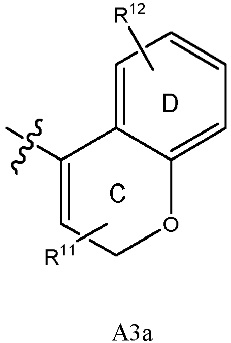 wherein:
ring C is optionally substituted with one to three R¹¹, wherein each R¹¹ is selected from (C₁-C₆)alkyl or halo(C₁-C₆)alkyl; or two R¹¹ taken together with the carbon atom(s) to which they are attached form a (C₃-C₆)cycloalkyl or a (4- to 6-membered)heterocycloalkyl; ring D is optionally substituted with one to three R¹², wherein each R¹² is selected from halogen, (C₁-C₆)alkyl, (C₁-C₆)alkoxy, halo(C₁-C₆)alkyl, halo(C₁-C₆)alkoxy, -SF₅, and (C₃-C₆)cycloalkyl.

9. The compound according to claim 8, or a pharmaceutically acceptable salt thereof, wherein:
each R¹¹ is selected from:
i) (C₁-C₆)alkyl, wherein the (C₁-C₆)alkyl is methyl; or
ii) halo(C₁-C₆)alkyl, wherein the halo(C₁-C₆)alkyl is selected from fluoromethyl, trifluoromethyl, or trifluoroethyl; or
two R¹¹ taken together with the carbon atom(s) to which they are attached form:
i) a (C₃-C₆)cycloalkyl, wherein the (C₃-C₆)cycloalkyl is cyclobutyl; or
ii) a (4- to 6-membered)heterocycloalkyl wherein the (4- to 6-membered)heterocycloalkyl is an oxetanyl, and
each R¹² is selected from:
i) halogen selected from fluoro or chloro;
ii) (C₁-C₆)alkyl, wherein the (C₁-C₆)alkyl is methyl;
iii) (C₁-C₆)alkoxy, wherein the (C₁-C₆)alkoxy is methoxy;
iv) halo(C₁-C₆)alkyl, wherein the halo(C₁-C₆)alkyl is selected from fluoromethyl, trifluoromethyl, or trifluoroethyl;
v) halo(C₁-C₆)alkoxy, wherein the halo(C₁-C₆)alkoxy is selected from fluoromethoxy, fluoroethoxy, or difluoroethoxy;
vi) -SF₅; or
vii) (C₃-C₆)cycloalkyl, wherein the (C₃-C₆)cycloalkyl is cyclopropyl optionally substituted with one to two substituents selected from methyl and trifluoromethyl.

10. A compound of claim 1, or a pharmaceutically acceptable salt thereof, wherein the compound is selected from the group consisting of:
2-[(6-Chloro-8-fluoro-2,2-dimethyl-2*H*-chromen-4-yl)methyl]-7-(4-methyl-1*H-*imidazol-1-yl)-3,4-dihydro-2*H*-pyrido[1,2-*a*]pyrazine-1,6-dione;
2-{[2,2-Dimethyl-6-(trifluoromethyl)-2*H*-chromen-4-yl]methyl}-7-(4-methyl-1*H-*imidazol-1-yl)-3,4-dihydro-2*H*-pyrido[1,2-*a*]pyrazine-1,6-dione;
2-{[(1a*S*,7b*S*)-2,2-Dimethyl-6-(trifluoromethoxy)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione;
2-{[(1a*S*,7b*S*)-2,2-Dimethyl-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione;
2-{(1*S*)-1-[2,2-dimethyl-6-(trifluoromethyl)-2*H*-chromen-4-yl]ethyl}-7-(4-methyl-1*H-*imidazol-1-yl)-3,4-dihydro-2*H*-pyrido[1,2-*a*]pyrazine-1,6-dione;
2-{[2,2-dimethyl-6-(trifluoromethoxy)-2*H*-chromen-4-yl]methyl}-7-(4-methyl-1*H-*imidazol-1-yl)-3,4-dihydro-2*H*-pyrido[1,2-*a*]pyrazine-1,6-dione;
2-{[(1a*S*,7b*S*)-6-(1,1-difluoroethoxy)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione;
2-{[(1a*S*,7b*S*)-4-chloro-6-(trifluoromethyl)-1a,2-dihydrocyclopropa[*c*]chromen-7b(1*H*)-yl]methyl}-7-(4-methyl-1*H*-imidazol-1-yl)-3,4-dihydro-2*H*-pyrido[1,2-*a*]pyrazine-1,6-dione;
7-(4-methyl-1*H*-imidazol-1-yl)-2-{[(1a*R*,7b*R*)-6-[1-(trifluoromethyl)cyclopropyl]-1a,2-dihydrocyclopropa[*c*]chromen-7b(1*H*)-yl]methyl}-3,4-dihydro-2*H*-pyrido[1,2-*a*]pyrazine-1,6-dione; and
7-(4-methyl-1*H*-imidazol-1-yl)-2-{[(1a*S*,7b*S*)-6-(trifluoromethoxy)-1a,2-dihydrocyclopropa[c]chromen-7b(1*H*)-yl]methyl}-3,4-dihydro-2*H*-pyrido[1,2-a]pyrazine-1,6-dione.

11. The compound according to any one of claims 1 to 10, or a pharmaceutically acceptable salt thereof, for use in the treatment of neurodegeneration and psychiatric disorders, including Alzheimer's disease or Niemann-Pick disease type C.

12. A pharmaceutical composition comprising a compound of any one of claims 1 to 10, or a pharmaceutically acceptable salt thereof, and a pharmaceutically acceptable excipient.

## Patentansprüche

1. Verbindung mit der Struktur der Formel I: 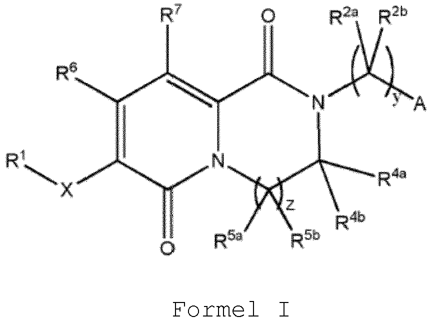 oder ein pharmazeutisch annehmbares Salz davon, wobei:
A ausgewählt ist aus der Gruppe, bestehend aus A1a, A1b und A3a: 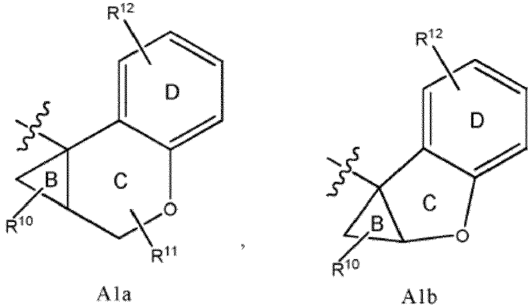 oder 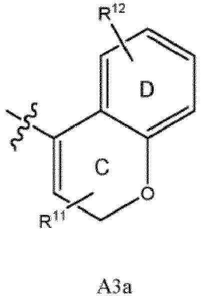
X ein (5-gliedriges) Heteroaryl ist, wobei das Heteroaryl Imidazolyl ist;
R¹ ein (C₁-C₆)-Alkyl ist, wobei das Alkyl Methyl ist;
R^{2a}, R^{2b}, R^{4a}, R^{4b}, R^{5a} und R^{5b} jeweils unabhängig sind:
i) Wasserstoff oder
ii) (C₁-C₆)-Alkyl, wobei das (C₁-C₆)-Alkyl Methyl ist;
R⁶ und R⁷ jeweils unabhängig Wasserstoff sind;
z und y jeweils 1 sind;
der Ring B gegebenenfalls mit bis zu drei R¹⁰ substituiert ist, wobei jedes R¹⁰ unabhängig aus Halogen oder (C₁-C₆)-Alkyl ausgewählt ist;
der Ring C gegebenenfalls mit bis zu drei R¹¹ substituiert ist, so dass eine Substitution an einem beliebigen Kohlenstoffatom, die chemisch zulässig ist, auftritt, und wobei jedes R¹¹ unabhängig ausgewählt ist aus Halogen, (C₁-C₆)-Alkyl oder Halogen(C₁-C₆)alkyl; oder zwei R¹¹ zusammen mit dem Kohlenstoffatom/den Kohlenstoffatomen, an das/die sie gebunden sind, ein (C₃-C₆)-Cycloalkyl oder ein (4- bis 6-gliedriges) Heterocycloalkyl bilden, wobei die (C₃-C₆)-Cycloalkyl- und (4-bis 6-gliedrigen) Heterocycloalkyl-Gruppierungen jeweils gegebenenfalls mit einem bis drei Substituenten, unabhängig ausgewählt aus Halogen oder (C₁-C₆)-Alkyl, substituiert sind; und
der Ring D gegebenenfalls mit bis zu drei R¹² substituiert ist, wobei jedes R¹² unabhängig ausgewählt ist aus der Gruppe, bestehend aus Halogen, (C₁-C₆)-Alkyl, (C₁-C₆)-Alkoxy, Halogen (C₁-C₆)alkyl, Halogen(C₁-C₆)alkoxy, -SF₅ und (C₃-C₆)-Cycloalkyl, wobei das (C₃-C₆)-Cycloalkyl gegebenenfalls mit einem bis drei Substituenten, unabhängig ausgewählt aus Halogen, (C₁-C₆)-Alkyl oder Halogen(C₁-C₆)alkyl, substituiert ist.

2. Verbindung gemäß Anspruch 1 oder ein pharmazeutisch annehmbares Salz davon, wobei R^{2a}, R^{2b}, R^{4a}, R^{4b}, R^{5a} und R^{5b} jeweils unabhängig Wasserstoff sind.

3. Verbindung gemäß Anspruch 1 oder ein pharmazeutisch annehmbares Salz davon, wobei R^{2a}, R^{2b}, R^{5a} und R^{5b} jeweils unabhängig Wasserstoff sind; und eines von R^{4a} und R^{4b} Wasserstoff ist und das andere Methyl ist.

4. Verbindung gemäß Anspruch 1 oder ein pharmazeutisch annehmbares Salz davon, wobei eines von R^{2a} und R^{2b} Wasserstoff ist und das andere Methyl ist; R^{4a}, R^{4b}, R^{5a} und R^{5b} jeweils unabhängig Wasserstoff sind.

5. Verbindung gemäß Anspruch 1 oder ein pharmazeutisch annehmbares Salz davon, wobei A durch die Formel A1a dargestellt ist: 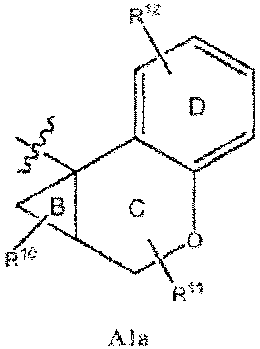 der Ring B ist gegebenenfalls mit einem bis zwei R¹⁰ substituiert ist, wobei jedes R¹⁰ aus Halogen oder (C₁-C₆)-Alkyl ausgewählt ist; der Ring C gegebenenfalls mit einem bis zwei R¹¹ substituiert ist, wobei jedes R¹¹ aus (C₁-C₆)-Alkyl oder Halogen(C₁-C₆)alkyl ausgewählt ist; oder zwei R¹¹ zusammen mit dem Kohlenstoffatom/den Kohlenstoffatomen, an das/die sie gebunden sind, ein (C₃-C₆)-Cycloalkyl oder ein (4- bis 6-gliedriges) Heterocycloalkyl bilden; und der Ring D gegebenenfalls mit einem bis drei R¹² substituiert ist, wobei jedes R¹² aus Halogen, (C₁-C₆)-Alkyl, (C₁-C₆)-Alkoxy, Halogen(C₁-C₆)alkyl, Halogen(C₁-C₆)alkoxy, -SF₅ und (C₃-C₆)-Cycloalkyl, gegebenenfalls substituiert mit einem oder zwei Substituenten, ausgewählt aus Methyl oder Trifluormethyl, ausgewählt ist.

6. Verbindung gemäß Anspruch 5 oder ein pharmazeutisch annehmbares Salz davon, wobei:
jedes R¹⁰ ist:
i) Halogen, ausgewählt aus Fluor oder Chlor, oder
ii) (C₁-C₆)-Alkyl, wobei das (C₁-C₆)-Alkyl Methyl ist;
jedes R¹¹ ist:
i) (C₁-C₆)-Alkyl, wobei das (C₁-C₆)-Alkyl Methyl ist; oder
ii) Halogen(C₁-C₆)alkyl, wobei das Halogen(C₁-C₆)alkyl aus Fluormethyl, Trifluormethyl oder Trifluorethyl ausgewählt ist; oder
zwei R¹¹, zusammen mit dem Kohlenstoffatom/den Kohlenstoffatomen, an das/die sie gebunden sind, das Folgende bilden:
i) ein (C₃-C₆)-Cycloalkyl, wobei das (C₃-C₆)-Cycloalkyl Cyclobutyl ist; oder
ii) ein (4- bis 6-gliedriges) Heterocycloalkyl, wobei das (4- bis 6-gliedrige) Heterocycloalkyl ein Oxetanyl ist, und
jedes R¹² ist:
i) Halogen, ausgewählt aus Fluor oder Chlor;
ii) (C₁-C₆)-Alkyl, wobei das (C₁-C₆)-Alkyl Methyl ist;
iii) (C₁-C₆)-Alkoxy, wobei das (C₁-C₆)-Alkoxy Methoxy ist;
iv) Halogen(C₁-C₆)alkyl, wobei das Halogen(C₁-C₆)alkyl aus Fluormethyl, Trifluormethyl oder Trifluorethyl ausgewählt ist;
v) Halogen(C₁-C₆)alkoxy, wobei das Halogen(C₁-C₆)alkoxy aus Fluormethoxy, Fluorethoxy oder Difluorethoxy ausgewählt ist;
vi) -SF₅ oder
vii) (C₃-C₆)-Cycloalkyl, wobei das (C₃-C₆)-Cycloalkyl Cyclopropyl, gegebenenfalls substituiert mit einem bis zwei Substituenten, ausgewählt aus Methyl und Trifluormethyl, ist.

7. Verbindung gemäß Anspruch 1 oder ein pharmazeutisch annehmbares Salz davon, wobei A durch die Formal A1b dargestellt ist: 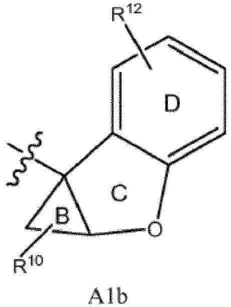 wobei:
der Ring B gegebenenfalls mit einem bis zwei R¹⁰ substituiert ist, wobei jedes R¹⁰ aus Halogen oder (C₁-C₆)-Alkyl ausgewählt ist; und der Ring D gegebenenfalls mit einem bis drei R¹² substituiert ist, wobei jedes R¹² aus Halogen, (C₁-C₆)-Alkyl, (C₁-C₆)-Alkoxy, Halogen(C₁-C₆)alkyl, Halogen(C₁-C₆)alkoxy, -SF₅ und (C₃-C₆)-Cycloalkyl, gegebenenfalls substituiert mit einem oder zwei Substituenten, ausgewählt aus Methyl oder Trifluormethyl, ausgewählt ist.

8. Verbindung gemäß Anspruch 1 oder ein pharmazeutisch annehmbares Salz davon, wobei A durch die Formel A3a dargestellt ist: 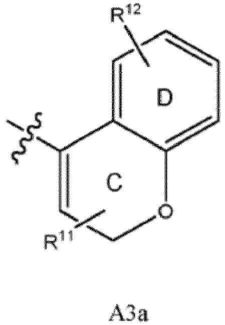 wobei:
der Ring C gegebenenfalls mit einem bis drei R¹¹ substituiert ist, wobei jedes R¹¹ aus (C₁-C₆)-Alkyl oder Halogen(C₁-C₆)alkyl ausgewählt ist; oder zwei R¹¹, zusammen mit dem Kohlenstoffatom/den Kohlenstoffatomen, an das/die sie gebunden sind, ein (C₃-C₆)-Cycloalkyl oder ein (4- bis 6-gliedriges) Heterocycloalkyl bilden; der Ring D gegebenenfalls mit einem bis drei R¹² substituiert ist, wobei jedes R¹² aus Halogen, (C₁-C₆)-Alkyl, (C₁-C₆)-Alkoxy, Halogen(C₁-C₆)alkyl, Halogen(C₁-C₆)-Alkoxy, -SF₅ und (C₃-C₆)-Cycloalkyl ausgewählt ist.

9. Verbindung gemäß Anspruch 8 oder ein pharmazeutisch annehmbares Salz davon, wobei:
jedes R¹¹ ausgewählt ist aus:
i) (C₁-C₆)-Alkyl, wobei das (C₁-C₆)-Alkyl Methyl ist; oder
ii) Halogen(C₁-C₆)alkyl, wobei das Halogen(C₁-C₆)alkyl aus Fluormethyl, Trifluormethyl oder Trifluorethyl ausgewählt ist; oder
zwei R¹¹, zusammen mit dem Kohlenstoffatom/den Kohlenstoffatomen, an das/die sie gebunden sind, das Folgende bilden:
i) ein (C₃-C₆)-Cycloalkyl, wobei das (C₃-C₆)-Cycloalkyl Cyclobutyl ist; oder
ii) ein (4- bis 6-gliedriges) Heterocycloalkyl, wobei das (4- bis 6-gliedrige) Heterocycloalkyl ein Oxetanyl ist, und
jedes R¹² ausgewählt ist aus:
i) Halogen, ausgewählt aus Fluor oder Chlor;
ii) (C₁-C₆)-Alkyl, wobei das (C₁-C₆)-Alkyl Methyl ist;
iii) (C₁-C₆)-Alkoxy, wobei das (C₁-C₆)-Alkoxy Methoxy ist;
iv) Halogen(C₁-C₆)alkyl, wobei das Halogen(C₁-C₆)alkyl aus Fluormethyl, Trifluormethyl oder Trifluorethyl ausgewählt ist;
v) Halogen(C₁-C₆)alkoxy, wobei das Halogen(C₁-C₆)alkoxy aus Fluormethoxy, Fluorethoxy oder Difluorethoxy ausgewählt ist;
vi) -SF₅ oder
vii) (C₃-C₆)-Cycloalkyl, wobei das (C₃-C₆)-Cycloalkyl Cyclopropyl, gegebenenfalls substituiert mit einem bis zwei Substituenten, ausgewählt aus Methyl und Trifluormethyl, ist.

10. Verbindung nach Anspruch 1 oder ein pharmazeutisch annehmbares Salz davon, wobei die Verbindung ausgewählt ist aus der Gruppe, bestehend aus:
2-[(6-Chlor-8-fluor-2,2-dimethyl-2*H*-chromen-4-yl)methyl]-7-(4-methyl-1*H*-imidazol-1-yl)-3,4-dihydro-2*H*-pyrido[1,2-*a*]pyrazin-1,6-dion;
2-{[2,2-Dimethyl-6-(trifluormethyl)-2*H*-chromen-4-yl]methyl}-7-(4-methyl-1*H*-imidazol-1-yl)-3,4-dihydro-2*H-*pyrido[1,2-*a*]pyrazin-1,6-dion;
2-{[(1a*S*,7b*S*)-2,2-Dimethyl-6-(trifluormethoxy)-1a,2-dihydrocyclopropa[*c*]chromen-7b(1*H*)-yl]methyl}-7-(4-methyl-1*H-*imidazol-1-yl)-3,4-dihydro-2*H*-pyrido[1,2-*a*]pyrazin-1,6-dion;
2-{[(1a*S*,7b*S*)-2,2-Dimethyl-6-(trifluormethyl)-1a,2-dihydrocyclopropa[*c*]chromen-7b(1*H*)-yl]methyl}-7-(4-methyl-1*H-*imidazol-1-yl)-3,4-dihydro-2*H*-pyrido[1,2-*a*]pyrazin-1,6-dion;
2-{(1*S*)-1-[2,2-Dimethyl-6-(trifluormethyl)-2*H*-chromen-4-yl]ethyl}-7-(4-methyl-1*H*-imidazol-1-yl)-3,4-dihydro-2*H-*pyrido[1,2-*a*]pyrazin-1,6-dion;
2-{[2,2-Dimethyl-6-(trifluormethoxy)-2*H*-chromen-4-yl]methyl}-7-(4-methyl-1*H*-imidazol-1-yl)-3,4-dihydro-2*H-*pyrido[1,2-*a*]pyrazin-1,6-dion;
2-{[(1a*S*,7b*S*)-6-(1,1-Difluorethoxy)-1a,2-dihydrocyclopropa[*c*]chromen-7b(1*H*)-yl]methyl}-7-(4-methyl-1*H-*imidazol-1-yl)-3,4-dihydro-2*H*-pyrido[1,2-*a*]pyrazin-1,6-dion;
2-{[(1a*S*,7b*S*)-4-Chlor-6-(trifluormethyl)-1a,2-dihydrocyclopropa[*c*]chromen-7b(1*H*)-yl]methyl}-7-(4-methyl-1*H-*imidazol-1-yl)-3,4-dihydro-2*H*-pyrido[1,2-*a*]pyrazin-1,6-dion;
7-(4-Methyl-1*H*-imidazol-1-yl)-2-{[(1a*R*,7b*R*)-6-[1-(trifluormethyl)cyclopropyl]-1a,2-dihydrocyclopropa[*c*]chromen-7b(1*H*)-yl]methyl}-3,4-dihydro-2*H-*pyrido[1,2-*a*]pyrazin-1,6-dion; und
7-(4-Methyl-1*H*-imidazol-1-yl)-2-{[(1a*S*,7b*S*)-6-(trifluormethoxy)-1a,2-dihydrocyclopropa[*c*]chromen-7b(1*H*)-yl]methyl}-3,4-dihydro-2*H*-pyrido[1,2-*a*]pyrazin-1,6-dion.

11. Verbindung gemäß einem der Ansprüche 1 bis 10 oder ein pharmazeutisch annehmbares Salz davon zur Verwendung bei der Behandlung von Neurodegeneration und psychiatrischen Störungen, einschließlich Alzheimer'scher Erkrankung oder Niemann-Pick-Erkrankung Typ C.

12. Pharmazeutische Zusammensetzung, umfassend eine Verbindung nach einem der Ansprüche 1 bis 10 oder ein pharmazeutisch annehmbares Salz davon und ein pharmazeutisch annehmbares Exzipiens.

## Revendications

1. Composé ayant la structure de formule I : 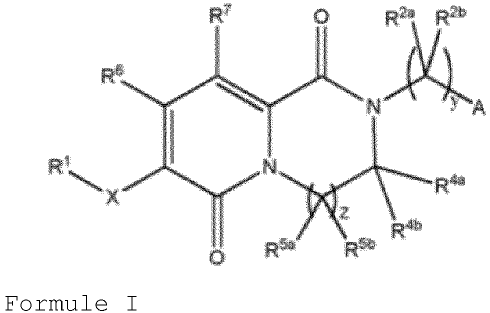 ou un de ses sels pharmaceutiquement acceptables, formule dans laquelle :
A est choisi dans le groupe constitué par A1a, A1b et A3a : 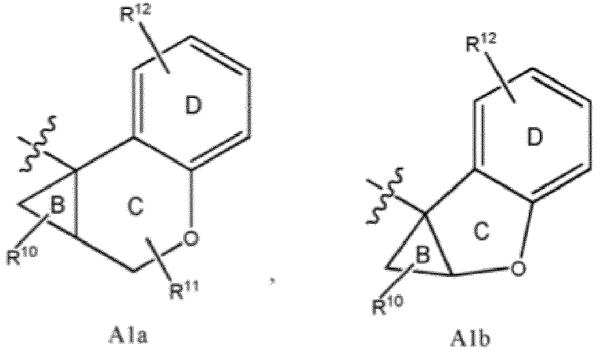 ou 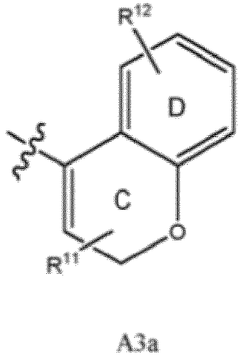
X est un groupe hétéroaryle (pentagonal) où le groupe hétéroaryle est l'imidazolyle ;
R¹ est un groupe alkyle en C₁ à C₆, où le groupe alkyle est un groupe méthyle ;
R^{2a}, R^{2b}, R^{4a}, R^{4b}, R^{5a} et R^{5b} sont chacun indépendamment :
i) un atome d'hydrogène ; ou
ii) un groupe alkyle en C₁ à C₆, où le groupe alkyle en C₁ à C₆ est un groupe méthyle ;
R⁶ et R⁷ sont chacun indépendamment un atome d'hydrogène ;
z et y valent chacun 1 ;
le noyau B est éventuellement substitué avec jusqu'à trois R¹⁰, où chaque R¹⁰ est choisi indépendamment parmi un atome d'halogène ou un groupe alkyle en C₁ à C₆ ;
le noyau C est éventuellement substitué avec jusqu'à trois R¹¹ de telle manière qu'une substitution se produit à un quelconque atome de carbone qui est chimiquement permissible, et où chaque R¹¹ est choisi indépendamment parmi un atome d'halogène, un groupe alkyle en C₁ à C₆ ou halogénoalkyle en C₁ à C₆ ; ou deux R¹¹ pris conjointement avec l'(les) atome (s) de carbone au(x)quel(s) ils sont attachés forment un groupe cycloalkyle en C₃ à C₆ ou hétérocycloalkyle (tétra- à hexagonal), où les groupements cycloalkyle en C₃ à C₆ et hétérocycloalkyle (tétra- à hexagonal) sont chacun éventuellement substitués avec un à trois substituants choisis indépendamment parmi un atome d'halogène ou un groupe alkyle en C₁ à C₆ ; et
le noyau D est éventuellement substitué avec trois R¹², où chaque R¹² est choisi indépendamment dans le groupe constitué par un atome d'halogène, un groupe alkyle en C₁ à C₆, alkoxy en C₁ à C₆, halogénoalkyle en C₁ à C₆, halogénoalkoxy en C₁ à C₆, -SF₅ et cycloalkyle en C₃ à C₆, où le groupe cycloalkyle en C₃ à C₆ est éventuellement substitué avec un à trois substituants choisis indépendamment parmi un atome d'halogène, un groupe alkyle en C₁ à C₆ ou halogénoalkyle en C₁ à C₆.

2. Composé selon la revendication 1, ou un de ses sels pharmaceutiquement acceptables, dans lequel R^{2a}, R^{2b}, R^{4a}, R^{4b}, R^{5a} et R^{5b} sont chacun indépendamment un atome d'hydrogène.

3. Composé selon la revendication 1, ou un de ses sels pharmaceutiquement acceptables, dans lequel R^{2a}, R^{2b}, R^{5a} et R^{5b} sont chacun indépendamment un atome d'hydrogène ; et l'un de R^{4a} et R^{4b} est un atome d'hydrogène et l'autre est un groupe méthyle.

4. Composé selon la revendication 1, ou un de ses sels pharmaceutiquement acceptables, dans lequel l'un de R^{2a} et R^{2b} est un atome d'hydrogène et l'autre est un groupe méthyle ; R^{4a}, R^{4b}, R^{5a} et R^{5b} sont chacun indépendamment un atome d'hydrogène.

5. Composé selon la revendication 1, ou un de ses sels pharmaceutiquement acceptables, dans lequel A est représenté par la formule A1a : 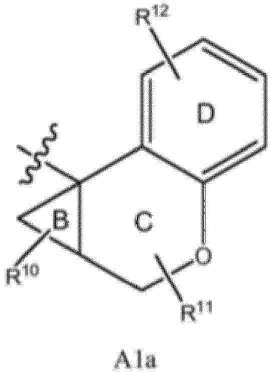 le noyau B est éventuellement substitué avec un à deux R¹⁰, où chaque R¹⁰ est choisi parmi un atome d'halogène ou un groupe alkyle en C₁ à C₆ ; le noyau C est éventuellement substitué avec un à deux R¹¹, où chaque R¹¹ est choisi parmi un groupe alkyle en C₁ à C₆ ou halogénoalkyle en C₁ à C₆ ; ou deux R¹¹ pris conjointement avec l'(les) atome (s) de carbone au(x)quel(s) ils sont attachés forment un groupe cycloalkyle en C₃ à C₆ ou hétérocycloalkyle (tétra- à hexagonal) ; et le noyau D est éventuellement substitué avec un à trois R¹², où chaque R¹² est choisi parmi un atome d'halogène, un groupe alkyle en C₁ à C₆, alkoxy en C₁ à C₆, halogénoalkyle en C₁ à C₆, halogénoalkoxy en C₁ à C₆, -SF₅ et cyloalkyle en C₃ à C₆ éventuellement substitué avec un à deux substituants choisis parmi des groupes méthyle ou trifluorométhyle.

6. Composé selon la revendication 5, ou un de ses sels pharmaceutiquement acceptables, dans lequel :
chaque R¹⁰ est :
i) un atome d'halogène choisi parmi un chloro ou un fluoro, ou
ii) un groupe alkyle en C₁ à C₆, où le groupe alkyle en C₁ à C₆ est un groupe méthyle ;
chaque R¹¹ est :
i) un groupe alkyle en C₁ à C₆, où le groupe alkyle en C₁ à C₆ est un groupe méthyle ; ou
ii) un groupe halogénoalkyle en C₁ à C₆, où le groupe halogénoalkyle en C₁ à C₆ est choisi parmi des groupes fluorométhyle, trifluorométhyle ou trifluoro-éthyle ; ou
deux R¹¹ pris conjointement avec l'(les) atome (s) de carbone au(x)quel(s) ils sont attachés forment :
i) un groupe cycloalkyle en C₃ à C₆, où le groupe cycloalkyle en C₃ à C₆ est un groupe cyclobutyle ; ou
ii) un groupe hétérocycloalkyle (tétra- à hexagonal) où le groupe hétérocycloalkyle (tétra- à hexagonal) est un groupe oxétannyle, et
chaque R¹² est :
i) un atome d'halogène choisi parmi un fluoro ou un chloro ;
ii) un groupe alkyle en C₁ à C₆, où le groupe alkyle en C₁ à C₆ est un groupe méthyle ;
iii) un groupe alkoxy en C₁ à C₆, où le groupe alkoxy en C₁ à C₆ est un groupe méthoxy ;
iv) un groupe halogénoalkyle en C₁ à C₆, où le groupe halogénoalkyle en C₁ à C₆ est choisi parmi des groupes fluorométhyle, trifluorométhyle ou trifluoro-éthyle ;
v) un groupe halogénoalkoxy en C₁ à C₆, où le groupe halogénoalkoxy est choisi parmi des groupes fluorométhoxy, fluoroéthoxy ou difluoroéthoxy ;
vi) -SF₅ ; ou
vii) un groupe cycloalkyle en C₃ à C₆, où le groupe cycloalkyle en C₃ à C₆ est un groupe cyclopropyle éventuellement substitué avec un à deux substituants choisis parmi des groupes méthyle et trifluorométhyle.

7. Composé selon la revendication 1, ou un de ses sels pharmaceutiquement acceptables, dans lequel A est représenté par la formule A1b : 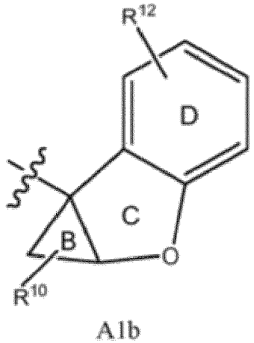 dans laquelle :
le noyau B est éventuellement substitué avec un à deux R¹⁰, où chaque R¹⁰ est choisi parmi un atome d'halogène ou un groupe alkyle en C₁ à C₆ ; et le noyau D est éventuellement substitué avec un à trois R¹², où chaque R¹² est choisi parmi un atome d'halogène, un groupe alkyle en C₁ à C₆, alkoxy en C₁ à C₆, halogénoalkyle en C₁ à C₆, halogénoalkoxy en C₁ à C₆, -SF₅ et cycloalkyle en C₃ à C₆ éventuellement substitué avec un à deux substituants choisis parmi des groupes méthyle ou trifluorométhyle.

8. Composé selon la revendication 1, ou un de ses sels pharmaceutiquement acceptables, dans lequel A est représenté par la formule A3a : 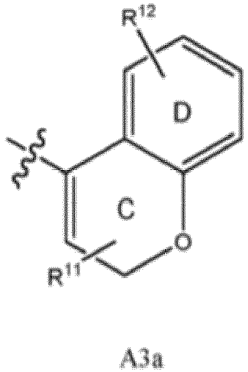 dans laquelle :
le noyau C est éventuellement substitué avec un à trois R¹¹, où chaque R¹¹ est choisi parmi un groupe alkyle en C₁ à C₆ ou halogénoalkyle en C₁ à C₆ ; ou deux R¹¹ pris conjointement avec l'(les) atome(s) au(x)quel(s) ils sont attachés forment un groupe cycloalkyle en C₃ à C₆ ou hétérocycloalkyle (tétra- à hexagonal) ; le noyau D est éventuellement substitué avec un à trois R¹², où chaque R¹² est choisi parmi un atome d'halogène, un groupe alkyle en C₁ à C₆, alkoxy en C₁ à C₆, halogénoalkyle en C₁ à C₆, halogénoalkoxy en C₁ à C₆, -SF₅ et cycloalkyle en C₃ à C₆.

9. Composé selon la revendication 8, ou un de ses sels pharmaceutiquement acceptables, dans lequel :
chaque R¹¹ est choisi parmi :
i) un groupe alkyle en C₁ à C₆, où le groupe alkyle en C₁ à C₆ est un groupe méthyle ; ou
ii) un groupe halogénoalkyle en C₁ à C₆, où le groupe halogénoalkyle en C₁ à C₆ est choisi parmi des groupes fluorométhyle, trifluorométhyle ou trifluoro-éthyle ; ou
deux R¹¹ pris conjointement avec l'(les) atome(s) de carbone au(x)quel(s) ils sont attachés forment :
i) un groupe cycloalkyle en C₃ à C₆, où le groupe cycloalkyle en C₃ à C₆ est un groupe cyclobutyle ; ou
ii) un groupe hétérocycloalkyle (tétra- à hexagonal) où le groupe hétéroalkyle (tétra- à hexagonal) est un groupe oxétannyle, et
chaque R¹² est choisi parmi :
i) un atome d'halogène choisi parmi un fluoro ou un chloro ;
ii) un groupe alkyle en C₁ à C₆, où le groupe alkyle en C₁ à C₆ est un groupe méthyle ;
iii) un groupe alkoxy en C₁ à C₆ où le groupe alkoxy en C₁ à C₆ est un groupe méthoxy ;
iv) un groupe halogénoalkyle en C₁ à C₆ où le groupe halogénoalkyle en C₁ à C₆ est choisi parmi des groupes fluorométhyle, trifluorométhyle ou trifluoro-éthyle ;
v) un groupe halogénoalkoxy en C₁ à C₆, où le groupe halogénoalkoxy en C₁ à C₆ est choisi parmi des groupes fluorométhoxy, fluoroéthoxy ou difluoroéthoxy ;
vi) -SF₅ ; ou
vii) un groupe cycloalkyle en C₃ à C₆, où le groupe cycloalkyle en C₃ à C₆ est un groupe cyclopropyle éventuellement substitué avec un à deux substituants choisis parmi des groupes méthyle et trifluorométhyle.

10. Composé selon la revendication 1, ou un de ses sels pharmaceutiquement acceptables, où le composé est choisi dans le groupe constitué par :
la 2-[(6-chloro-8-fluoro-2,2-diméthyl-2*H*-chromène-4-yl)méthyl]-7-(4-méthyl-1*H*-imidazole-1-yl)-3,4-dihydro-2*H-*pyrido[1,2-*a*]pyrazine-1,6-dione ;
la 2-{[2,2-diméthyl-6-(trifluorométhyl)-2*H*-chromène-4-yl]méthyl}-7-(4-méthyl-1*H*-imidazole-1-yl)-3,4-dihydro-2*H-*pyrido[1,2-*a*]pyrazine-1,6-dione ;
la 2-{[(1a*S*,7b*S*)-2,2-diméthyl-6-(trifluorométhoxy)-1a,2-dihydrocyclopropa[*c*]chromène-7b(1*H*)-yl]méthyl}-7-(4-méthyl-1*H*-imidazole-1-yl)-3,4-dihydro-2*H*-pyrido[1,2-*a*]-pyrazine-1,6-dione ;
la 2-{[(1a*S*,7b*S*)-2,2-diméthyl-6-(trifluorométhyl)-1a,2-dihydrocyclopropa[*c*]chromène-7b(1*H*)-yl]méthyl}-7-(4-méthyl-1*H*-imidazole-1-yl)-3,4-dihydro-2*H*-pyrido[1,2-*a*]-pyrazine-1,6-dione ;
la 2-{(1*S*)-1-[2,2-diméthyl-6-(trifluorométhyl)-2*H-*chromène-4-yl]éthyl}-7-(4-méthyl-1*H*-imidazole-1-yl)-3,4-dihydro-2*H*-pyrido[1,2-a]pyrazine-1,6-dione ;
la 2-{[2,2-diméthyl-6-(trifluorométhoxy)-2*H*-chromène-4-yl]méthyl}-7-(4-méthyl-1*H*-imidazole-1-yl)-3,4-dihydro-2*H-*pyrido[1,2-*a*]pyrazine-1,6-dione ;
la 2-{[(1a*S*,7b*S*)-6-(1,1-difluoroéthoxy)-1a,2-dihydro-cyclopropa[*c*]chromène-7b(1*H*)-yl]méthyl}-7-(4-méthyl-1*H-*imidazole-1-yl)-3,4-dihydro-2*H*-pyrido[1,2-*a*]pyrazine-1,6-dione ;
la 2-{[(1a*S*,7b*S*)-4-chloro-6-(trifluorométhyl)-1a,2-dihydrocyclopropa[*c*]chromène-7b(1*H*)-yl]méthyl}-7-(4-méthyl-1*H*-imidazole-1-yl)-3,4-dihydro-2*H*-pyrido[1,2-*a*]pyrazine-1,6-dione ;
la 7-(4-méthyl-1*H*-imidazole-1-yl)-2-{[(1a*R*,7b*R*)-6-[1-(trifluorométhyl)cyclopropyl]-1a,2-dihydrocyclopropa[*c*]chromène-7b(1*H*)-yl]méthyl}-3,4-dihydro-2*H*-pyrido[1,2-*a*]pyrazine-1,6-dione ; et
la 7-(4-méthyl-1*H*-imidazole-1-yl)-2-{[(1a*S*,7b*S*)-6-(trifluorométhoxy)-1a,2-dihydrocyclopropa[*c*]chromène-7b(1*H*)-yl]méthyl}-3,4-dihydro-2*H*-pyrido[1,2-*a*]pyrazine-1,6-dione.

11. Composé selon l'une quelconque des revendications 1 à 10, ou un de ses sels pharmaceutiquement acceptables, pour utilisation dans le traitement de la neuro-dégénérescence et de troubles psychiatriques, y compris la maladie d'Alzheimer ou le type C de la maladie de Niemann-Pick.

12. Composition pharmaceutique comprenant un composé selon l'une quelconque des revendications 1 à 10, ou un de ses sels pharmaceutiquement acceptables, et un excipient pharmaceutiquement acceptables.
